(19) Europäisches Patentamt
European Patent Office
Office européen des brevets

(11) EP 2 143 721 B1

(12) **EUROPEAN PATENT SPECIFICATION**

(45) Date of publication and mention of the grant of the patent:
**15.08.2012 Bulletin 2012/33**

(21) Application number: **08740165.9**

(22) Date of filing: **10.04.2008**

(51) Int Cl.:
***C07D 401/04*** *(2006.01)* ***A01N 47/12*** *(2006.01)*
***A01N 47/34*** *(2006.01)*

(86) International application number:
**PCT/JP2008/057064**

(87) International publication number:
**WO 2008/126889 (23.10.2008 Gazette 2008/43)**

(54) **HYDRAZIDE COMPOUND AND HARMFUL ARTHROPOD-CONTROLLING AGENT CONTAINING THE SAME**

HYDRAZIDVERBINDUNG UND DIESE ENTHALTENDES MITTEL ZUR BEKÄMPFUNG SCHÄDLICHER ARTHROPODEN

COMPOSÉ D'HYDRAZIDE ET AGENT CONTRÔLANT L'ARTHROPODE NOCIF LE CONTENANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.04.2007 JP 2007104646**

(43) Date of publication of application:
**13.01.2010 Bulletin 2010/02**

(73) Proprietor: **Sumitomo Chemical Company, Limited**
**Tokyo 104-8260 (JP)**

(72) Inventors:
- **JACHMANN, Markus**
**Kobe-shi**
**Hyogo 652-0897 (JP)**
- **IKEGAMI, Hiroshi**
**Ikeda-shi**
**Osaka 563-0026 (JP)**
- **NOKURA, Yoshihiko**
**Toyonaka-shi**
**Osaka 561-0802 (JP)**

(74) Representative: **Vossius & Partner**
**Siebertstrasse 4**
**81675 München (DE)**

(56) References cited:
**WO-A1-2007/043677**
**JP-A- 09 510 471**
**JP-A- 62 167 747**
**JP-A- 2003 528 070**
**JP-A- 2004 538 327**
**JP-A- 2005 502 716**
**JP-A- 2005 503 384**

- **TOYA T. ET AL.: 'Cyclic dibenzoylhydrazines reproducing the conformation of ecdysone agonists, RH-5849' BIOORG. MED. CHEM. vol. 10, no. 4, 2002, pages 953 - 961, XP002257781**

EP 2 143 721 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The present invention relates to a hydrazide compound and a harmful arthropod controlling agent containing the same.

Background Art

WO 01/70671, WO 03/015518, WO 03/016284, WO 03/016300 and WO 03/024222 disclose certain amide compounds for controlling harmful arthropods.

Disclosure of the Invention

Problem to be solved by the invention

An object of the present invention is to provide a novel compound having an excellent controlling activity on harmful arthropods.

Means for solving the problem

As a result of the present inventors' intensive study, they have found a hydrazide compound represented by the following formula (1) which has an excellent controlling activity against harmful arthropods, and thus the present invention has been completed.
That is, the present invention provides:

[1] A hydrazide compound represented by the formula (1)

[Chemical Formula 1]:

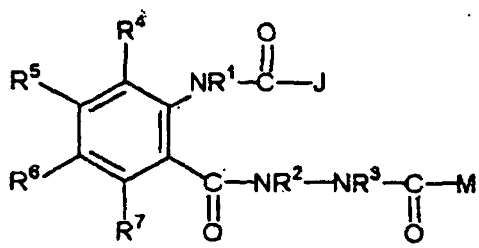

(1)

wherein

$R^1$ represents a hydrogen atom, an optionally halogenated C1-C6 alkyl group, a C2-C6 cyanoalkyl group, a C2-C6 alkoxyalkyl group, an optionally halogenated C3-C6 alkenyl group, an optionally halogenated C3-C6 alkynyl group, or a C7-C9 phenylalkyl group whose benzene ring moiety is optionally substituted with a substituent A shown below;
each of $R^2$ and $R^3$ independently represents a hydrogen atom, an optionally halogenated C1-C6 alkyl group, or $R^G$ (in which at least one of $R^2$ and $R^3$ is $R^G$,
$R^G$ represents a C1-C6 alkyl group substituted with at least one substituent selected from the group D shown below, an optionally halogenated C3-C6 alkenyl group, an optionally halogenated C3-C6 alkynyl group, a C3-C6 cycloalkyl group optionally substituted with a substituent B shown below, a 5- to 6-membered heteroaryl group optionally substituted with a substituent A shown below, a 3- to 8-membered non-aromatic heterocyclic group optionally substituted with a substituent B shown below, an optionally halogenated C1-C6 alkylthio group, an optionally halogenated C1-C6 alkylsulfinyl group, or an optionally halogenated C1-C6 alkylsulfonyl group,
"Group D:

(a) a cyano group,
(b) a nitro group,
(c) -C(=O)OR$^{101}$ ($R^{101}$ represents a hydrogen atom or a C1-C4 alkyl group),

(d) -C(=O)N$R^{102}R^{103}$ (each of $R^{102}$ and $R^{103}$ independently represents a hydrogen atom or a C1-C4 alkyl group),
(e) -C(=O)$R^{104}$ ($R^{104}$ represents a hydrogen atom, an optionally halogenated C1-C6 alkyl group, or a phenyl group optionally substituted with a substituent A shown below),
(f) -C$R^{105}$=N-O$R^{106}$ ($R^{105}$ represents a hydrogen atom, a C1-C4 alkyl group, or a phenyl group optionally substituted with a substituent A shown below, and $R^{106}$ represents a hydrogen atom or a C1-C4 alkyl group),
(g) -O$R^{107}$ ($R^{107}$ represents a hydrogen atom, a C1-C4 alkyl group, or a phenyl group optionally substituted with a substituent A shown below),
(h) -$S(O)_j R^{108}$ ($R^{108}$ represents a hydrogen atom, a C1-C4 alkyl group, or a phenyl group optionally substituted with a substituent A shown below, and j represents an integer of 0 to 2),
(i) -N$R^{109}$ $R^{110}$ (each of $R^{109}$ and $R^{110}$ independently represents a hydrogen atom, a C1-C9 alkyl group, or a phenyl group optionally substituted with a substituent A shown below),
(j) a C3-C6 cycloalkyl group optionally substituted with a substituent B shown below,
(k) a phenyl group optionally substituted with a substituent A shown below,
(l) a 5- to 6-membered heteroaryl group optionally substituted with a substituent A shown below,
(m) a 3- to 8-membered non-aromatic heterocyclic group optionally substituted with a substituent B shown below, and
(n) a styryl group whose benzene ring moiety is optionally be substituted with a substituent A shown below"};

$R^4$ represents a halogen atom, or an optionally halogenated C1-C6 alkyl group;
each of $R^5$, $R^6$ and $R^7$ independently represents a hydrogen atom, a halogen atom, a cyano group, or an optionally halogenated C1-C6 alkyl group, or
$R^5$ and $R^6$ may be combined to form a 1,3-butadiene-1,4-diyl group optionally substituted with a substituent C shown below;
M represents -$R^8$, -O$R^9$, -S$R^{10}$, or -N$R^{11}R^{12}$ (in which $R^8$ represents a hydrogen atom, an optionally halogenated C1-C6 alkyl group, a C2-C6 alkoxyalkyl group, an optionally halogenated C2-C6 alkenyl group, or an optionally halogenated C2-C6 alkynyl group, each of $R^9$, $R^{10}$, $R^{11}$ and $R^{12}$ independently represents an optionally halogenated C1-C6 alkyl group, a C3-C6 alkoxyalkyl group, an optionally halogenated C3-C6 alkenyl group, or an optionally halogenated C3-C6 alkynyl group };
J represents any one of J1 to J3 shown below:

[Chemical Formula 2]

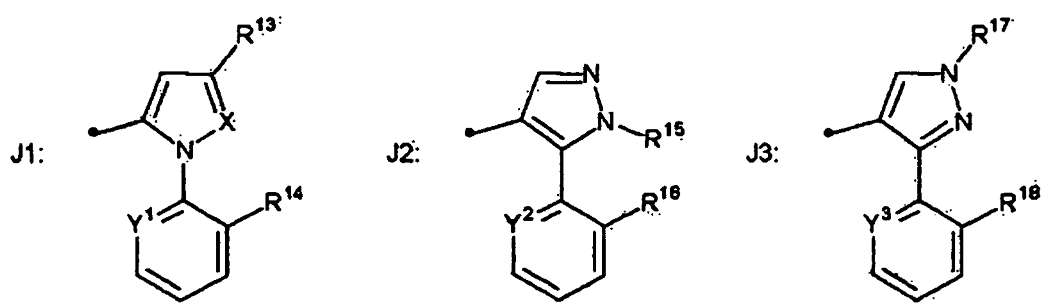

{in which X represents a nitrogen atom or C$R^{19}$; $Y^1$ represents a nitrogen atom or C$R^{20}$;
$Y^2$ represents a nitrogen atom or C$R^{21}$;
$Y^3$ represents a nitrogen atom or C$R^{22}$;
each of $R^{13}$ and $R^{19}$ independently represents a hydrogen atom, a halogen atom, a cyano group, an optionally halogenated C1-C6 alkyl group, an optionally halogenated C1-C6 alkoxy group, an optionally halogenated C1-C6 alkylthio group, an optionally halogenated C1-C6 alkylsulfinyl group, or an optionally halogenated C1-C6 alkylsulfonyl group;
$R^{15}$ and $R^{17}$ each independently represents an optionally halogenated C1-C6 alkyl group;
each of $R^{14}$, $R^{16}$, $R^{18}$, $R^{20}$, $R^{21}$ and $R^{22}$ independently represents a hydrogen atom, a halogen atom, or an optionally halogenated C1-C6 alkyl group};

substituent A: a substituent selected from the group consisting of a halogen atom, a cyano group, a nitro group, an optionally halogenated C1-C6 alkyl group, and an optionally halogenated C1-C6 alkoxy group;
substituent B: a substituent selected from the group consisting of a halogen atom and a C1-C6 alkyl group; and

substituent C: a substituent selected from the group consisting of a halogen atom, a cyano group and an optionally halogenated C1-C6 alkyl group (hereinafter, sometimes, referred to as the present compound);

[2] The hydrazide compound according to the above [1], wherein, in the formula (1), $R^G$ is a C1-C6 alkyl group substituted with at least one substituent selected from the group D1 shown below, an optionally halogenated C3-C6 alkenyl group, an optionally halogenated C3-C6 alkynyl group, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B;
"group D1: (a) a cyano group, (g) $-OR^{107}$ ($R^{107}$ represents a hydrogen atom, a C1-C4 alkyl group or a phenyl group optionally substituted with a substituent A), (j) a C3-C6 cycloalkyl group optionally substituted with a substituent B, (k) a phenyl group optionally substituted with a substituent A, (l) a 5- to 6-membered heteroaryl group optionally substituted with a substituent A; and (m) a 3-to 8-membered non-aromatic heterocyclic group optionally substituted with a substituent B";
[3] The hydrazide compound according to the above [2], wherein, in the formula (1), $R^G$ is a C1-C6 alkyl group substituted with at least one substituent selected from the group D2 shown below, an optionally halogenated C3-C6 alkenyl group, or an optionally halogenated C3-C6 alkynyl group;
"group D2: (a) a cyano group and (g) $-OR^{107}$ ($R^{107}$ represents a hydrogen atom, a C1-C4 alkyl group or a phenyl group optionally substituted with a substituent A)";
[4] The hydrazide compound according to the above [2], wherein, in the formula (1), $R^G$ is a C1-C6 alkyl group substituted with at least one substituent selected from the group D3 shown below, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B; "group D3: (j) a C3-C6 cycloalkyl group optionally substituted with a substituent B, (k) a phenyl group optionally substituted with a substituent A, (l) a 5- to 6-membered heteroaryl group optionally substituted with a substituent A, and (m) a 3- to 8-membered non-aromatic heterocyclic group optionally substituted with a substituent B";
[5] The hydrazide compound according to the above [3], wherein, in the formula (1), one of $R^2$ and $R^3$ is $R^G$, and the other one is a hydrogen atom or an optionally halogenated C1-C6 alkyl group, and $R^3$ is a C1-C6 alkyl group substituted with a cyano group, an optionally halogenated C3-C6 alkenyl group, or an optionally halogenated C3-C6 alkynyl group;
[6] The hydrazide compound according to the above [1], wherein, in the formula (1), $R^G$ is a C1-C6 alkyl group substituted with at least one substituent selected from the group D;
[7] The hydrazide compound according to the above [6], wherein in the formula (1), $R^G$ is a C1-C6 alkyl group substituted with a cyano group;
[8] The hydrazide compound according to the above [7], wherein, in the formula (1), $R^2$ is a C1-C6 alkyl group substituted with a cyano group; and $R^3$ is a hydrogen atom or an optionally halogenated C1-C6 alkyl group;
[9] The hydrazide compound according to the above [7], wherein, in the formula (1), $R^2$ is a hydrogen atom or an optionally halogenated C1-C6 alkyl group; and $R^3$ is a C1-C6 alkyl group substituted with a cyano group;
[10] A harmful arthropod controlling agent comprising the hydrazide compound according to any one of the above [1] to [9] as an active ingredient;
[11] Use of the hydrazide compound according to any one of the above [1] to [9] as an active ingredient of a harmful arthropod controlling agent;
[12] A method for controlling harmful arthropods, which comprises applying the hydrazide compound according to any one of the above [1] to [9] directly to harmful arthropods, or applying to habitats of harmful arthropods, wherein the term "habitats" means plants, soils and houses;
[13] Use of the hydrazide compound according to any one of the above [1] to [9] for the production of a harmful arthropod controlling agent.

Effect of the invention

The present compound has an excellent controlling activity against harmful arthropods and is therefore useful as an active ingredient of a harmful arthropod controlling agent.

Best Mode for Carrying Out the Invention

In the present invention, examples of the "halogen atom" include a fluorine atom, a chlorine atom, a bromine atom and an iodine atom.
Examples of the "C1-C6 alkyl group substituted with a cyano group" include a cyanomethyl group, a 1-cyanvethyl group, a 2-cyanoethyl group, a 2-cyanopropyl group, a 1-cyano-2-propyl group, a 1-cyano-2-methyl-2-propyl group, a

3-cyano-2-butyl group, a 3-cyanopropyl group, a 4-cyanobutyl group and a 6-cyanohexyl group.

Examples of the "C1-C4 alkyl group" include a methyl group, an ethyl group, a propyl group, a butyl group, an isopropyl group, an isobutyl group, a sec-butyl group and a tert-butyl group.

Examples of the "optionally halogenated C1-C6 alkyl group" include a methyl group, a trifluoromethyl group, a trichloromethyl group, a chloromethyl group, a dichloromethyl group, a fluoromethyl group, a difluoromethyl group, an ethyl group, a pentafluoroethyl group, a 2,2,2-trifluoroethyl group, a 2,2,2-trichloroethyl group, a propyl group, an isopropyl group, a heptafluoroisopropyl group, a butyl group, an isobutyl group, a sec-butyl group, a tert-butyl group, a pentyl group and a hexyl group.

Examples of the "optionally halogenated C2-C6 alkenyl group" include a vinyl group, a 1-propenyl group, a 2-propenyl group, a 1-methylvinyl group, a 2-chlorovinyl group and a 2-methyl-1-propenyl group.

Examples of the "optionally halogenated C3-C6 alkenyl group" include a 2-propenyl group, a 3-chloro-2-propenyl group, a 2-chloro-2-propenyl group, a 3,3-dichloro-2-propenyl group, a 3-bromo-2-propenyl group, a 2-bromo-2-propenyl group, a 3,3-dibromo-2-propenyl group, a 2-butenyl group, a 3-butenyl group, a 1-methyl-2-propenyl group, a 2-methyl-2-propenyl group, a 3-methyl-2-butenyl group, a 2-pentenyl group and a 2-hexenyl group.

Examples of the "optionally halogenated C2-C6 alkynyl group" include an ethynyl group, a 2-propynyl group, a 3-chloro-2-propynyl group, a 3-bromo-2-propynyl group, a 2-butynyl group and a 3-butynyl group.

Examples of the "optionally halogenated C3-C6 alkynyl group" include a 2-propynyl group, a 3-chloro-2-propynyl group, a 3-bromo-2-propynyl group, a 1-methyl-2-propynyl group, a 2-butynyl group and a 3-butynyl group.

Examples of the "C2-C6 cyanoalkyl group" include a cyanomethyl group, a 1-cyanoethyl group, a 2-cyanoethyl group, a 2-cyanopropyl group, a 1-cyano-2-propyl group, a 1-cyano-2-methyl-2-propyl group, a 3-cyano-2-butyl group, a 3-cyanopropyl group and a 4-cyanobutyl group.

Examples of the "C2-C6 alkoxyalkyl group" include a methoxymethyl group, a 1-methoxyethyl group, a 2-methoxyethyl group, a 2-ethoxyethyl group and a 2-isopropyloxyethyl group.

Examples of the "C3-C6 alkoxyalkyl group" include a 2-methoxyethyl group, a 2-ethoxyethyl group and a 2-isopropyloxyethyl group.

Examples of the "C2-C6 alkoxycarbonyl group" include a methoxycarbonyl group, an ethoxycarbonyl group, an isopropoxycarbonyl group and a tert-butoxycarbonyl group.

Examples of the "1,3-butadiene-1,4-diyl group optionally substituted with a substituent C" include a 1,3-butadiene-1,4-diyl group, a 2-bromo-1,3-butadiene-1,4-diyl group, a 2-chloro-1,3-butadiene-1,4-diyl group, a 2-cyano-1,3-butadiene-1,4-diyl group and a 1-methyl-1,3-butadiene-1,4-diyl group.

Examples of the "optionally halogenated C1-C6 alkoxy group" include a methoxy group, a trifluoromethoxy group, an ethoxy group, a 2,2,2-trifluoroethoxy group, a propyloxy group, an isopropyloxy group, a butoxy group, an isobutyloxy group, a sec-butoxy group, a tert-butoxy group, a pentyloxy group and a hexyloxy group.

Examples of the "optionally halogenated C1-C6 alkylthio group" include a methylthio group, a trifluoromethylthio group and an ethylthio group.

Examples of the "optionally halogenated C1-C6 alkylsulfinyl group" include a methylsulfinyl group, a trifluoromethylsulfinyl group and an ethylsulfinyl group.

Examples of the "optionally halogenated C1-C6 alkylsulfonyl group" include a methylsulfonyl group, a trifluoromethylsulfonyl group and an ethylsulfonyl group.

Examples of the "C3-C6 trialkylsilyl group" include a trimethylsilyl group and a tert-butyldimethylsilyl group.

Examples of the "phenyl group optionally substituted with a substituent A" include a phenyl group, a 2-chlorophenyl group, a 3-chlorophenyl group, a 4-chlorophenyl group, a 4-fluorophenyl group, a 4-bromophenyl group, a 4-iodophenyl group, a 2-cyanophenyl group, a 3-cyanophenyl group, a 4-cyanophenyl group, a 2-nitrophenyl group, a 3-nitrophenyl group, a 4-nitrophenyl group, a 2-methylphenyl group, a 3-methylphenyl group, a 4-methylphenyl group, a 2-(trifluoromethyl)phenyl group, a 3-(trifluoromethyl)phenyl group, a 4-(trifluoromethyl)phenyl group, a 2-methoxyphenyl group, a 3-methoxyphenyl group, a 4-methoxyphenyl group, a 4-(trifluoromethoxy)phenyl group and a 4-(methylthio) phenyl group.

Examples of the 5- to 6-membered heteroaryl group in the "5- to 6-membered heteroaryl group optionally substituted with a substituent A" include a 1-pyrrolyl group, a 2-pyrrolyl group, a 3-pyrrolyl group, a 2-furyl group, a 3-furyl group, a 2-thienyl group, a 3-thienyl group, a 1-pyrazolyl group, a 3(5)-pyrazolyl group, a 4-pyrazolyl group, a 1-imidazolyl group, a 2-imidazolyl group, a 4-imidazolyl group, a 2-pyridinyl group, a 3-pyridinyl group, a 4-pyridinyl group, a 2-pyrimidinyl group, a 4-pyrimidinyl group, a 5-pyrimidinyl group and a pyrazinyl group.

Examples of the "C3-C6 cycloalkyl group optionally substituted with a substituent B" include a cyclopropyl group, a 2-methylcyclopropyl group, a cyclobutyl group, a cyclopentyl group, a cyclohexyl group, a 4-methylcyclohexyl group, a 4-ethylcyclohexyl group, a 4-isopropylcyclohexyl group and a 4-tert-butylcyclohexyl group.

Examples of the "3- to 8-membered non-aromatic heterocyclic group optionally substituted with a substituent B" include an oxetan-3-yl group, a tetrahydrofuran-2-yl group, a tetrahydrofuran-3-yl group, a tetrahydropyran-2-yl group, a tetrahydropyran-3-yl group, a tetrahydropyran-4-yl group, a 1,3-dioxolan-2-yl group, a 1,3-dioxan-2-yl group and a

2,2-dimethyl-1,3-dioxolan-4-yl group.

Examples of the "C7-C9 phenylalkyl group whose benzene ring moiety is optionally substituted with a substituent A" include a benzyl group, a 1-phenylethyl group, a 2-phenylethyl group, a 2-chlorobenzyl group, a 3-chlorobenzyl group, a 4-chlorobenzyl group, a 4-bromobenzyl group, a 2-cyanobenzyl group, a 3-cyanobenzyl group, a 4-cyanobenzyl group, a 2-nitrobenzyl group, a 3-nitrobenzyl group, a 4-nitrobenzyl group, a 2-methylbenzyl group, a 3-methylbenzyl group, a 4-methylbenzyl group, a 2-methoxybenzyl group, a 3-methoxybenzyl group and a 4-methoxybenzyl group.

The styryl group is a group represented by -CH=CH-Ph (Ph represents a phenyl group) and examples of the "styryl group whose benzene ring moiety is optionally substituted with a substituent A" include a styryl group, a 2-chlorostyryl group, a 3-chlorostyryl group, a 4-chlorostyryl group, a 4-bromostyryl group, a 2-cyanostyryl group, a 3-cyanostyryl group, a 4-cyanostyryl group, a 2-nitrostyryl group, a 3-nitrostyryl group, a 4-nitrostyryl group, a 2-methylstyryl group, a 3-methylstyryl group, a 4-methylstyryl group, a 2-methoxystyryl group, a 3-methoxystyryl group and a 4-methoxystyryl group.

Examples of the "C1-C6 alkyl group substituted with at least one group selected from the group D" include a cyanomethyl group, a 1-cyanoethyl group, a 2-cyanoethyl group, a 2-cyanopropyl group, a 1-cyano-2-propyl group, a 1-cyano-2-methyl-2-propyl group, a 3-cyano-2-butyl group, a 3-cyanopropyl group, a 4-cyanobutyl group, a 2-nitroethyl group, a methoxycarbonylmethyl group, an ethoxycarbonylmethyl group, an isopropoxycarbonylmethyl group, a tert-butoxycarbonylmethyl group, an N,N-dimethylcarbamoylmethyl group, a 2-oxopropyl group, a 2-oxo-3,3,3-trifluoropropyl group, a 2-oxo-2-phenylethyl group, a 2-(methoxyimino)ethyl group, a methoxymethyl group, a 1-methoxyethyl group, a 2-methoxyethyl group, a 2-ethoxyethyl group, a 2-isopropyloxyethyl group, a 2-phenoxyethyl group, a 2-(methylthio)ethyl group, a 2-(methylsulfinyl)ethyl group, a 2-(methylsulfonyl)ethyl group, a 2-(N,N-dimethylamino)ethyl group, a cyclopropylmethyl group, a 1-(cyclopropyl)ethyl group, a 1-(1-methylcyclopropyl)ethyl group, a cyclobutylmethyl group, a cyclopentylmethyl group, a cyclohexylmethyl group, a benzyl group, a 2-pyridylmethyl group, a 3-pyridylmethyl group, a 4-pyridylmethyl group, a 2-furylmethyl group, a oxetan-3-ylmethyl group, a tetrahydrofuran-2-ylmethyl group, a tetrahydrofuran-3-ylmethyl group, a tetrahydropyran-2-ylmethyl group, a tetrahydropyran-3-ylmethyl group, a tetrahydropyran-4-ylmethyl group, a 1,3-dioxolan-2-ylmethyl group, a 1,3-dioxan-2-ylmethyl group, a 2,2-dimethyl-1,3-dioxolan-4-ylmethyl group and a 3-phenyl-2-propenyl group.

Examples of the present compound include the following aspects.

"Aspect 1"

A hydrazide compound represented by the formula (1), wherein $R^G$ is a C1-C6 alkyl group substituted with at least one substituent selected from the group D.

"Aspect 2"

A hydrazide compound represented by the formula (1), wherein $R^G$ is a C1-C6 alkyl group substituted with a cyano group.

"Aspect 3"

A hydrazide compound represented by the formula (1), wherein $R^2$ is a C1-C6 alkyl group substituted with a cyano group; and $R^3$ is a hydrogen atom or an optionally halogenated C1-C6 alkyl group.

"Aspect 4"

A hydrazide compound represented by the formula (1), wherein $R^2$ is a hydrogen atom or an optionally halogenated C1-C6 alkyl group; and $R^3$ is a C1-C6 alkyl group substituted with a cyano group.

"Aspect 5"

A hydrazide compound represented by the formula (1), wherein $R^1$ is a hydrogen atom.

"Aspect 6"

A hydrazide compound represented by the formula (1), wherein $R^1$ is a hydrogen atom; and any one of $R^2$ and $R^3$ is a C1-C6 alkyl group substituted with a cyano group, and the other one is a hydrogen atom or an optionally halogenated C1-C6 alkyl group.

"Aspect 7"

A hydrazide compound represented by the formula (1), wherein J is J1.

"Aspect 8"

A hydrazide compound represented by the formula (1), wherein M is -$R^8$ or a -$OR^9$.

"Aspect 9"

A hydrazide compound represented by the formula (1), wherein $R^1$ is a hydrogen atom or an optionally halogenated C1-C6 alkyl group; $R^2$ is a C1-C6 alkyl group substituted with a cyano group; $R^3$ is a hydrogen atom or an optionally halogenated C1-C6 alkyl group; $R^4$ is a halogen atom or an optionally halogenated C1-C6 alkyl group; $R^5$, $R^6$ and $R^7$ each independently represents a hydrogen atom, a halogen atom, a cyano group or an optionally halogenated C1-C6 alkyl group; M is -$R^8$, -$OR^9$ or a -$NR^{11}R^{12}$; $R^8$ is a hydrogen atom or an optionally halogenated C1-C6 alkyl group; $R^9$ is an optionally halogenated C1-C6 alkyl group; each of $R^{11}$ and $R^{12}$ independently represents an optionally halogenated C1-C6 alkyl group; J is J1; X is a nitrogen atom or CH; $Y^1$ is a nitrogen atom or CH; $R^{13}$ is a hydrogen atom, a halogen atom, a cyano group, an optionally halogenated C1-C6 alkyl group, an optionally halogenated C1-C6 alkoxy group, or an optionally halogenated C1-C6 alkylthio group; and $R^{14}$ is a hydrogen atom, a halogen atom or an optionally halogenated C1-C6 alkyl group.

"Aspect 10"

A hydrazide compound represented by the formula (1), wherein $R^1$ is a hydrogen atom or an optionally halogenated C1-C6 alkyl group; $R^2$ is a hydrogen atom or an optionally halogenated C1-C6 alkyl group; $R^3$ is a C1-C6 alkyl group substituted with a cyano group; $R^4$ is a halogen atom or an optionally halogenated C1-C6 alkyl group; each of $R^5$, $R^6$ and $R^7$ independently represents a hydrogen atom, a halogen atom, a cyano group or an optionally halogenated C1-C6 alkyl group; M is -$R^8$, -$OR^9$ or -$NR^{11}R^{12}$; $R^8$ is a hydrogen atom or an optionally halogenated C1-C6 alkyl group; $R^9$ is an optionally halogenated C1-C6 alkyl group; each of $R^{11}$ and $R^{12}$ independently represents an optionally halogenated C1-C6 alkyl group; J is J1; X is a nitrogen atom or CH; $Y^1$ is a nitrogen atom or CH; $R^{13}$ is a hydrogen atom, a halogen atom, a cyano group, an optionally halogenated C1-C6 alkyl group, an optionally halogenated C1-C6 alkoxy group, or an optionally halogenated C1-C6 alkylthio group; and $R^{14}$ is a hydrogen atom, a halogen atom or an optionally halogenated C1-C6 alkyl group.

"Aspect 11"

A hydrazide compound represented by the formula (1), wherein $R^1$ is a hydrogen atom or a methyl group; $R^2$ is a C1-C6 alkyl group substituted with a cyano group; $R^3$ is a hydrogen atom or an optionally halogenated alkyl group; $R^4$ is a halogen atom or a methyl group; $R^5$ and $R^7$ are hydrogen atom; $R^6$ is a hydrogen atom, a halogen atom, a cyano group or a methyl group; M is a hydrogen atom, a methyl group, a methoxy group or a dimethylamino group; J is J1; X is a nitrogen atom or CH; $Y^1$ is a nitrogen atom; $R^{13}$ is a halogen atom, a cyano group or a trifluoromethyl group; and $R^{14}$ is a halogen atom.

"Aspect 12"

A hydrazide compound represented by the formula (1), wherein $R^1$ is a hydrogen atom or a methyl group; $R^2$ is a hydrogen atom or an optionally halogenated alkyl group; $R^3$ is a C1-C6 alkyl group substituted with a cyano group; $R^4$ is a halogen atom or a methyl group; $R^5$ and $R^7$ are hydrogen atoms; $R^6$ is a hydrogen atom, a halogen atom, a cyano group or a methyl group; M is a hydrogen atom, a methyl group, a methoxy group or a dimethylamino group; J is J1; X is a nitrogen atom or CH; $Y^1$ is a nitrogen atom; $R^{13}$ is a halogen atom, a cyano group or a trifluoromethyl group; and $R^{14}$ is a halogen atom.

"Aspect 13"

A hydrazide compound represented by the formula (1), wherein $R^G$ is a C1-C6 alkyl group substituted with at least one substituent selected from the group D1 shown below, an optionally halogenated C3-C6 alkenyl group, an optionally halogenated C3-C6 alkynyl group, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B:

"group D1: (a) a cyano group, (g) -$OR^{107}$ ($R^{107}$ represents a hydrogen atom, a C1-C4 alkyl group or a phenyl group optionally substituted with a substituent A), (j) a C3-C6 cycloalkyl group optionally substituted with a substituent B, (k) a phenyl group optionally substituted with a substituent A, (l) a 5- to 6-membered heteroaryl group optionally substituted with a substituent A, and (m) a 3-to 8-membered non-aromatic heterocyclic group optionally substituted with a substituent B".

"Aspect 14"

A hydrazide compound represented by the formula (1), wherein $R^G$ is a C1-C6 alkyl group substituted with at least one substituent selected from the group D2 shown below, an optionally halogenated C3-C6 alkenyl group, or an optionally halogenated C3-C6 alkynyl group; "group D2: (a) a cyano group and (g) -$OR^{107}$ ($R^{107}$ represents a hydrogen atom, a C1-C4 alkyl group or a phenyl group optionally substituted with a substituent A)".

"Aspect 15"

A hydrazide compound represented by the formula (1), wherein $R^G$ is a C1-C6 alkyl group substituted with a cyano group, an optionally halogenated C3-C6 alkenyl group, or an optionally halogenated C3-C6 alkynyl group.

"Aspect 16"

A hydrazide compound represented by the formula (1), wherein $R^G$ is an optionally halogenated C3-C6 alkenyl group or an optionally halogenated C3-C6 alkynyl group.

"Aspect 17"

A hydrazide compound represented by the formula (1), wherein $R^G$ is an optionally halogenated C3-C6 alkenyl group.

"Aspect 18"

A hydrazide compound represented by the formula (1), wherein $R^G$ is an optionally halogenated C3-C6 alkynyl group.

"Aspect 19"

A hydrazide compound represented by the formula (1), wherein $R^G$ is a C1-C6 alkyl group substituted with at least one substituent selected from the group D3 shown below, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B;
"group D3: (j) a C3-C6 cycloalkyl group optionally substituted with a substituent B, (k) a phenyl group optionally substituted with a substituent A, (l) a 5- to 6-membered heteroaryl group optionally substituted with a substituent A, and (m) a 3- to 8-membered non-aromatic heterocyclic group optionally substituted with a substituent B".

"Aspect 20"

A hydrazide compound represented by the formula (1), wherein $R^G$ is a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B.

"Aspect 21"

A hydrazide compound represented by the formula (1), wherein $R^G$ is a C1-C6 alkyl group substituted with at least one substituent selected from the group D3 shown below: "group D3: (j) a C3-C6 cycloalkyl group optionally substituted with a substituent B, (k) a phenyl group optionally substituted with a substituent A, (l) a 5- to 6-membered heteroaryl group optionally substituted with a substituent A, and (m) a 3- to 8-membered non-aromatic heterocyclic group optionally substituted with a substituent B".

"Aspect 22"

A hydrazide compound represented by the formula (1), wherein $R^G$ is a C1-C6 alkyl group substituted with at least one substituent selected from the group D4 shown below, a C3-C6 cycloalkyl group optionally substituted with a substituent B, or a 3- to 8-membered non-aromatic heterocyclic group optionally substituted with a substituent B;
"group D4: (j) a C3-C6 cycloalkyl group optionally substituted with a substituent B and (m) a 3- to 8-membered non-aromatic heterocyclic group optionally substituted with a substituent B".

"Aspect 23"

A hydrazide compound represented by the formula (1), wherein $R^G$ is a C1-C6 alkyl group substituted with at least one substituent selected from the group D5 shown below, a 5- to 6-membered heteroaryl group optionally substituted with a substituent A, or a 3- to 8-membered non-aromatic heterocyclic group optionally substituted with a substituent B:
"group D5: (1) a 5- to 6-membered heteroaryl group optionally substituted with a substituent A and (m) a 3- to 8-membered non-aromatic heterocyclic group optionally substituted with a substituent B".

"Aspect 24"

A hydrazide compound represented by the formula (1), wherein one of $R^2$ and $R^3$ is $R^G$, and the other is a hydrogen atom or an optionally halogenated C1-C6 alkyl group.

"Aspect 25"

A hydrazide compound represented by the formula (1), wherein $R^2$ is $R^G$ and $R^3$ is a hydrogen atom.

"Aspect 26"

A hydrazide compound represented by the formula (1), wherein $R^2$ is a hydrogen atom and $R^3$ is $R^G$.

"Aspect 27"

A hydrazide compound represented by the formula (1), wherein $R^2$ is $R^G$ and $R^3$ is an optionally halogenated C1-C6 alkyl group.

"Aspect 28"

A hydrazide compound represented by the formula (1), wherein $R^2$ is an optionally halogenated C1-C6 alkyl group and $R^3$ is $R^G$.

"Aspect 29"

A hydrazide compound represented by the formula (1), wherein one of $R^2$ and $R^3$ is $R^G$, and the other is a hydrogen atom, a methyl group or an ethyl group.

"Aspect 30"

A hydrazide compound represented by the formula (1), wherein $R^2$ is $R^G$ and $R^3$ is a methyl group or an ethyl group.

"Aspect 31"

A hydrazide compound represented by the formula (1), wherein $R^2$ is a methyl group or an ethyl group and $R^3$ is $R^G$.

"Aspect 32"

A hydrazide compound represented by the formula (1), wherein one of $R^2$ and $R^3$ is $R^G$, and the other is a hydrogen atom or an optionally halogenated C1-C6 alkyl group; and $R^G$ is a C1-C6 alkyl group substituted with a cyano

group, an optionally halogenated C3-C6 alkenyl group, or an optionally halogenated C3-C6 alkynyl group.

"Aspect 33"

A hydrazide compound represented by the formula (1), wherein M is $-OR^9$.

"Aspect 34"

A hydrazide compound represented by the formula (1), wherein M is $-OR^9$ and $R^9$ is an optionally halogenated C1-C6 alkyl group.

"Aspect 35"

A hydrazide compound represented by the formula (1), wherein M is $-R^8$, $-OR^9$ or $-NR^{11}R^{12}$, $R^8$ is a hydrogen atom or an optionally halogenated C1-C6 alkyl group, $R^9$ is an optionally halogenated C1-C6 alkyl group, and each of $R^{11}$ and $R^{12}$ independently represents an optionally halogenated C1-C6 alkyl group.

"Aspect 36"

A hydrazide compound represented by the formula (1), wherein M is a hydrogen atom, a methyl group, a methoxy group, an ethoxy group or a dimethylamino group.

"Aspect 37"

A hydrazide compound represented by the formula (1), wherein M is a methoxy group or an ethoxy group.

"Aspect 38"

A hydrazide compound represented by the formula (1), wherein M is a hydrogen atom or a methoxy group.

"Aspect 39"

A hydrazide compound represented by the formula (1), wherein $R^4$ is a halogen atom or an optionally halogenated C1-C6 alkyl group, and each of $R^5$, $R^6$ and $R^7$ independently represents a hydrogen atom, a halogen atom, a cyano group or an optionally halogenated C1-C6 alkyl group.

"Aspect 40"

A hydrazide compound represented by the formula (1), wherein $R^4$ is a halogen atom or a methyl group, $R^5$ and $R^7$ are hydrogen atoms, and $R^6$ is a hydrogen atom, a halogen atom or a cyano group.

"Aspect 41"

A hydrazide compound represented by the formule (1), wherein J is J1, X is a nitrogen atom or CH, $Y^1$ is a nitrogen atom or CH, and $R^{13}$ is a hydrogen atom, a halogen atom, cyano group, an optionally halogenated C1-C6 alkyl group, or an optionally halogenated C1-C6 alkoxy group.

"Aspect 42"

A hydrazide compound represented by the formula (1), wherein J is J1, X is a nitrogen atom, $Y^1$ is a nitrogen atom, $R^{13}$ is a halogen atom, a cyano group, a trifluoromethyl group or a 2,2,2-trifluoroethoxy group, and $R^{14}$ is a halogen atom.

"Aspect 43"

A hydrazide compound represented by the formula (1), wherein $R^1$ is a hydrogen atom or an optionally halogenated C1-C6 alkyl group; one of $R^2$ and $R^3$ is $R^G$, and the other is a hydrogen atom or an optionally halogenated C1-C6 alkyl group; $R^G$ is a C1-C6 alkyl group substituted with a cyano group, an optionally halogenated C3-C6 alkenyl

group, or an optionally halogenated C3-C6 alkynyl group; $R^4$ is a halogen atom or an optionally halogenated C1-C6 alkyl group; each of $R^5$, $R^6$ and $R^7$ independently represents a hydrogen atom, a halogen atom, a cyano group or an optionally halogenated C1-C6 alkyl group; J is J1; X is a nitrogen atom or CH; $Y^1$ is a nitrogen atom or CH; and $R^{13}$ is a hydrogen atom, a halogen atom, a cyano group, an optionally halogenated C1-C6 alkyl group, or an optionally halogenated C1-C6 alkoxy group.

"Aspect 44"

A hydrazide compound represented by the formula (1), wherein $R^1$ is a hydrogen atom or an optionally halogenated C1-C6 alkyl group; one of $R^2$ and $R^3$ is $R^G$, and the other is a hydrogen atom or an optionally halogenated C1-C6 alkyl group; $R^G$ is a C1-C6 alkyl group substituted with a cyano group, an optionally halogenated C3-C6 alkenyl group, or an optionally halogenated C3-C6 alkynyl group; $R^4$ is a halogen atom or an optionally halogenated C1-C6 alkyl group; each of $R^5$, $R^6$ and $R^7$ independently represents a hydrogen atom, a halogen atom, a cyano group or an optionally halogenated C1-C6 alkyl group; M is $-OR^9$; J is J1; X is a nitrogen atom or CH; $Y^1$ is a nitrogen atom or CH; and $R^{13}$ is a hydrogen atom, a halogen atom, a cyano group, an optionally halogenated C1-C6 alkyl group, or an optionally halogenated C1-C6 alkoxy group.

"Aspect 45"

A hydrazide compound represented by the formula (1), wherein $R^1$ is a hydrogen atom or a methyl group; one of $R^2$ and $R^3$ is a cyanomethyl group, a 2-propenyl group or a 2-propynyl group, and the other is a hydrogen atom, a methyl group or an ethyl group; $R^4$ is a halogen atom or a methyl group; $R^5$ and $R^7$ are hydrogen atoms; $R^6$ is a hydrogen atom, a halogen atom or a cyano group; M is a hydrogen atom, a methyl group, a methoxy group, an ethoxy group or a dimethylamino group; J is J1; X is a nitrogen atom; $Y^1$ is a nitrogen atom; $R^{13}$ is a halogen atom, a cyano group, a trifluoromethyl group or a 2,2,2-trifluoroethoxy group; and $R^{14}$ is a halogen atom.

"Aspect 46"

A hydrazide compound represented by the formula (1), wherein $R^1$ is a hydrogen atom; one of $R^2$ and $R^3$ is a cyanomethyl group, a 2-propenyl group or a 2-propynyl group, and the other is a hydrogen atom, a methyl group or an ethyl group; $R^4$ is a halogen atom or a methyl group; $R^5$ and $R^7$ are hydrogen atoms; $R^6$ is a hydrogen atom, a halogen atom or a cyano group; M is a methoxy group or an ethoxy group; J is J1; X is a nitrogen atom; $Y^1$ is a nitrogen atom; $R^{13}$ is a halogen atom, a cyano group, a trifluoromethyl group or a 2,-2,2-trifluoroethoxy group; and $R^{14}$ is a halogen atom.

Hereinafter, a process for producing the present compound will be explained.
The present compound can be produced, for example, by the following Process A-1 to Process C-3.

(Process A)

The present compound can be produced by reacting a compound represented by the formula (2):

[Chemical Formula 3]

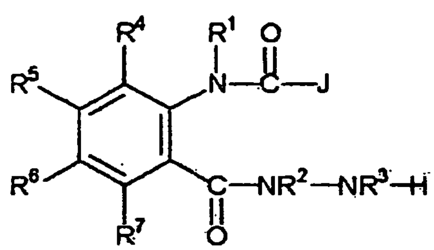

(2)

wherein $R^1$, $R^2$, $R^3$, $R^4$, $R^5$, $R^6$, $R^7$ and J are as defined above (hereinafter referred to as the compound (2)) with a compound represented by the formula (3):

[Chemical Formula 4]

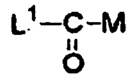 (3)

wherein M is as defined above and $L^1$ represents a halogen atom or an M-C(=O)O- group (hereinafter referred to as the compound (3)).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (3) used in the reaction is usually from 1 to 2 mol per mol of the compound (2).

The reaction is carried out in the presence of a base, if necessary. Examples of the base when the reaction is carried out in the presence of a base include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base when the reaction is carried out in the presence of the base is usually from 1 mol or more per mol of the compound (2).

The reaction temperature is usually from 0 to 100˚C and the reaction time is usually from 0.1 to 24 hours.

After completion of the reaction, the present compound can be isolated by pouring the reaction mixture into water and extracting the mixture with an organic solvent, or collecting a deposited precipitate by filtration. The isolated present compound may be further purified, for example, by recrystallization, or chromatography.

(Process B-1)

The present compound can be produced by reacting a compound represented by the formula (6):

[Chemical Formula 5]

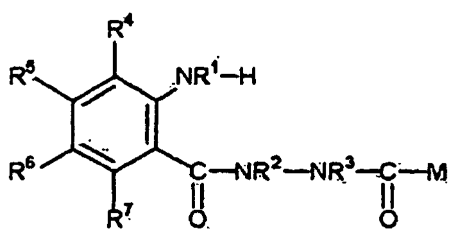 (6)

wherein $R^1$, $R^2$, $R^3$, $R^4$, $R^5$, $R^6$, $R^7$ and M are as defined above (hereinafter referred to as the compound (6)) with a compound represented by the formula (7):

[Chemical Formula 6]

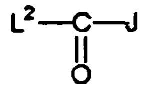 (7)

wherein $L^2$ represents a halogen atom and J is as defined above (hereinafter referred to as the compound (7)).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (7) used in the redaction is usually 1 mol or more per mol of the compound (6).

The reaction is carried out in the presence of a base, if necessary. Examples of the base when the reaction is carried out in the presence of a base include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base when the reaction is carried out in the presence of the base is usually from 1 mol or more per mol of the compound (6).

The reaction temperature is usually from 0 to 100°C and the reaction time is usually from 0.1 to 24 hours.

After completion of the reaction, the present compound can be isolated by pouring the reaction mixture into water and extracting the mixture with an organic solvent, or collecting a deposited precipitate by filtration. The isolated present compound may be further purified, for example, by recrystallization, or chromatography.

(Process B-2)

The present compound can be produced by reacting the compound (6) with a compound represented by the formula (8) :

[Chemical Formula 7]

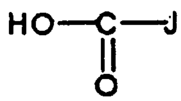

(8)

wherein J are as defined above (hereinafter referred to as the compound (8)) in the presence of a dehydrating agent.

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (8) used in the reaction is usually 1 mol or more per mol of the compound (6).

Examples of the dehydrating agent used in the reaction include carbodiimides such as dicyclohexylcarbodiimide (DCC), and 1-ethyl-3-(3-dimethylaminopropyl) carbodiimide hydrochloride (WSC). The amount of the dehydrating agent to be used is usually from 1 or more per mol of the compound (6).

The reaction temperature is usually from 0 to 100°C and the reaction time is usually from 0.1 to 24 hours.

After completion of the reaction, the present compound can be isolated by pouring the reaction mixture into water and extracting the mixture with an organic solvent, or collecting a deposited precipitate by filtration. The isolated present compound may be further purified, for example, by recrystallization, chromatography, or the like.

(Process B-3)

Among the present compounds, a compound represented by the formula (1-i):

[Chemical Formula 8]

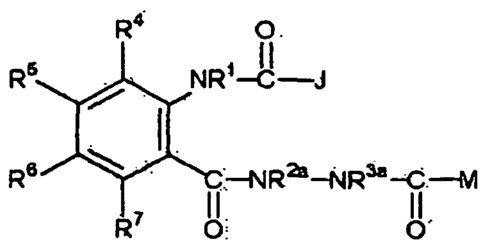

(1-i)

wherein $R^{2a}$ represents $R^G$, $R^{3a}$ represents a hydrogen atom, an optionally halogenated C1-C6 alkyl group or $R^G$, or

$R^{2a}$ represents an optionally halogenated C1-C6 alkyl group, $R^{3a}$ represents $R^G$, and $R^1$, $R^4$, $R^5$, $R^6$, $R^7$, J and M are as defined above (hereinafter referred to as the compound (1-i)) can be produced by reacting a compound (6-i) represented by the formula (6-i):

[Chemical Formula 9]

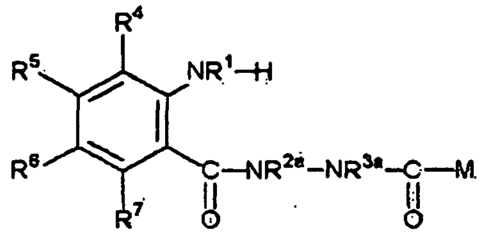

(6-i)

wherein $R^1$, $R^{2a}$, $R^{3a}$, $R^4$, $R^5$, $R^6$, $R^7$ and M are as defined above (hereinafter referred to as the compound (6-i)) with a compound represented by the formula (4):

[Chemical Formula 10]

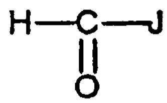

(4)

wherein J are as defined above (hereinafter referred to as the compound (4)) in the presence of an oxidizing agent, for example, peracids such as methachloroperbenzoic acid; and quinone compounds such as o-chloranil, and p-chloranil.

The reaction is carried out in the presence of a solvent. Examples of the solvent used in the reaction include ether solvents such as 1,4-dioxane, diethylether, tetrahydrofuran, and methyl tert-butyl ether; halogenated hydrocarbon solvents such as dichloromethane, chloroform, carbon tetrachloride, 1,2-dichloroethane, and chlorobenzene; hydrocarbon solvents such as hexane, heptane, toluene, benzene, and xylene; nitrite solvents such as acetonitrile; amide solvents such as N,N-dimethylformamide; nitrogen-containing cyclic compound solvents such as N-methyl pyrrolidone, and 1,3-dimethyl-2-imidazolidinone; aprotic solvents, for example, sulfoxide solvents such as dimethyl sulfoxide; carboxylic acid solvents such as acetic acid; ketone solvents such as acetone, and isobutyl methyl ketone; ester solvents such as ethyl acetate; alcohol solvents such as 2-propanol, and tert-butyl alcohol; water; and a mixture thereof.

The amount of the compound (4) used in the reaction is usually 1 mol or more per mol of the compound (6-i).

The reaction temperature is usually from 0 to 150°C and the reaction time is usually from instant to 72 hours.

After completion of the reaction, the compound (1-i) can be isolated by pouring the reaction mixture into water and extracting the mixture with an organic solvent, or collecting a deposited precipitate by filtration. The isolated compound (1-i) may be further purified, for example, by recrystallization, or chromatography.

(Process C-1)

Among the present compounds, a compound represented by the formula (1-ii):

[Chemical Formula 11]

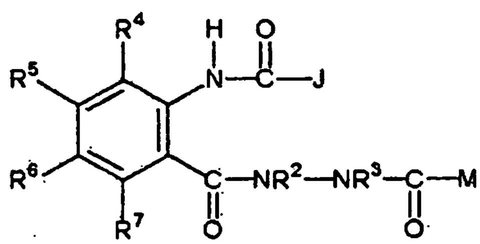

(1-ii)

wherein $R^2$, $R^3$, $R^4$, $R^5$, $R^6$, $R^7$, J and M are as defined above (hereinafter referred to as the compound (1-ii)) is produced by reacting a compound represented by the formula (9):

[Chemical Formula 12]

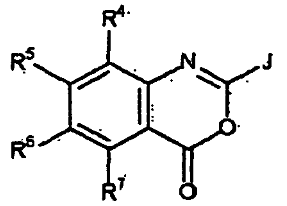

(9)

wherein $R^4$, $R^5$, $R^6$, $R^7$ and J are as defined above (hereinafter referred to as the compound (9)) with a compound represented by the formula (10):

[Chemical Formula 13]

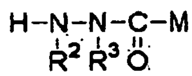

(10)

wherein $R^2$, $R^3$ and M are as defined above (hereinafter referred to as the compound (10)).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (10) used in the reaction is usually 1 mol or more per mol of the compound (9).

The reaction temperature is usually from 0 to 100°C and the reaction time is usually from 0.1 to 48 hours.

After completion of the reaction, the compound (1-ii) can be isolated by pouring the reaction mixture into water and extracting the mixture with an organic solvent, or collecting a deposited precipitate by filtration. The isolated compound (1-ii) may be further purified, for example, by recrystallization, or chromatography.

(Process C-2)

Among the present compounds, a compound represented by the formula (1-iii):

[Chemical Formula 14]

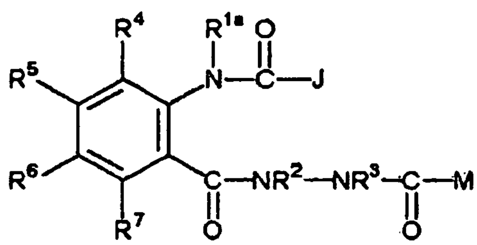

(1-iii)

wherein $R^{1a}$ represents an optionally halogenated C1-C6 alkyl group, a C2-C6 cyanoalkyl group, a C2-C6 alkoxyalkyl group, an optionally halogenated C3-C6 alkenyl group, an optionally halogenated C3-C6 alkynyl group, or a C7-C9 phenylalkyl group whose benzene ring moiety is optionally substituted with a substituent A, and $R^2$, $R^3$, $R^4$, $R^5$, $R^6$, $R^7$, J and M are as defined above (hereinafter referred to as the compound (1-iii)) is produced by reacting a compound represented by the formula (11):

[Chemical Formula 15]

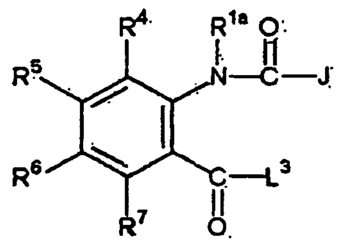

(11)

wherein $L^3$ represents a halogen atom, and $R^{1a}$, $R^4$, $R^5$, $R^6$, $R^7$ and J are as defined above (hereinafter referred to as the compound (11)) with the compound (10).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (10) used in the reaction is usually 1 mol or more per mol of the compound (11).

The reaction is carried out in the presence of a base, if necessary. Examples of the base when the reaction is carried out in the presence of a base include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base when the reaction is carried out in the presence of the base is usually from 1 mol or more per mol of the compound (6).

The reaction temperature is usually from 0 to 100°C and the reaction time is usually from 0.1 to 24 hours.

After completion of the reaction, the compound (1-iii) can be isolated by pouring the reaction mixture into water and extracting the mixture with an organic solvent, or collecting a deposited precipitate by filtration. The isolated compound (1-iii) may be further purified, for example, by recrystallization, or chromatography.

(Process C-3)

The compound (1-iii) can also be produced by reacting a compound represented by the formula (12):

[Chemical Formula 16]

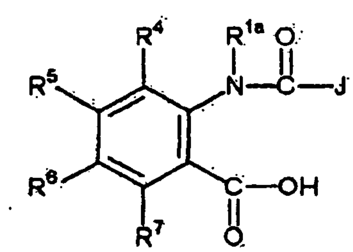

(12)

wherein $R^{1a}$, $R^4$, $R^5$, $R^6$, $R^7$ and J are as defined above (hereinafter referred to as the compound (12)) with the compound (10) in the presence of a dehydrating agent.

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (10) used in the reaction is usually 1 mol or more per mol of the compound (12).

Examples of the dehydrating agent used in the reaction include carbodiimides such as dicyclohexylcarbodiimide (DCC), and 1-ethyl-3-(3-dimethylaminopropyl)carbodiimide hydrochloride (WSC). The amount of the dehydrating agent used is usually 1 mol or more per mol of the compound (12).

The reaction temperature is usually from 0 to 100°C and the reaction time is usually from 0.1 to 24 hours.

After completion of the reaction, the compound (1-iii) can be isolated by pouring the reaction mixture into water and extracting the mixture with an organic solvent, or collecting a deposited precipitate by filtration. The isolated compound (1-iii) may be further purified, for example, by recrystallization, or chromatography.

Hereinafter, a method for producing intermediates for producing the present compound will be explained.

Among the compound (2), a compound represented by the formula (2-i):

[Chemical Formula 17]

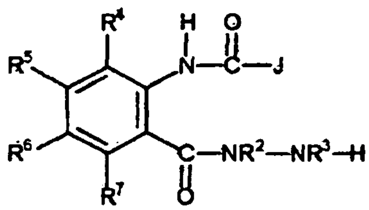

(2-i)

wherein $R^2$, $R^3$, $R^4$, $R^5$, $R^6$, $R^7$ and J are as defined above (hereinafter referred to as the compound (2-i)) can be produced by reacting the compound (9) with a compound represented by the formula (13):

[Chemical Formula 18]

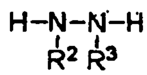

(13)

wherein $R^2$ and $R^3$ are as defined above (hereinafter referred to as the compound (13)).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (13) used in the reaction is usually 1 mol or more per mol of the compound (9).

The reaction temperature is usually from -50 to 100°C and the reaction time is usually from 0.1 to 24 hours.

After completion of the reaction, the compound (2-i) can be isolated by pouring the reaction mixture into water and extracting the mixture with an organic solvent, or collecting a deposited precipitate by filtration. The isolated compound (2-i) may be further purified, for example, by recrystallization, or chromatography.

Among the compound (2), a compound represented by the formula (2-ii):

[Chemical Formula 19]

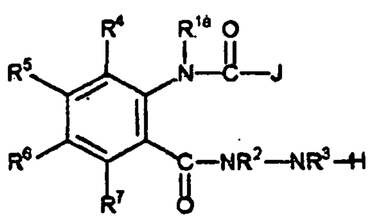

(2-ii)

wherein $R^{1a}$, $R^2$, $R^3$, $R^4$, $R^5$, $R^6$, $R^7$ and J are as defined (hereinafter referred to as the compound (2-ii)) can be produced by reacting the compound (11) with the compound (13).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (13) used in the reaction is usually 1 mol or more per mol of the compound (11).

The reaction temperature is usually from -50 to 100˚C and the reaction time is usually from 0.1 to 24 hours.

After completion of the reaction, the compound (2-ii) can be isolated by pouring the reaction mixture into water and extracting the mixture with an organic solvent, or collecting a deposited precipitate by filtration. The isolated compound (2-ii) may be further purified, for example, by recrystallization, or chromatography.

The compound (9) can be produced by reacting a compound represented by the formula (14):

[Chemical Formula 20]

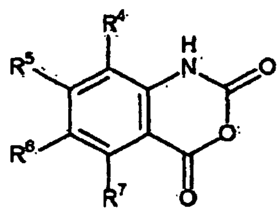

(14)

wherein $R^4$, $R^5$, $R^6$ and $R^7$ are as defined above (hereinafter referred to as the compound (14)) with the compound (7).

The reaction is carried out in the presence of a base in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (7) used in the reaction is usually from 0.5 to 2 mol per mol of the compound (14).

Examples of the base used in the reaction include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base is usually 1 mol or more per mol of the compound (14).

The reaction temperature is usually from 50 to 150˚C and the reaction time is usually from 1 to 24 hours.

After completion of the reaction, the compound (9) can be isolated by pouring the reaction mixture into water and extracting the mixture with an organic solvent, or collecting a deposited precipitate by filtration. The isolated compound (9) may be further purified, for example, by recrystallization, or chromatography.

The compound (9) can be produced by reacting a compound represented by the formula (15):

[Chemical Formula 21]

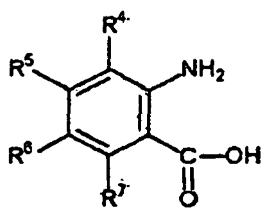

(15)

wherein $R^4$, $R^5$, $R^6$ and $R^7$ are as defined above (hereinafter referred to as the compound (15)) with the compound (7).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The process comprises the following (step 5-1) and (step 5-2).

(Step 5-1)

The step is carried out by reacting the compound (15) with the compound (7) in the presence of a base.

The amount of the compound (7) used in the step is usually 1 mol or more per mol of the compound (15). Examples of the base used in the step include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base used is usually 1 mol or more per mol of the compound (15).

The reaction temperature of the step is usually from 0 to 50°C and the reaction time is usually from 0.1 to 24 hours.

After completion of the step, the reaction mixture is used as it is for the following (step 5-2).

(Step 5-2)

The step is carried out by reacting the reaction mixture in the (step 5-1) with a sulfonyl halide in the presence of a base.

Examples of the sulfonyl halide used in the step include methanesulfonyl chloride, p-toluenesulfonyl chloride, and trifluoromethanesulfonyl chloride. The amount of the sulfonyl halide used in the step is usually from 1 mol or more per mol of the compound (15) used in the (step 5-1).

Examples of the base used in the step include the same bases as those described with respect to the (step 5-1) and usually include the same bases as those described with respect to the (step 5-1). The amount of the base used is usually 1 mol or more per mol of the compound (15) used in the (step 5-1).

The reaction temperature of the step is usually from 0 to 50°C and the reaction time is usually from 0.1 to 24 hours.

After completion of this step, the compound (9) can be isolated by pouring the reaction mixture into water, followed by conventional extraction with an organic solvent. The isolated compound (9) may be further purified, for example, by recrystallization, or chromatography.

The compound (11) can be produced by reacting the compound (12) with a halogenating agent.

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

Examples of the halogenating agent used in the reaction include thionyl chloride, thionyl bromide, phosphorus oxychloride, phosphorus oxybromide, phosphorus pentachloride, oxalyl chloride and phosgene.

The amount of the halogenating agent used in the reaction is usually 1 mol or more per mol of the compound (12).

The reaction temperature is usually from 0°C to 150°C and the reaction time is usually from 0.1 to 24 hours.

After completion of the reaction, the compound (11) can be isolated by collecting a precipitate deposited in the reaction mixture by filtration, or extracting the reaction mixture with an organic solvent. The isolated compound (11) may be usually used in the next step as it is. If necessary, it can be further purified, for example, by recrystallization, or chromatography.

The compound (12) can be produced by reacting a compound represented by the formula (16):

[Chemical Formula 22]

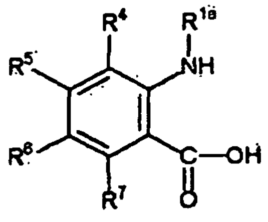

(16)

wherein $R^{1a}$, $R^4$, $R^5$, $R^6$ and $R^7$ are as defined above (hereinafter referred to as the compound (16)) with the compound (7).

The reaction is carried out in the presence or the absence of a solvent. Examples of the solvent used in the reaction include ethers such as 1,4-dioxane, diethyl ether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidadzolidinone, and dimethyl sulfoxide; and a mixture thereof.

The amount of the compound (7) used in the reaction is usually 1 mol or more per mol of the compound (16).

The reaction is carried out in the presence of a base. Examples of the based used include nitrogen-containing heterocyclic compounds such as pyridine, picoline, 2,6-lutidine, 1,8-diazabicyclo[5,4,0]7-undecene (DBU), and 1,5-diazabicyclo[4,3,0]5-nonene (DBN); tertiary amines such as triethylamine, and N,N-diisopropylethylamine; and inorganic bases such as potassium carbonate, and sodium hydride. The amount of the base used is usually 1 mol or more per mol of the compound (16).

The reaction temperature of the step is usually from 0 to 50°C and the reaction time is usually from 0.1 to 24 hours.

After completion of the reaction, the compound (12) can be isolated by pouring the reaction mixture into water and extracting the mixture with an organic solvent, or collecting a deposited precipitate by filtration. The isolated compound (12) may be further purified, for example, by recrystallization, or chromatography.

The compound (6) can be produced by reacting a compound represented by the formula (17):

[Chemical Formula 23]

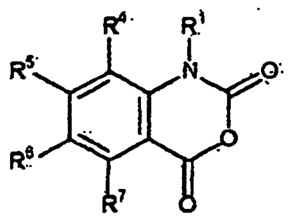

(17)

wherein $R^1$, $R^4$, $R^5$, $R^6$ and $R^7$ are as defined above (hereinafter referred to as the compound (17)) with the compound (10).

The reaction is carried out in the presence or the absence of a solvent. Examples of the solvent used in the reaction include ethers such as 1,4-dioxane, diethylether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidazolidinone, and dimethyl sulfoxide; alcohols such as methanol, ethanol, and isopropyl alcohol; and a mixture thereof.

The amount of the compound (10) used in the reaction is usually 1 mol or more per mol of the compound (17).

The reaction temperature is usually from -20 to 150°C and the reaction time is usually from 0.1 to 24 hours.

After completion of the reaction, the compound (6) can be isolated by pouring the reaction mixture into water and extracting the mixture with an organic solvent, or collecting a deposited precipitate by filtration. The isolated compound (6) may be further purified, for example, by recrystallization, or chromatography.

The compound (6) can be produced according to the following scheme:

[Chemical Formula 24]

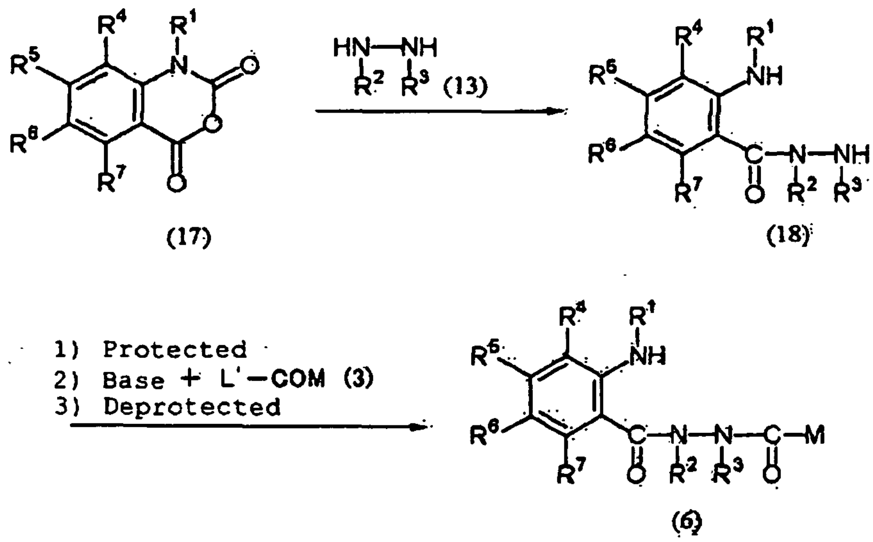


(17) (18) (6)

wherein $L^1$, $R^1$, $R^2$, $R^3$, $R^4$, $R^5$, $R^6$ $R^7$ and M are as defined above.

Compound (17) → Compound (18)

The amount of the compound (13) is usually 1 mol per mol of the compound (17).

The reaction is usually carried out in the presence of a solvent, and examples of the solvent include ethers such as 1,4-dioxane, diethylether, tetrahydrofuran, and methyl tert-butyl ether; halogenated hydrocarbons such as dichloromethane, chloroform, carbon tetrachloride, 1,2-dichloroethane, and chlorobenzene; hydrocarbons such as toluene, benzene, and xylene; nitriles such as acetonitrile; aprotic polar solvents such as N,N-dimethylformamide, N-methyl pyrrolidone, 1,3-dimethyl-2-imidazolidinone, and dimethyl sulfoxide; alcohols such as methanol, ethanol, and isopropyl alcohol; and a mixture thereof.

Compound (18) → Compound (6)

1) The amino group (-$NHR^1$ group) on the benzene ring of the compound (18) can be protected with a suitable protecting group (e.g. N-benzylidene group, N-(1-methyl)ethylidene group, and benzyloxycarbonyl group) described in Greene's Protective Groups in Organic Synthesis (WILEY) etc., if necessary.
2) The amount of the compound (3) used is usually 1 mol per mol of the compound (18) or a derivative thereof in which the amino group is protected. Examples of a base used in the reaction include metal carbonates such as potassium carbonate.
3) The compound (6) in which the amino group is protected can be deprotected under known conditions.

Among the compound (6), a compound represented by the formula (6-ii) can be produced according to the following scheme:

[Chemical Formula 25]

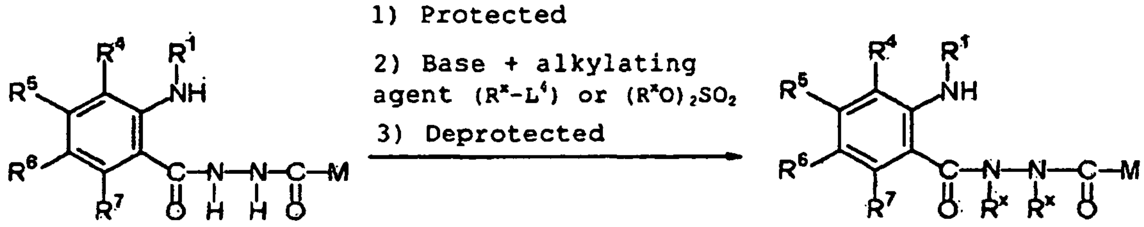


(6-i) (6-ii)

wherein $R^x$ represents an optionally halogenated C1-C6 alkyl group or $R^G$, $L^4$ represents a leaving group (e.g., a halogen atom, methanesulfonyloxy group, and p-toluenesulfonyloxy group) and $R^1$, $R^4$, $R^5$, $R^6$, $R^7$, $R^G$ and M are as defined above.

1) Protection
The amino group (-NHR$^1$ group) on the benzene ring of the compound (6-i) can be protected with a suitable protecting group (e.g., an N-benzylidene group, an N-(1-methyl)ethylidene group, and a benzyloxycarbonyl group) described in Greene's Protective Groups in Organic Synthesis (WILEY) etc., if necessary.
2) The amount of an alkylating agent used is usually 2 mol per mol of the compound (6-i) or a derivative thereof in which the amino group is protected. Examples of a base used in the reaction include metal carbonates such as potassium carbonate.
3) The compound (6-ii) in which the amino group is protected can be deprotected under known conditions.

The compound (6-iv) can be produced according to the following scheme:

[Chemical Formula 26]

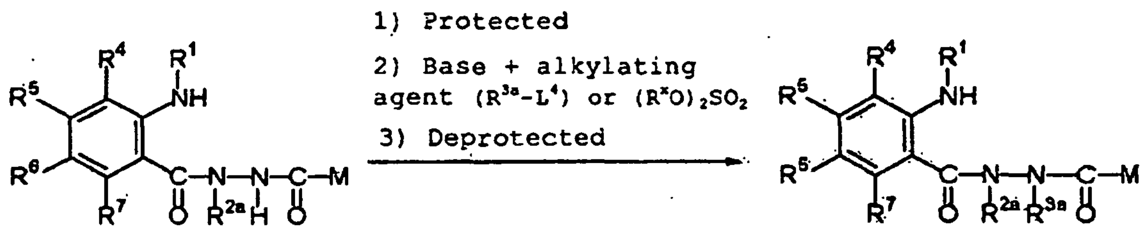


(6-iii) (6-iv)

wherein $R^{2a}$ and $R^{3a}$ each independently represents an optionally halogenated C1-C6 alkyl group or $R^6$, $R^1$, $R^4$, $R^5$, $R^6$, $R^7$, $R^G$ and M are as defined above, and $L^4$ is as defined above.

1) Protection
The amino group (-NHR$^1$ group) on the benzene ring of the compound (6-iii) can be protected with a suitable protecting group (e.g., an N-benzylidene group, an N-(1-methyl)ethylidene group, and a benzyloxycarbonyl group) described in Greene's Protective Groups in Organic Synthesis (WILEY) etc., if necessary.
2) The amount of an alkylating agent used is usually 1 mol per mol of the compound (6-iii) or a derivative thereof in which the amino group is protected. Examples of a base used in the reaction include metal carbonates such as potassium carbonate.
3) The compound (6-iv) in which the amino group is protected can be deprotected under known conditions.

The compound (6-iv) can be produced according to the following scheme:

[Chemical Formula 27]

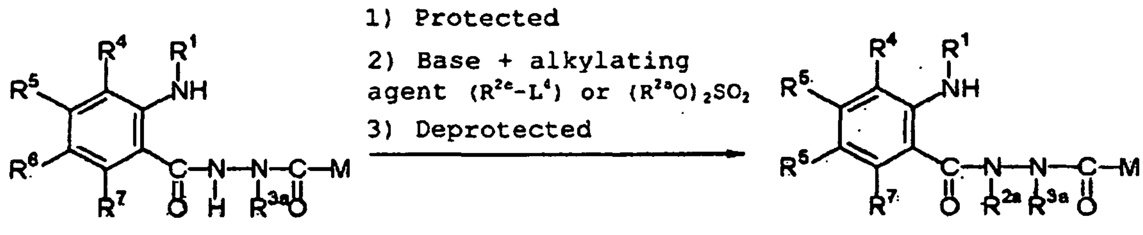


(6-v) (6-iv)

wherein $R^{2a}$ and $R^{3a}$ each independently represents an optionally halogenated C1-C6 alkyl group, or $R^G$, $R^1$, $R^4$, $R^5$, $R^6$, $R^7$, $R^G$ and M are as defined above, and $L^4$ is as defined above.

1) Protection
The amino group (-NHR[1] group) on the benzene ring of the compound (6-v) can be protected with a suitable protecting group (e.g., an N-benzylidene group, an N-(1-methyl)ethylidene group, and a benzyloxycarbonyl group) described in Greene's Protective Groups in Organic Synthesis (WILEY) etc., if necessary.
2) The amount of an alkylating agent used is usually 1 mol per mol of the compound (6-v) or a derivative thereof in which the amino group is protected. Examples of a base used in the reaction include metal carbonates such as potassium carbonate.
3) The compound (6-iv) in which the amino group is protected can be deprotected under known conditions.

The compounds (3) and (13) are known compounds, or can be produced from known compounds according to known processes (see, for example, Organic Functional Group Preparations, 2nd edition, Vol. 1, chapter 12, P. 359-376, Stanley R. Sandler, Wolf Karo, or Organic Functional Group Preparations, 2nd edition, Vol. 1, chapter 14, P. 434-465, Stanley R. Sandler, Wolf Karo.).
The compound (10) can be produced according to a method, for example, shown in the following scheme:
[Chemical Formula 28]

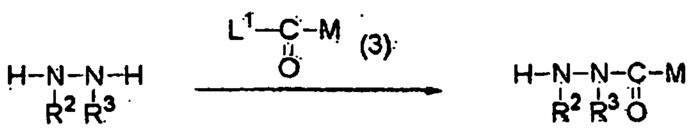


(13) (10)

wherein $L^1$, $R^2$, $R^3$ and M are as defined above.
The compound (15) can be produced according to a method, for example, shown in the following scheme:
[Chemical Formula 29]

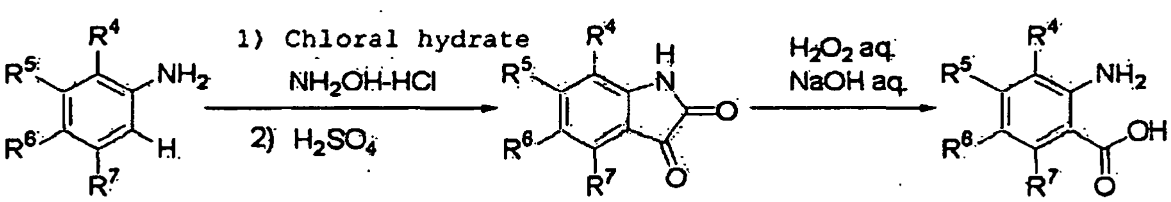


(15)

wherein $R^4$, $R^5$, $R^6$ and $R^7$ are as defined above.

The compounds (14), (16) and (17) can be produced according to a method, for example, shown in the following scheme:

[Chemical Formula 30]

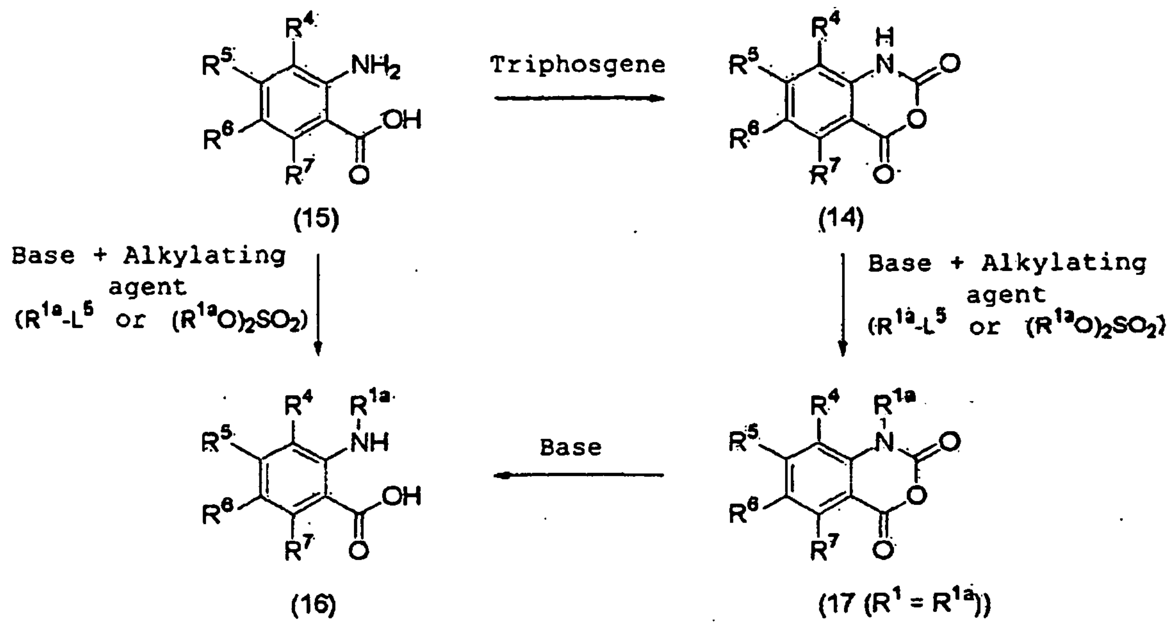


(15) (14) (16) (17 ($R^1$ = $R^{1a}$))

wherein $R^{1a}$, $R^4$, $R^5$, $R^6$ and $R^7$ are as defined above and $L^5$ represents a leaving group (e.g., a halogen atom, a methanesulfonyloxy group, and a p-toluenesulfonyloxy group).

Among the compound (8), a compound represented by the formula (8-i):

[Chemical Formula 31]

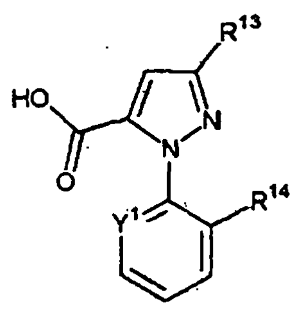

(8-i)

wherein $R^{13}$, $R^{14}$ and $Y^1$ are as defined above, can be produced according to a process, for example, shown in the following scheme:

[Chemical Formula 32]

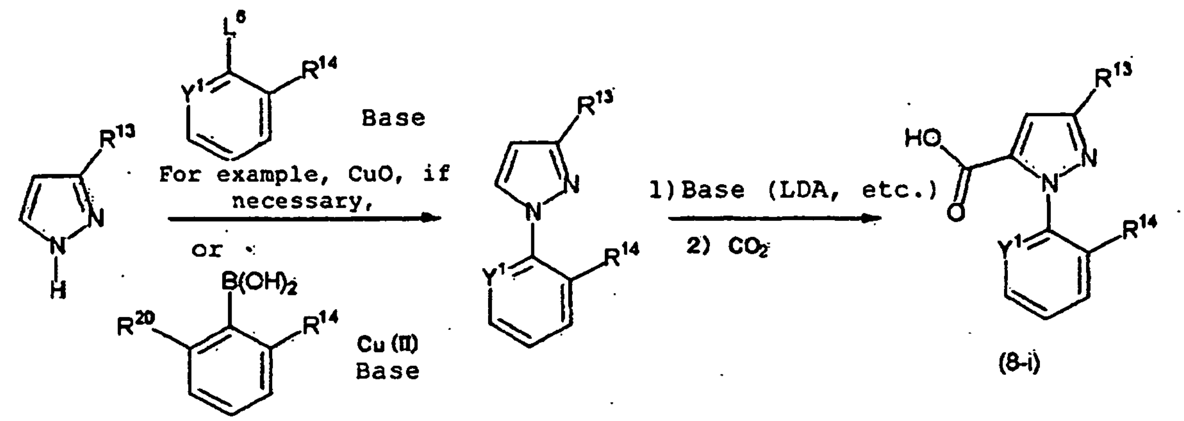


(8-i)

wherein $R^{14}$, $R^{13}$, $R^{20}$ and $Y^1$ are as defined above and $L^6$ represents a leaving group (e.g., a halogen atom, and methylsulfonyl group).

Among the compound (8), compounds represented by the formula (8-ii) and the formula (8-iii):

[Chemical Formula 33]

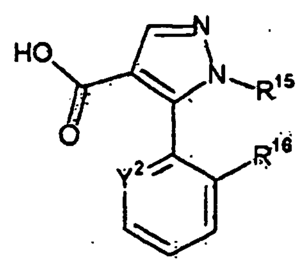

(8-ii)

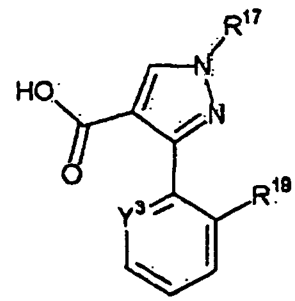

(8-iii)

wherein $R^{15}$, $R^{16}$, $R^{17}$, $R^{18}$, $Y^2$ and $Y^3$ are as defined above, can be produced according to a process, for example, shown in the following scheme:

[Chemical Formula 34]

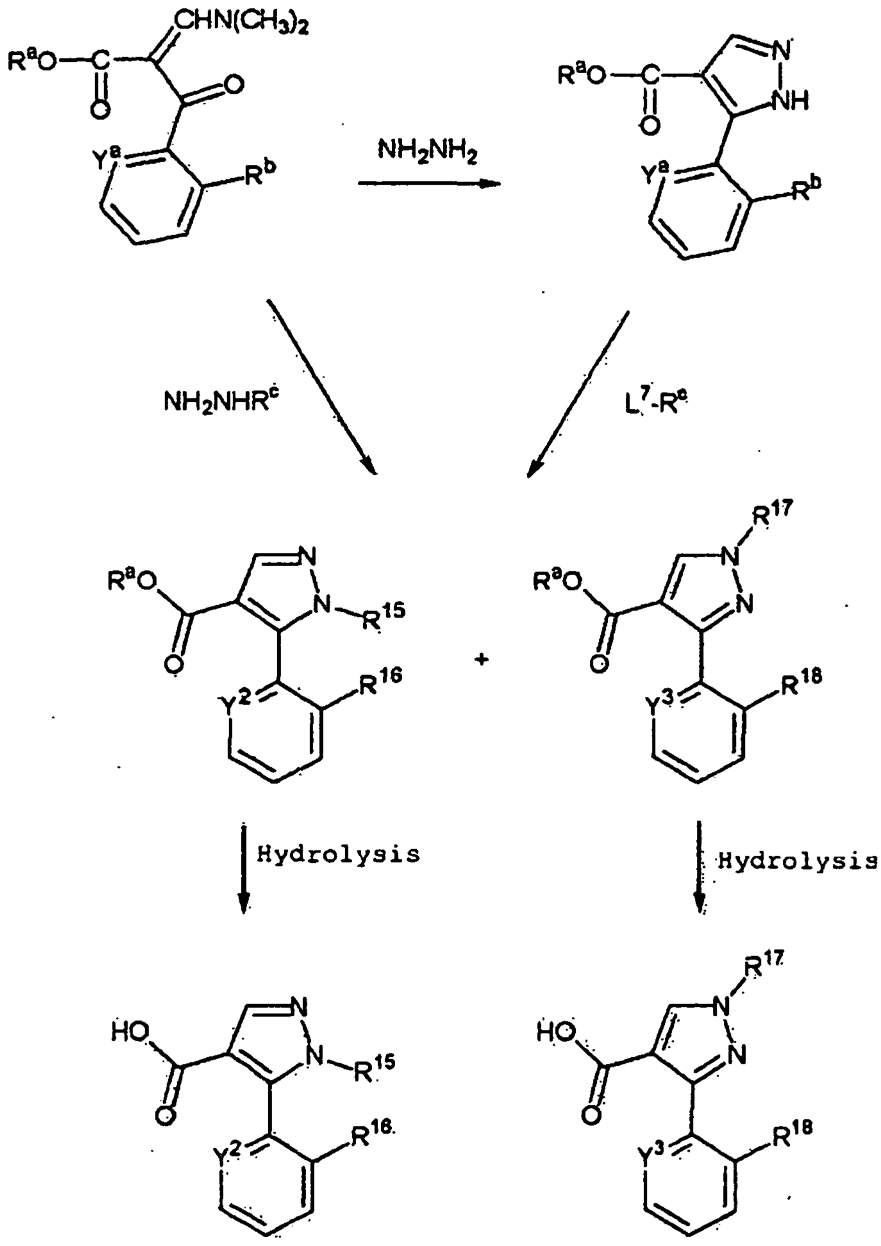


(8-ii) (8-iii)

wherein $R^{15}$, $R^{16}$, $R^{17}$, $R^{18}$, $Y^2$ and $Y^3$ are as defined above, $R^a$ represents a methyl group or an ethyl group, $Y^a$ is as defined in $Y^2$ or $Y^3$, $R^b$ is as defined in $R^{16}$ or $R^{18}$, $R^c$ is as defined in $R^{15}$ or $R^{17}$, and $L^7$ represents a leaving group (e.g., a halogen atom, methanesulfonyloxy group, and p-toluenesulfonyloxy group).

Among the compound (8), a compound represented by the formula (8-iv):

[Chemical Formula 35]

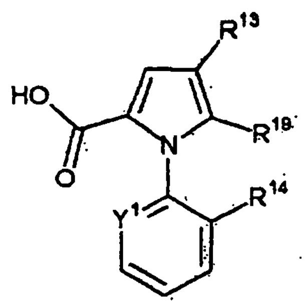

(8-iv)

wherein $R^{13}$, $R^{14}$, $R^{19}$ and $Y^1$ are as defined above, can be produced according to a process, for example, shown in the following scheme:

[Chemical Formula 36]

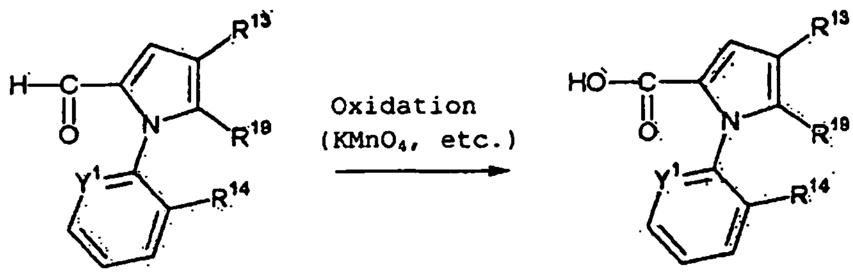


(4-i) (8-iv)

wherein $R^{13}$, $R^{14}$, $R^{19}$ and $Y^1$ are as defined above.

Among the compound (4), a compound (4-i) can be produced according to a process, for example, shown in the following scheme:

[Chemical Formula 37]

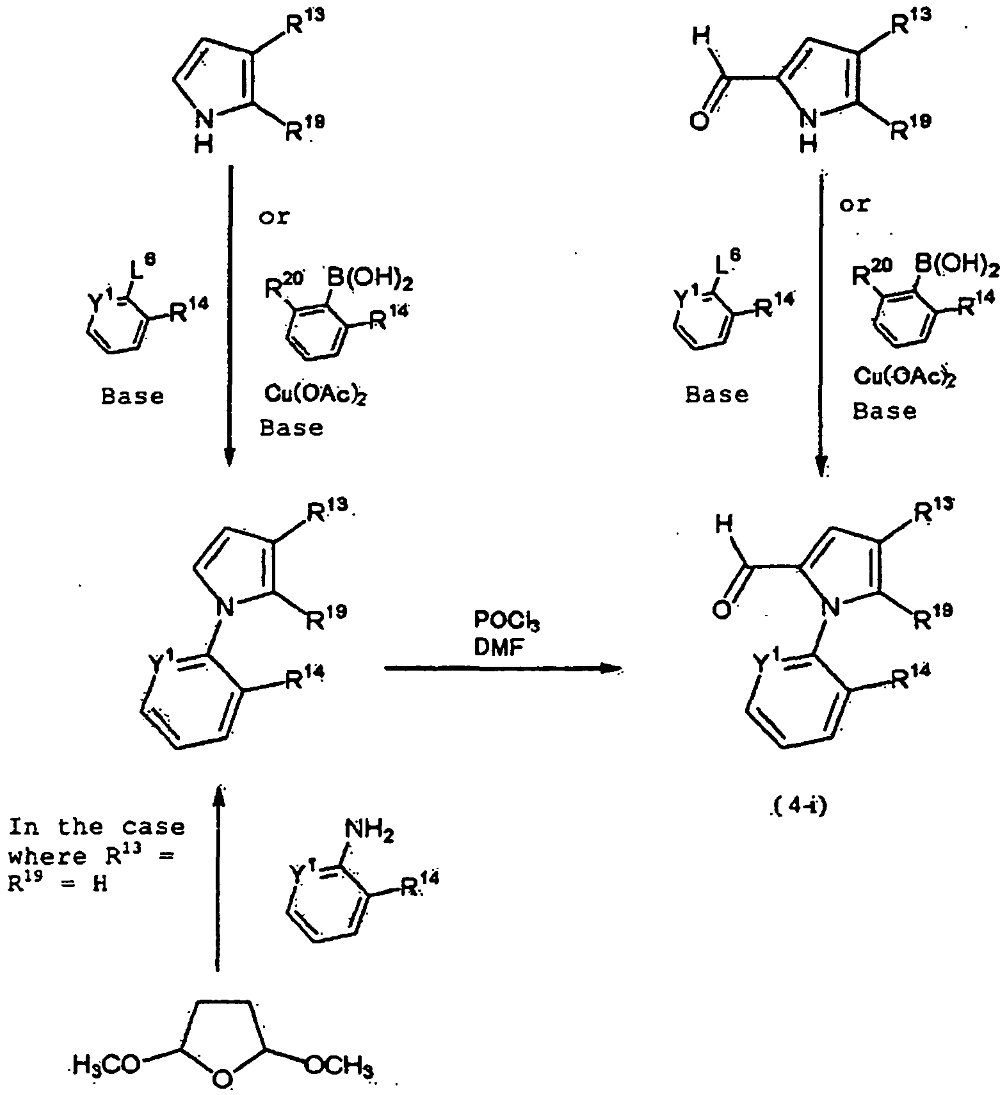


(4-i)

wherein $R^{13}$, $R^{14}$, $R^{19}$, $R^{20}$, $Y^1$ and $L^6$ are as defined above.

Among the compound (4), compounds represented by the formula (4-ii), the formula (4-iii) and the formula (4-iv):

[Chemical Formula 38]

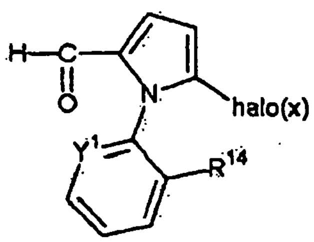

(4-ii)

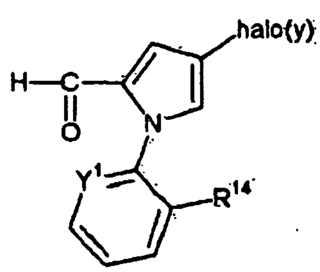

(4-iii)

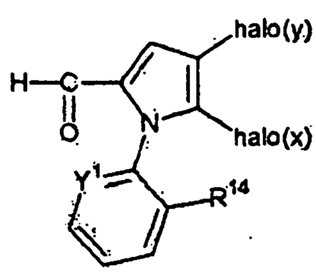

(4-iv)

wherein $R^{14}$ and $Y^1$ are as defined above, and each of halo(x) and halo(y) independently represents a halogen atom can be produced according to a process, for example, shown in the following scheme:

[Chemical Formula 39]

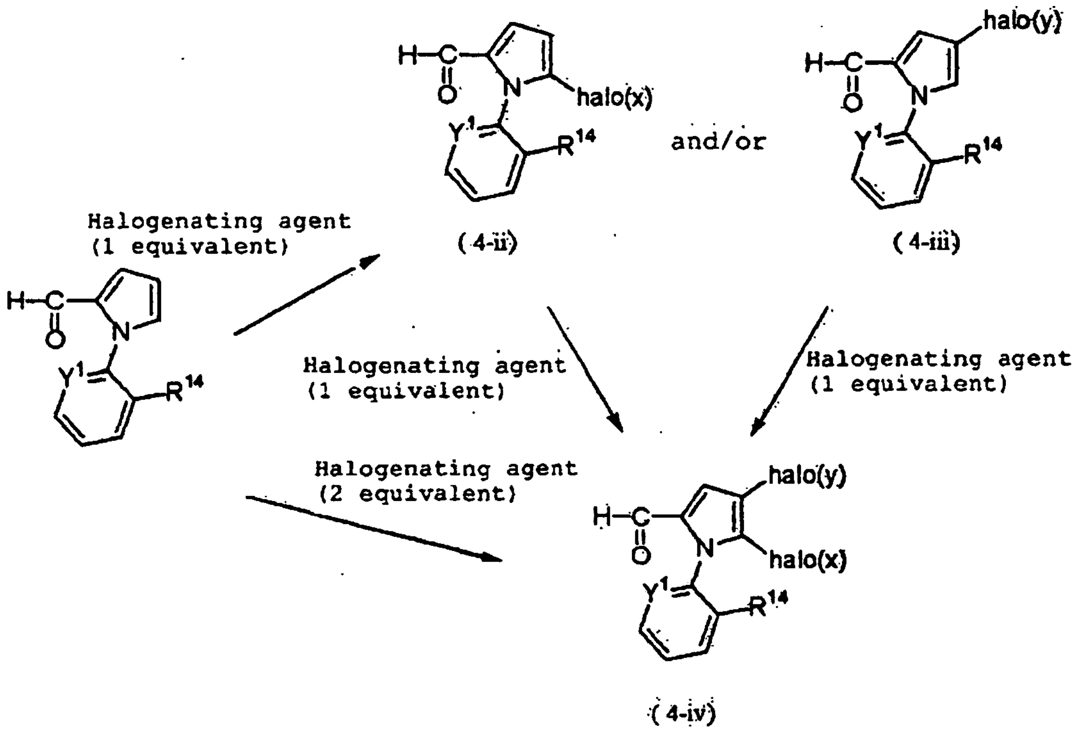


wherein $R^{14}$, $Y^1$, halo(x) and halo(y) are as defined above.

Among the compound (4), a compound represented by the formula (4-v):

[Chemical Formula 40]

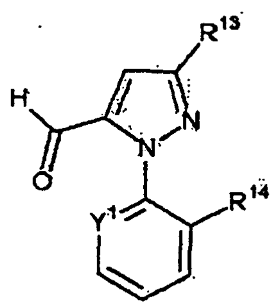

(X-v)

wherein $R^{13}$, $R^{14}$ and $Y^1$ are as defined above, can be produced according to a process, for example, shown in the following scheme:

[Chemical Formula 41]

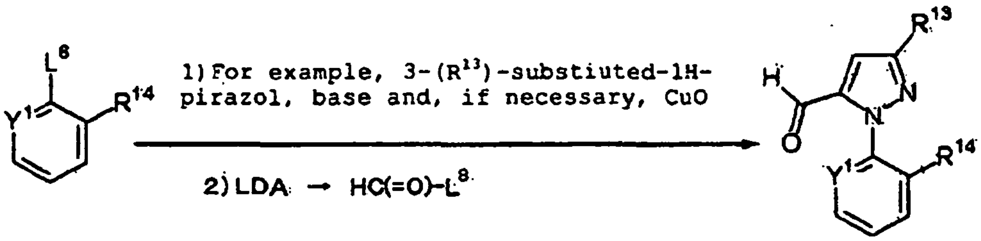


(4-v)

wherein $L^6$ represents a leaving group (e.g., a halogen atom, and methylsulfonyl group), $L^8$ represents a leaving group (e.g., a methoxy group, an ethoxy group, and an N,N-dimethylamino group) and $R^{13}$, $R^{14}$ and $Y^1$ are as defined above.

The compound (7) can be produced according to the following scheme:

[Chemical Formula 42]

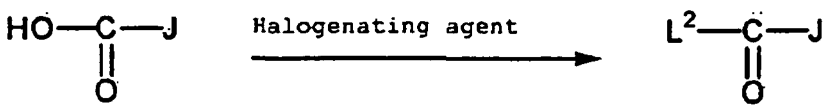


(8) (7)

wherein $L^2$ and J are as defined above.

The compounds obtained by the processes described above can be isolated and purified by a conventional method such as grinding, pulverization, recrystallization, column chromatography, high performance column chromatography (HPLC), medium pressure preparative HPLC, desalting resin column chromatography, or re-precipitation.

The present compound may be isolated in the form of, for example, a salt (a salt obtained by reacting the present compound with an acid or a base), or a solvate (e.g., a hydrate) according to conditions, and the compounds in these forms are also included in the present invention.

The present compound may exist as a tautomer, and the tautomer is also included in the present compound.

Specific examples of the present compound are shown below.

A compound represented by the formula (A-1):

[Chemical Formula 43]

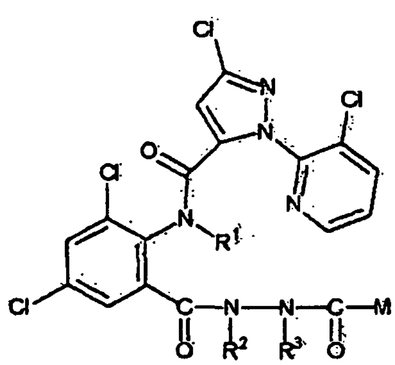

(A-1)

$R^1$, $R^2$, $R^3$ and M in the formula represent the combinations described in Table 1 to Table 68.

Table 1

| $R^1$ | $R^2$ | $R^3$ | M |
|---|---|---|---|
| H | $CH_2CH{=}CH_2$ | H | H |
| H | $CH_2C{\equiv}CH$ | H | H |
| H | cyclopropyl | H | H |
| H | $CH_2$(cyclopropyl) | H | H |
| H | $CH(CH_2)$ (cyclopropyl) | H | H |
| H | $CH_2CN$ | H | H |
| H | $CH(CH_2)CN$ | H | H |
| H | $CH_2OCH_2$ | H | H |
| H | $CH_2OCH_2CH_3$ | H | H |
| H | $CH(CH_3)OCH_3$ | H | H |
| H | $CH_2CH_2OCH_3$ | H | H |
| H | $CH_2CH_2OCH_2$ | H | H |
| H | $CH_2CH_2OCH(CH_3)_2$ | H | H |
| H | $CH_2CH_2OC(CH_3)_2$ | H | H |
| H | $CH_2C(=O)OCH_3$ | H | H |
| H | $CH_2C(=O)OCH_2CH_2$ | H | H |
| H | $CH_2C(=O)OCH(CH_3)_2$ | H | H |
| H | $CH_2C(=O)OC(CH_2)_3$ | H | H |
| H | $CH(CH_3)C(=O)OCH_3$ | H | H |
| H | $CH(CH_3)C(=O)OCH_2CH_2$ | H | H |

Table 2

| $R^1$ | $R^2$ | $R^3$ | M |
|---|---|---|---|
| H | $CH_2CH{=}CH_2$ | $CH_3$ | H |
| H | $CH_2C{\equiv}CH$ | $CH_3$ | H |
| H | cyclopropyl | $CH_3$ | H |
| H | $CH_2$(cyclopropyl) | $CH_3$ | H |
| H | $CH(CH_3)$ (cyclopropyl) | $CH_3$ | H |
| H | $CH_2CN$ | $CH_3$ | H |
| H | $CH(CH_3)CN$ | $CH_3$ | H |
| H | $CH_2OCH_3$ | $CH_3$ | H |
| H | $CH_2OCH_2CH_3$ | $CH_2$ | H |
| H | $CH(CH_3)OCH_3$ | $CH_3$ | H |
| H | $CH_2CH_2OCH_3$ | $CH_3$ | H |
| H | $CH_2CH_2OCH_2CH_3$ | $CH_3$ | H |
| H | $CH_2CH_2OCH(CH_3)_2$ | $CH_2$ | H |

(continued)

| $R^1$ | $R^2$ | $R^3$ | M |
|---|---|---|---|
| H | $CH_2CH_2OC(CH_3)_3$ | $CH_3$ | H |
| H | $CH_2C(=O)OCH_3$ | $CH_3$ | H |
| H | $CH_2C(=O)OCH_2CH_3$ | $CH_3$ | H |
| H | $CH_2C(=O)OCH(CH_3)_2$ | $CH_3$ | H |
| H | $CH_2C(=O)OC(CH_3)_3$ | $CH_2$ | H |
| H | $CH(CH_3)C(=O)OCH_3$ | $CH_3$ | H |
| H | $CH(CH)C(=O)OCH_2CH_3$ | $CH_3$ | H |

Table 3

| $R^1$ | $R^2$ | $R^3$ | M |
|---|---|---|---|
| H | $CH_2CH{=}CH_2$ | $CH_2CH_3$ | H |
| H | $CH_2C{\equiv}CH$ | $CH_2CH_3$ | H |
| .H | cyclopropyl | $CH_2CH_3$ | H |
| H | $CH_2$(cyclopropyl) | $CH_2CH_3$ | H |
| H | $CH(CH_2)$ (cyclopropyl) | $CH_2CH_3$ | H |
| H | $CH_2CN$ | $CH_3CH_3$ | H |
| .H | $CH(CH_3)CN$ | $CH_3CH_3$ | H |
| H | $CH_2OCH_2$ | $CH_2CH_2$ | H |
| H. | $CH_2OCH_2CH_2$ | $CH_2CH_3$ | H |
| H | $CH(CH_2)OCH_3$ | $CH_2CH_3$ | H |
| H | $CH_2CH_3OCH_2$ | $CH_2CH_3$, | H |
| H | $CH_2CH_2OCH_2CH_3$ | $CH_2CH_3$ | H |
| H | $CH_2CH_2OCH(CH_3)_2$ | $CH_2CH_3$ | H |
| H | $CH_2CH_2OC(CH_3)_3$ | $CH_2CH_2$ | H |
| H | $CH_2C(=O)OCH_3$ | $CH_2CH_3$ | H |
| H | $CH_2C(=C)OCH_2CH_2$ | $CH_2CH_3$ | H |
| H | $CH_2C(=O)OCH(CH_3)_2$ | $CH_2CH_2$ | H |
| H | $CH_2C(=O)OC(CH_3)_2$ | $CH_2CH_2$ | H |
| H | $CH(CH_2)C(=O)OCH_3$ | $CH_2CH_3$ | H |
| H | $CH(CH_2)C(=O)OCH_2CH_3$ | $CH_2CH_3$ | H |

Table 4

| $R^1$ | $R^2$ | $R^3$ | M |
|---|---|---|---|
| H | H | $CH_2CH{=}CH_2$ | H |
| H | H | $CH_2C{\equiv}CH$ | H |
| H | H | cyclopropyl | H |

(continued)

| $R^1$ | $R^2$ | $R^3$ | M |
|---|---|---|---|
| H | H | $CH_2$ (cyclopropyl) | H |
| H | H | $CH(CH_3)$ (cyclopropyl) | H |
| H | H | $CH_3CN$ | H |
| H | H | $CH(CH_3)$ CN | H |
| H | H | $CH_2OCH_3$ | H |
| H | H | $CH_2OCH_2CH_3$ | H |
| H | H | $CH(CH_3)OCH_2$ | H |
| H | H | $CH_2CH_2OCH_3$ | H |
| H | H | $CH_2CH_2OCH_2CH_2$ | H |
| H | H | $CH_2CH_2OCH(CH_3)_2$ | H |
| H | H | $CH_2CH_2OC(CH_3)_3$ | H |
| H | H | $CH_2C(=O)OCH_3$ | H |
| H | H | $CH_2C(=O)OCH_2CH_3$ | H |
| H | H | $CH_2C(=O)OCH(CH_2)_2$ | H |
| H | H | $CH_2C(=O)OC(CH_3)_3$ | H |
| H | H | $CH(CH_2)C(=O)OCH_3$ | H |
| H | H | $CH(CH_3)C(=O)OCH_2CH_2$ | H |

Table 5

| $R^1$ | $R^2$ | $R^3$ | M |
|---|---|---|---|
| H | $CH_3$ | $CH_2CH=CH_2$ | H |
| H | $CH_3$ | $CH_2C\equiv CH$ | H |
| H | $CH_3$ | cyclopropyl | H |
| H | $CH_3$ | $CH_2$ (cyclopropyl) | H |
| H | $CH_3$ | $CH(CH_3)$(cyclopropyl) | H |
| H | $CH_3$ | $CH_2CH$ | H |
| H | $CH_3$ | $CH(CH_3)CN$ | H |
| H | $CH_2$ | $CH_2OCH_2$ | H |
| H | $CH_3$ | $CH_2OCH_2CH_3$ | H |
| H | $CH_3$ | $CH(CH_3)OCH_3$ | H |
| H | $CH_2$ | $CH_2CHO_2CH_3$ | H |
| H | $CH_3$ | $CH_2CH_2OCH_2CH_3$ | H |
| H | $CH_3$ | $CH_2CH_2OCH(CH_3)_2$ | H |
| H | $CH_3$ | $CH_2CH_2OC(CH_2)_3$ | H |
| H | $CH_3$ | $CH_2C(=O)OCH_3$ | H |
| H | $CH_3$ | $CH_2C(=O)OCH_2CH_3$ | H |
| H | $CH_{3.}$ | $CH_2C(=O)OCH(CH_3)_2$ | H |

(continued)

| $R^1$ | $R^2$ | $R^3$ | M |
|---|---|---|---|
| H | $CH_3$ | $CH_2C(=O)OC(CH_3)_2$ | H |
| H | $CH_3$ | $CH(CH_3)C(=O)OCH_2$ | H |
| H | $CH_3$ | $CH(CH_3)C(=O)OCH_2CH_3$ | H |

Table 6

| $R^1$ | $R^2$ | $R^3$ | M |
|---|---|---|---|
| H | $CH_2CH_3$ | $CH_2CH=CH_2$ | H |
| H | $CH_2CH_3$ | $CH_2C\equiv CH$ | H |
| H | $CH_2CH_3$ | cyclopropyl | H |
| H | $CH_2CH_3$ | $CH_2$ (cyclopropyl) | H |
| H | $CH_2CH_3$ | $CH(CH_3)$ (cyclopropyl) | H |
| H | $CH_2CH_3$ | $CH_2CN$ | H |
| H | $CH_2CH_3$ | $CH(CH_3)CN$ | H |
| H | $CH_2CH_3$ | $CH_2OCH_3$ | H |
| H | $CH_2CH_3$ | $CH_2OCH_2CH_3$ | H |
| H | $CH_2CH_3$ | $CH(CH_3)OCH_3$ | H |
| H | $CH_2CH_3$ | $CH_2CH_2OCH_3$ | H |
| H | $CH_2CH_3$ | $CH_2CH_2OCH2CH_3$ | H |
| H | $CH_2CH_3$ | $CH_2CH_2OCH(CH_2)_2$ | H |
| H | $CH_2CH_2$ | $CH_2CH_2OC(CH_2)_3$ | H |
| H | $CH_2CH_3$ | $CH_2C(=O)OCH_3$ | H |
| H | $CH_2CH_2$ | $CH_2C(=O)OCH_2CH_3$ | H |
| H | $CH_2CH_3$ | $CH_2C(=O)OCH(CH_3)_3$ | H |
| H. | $CH_2CH_3$ | $CH_2C(=O)OC(CH_3)_3$ | H |
| H | $CH_2CH_3$ | $CH(CH_3)C(=O)OCH_3$ | H |
| H | $CH_2CH_3$ | $CH(CH_2)C(=O)OCH_2CH_3$ | H |

Table 7

| $R^1$ | $R^2$ | $R^3$ | M |
|---|---|---|---|
| H | $CH_2CH=CH_2$ | $CH_2CH=CH_2$ | H |
| H | $CH_2C\equiv CH$ | $CH_2C\equiv CH$ | H |
| H | cyclopropyl | cyclopropyl | H |
| H | $CH_2$ (cyclopropyl) | $CH_2$ (cyclopropyl) | H |
| H | $CH(CH_2)$ (cyclopropyl) | $CH(CH_3)$(cyclopropyl) | H |
| H | $CH_2CN$ | $CH_2CN$ | H |
| H | $CH(CH_3)CN$ | $CH(CH_3)CN$ | H |

(continued)

| $R^1$ | $R^2$ | $R^3$ | M |
|---|---|---|---|
| H | $CH_2OCH_3$ | $CH_2OCH_3$ | H |
| H | $CH_2OCH_2CH_3$ | $CH_2OCH_2CH_3$ | H |
| H | $CH(CH_3)OCH_3$ | $CH(CH_3)OCH_3$ | H |
| H | $CH_2CHOCH_3$ | $CH_2CH_2OCH_3$ | H |
| H | $CH_2CH_2OCH_2CH_3$ | $CH_2CH_2OCH_2CH_3$ | H |
| H | $CH_2CH_2OCH(CH_3)_2$ | $CH_2CH_2OCH(CH_3)_2$ | H |
| H | $CH_2CH_2OC(CH_2)_3$ | $CH_2CH_2O(CH_2)_3$ | H |
| H | $CH_2C(=O)OCH_3$ | $CH_2C(=O)\text{-}OCH_3$ | H |
| H | $CH_2C(=O)OCH_2CH_3$ | $CH_2C(=O)OCH_2CH_3$ | H |
| H | $CH_2C(=O)OCH(CH_3)_2$ | $CH_2C(=O)OCH(CH_3)_2$ | H |
| H | $CH_2C(=O)OC(CH_3)_2$ | $CH_3C(=O)OC(CH_3)_3$ | H |
| H | $CH(CH_3)C(=O)OCH_3$ | $CH(CH_3)C(=O)OCH_3$ | H |
| H | $CH(CH_3)C(=O)OCH_2CH_3$ | $CH(CH_3)C(=O)OCH_2CH_2$ | H |

Table 8

| $R^1$ | $R^2$ | $R^3$ | M |
|---|---|---|---|
| H | $CH_2CH=CH_2$. | H | $OCH_3$ |
| H | $CH_2C\equiv CH$ | H | $OCH_3$ |
| H | cyclopropyl | H | $OCH_3$ |
| H | $CH_2$ (cyclopropyl) | H | $OCH_3$ |
| H | $CH(CH_3)$ (cyclopropyl) | H | $OCH_3$ |
| H | $CH_2CN$ | H | $OCH_2$ |
| H | $CH(CH_3)CN$ | H | $OCH_2$ |
| H | $CH_2OCH_2$ | H | $OCH_3$ |
| H | $CH_2OCH_2CH_3$ | H | $OCH_3$ |
| H | $CH(CH_3)OCH_3$ | H | $OCH_3$ |
| H | $CH_2CH_2OCH_3$ | H | $OCH_3$ |
| H | $CH_2CH_2OCH_2CH_3$ | H | $OCH_3$ |
| H | $CH_2CH_2OCH(CH_3)_2$ | H | $OCH_3$ |
| H | $CH_2CH_2OC(CH_3)_3$ | H | $OCH_3$ |
| H | $CH_2C(=O)OCH_3$ | H | $OCH_3$ |
| H | $CH=C(=O)OCH_2CH_3$ | H | $OCH_3$ |
| H | $CH_2C(=O)OCH(CH_3)_2$ | H | $OCH_2$ |
| H | $CH_2C(=O)OC(CH_3)_2$ | H | $OCH_3$ |
| H | $CH(CH_3)CH(=O)OCH,$ | H | $OCH_3$ |
| H | $CH(CH_3)C(=O)OCH_2CH_3$ | H | $OCH_2$ |

Table 9

| $R^1$ | $R^2$ | $R^3$ | M |
|---|---|---|---|
| H | $CH_2CH{=}CH_2$ | $CH_3$ | $OCR_3$ |
| H | $CH_2C{\equiv}CH$ | $CH_2$ | $OCH_3$ |
| H | cyclopropyl | $CH_2$ | $OCH_3$ |
| H | $CH_2$ (cyclopropyl) | $CH_3$ | $OCH_2$ |
| H | $CH(CH_3)$ (cyclopropyl) | $CH_3$ | $OCH_3$ |
| H | $CH_2CN$ | $CH_3$ | $OCH_3$ |
| H | $CH(CH_2)CN$ | $CH_3$ | $OCH_2$ |
| H | $CH_2OCH_2$ | $CH_2$ | $OCH_3$ |
| H | $CH_2OCH_2CH_2$ | $CH_3$ | $OCH_3$ |
| H | $CH(CH_2)OCH_3$ | $CH_2$ | $OCH_3$ |
| H | $CH_2CH_2OCH_2$ | $CH_3$ | $-OCH_3$ |
| H | $CH_2CH_2OH_2CH_2$ | $CH_3$ | $OCH_2$ |
| H | $CH_2CH_2OCH(CH_3)_2$ | $CH_3$ | $OCH_3$ |
| H | $CH_2CH_2OC\ (CH_3)_2$ | $CH_3$ | $OCH_3$ |
| H | $CH_2C(=O)OCH_3$ | $CH_3$ | $OCH_3$ |
| H | $CH_2C(=O)OCH_2CH_3$ | $CH_3$ | $OCH_3$ |
| H | $CH_2C(=O)OCH(CH_3)_2$ | $CH_3$ | $OCH_3$ |
| H | $CH_2C(=O)OC(CH_3)_3$ | $CH_3$ | $OCH_3$ |
| H | $CH(CH_3)C(=O)OCH_3$ | $CH_2$ | $OCH_3$ |
| H | $CH(CH_2)C(=O)OCH_2CH_3$ | $CH_3$ | $OCH_3$ |

Table 10

| $R^1$ | $R^2$ | $R^3$ | M |
|---|---|---|---|
| H | $CH_2CH{=}CH_2$ | $CH_2CH_3$ | $OCH_3$ |
| H | $CH_2C{\equiv}CH$ | $CH_2CH_2$ | $OCH_2$ |
| H | cyclopropyl | $CH_2CH_3$ | $OCH_3$ |
| H | $CH_2$ (cyclopropyl) | $CH_2CH_3$ | $OCH_3$ |
| H | $CH(CH_3)$ (cyclopropyl) | $CH_2CH_3$ | $OCH_3$ |
| H | $CH_2CN$ | $CH_2CH_3$ | $OCH_3$ |
| H | $CH(CH_3)CN$ | $CH_2CH_3$ | $OCH_3$ |
| H | $CH_2OCH_3$ | $CH_2CH_3$ | $OCH_3$ |
| H | $CH_2OCH_2CH_3$ | $CH_2CH_3$ | CH3 |
| H | $CH(CH_3)OCH_2$ | $CH_2H_3$ | $OCH_3$ |
| H | $CH_2H_2OCH_3$ | $CH_2CH_3$ | $OCH_3$ |
| H | $CH_2CH_2OCH_2CH_3$ | $CH_2CH_2$ | $OCH_3$ |

(continued)

| $R^1$ | $R^2$ | $R^3$ | M |
|---|---|---|---|
| H | $CH_2CH_2OCH(CH_3)_2$ | $CH_2CH_3$ | $OCH_3$ |
| H | $CH_2CH_2OC(CH_2)_3$ | $CH_2CH_3$ | $OCH_3$ |
| H | $CH_2C(=O)OCH_3$ | $CH_2CH_3$ | $OCH_3$ |
| H | $CH_2C(=O)OCH_2CH_3$ | $CH_2CH_3$ | $-OCH_3$ |
| H | $CH_2C(=O)OCH(CH_3)_2$ | $CH_2CH_3$ | $OCH_3$ |
| H | $CH_2C(=O)OC(CH_3)_3$ | $CH_2CH_3$ | $OCH_3$ |
| H | $CH(CH_3)C(=O)OCH_3$ | $CH_2CH_3$ | $OCH_3$ |
| H | $CH(CH_3)C(=O)OCH_2CH_2$ | $CH_2CH_3$ | $OCH_3$ |

Table 11

| $R^1$ | $R^2$ | $R^3$ | M |
|---|---|---|---|
| H | H | $CH_2CH=CH_2$ | $OCH_3$ |
| H | H | $CH_2C\equiv CH$ | $OCH_3$ |
| H | H | cyclopropyl | $OCH_3$ |
| H | H | $CH_2$ (cyclopropyl) | $OCH_3$ |
| H | H | $CH(CH_3)$ (cyclopropyl) | $OCH_3$ |
| H | H | $CH_2CN$ | $OCH_3$ |
| H | H | $CH(CH_3)CN$ | $OCH_3$ |
| H | H | $CH_2OCH_3$ | $OCH_3$ |
| H | H | $CH_2OCH3CH_3$ | $OCH_3$ |
| H | H | $CH(CH_2)OCH_2$ | $OCH_2$ |
| H | H | $CH_2CH_2OCH_3$ | $OCH_3$ |
| H | H | $CH_2OH_2OCH_2CH_3$ | $OCH_3$ |
| H | H | $CH_2CH_2OCH(CH_3)_2$ | $OCH_3$ |
| H | H | $CH_2CH_2OC(CH_3)_3$ | $OCH_3$ |
| H | H | $CH_2C(=O)OCH_3$ | $OCH_3$ |
| H | H | $CH_2C(=O)OCH_2CH_3$ | $OCH_3$ |
| H | H | $CH_2C(=O)OCH(CH_2)_2$ | $OCH_3$ |
| H | H | $CHC(=O)OC(CH_3)_3$ | $OCH_3$ |
| H | H | $CH(CH_3)C(=O)OCH_3$ | $OCH_3$ |
| H | H | $CH(CH_3)C(=O)OCH_2CH_3$ | $OCH_3$ |

Table 12

| $R^1$ | $R^2$ | $R^3$ | M |
|---|---|---|---|
| H | $CH_3$ | $CH_2CH=CH_2$ | $OCH_3$ |
| H | $CH_3$ | $CH_2C\equiv CH$ | $OCH_3$ |

(continued)

| $R^1$ | $R^2$ | $R^3$ | M |
|---|---|---|---|
| H | $CH_3$ | cyclopropyl | $OCH_2$ |
| H | $CH_3$ | $CH_2$ (cyclopropyl) | $OCH_3$ |
| H | $CH_3$ | $CH(CH_2)$ (cyclopropyl) | $OCH_3$ |
| H | $CH_3$ | $CH_2CN$ | $OCH_3$ |
| H | $CH_3$ | $CH(CH_3)CN$ | $OCH_3$ |
| H | $CH_3$ | $CH_2OCH_3$ | $OCH_3$ |
| H | $CH_3$ | $CH_2OCH_2CH_3$ | $OCH_3$ |
| H | $CH_3$ | $CH(CH_3)OCH_3$ | $OCH_3$ |
| H | $CH_3$ | $CH_2CH_2OCH_3$ | $OCH_3$ |
| H | $CH_3$ | $CH_2CH_2OCH_2CH_3$ | $OCH_3$ |
| H | $CH_3$ | $CH_2CH_2OCH(CH_3)_2$ | $OCH_3$ |
| H | $CH_3$ | $CH_2OCOC(CH_3)_3$ | $OCH_3$ |
| H | $CH_3$ | $CH_2C(=O)OCH_3$ | $OCH_3$ |
| H | $CH_3$ | $CH_2C(=O)OCH_2CH_3$ | $OCH_3$ |
| H | $CH_3$ | $CH_2C(=O)OCH(CH_3)_2$ | $OCH_3$ |
| H | $CH_3$ | $CH_2C(=O)OC(CH_2)_3$ | $OCH_3$ |
| H | $CH_3$ | $CH(CH_3)C(=O)OCH_3$ | $OCH_3$ |
| H | $CH_3$ | $CH(CH_3)C(=O)OCH_2CH_3$ | $OCH_3$ |

Table 13

| $R^1$ | $R^2$ | $R^3$ | M |
|---|---|---|---|
| H | $CH_2CH_3$ | $CH_2CH=CH_3$ | $OCH_3$ |
| H | $CH_2CH_3$ | $CH_2C\equiv CH$ | $OCH_3$ |
| H | $CH_2CH_3$ | cyclopropyl | $OCH_3$ |
| H | $CH_2CH_3$ | $CH_2$(cyclopropyl) | $OCH_3$ |
| H | $CH_2H_3$ | $CH(CH_3)$(cyclopropyl) | $OCH_3$ |
| H | $CH_2CH_3$ | $CH_2CN$ | $OCH_3$ |
| H | $CH_2CH_3$ | $CH(CH_3)CN$ | $OCH_3$ |
| H | $CH_2CH_3$ | $CH_2OCH_3$ | $OCH_3$ |
| H | $CH_2CH_3$ | $CH_2OCH_2CH_3$ | $OCH_3$ |
| H | $CH_2CH_3$ | $CH(CH_2)OCH_3$ | $OCH_3$ |
| H | $CH_2CH_3$ | $CH_2CH_3OCH_3$ | $OCH_3$ |
| H | $CH_2CH_3$ | $CH_2CH_2OCH_2CH_3$ | $OCH_3$ |
| H | $CH_2CH_3$ | $CH_2CH_2OC(CH_3)_2$ | $OCH_3$ |
| H | $CH_2CH_3$ | $CH_2CH_2OC(CH_3)_3$ | $OCH_3$ |
| H | $CH_2CH_3$ | $CH_2C(=O)OCH_3$ | $OCH_3$ |
| H | $CH_2CH_3$ | $CH_2C(=O)OCH_2CH_3$ | $OCH_3$ |

(continued)

| $R^1$ | $R^2$ | $R^3$ | M |
|---|---|---|---|
| H | $CH_2CH_3$ | $CH_2C(=O)OCH(CH_3)_2$ | $OCH_3$ |
| H | $CH_2CH_3$ | $CH_2C(=O)OC(CH_3)_3$ | $OCH_3$ |
| H | $CH_2CH_3$ | $CH(CH_3)C(=O)OCH_3$ | $OCH_3$ |
| H | $CH_2CH_3$ | $CH(CH_3)C(=O)OCH_2CH_3$ | $OCH_3$ |

Table 14

| $R^1$ | $R^2$ | $R^3$ | M |
|---|---|---|---|
| H | $CH_2CH=CH_3$ | $CH_2CH=CH_2$ | $OCH_3$ |
| H | $CH_2C{\equiv}CH$ | $CH_2C{\equiv}CH$ | $OCH_3$ |
| H | cyclopropyl | cyclopropyl | $OCH_3$ |
| H | $CH_2$ (cyclopropyl) | $CH_2$(cyclopropyl) | $OCH_3$ |
| H | $CH(CH_2)$ (cyclopropyl) | $CH(CH_3)$ (cyclopropyl) | $OCH_3$ |
| H | $CH_2CN$ | $CH_2CN$ | $OCH_3$ |
| H | $CH(CH_3)CN$ | $CH(CH_3)CN$ | $OCH_3$ |
| H | $CH_2OCH_3$ | $CH_2OCH_3$ | $OCH_3$ |
| H | $CH_2OCH_2CH_3$ | $CH_2OCH_2CH_3$ | $OCH_3$ |
| H | $CH(CH_3)OCH_3$ | $CH(CH_3)OCH_3$ | $OCH_3$ |
| H | $CH_2CH_2OCH_3$ | $CH_2CH_2OCH_3$ | $OCH_3$ |
| H | $CH_2CH_2OCH_2CH_3$ | $CH_2CH_2OCH_2CH_3$ | $OCH_3$ |
| H | $CH_2CH_2OCH(CH_3)_2$ | $CH_2CH_2OCH(CH_3)_2$ | $OCH_3$ |
| H | $CH_2CH_2OC(CH_3)_3$ | $CH_2CH_2OC(CH_3)_3$ | $OCH_3$ |
| H | $CH_2C(=O)OCH_3$ | $CH_2C(=O)OCH_3$ | $OCH_3$ |
| H | $CH_2C(=O)OCH_2CH_3$ | $CH_2C(=O)OCH_2CH_3$ | $OCH_3$ |
| H | $CH_2C(=O)OCH(CH_3)_2$ | $CH_2C(=O)OCH(CH_3)_2$ | $OCH_3$ |
| H | $CH_2C(=O)OC(CH_3)_2$ | $CH_2C(=O)OC(CH_3)_2$ | $OCH_3$ |
| H | $CH(CH_2)C(=O)OCH_3$ | $CH(CH_3)C(=O)OCH_3$ | $OCH_3$ |
| H | $CH(CH_3)C(=O)OCH_2CH_3$ | $CH(CH_3)C(=O)OCH_2CH_3$ | $OCH_3$ |

Table 15

| $R^1$ | $R^2$ | $R^3$ | M |
|---|---|---|---|
| H | $CH_2CH=CH_2$ | H | $N(CH_3)_2$ |
| H | $CH_2C{\equiv}CH$ | H | $N(CH_3)_2$ |
| H | cyclopropyl | H | $N(CH_3)_2$ |
| H | $CH_2$ (cyclopropyl) | H | $N(CH_3)_2$ |
| H | $CH(CH_3)$ (cyclopropyl) | H | $N(CH_3)_2$ |
| H | $CH_2CN$ | H | $N(CH_3)_2$ |

(continued)

| $R^1$ | $R^2$ | $R^3$ | M |
|---|---|---|---|
| H | $CH(CH_3)CN$ | H | $N(CH_3)_2$ |
| H | $CH_2OCH_3$ | H | $N(CH_3)_2$ |
| H | $CH_2OCH_2CH_3$ | H | $N(CH_3)_2$ |
| H | $CH(CH_3)OCH_3$ | H | $N(CH_3)_2$ |
| H | $CH_2CH_2OCH_3$ | H | $N(CH_3)_2$ |
| H | $CH_2CH_2OCH_2CH_3$ | H | $N(CH_3)_2$ |
| H | $CH_2CH_2OCH(CH_3)_2$ | H | $N(CH_3)_2$ |
| H | $CH_2CH_2OC(CH_3)_3$ | H | $N(CH_3)_2$ |
| H | $CH_2C(=O)OCH_3$ | H | $N(CH_3)_2$ |
| H | $CH_2C(=O)OCH_2CH_3$ | H | $N(CH_2)_2$ |
| H | $CH_2C(=O)OCH(CH_2)_2$ | H | $N(CH_3)_2$ |
| H | $CH_2C(=O)OC(CH_3)_3$ | H | $N(CH_2)_2$ |
| H | $CH(CH_3)C(=O)OCH_3$ | H | $N(CH_3)_2$ |
| H | $CH(CH_2)C(=O)OCH_2CH_2$ | H | $N(CH_3)_2$ |

Table 16

| $R^1$ | $R^2$ | $R^3$ | M |
|---|---|---|---|
| H | $CH_2CH{=}CH_2$ | $CH_3$ | $N(CH_2)_2$ |
| H | $CH_2C{\equiv}CH$ | $CH_3$ | $N(CH_3)_2$ |
| H | cyclopropyl | $CH_3$ | $N(CH_3)_2$ |
| H | $CH_3$(cyclopropyl) | $CH_3$ | $N(CH_3)_2$ |
| H | $CH(CH_3)$ (cyclopropyl) | $CH_3$ | $N(CH_3)_2$ |
| H | $CH_2CN$ | $CH_3$ | $N(CH_3)_2$ |
| H | $CH(CH_3)CN$ | $CH_3$ | $N(CH_3)_2$ |
| H | $CH_2OCH_3$ | $CH_3$ | $N(CH)_2$ |
| H | $CH_2OCH_2CH_3$ | $CH_3$ | $N(CH_3)_2$ |
| H | $CH(CH_3)OCH_3$ | $CH_3$ | $N(CH_3)_2$ |
| H | $CH_2CH_2OCH_3$ | $CH_3$ | $N(CH_3)_2$ |
| H | $CH_2CH_2OCH_2CH_3$ | $CH_3$ | $N(CH_3)_2$ |
| H | $CH_2CH_2OCH(CH_2)_2$ | $CH_3$ | $N(CH_3)_2$ |
| H | $CH_2CH_2OC(CH_3)_2$ | $CH_3$ | $N(CH_3)_2$ |
| H | $CH_2C(=O)OCH_3$ | $CH_3$ | $N(CH_3)_2$ |
| H | $CH_2C(=O)OCH_2CH_3$ | $CH_3$ | $N(CH_3)_2$ |
| H | $CH_2C(=O)OCH(CH_3)_2$ | $CH_3$ | $N(CH_3)_2$ |
| H | $CH_2C(=O)OC(CH_3)_2$ | $CH_2$ | $N(CH_3)_2$ |
| H | $CH(CH_3)C(=O)OCH_3$ | $CH_2$ | $N(CH_3)_2$ |
| H | $CH(CH_3)C(=O)OCH_2CH_3$ | $CH_3$ | $N(CH_3)_2$ |

Table 17

| $R^1$ | $R^2$ | $R^3$ | M |
|---|---|---|---|
| H | $CH_2CH=CH_2$ | $CH_2CH_3$ | $N(CH_3)_2$ |
| H | $CH_2C{\equiv}CH$ | $CH_2CH_3$ | $N(CH_3)_2$ |
| H | cyclopropyl | $CH_2CH_3$ | $N(CH_3)_2$ |
| H | $CH_2$(cyclopropyl) | $CH_2CH_3$ | $N(CH_3)_2$ |
| H | $CH(CH_3)$ (cyclopropyl) | $CH_2CH_3$ | $N(CH_3)_2$ |
| H | $CH_2CN$ | $CH_2CH_3$ | $N(CH_3)_2$ |
| H | $CH(CH_3)CN$ | $CH_2CH_3$ | $N(CH_3)_2$ |
| H | $CH_2OCH_3$ | $CH_2CH_3$ | $N(CH_3)_2$ |
| H | $CH_2OCH_2CH_3$ | $CH_2CH_3$ | $N(CH_3)_2$ |
| H | $CH(CH_3)OCH_3$ | $CH_2CH_2$ | $N(CH_3)_2$ |
| H | $CH_2CH_2OCH_3$ | $CH_2CH_3$ | $N(CH_3)_2$ |
| H | $CH_2CH_2OCH_2CH_3$ | $CH_2CH_3$ | $N(CH_3)_2$ |
| H | $CH_2CH_2OCH(CH_3)_2$ | $CH_2CH_3$ | $N(CH_3)_2$ |
| H | $CH_2CH_2OC(CH_3)_3$ | $CH_2CH_8$ | $N(CH_3)_2$ |
| H | $CH_2C(=O)OCH_3$ | $CH_2CH_3$ | $N(CH_3)_2$ |
| H | $CH_2C(=O)OCH_2CH_3$ | $CH_2CH_3$ | $N(CH_3)_2$ |
| H | $CH_2C(=O)OCH\text{-}(CH_2)_2$ | $CH_2CH_3$ | $N(CH_3)_2$ |
| H | $CH_2C(=O)OC(CH_3)_3$ | $CH_2CH_3$ | $N(CH_3)_2$ |
| H | $CH(CH_3)C(=O)OCH_2$ | $CH_2CH_3$ | $N(CH_3)_2$ |
| H | $CH(CH_3)C(=O)OCH_2CH_3$ | $CH_2CH_3$ | $N(CH_3)_2$ |

Table 18

| $R^1$ | $R^2$ | $R^3$ | M |
|---|---|---|---|
| H | H | $CH_2CH=CH_2$ | $N(CH_3)_2$ |
| H | H | $CH_2C{\equiv}CH$ | $N(CH_3)_2$ |
| H | H | cyclopropyl | $N(CH_3)_2$ |
| H | H | $CH_2$(cyclopropyl) | $N(CH_3)_2$ |
| H | H | $CH(CH_3)$ (cyclopropyl) | $N(CH_3)_2$ |
| H | H | $CH_2CN$ | $N(CH_3)_2$ |
| H | H | $CH(CH_3)CN$ | $N(CH_3)_2$ |
| H | H | $CH_2OCH_3$ | $N(CH_3)_2$ |
| H | H | $CH_2OCH_2CH_3$ | $N(CH_3)_2$ |
| H | H | $CH(CH_3)OCH_3$ | $N(CH_2)_3$ |
| H | H | $CH_2CH_2OCH_3$ | $N(CH_3)_2$ |
| H | H | $CH_2CH_2OCH_2CH_3$ | $N(CH_3)_2$ |

(continued)

| $R^1$ | $R^2$ | $R^3$ | M |
|---|---|---|---|
| H | H | $CH_2CH_2OCH(CH_3)_3$ | $N(CH_3)_2$ |
| H | H | $CH_2CH_2OC(CH_3)_3$ | $N(CH_3)_2$ |
| H | H | $CH_2C(=O)OCH_3$ | $N(CH_3)_2$ |
| H | H | $CH_2C(=O)OCH_2CH_3$ | $N(CH_3)_2$ |
| H | H | $CH_2C(=O)OCH(CH_3)_2$ | $N(CH_2)_2$ |
| H | H | $CH_2C(=O)OC(CH_2)_3$ | $N(CH_3)_2$ |
| H | H | $CH(CH_3)C(=O)OCH_3$ | $N(CH_3)_2$ |
| H | H | $CH(CH_3)C(=O)OCH_2CH_3$ | $N(CH_3)_2$ |

Table 19

| $R^1$ | $R^2$ | $R^3$ | M |
|---|---|---|---|
| H | $CH_3$ | $CH_2CH=CH_2$ | $N(CH_3)_2$ |
| H | $CH_3$ | $CH_2C\equiv CH$ | $N(CH_3)_2$ |
| H | $CH_3$ | cyclopropyl | $N(CH_2)_2$ |
| H | $CH_2$ | $CH_2$ (cyclopropyl) | $N(CH_3)_2$ |
| H | $CH_3$ | $CH(CH_3)$ (cyclopropyl) | $N(CH_3)_2$ |
| H | $CH_3$ | $CH_2CN$ | $N(CH_3)_2$ |
| H | $CH_3$ | $CH(CH_3)CN$ | $N(CH_3)_2$ |
| H | $CH_3$ | $CH_2OCH_3$ | $N(CH_3)_2$ |
| H | $CH_3$ | $CH_2OCH_2CH_3$ | $N(CH_3)_2$ |
| H | $CH_3$ | $CH(CH_3)OCH_3$ | $N(CH_3)_2$ |
| H | $CH_3$ | $CH_2CH_2OCH_3$ | $N(CH_2)_2$ |
| H | $CH_3$ | $CH_3CH_2OCH_2CH_3$ | $N(CH_3)_2$ |
| H | $CH_3$ | $CH_2CH_2OCH(CH_3)_2$ | $N(CH_3)_2$ |
| H | $CH_3$ | $CH_2CH_2OC(CH_3)_3$ | $N(CH_3)_2$ |
| H | $CH_3$ | $CH_2C(=O)OCH_3$ | $N(CH_3)_2$ |
| H | $CH_3$ | $CH_2C(=O)OCH_2CH_3$ | $N(CH_3)_2$ |
| H | $CH_3$ | $CH_2C(=O)OCH(CH_3)_2$ | $N((CH_3)_2$ |
| H | $CH_2$ | $CH_2C(=O)OC(CH_3)_3$ | $N(CH_2)_2$ |
| H | $CH_3$ | $CH(CH_3)C(=O)OCH_3$ | $N(CH_3)_2$ |
| H | $CH_3$ | $CH(CH_3)C(=O)OCH_2CH_3$ | $N(CH_3)_2$ |

Table 20

| $R^1$ | $R^2$ | $R^3$ | M |
|---|---|---|---|
| H | $CH_2CH_3$ | $CH_2CH=CH_2$ | $N(CH_3)_2$ |
| H | $CH_2CH_3$ | $CH_2C\equiv CH$ | $N(CH_3)_2$ |

(continued)

| $R^1$ | $R^2$ | $R^3$ | M |
|---|---|---|---|
| H | $CH_2CH_3$ | cyclopropyl | $N(CH_3)_2$ |
| H | $CH_2CH_3$ | $CH_2$(cyclopropyl) | $N(CH_3)_2$ |
| H | $CH_2CH_3$ | $CH(CH_3)$ (cyclopropyl) | $N(CH_3)_2$ |
| H | $CH_2CH_3$ | $CH_2CN$ | $N(CH_3)_2$ |
| H | $CH_2CH_3$ | $CH(CH_3)CN$ | $N(CH_3)_2$ |
| H | $CH_2CH_3$ | $CH_2OCH_3$ | $N(CH_3)_2$ |
| H | $CH_2CH_3$ | $CH_2OCH_2CH_3$ | $N(CH_3)_2$ |
| H | $CH_2CH_3$ | $CH(CH_3)OCH_3$ | $N(CH_3)_2$ |
| H | $CH_2CH_3$ | $CH_2CH_2OCH_3$ | $N(CH_3)_2$ |
| H | $CH_2CH_3$ | $CH_2CH_2OCH_2CH_3$ | $N(CH_3)_2$ |
| H | $CH_2CH_3$ | $CH_2CH_2OCH(CH_3)_2$ | $N(CH_3)_2$ |
| H | $CH_2CH_3$ | $CH_2CH_2OC(CH_3)_3$ | $N(CH_3)_2$ |
| H | $CH_2CH_3$ | $CH_2C(=O)OCH_3$ | $N(CH_3)_2$ |
| H | $CH_2CH_3$ | $CH_2C(=O)OCH_2CH_2$ | $N(CH_3)_2$ |
| H | $CH_2CH_3$ | $CH_2C(=O)OCH(CH_3)_2$ | $N(CH_3)_2$ |
| H | $CH_2CH_3$ | $CH_2C(=O)OC(CH_3)_3$ | $N(CH_2)_2$ |
| H | $CH_2CH_2$ | $CH(CH_3)C(=O)OCH_3$ | $N(CH_3)_2$ |
| H | $CH_2CH_3$ | $CN(CH_3)C(=O)OCH_2CH_3$ | $N(CH_3)_2$ |

Table 21

| $R^1$ | $R^2$ | $R^3$ | M |
|---|---|---|---|
| H | $CH_2CH=CH_2$ | $CH_2CH=CH_2$ | $N(CH_3)_2$ |
| H | $CH_2C{\equiv}CH$ | $CH_2C{\equiv}CH$ | $N(CH_3)_2$ |
| H | cyclopropyl | cyclopropyl | $N(CH_3)_2$ |
| H | $CH_2$(cyclopropyl) | $CH_2$(cyclopropyl) | $N(CH_3)_2$ |
| H | $CH(CH_3)$ (cyclopropyl) | $CH(CH_3)$ (cyclopropyl) | $N(CH_3)_2$ |
| H | $CH_2CN$ | $CH_2CH$ | $N(CH_3)_2$ |
| H | $CH(CH_3)CN$ | $CH(CH_3)CN$ | $N(CH_3)_2$ |
| H | $CH_2OCH_3$ | $CH_2OCH_3$ | $N(CH_3)_2$ |
| H | $CH_2OCH_2CH_2$ | $CH_2OCH_3CH_3$ | $N(CH_3)_2$ |
| H | $CH(CH_3)OCH_3$ | $CH(CH_3)OCH_3$ | $N(CH_3)_2$ |
| H | $CH_2CH_2OCH_3$ | $CH_2CH_2OCH_3$ | $N(CH_3)_2$ |
| H | $CH_2CH_2OCH_2CH_3$ | $CH_2CH_2OCH_3CH_3$ | $N(CH_2)_2$ |
| H | $CH_2CH_2OCH(CH_3)_2$ | $CH_2CH_2OCH(CH_3)_2$ | $N(CH_3)_2$ |
| H | $CH_2CH_2OC(CH_3)_3$ | $CH_2CH_2OC(CH_3)_3$ | $N(CH_3)_2$ |
| H | $CH_2C(=O)OCH_3$ | $CH_2C(=O)OCH_3$ | $N(CH_3)_2$ |
| H | $CH_2C(=O)OCH_2CH_3$ | $CH_2C(=O)OCH_2CH_3$ | $N(CH_3)_2$ |

(continued)

| $R^1$ | $R^2$ | $R^3$ | M |
|---|---|---|---|
| H | $CH_2C(=O)OCH(CH_2)_2$ | $CH_2C(=O)OCH(CH_2)_2$ | $N(CH_3)_2$ |
| H | $CH_2C(=O)OC(CH_3)_2$ | $CH_2C(=O)OC(CH_2)_3$ | $N(CH_3)_2$ |
| H | $CH(CH_3)C(=O)OCH_3$ | $CH(CH_2)C(=O)OCH_3$ | $N(CH_3)_2$ |
| H | $CH(CH_2)C(=O)OCH_2CH_3$ | $CH(CH_3)C(=O)OCH_2CH_3$ | $N(CH_3)_2$ |

Table 22

| $R^1$ | $R^2$ | $R^3$ | M |
|---|---|---|---|
| $CH_3$ | $CH_2CH=CH_2$ | H | H |
| $CH_3$ | $CH_2C{\equiv}cH$ | H | H |
| $CH_3$ | cyclopropyl | H | H |
| $CH_3$ | $CH_2$(cyclopropyl) | H | H |
| $CH_3$ | $CH(CH_3)$ (cyclopropyl) | H | H |
| $CH_3$ | $CH_2CN$ | H | H |
| $CH_3$ | $CH(CH_3)CN$ | H | H |
| $CH_3$ | $CH_2OCH_3$ | H | H |
| $CH_3$ | $CH_2OCH_2CH_3$ | H | H |
| $CH_3$ | $CH(CH_3)OCH_3$ | H | H |
| $CH_3$ | $CH_2CH_2OCH_3$ | H | H |
| $CH_3$ | $CH_2CH_2OCH_2CH_3$ | H | H |
| $CH_3$ | $CH_2CH_2OCH(CH_3)_2$ | H | H |
| $CH_3$ | $CH_2CH_2OC(CH_3)_3$ | H | H |
| $CH_3$ | $CH_2C(=O)OCH_3$ | H | H |
| $CH_3$ | $CH_2C(=O)OCH_2CH_3$ | H | H |
| $CH_3$ | $CH_2C(=O)OCH(CH_3)_2$ | H | H |
| $CH_3$ | $CH_2C(=O)OC(CH_3)_3$ | H | H |
| $CH_3$ | $CH(CH_3)C(=O)OCH_3$ | H | H |
| $CH_3$ | $CH(CH_3)C(=O)OCH_2CH_3$ | H | H |

Table 23

| $R^1$ | $R^2$ | $R^3$ | M |
|---|---|---|---|
| $CH_3$ | $CH_2CH=CH_2$ | $CH_3$ | H |
| $CH_2$ | $CH_2C{\equiv}CH$ | $CH_3$ | H |
| $CH_3$ | cyclopropyl | $CH_3$ | H |
| $CH_3$ | $CH_2$(cyclopropyl) | $CH_3$ | H |
| $CH_3$ | $CH(CH_3)$(cyclopropyl) | $CH_3$ | H |
| $CH_3$ | $CH_3CN$ | $CH_3$ | H |

(continued)

| $R^1$ | $R^2$ | $R^3$ | M |
|---|---|---|---|
| $CH_3$ | $CH(CH_3)CN$ | $CH_3$ | H |
| $CH_3$ | $CH_2OCH_3$ | $CH_3$ | H |
| $CH_3$ | $CH_2OCH_2CH_3$ | $CH_3$ | H |
| $CH_3$ | $CH(CH_3)OCH_3$ | $CH_3$ | H |
| $CH_3$ | $CH_2CH_2OCH_3$ | $CH_3$ | H |
| $CH_3$ | $CH_2CH_2OCH_2CH_3$ | $CH_3$ | H |
| $CH_3$ | $CH_2CH_2OCH(CH_3)_2$ | $CH_3$ | H |
| $CH_2$ | $CH_2CH_2OC(CH_2)_3$ | $CH_3$ | H |
| $CH_3$ | $CH_2C(=O)OCH_3$ | $CH_3$ | H |
| $CH_3$ | $CH_2C(=O)OCH_2CH_3$ | $CH_3$ | H |
| $CH_3$ | $CH_2C(=O)OCH(CH_3)_2$ | $CH_3$ | H |
| $CH_3$ | $CH_2C(=O)OC(CH_3)_3$ | $CH_2$ | H |
| $CH_3$ | $CH(CH_3)C(=O)OCH_3$ | $CH_3$ | H |
| $CH_3$ | $CH(CH_3)C(=O)OCH_2CH_2$ | $CH_3$ | H |

Table 24

| $R^1$ | $R^2$ | $R^3$ | M |
|---|---|---|---|
| $CH_2$ | $CH_2CH=CH_2$ | $CH_2CH_3$ | H |
| $CH_3$ | $CH_2C\equiv CH$ | $CH_2CH_3$ | H |
| $CH_3$ | cyclopropyl | $CH_2CH_3$ | H |
| $CH_3$ | $CH_2$(cyclopropyl) | $CH_2CH_3$ | H |
| $CH_3$ | $CH(CH_3)$ (cyclopropyl) | $CH_2CH_3$ | H |
| $CH_3$ | $CH_2CN$ | $CH_2CH_3$ | H |
| $CH_2$ | $CH(CH_3)CN$ | $CH_2CH_3$ | H |
| $CH_3$ | $CH_2OCH_3$ | $CH_2CH_3$ | H |
| $CH_3$ | $CH_2OCH_2CH_3$ | $CH_2CH_3$ | H |
| $CH_3$ | $CH(CH_3)OCH_3$ | $CH_2CH_3$ | H |
| $CH_3$ | $CH_2CH_2OCH_3$ | $CH_2CH_2$ | H |
| $CH_3$ | $CH_2CH_2OCH_2CH_3$ | $CH_2CH_2$ | H |
| $CH_3$ | $CH_2CH_2OCH(CH_3)_2$ | $CH_2CH_3$ | H |
| $CH_2$ | $CH_2CH_2OC(CH_2)_3$ | $CH_2CH_2$ | H |
| $CH_3$ | $CH_2C(=O)OCH_3$ | $CH_2CH_3$ | H |
| $CH_3$ | $CH_2C(=O)OCH_2CH_3$ | $CH_2CH_3$ | H |
| $CH_3$ | $CR_2C(=O)OCH(CH_3)_2$ | $CH_2CH_3$ | H |
| $CH_3$ | $CH_2C(=O)OC(CH_2)_3$ | $CH_2CH_3$ | H |
| $CH_3$ | $CH(CH_3)C(=O)OCH_3$ | $CH_2CH_3$ | H |
| $CH_3$ | $CH(CH_3)C(=O)OCH_2CH_3$ | $CH_2CH_3$ | H |

Table 25

| $R^1$ | $R^2$ | $R^3$ | M |
|---|---|---|---|
| $CH_2$ | H | $CH_2CH{=}CH_2$ | H |
| $CH_3$ | H | $CH_3C{\equiv}CH$ | H |
| $CH_2$ | H | cyclopropyl | H |
| $CH_3$ | H | $CH_2$(cyclopropyl) | H |
| $CH_2$ | H | $CH(CH_2)$ (cyclopropyl) | H |
| $CH_3$ | H | $CH_2CN$ | H |
| $CH_3$ | H | $CH(CH_3)CN$ | H |
| $CH_3$ | H | $CH_2OCH_3$ | H |
| $CH_3$ | H | $CH_2OCH_2CH_3$ | H |
| $CH_3$ | H | $CH(CH_3)OCH_3$ | H |
| $CH_3$ | H | $CH_2CH_2OCH_3$ | H |
| $CH_2$ | H | $CH_2CH_2OCH_2CH_3$ | H |
| $CH_2$ | H | $CH_2CH_2OCH(CH3)_2$ | H |
| $CH_3$ | H | $CH_2CH_2OC(CH_3)_3$ | H |
| $CH_3$ | H | $CH_2C(=O)OCH_3$ | H |
| $CH_3$ | H | $CH_2C(=O)OCH_2CH_3$ | H |
| $CH_3$ | H | $CH_2C(=O)OCH(CH_2)_2$ | H |
| $CH_3$ | H | $CH_2C(=O)OC(CH_2)_3$ | H |
| $CH_3$ | H | $CH(CH_3)C(=O)OCH_3$ | H |
| $CH_3$ | H | $CH(CH_3)C(=O)OCH_2CH_3$ | H |

Table 26

| $R^1$ | $R^2$ | $R^3$ | M |
|---|---|---|---|
| $CH_2$ | $CH_2$ | $CH_2CH{=}CH_2$ | H |
| $CH_3$ | $CH_3$ | $CH_2C{\equiv}CH$ | H |
| $CH_3$ | $CH_3$ | cyclopropyl | H |
| $CH_3$ | $CH_3$ | $CH_2$ (cyclopropyl) | H |
| $CH_3$ | $CH_3$ | $CH(CH_3)$ (cyclopropyl) | H |
| $CH_8$ | $CH_3$ | $CH_2CN$ | H |
| $CH_3$ | $CH_3$ | $CH(CH_3)CN$ | H |
| $CH_3$ | $CH_3$ | $CH_2OCH_3$ | H |
| $CH_3$ | $CH_3$ | $CH_2OCH_2CH_3$ | H |
| $CH_3$ | $CH_3$ | $CH(CH_3)OCH_3$ | H |
| $CH_3$ | $CH_3$ | $CH_2CH_2OCH_3$ | H |
| $CH_3$ | $CH_3$ | $CH_2CH_2OCH_2CH_3$ | H |

(continued)

| $R^1$ | $R^2$ | $R^3$ | M |
|---|---|---|---|
| $CH_2$ | $CH_3$ | $CH_2CH_2OCH(CH_3)_2$ | H |
| $CH_3$ | $CH_3$ | $CH_2CH_2OC(CH_2)_3$ | H |
| $CH_3$ | $CH_3$ | $CH_2C(=O)OCH_3$ | H |
| $CH_3$ | $CH_3$ | $CH_2C(=O)OCH_2CH_3$ | H |
| $CH_3$ | $CH_2$ | $CH_2C(=O)OCH(CH_2)_2$ | H |
| $CH_3$ | $CH_3$ | $CH_2C(=O)OC(CH_3)_3$ | H |
| $CH_3$ | $CH_2$ | $CH(CH_2)C(=O)OCH_2$ | H |
| $CH_2$ | $CH_2$ | $CH(CH_2)C(=O)OCH_2CH_3$ | H |

Table 27

| $R^1$ | $R^2$ | $R^3$ | M |
|---|---|---|---|
| $CH_2$ | $CH_2cH_3$ | $CH_2CH=CH_2$ | H |
| $CH_3$ | $CH_2CH_2$ | $CH_2C\equiv CH$ | H |
| $CH_3$ | $CH_2CH_2$ | cyclopropyl | H |
| $CH_3$ | $CH_2CH_3$ | $CH_2$ (cyclopropyl) | H |
| $CH_2$ | $CH_2CH_3$ | $CH(CH_3)$ (cyclopropyl) | H |
| $CH_3$ | $CH_2CH_3$ | $CH_2CN$ | H |
| $CH_3$ | $CH_2CH_3$ | $CH(CH_3)CN$ | H |
| $CH_3$ | $CH_2CH_3$ | $CH_2OCH_2$ | H |
| $CH_3$ | $CH_2CH_3$ | $CH_2OCH_2CH_3$ | H |
| $CH_3$ | $CH_2CH_3$ | $CH(CH_2)OCH_3$ | H |
| $CH_3$ | $CH_2CH_3$ | $CH_2CH_3CH_3$ | H |
| $CH_2$ | $CH_2CH_3$ | $CH_2CH_2OCH_2CH_3$ | H |
| $CH_3$ | $CH_2CH_3$ | $CH_2CH_2OCH(CH_3)_2$ | H |
| $CH_3$ | $CH_2CH_3$ | $CH_2CH_2OC(CH_4)_2$ | H |
| $CH_3$ | $CH_2CH_3$ | $CH_2C(=O)OCH_3$ | H |
| $CH_3$ | $CH_2CH_3$ | $CH_2C(=O)OCH_2CH_3$ | H |
| $CH_3$ | $CH_2CH_3$ | $CH_2C(=O)OCH(CH_2)_2$ | H |
| $CH_3$ | $CH_2CH_3$ | $CH_2C(=O)OC(CH_2)_3$ | H |
| $CH_3$ | $CH_2CH_3$ | $CH(CH_3)C(=O)OCH_2$ | H |
| $CH_3$ | $CH_2CH_3$ | $CH(CH_2)C(=O)OCH_2CH_2$ | H |

Table 28

| $R^1$ | $R^2$ | $R^3$ | M |
|---|---|---|---|
| $CH_3$ | $CH_2CH=CH_2$ | $CH_2CH=CH_2$ | H |
| $CH_3$ | $CH_2C\equiv CH.$ | $CH_2C=CH$ | H. |

(continued)

| $R^1$ | $R^2$ | $R^3$ | M |
|---|---|---|---|
| $CH_2$ | cyclopropyl | cyclopropyl | H |
| $CH_3$ | $CH_2$ (cyclopropyl) | $CH_2$(cyclopropyl) | H |
| $CH_3$ | $CH(CH_2)$(cyclopropyl) | $CH(CH_3)$ (cyclopropyl) | H |
| $CH_2$ | $CH_2CN$ | $CH_2CN$ | H |
| $CH_3$ | $CH(CH_3)CN$ | $CH(CH_3)CN$ | H |
| $CH_3$ | $CH_2OCH_2$ | $CH_2OCH_3$ | H |
| $CH_3$ | $CH_2OCH_2CH_3$ | $CH_2OCH_2CH_3$ | H |
| $CH_3$ | $CH(CH_3)OCH_3$ | $CH(CH_3)OCH_3$ | H |
| $CH_3$ | $CH_2CH_2OCH_3$ | $CH_2CH_2OCH_3$ | H |
| $CH_3$ | $CH_2CH_2OCH_3$ | $CH_2CH_2OCH_2CH_3$ | H |
| $CH_3$ | $CH_2CH_2OCH(CH_3)_2$ | $CH_2CH_2OCH(CH_3)_2$ | H |
| $CH_3$ | $CH_2CH_2O(CH_3)_3$ | $CH_2CH_2OC(CH_3)_3$ | H |
| $CH_3$ | $CH_2C(=O)OCH_2$ | $CH_2C(=O)OCH_2$ | H |
| $CH_3$ | $CH_2C(=O)OCH_2CH_3$ | $CH_2C(=O)OCHCH_3$ | H |
| $CH_3$ | $CH_2C$ $(=O)OCH(CH_2)_2$ | $CH_2C(=O)OCH(CH_3)_2$ | H |
| $CH_3$ | $CH_2C(=O)OC(CH_3)_3$ | $CH_2C(=O)OC(CH_3)_2$ | H |
| $CH_3$ | $CH(CH_3)C(=O)OCH_3$ | $CH(CH_3)C(=O)OCH_2$ | H |
| $CH_3$ | $CH(CH_3)C(=O)OCH_2CH_3$ | $CH(CH_3)C(=O)OCH_2CH_3$ | H |

Table 29

| $R^1$ | $R^2$ | $R^3$ | M |
|---|---|---|---|
| $CH_2$ | $CH_2CH=CH_2$ | H | $OCH_3$ |
| $CH_3$ | $CH_2C\equiv CH$ | H | $OCH_2$ |
| $CH_3$ | cyclopropyl | H | $OCH_2$ |
| $CH_3$ | $CH_2$ (cyclopropyl) | H | $OCH_3$ |
| $CH_3$ | $CH(CH_3)$ (cyclopropyl) | H | $OCH_3$ |
| $CH_3$ | $CH_2CN$ | H | $OCH_3$ |
| $CH_3$ | $CH(CH_3)CN$ | H | $OCH_3$ |
| $CH_2$ | $CH_2OCH_2$ | H | $OCH_2$ |
| $CH_3$ | $CH_2OCH_2CH_2$ | H | $OCH_3$ |
| $CH_3$ | $CH(CH_2)OCH_2$ | H | $OCH_3$ |
| $CH_2$ | $CH_2CH_2OCH_3$ | H | $OCH_3$ |
| $CH_3$ | $CH_2CH_2OCH_2CH_3$ | H | $OCH_2$ |
| $CH_3$ | $CH_2CH_2OCH(CH_2)_2$ | H | $OCH_3$ |
| $CH_3$ | $CH_2CH_2OC(CH_3)_3$ | H | $OCH_2$ |
| $CH_3$ | $CH_2C(=O)0CH_3$ | H | $OCH_3$ |
| $CH_3$ | $CH_2C(=0)OCH_2CH_2$ | H | $OCH_2$ |

(continued)

| $R^1$ | $R^2$ | $R^3$ | M |
|---|---|---|---|
| $CH_3$ | $CH_2C(=O)OCH(CH_3)_2$ | H | $OCH_2$ |
| $CH_3$ | $CH_2C(=O)OC(CH_2)_2$ | H | $OCH_3$ |
| $CH_3$ | $CH(CH_3)C(=O)OCH_3$ | H | $OCH_3$ |
| $CH_3$ | $CH(CH_3)C(=O)OCH_2CH_3$ | H | $OCH_3$ |

Table 30

| $R^1$ | $R^2$ | $R^3$ | M |
|---|---|---|---|
| $CH_3$ | $CH_2CH=CH_3$ | $CH_3$ | $OCH_2$ |
| $CH_3$ | $CH_2C \equiv CH$ | $CH_3$ | $OCH_3$ |
| $CH_3$ | cyclopropyl | $CH_3$ | $OCH_3$ |
| $CH_3$ | $CH_2$ (cyclopropyl) | $CH_3$ | $OCH_3$ |
| $CH_2$ | $CH(CH_3)$ (cyclopropyl) | $CH_3$ | $OCH_2$ |
| $CH_3$ | $CH_2CN$ | $CH_3$ | $OCH_3$ |
| $CH_3$ | $CH(CH_3)CN$ | $CH_3$ | $OCH_2$ |
| $CH_3$ | $CH_2OCH_3$ | $CH_3$ | $OCH_3$ |
| $CH_2$ | $CH_2OCH_2CH_3$ | $CH_3$ | $OCH_3$ |
| $CH_3$ | $CH(CH_2)OCH_3$ | $CH_3$ | $OCH_3$ |
| $CH_3$ | $CH_2CH_2OCH_3$ | $CH_3$ | $OCH_3$ |
| $CH_2$ | $CH_2CH_2OCH_2CH_3$ | $CH_3$ | $OCH_3$ |
| $CH_3$ | $CH_2CH_2OCH(CH_3)_2$ | $CH_3$ | $OCH_3$ |
| $CH_3$ | $CH_2CH_2OC(CH_3)_3$ | $CH_3$ | $OCH_3$ |
| $CH_3$ | $CH_2C(=O)OCH_3$ | $CH_3$ | $OCH_3$ |
| $CH_3$ | $CH_2C(=O)OCH_2CH_3$ | $CH_3$ | $OCH_3$ |
| $CH_2$ | $CH_2C(=O)OCH(CH_3)_2$ | $CH_2$ | $OCH_3$ |
| $CH_3$ | $CH_2C(=O)OC(CH_3)_3$ | $CH_3$ | $OCH_2$ |
| $CH_3$ | $CH(CH_3)C(=O)OCH_3$ | $CH_3$ | $OCH_3$ |
| $CH_3$ | $CH(CH_2)C(=O)OCH_2CH_3$ | $CH_3$ | $OCH_2$ |

Table 31

| $R^1$ | $R^2$ | $R^3$ | M |
|---|---|---|---|
| $CH_2$ | $CH_2CH=CH_2$ | $CH_2CH_3$ | $OCH_2$ |
| $CH_3$ | $CH_2C \equiv CH$ | $CH_2CH_3$ | $OCH_3$ |
| $CH_3$ | Cyclopropyl | $CH_2CH_3$ | $OCH_3$ |
| $CH_3$ | $CH_2$(cyclopropyl) | $CH_2CH_2$ | $OCH_3$ |
| $CH_3$ | $CH(CH_3)$ (cyclopropyl) | $CH_2CH_3$ | $OCH_3$ |
| $CH_3$ | $CH_2CN$ | $CH_2CH_3$ | $OCH_3$ |

(continued)

| $R^1$ | $R^2$ | $R^3$ | M |
|---|---|---|---|
| $CH_3$ | $CH(CH_3)CN$ | $CH_2CH_3$ | $OCH_3$ |
| $CH_3$ | $CH_2OCH_2$ | $CH_2CH_2$ | $OCH_2$ |
| $CH_3$ | $CH_2OCH_2CH_2$ | $CH_2CH_3$ | $OCH_3$ |
| $CH_3$ | $CH(CH_3)OCH_3$ | $CH_2CH_3$ | $OCH_3$ |
| $CH_2$ | $CH_2CH_2OCH_3$ | $CH_2CH_2$ | $OCH_3$ |
| $CH_3$ | $CH_2CH_2OCH_2CH_3$ | $CH_2CH_3$ | $OCH_3$ |
| $CH_3$ | $CH_2CH_2OCH(CH_3)_2$ | $CH_2CH_3$ | $OCH_3$ |
| $CH_3$ | $CH_2CH_2OC(CH_3)_3$ | $CH_2CH_3$ | $OCH_3$ |
| $CH_3$ | $CH_2C(=O)OCH_2$ | $CH_2CH_2$ | $OCH_3$ |
| $CH_3$ | $CH_2C(=O)OCH_2CH_3$ | $CH_2CH_3$ | $OCH_3$ |
| $CH_3$ | $CH_2C(=O)(CH_2)_2$ | $CH_2CH_3$ | $OCH_3$ |
| $CH_3$ | $CH_2C(=O)OC(CH_3)_3$ | $CH_2CH_3$ | $OCH_2$ |
| $CH_2$ | $CH(CH_2)C(=O)OCH_2$ | $CH_2CH_3$ | $OCH_3$ |
| $CH_3$ | $CH(CH_2)C(=O)OCH_2CH_2$ | $CH_2CH_3$ | $OCH_3$ |

Table 32

| $R^1$ | $R^2$ | $R^3$ | M |
|---|---|---|---|
| $CH_2$ | H | $CH_2H=CH_2$ | $OCH_2$ |
| $CH_3$ | H | $CH_2C{\equiv}CH$ | $OCH_3$ |
| $CH_2$ | H | cyclopropyl | $OCH_3$ |
| $CH_3$ | H | $CH_2$ (cyclopropyl) | $OCH_3$ |
| $CH_3$ | H | $CH(CH_3)$(cyclopropyl) | $OCH_2$ |
| $CH_3$ | H | $CH_2CN$ | $OCH_2$ |
| $CH_3$ | H | $CH(CH_3)CN$ | $OCH_3$ |
| $CH_3$ | H | $CH_2OCH_3$ | $OCH_3$ |
| $CH_3$ | H | $CH_2OCH_2CH_3$ | $OCH_3$ |
| $CH_3$ | H | $CH(CH_3)OCH_3$ | $OCH_3$ |
| $CH_3$ | H | $CH_2CH_2OCH_3$ | $OCH_3$ |
| $CH_3$ | H | $CH_2CH_2OCH_2CH_3$ | $OCH_3$ |
| $CH_3$ | H | $CH_2CH_2OCH(CH_3)_2$ | $OCH_2$ |
| $CH_3$ | H | $CH_2CH_2OC(CH_3)_3$ | $OCH_3$ |
| $CH_3$ | H | $CH_2C(=O)OCH_3$ | $OCH_3$ |
| $CH_3$ | H | $CH_2C(=O)OCH_2CH_3$ | $OCH_3$ |
| $CH_3$ | H | $CH_2C(=O)OCH(CH_3)_2$ | $OCH_3$ |
| $CH_2$ | H | $CH_2C(=O)OC(CH_3)_3$ | $OCH_3$ |
| $CH_3$ | H | $CH(CH_3)C(=O)OCH_3$ | $OCH_3$ |
| $CH_3$ | H | $CH(CH_2)C(=O)OCH_2CH_3$ | $OCH_3$ |

Table 33

| $R^1$ | $R^2$ | $R^3$ | M. |
|---|---|---|---|
| $CH_3$ | $CH_3$ | $CH_2CH{=}CH_2$ | $OCH_3$ |
| $CH_3$ | $CH_2$ | $CH_2C{\equiv}CH$ | $OCH_3$ |
| $CH_3$ | $CH_3$ | cyclopropyl | $OCH_3$ |
| $CH_3$ | $CH_3$ | $CH_2$(cyclopropyl) | $OCH_3$ |
| $CH_3$ | $CH_3$ | $CH(CH_2)$ (cyclopropyl) | $OCH_2$ |
| $CH_2$ | $CH_3$ | $CH_2CN$ | $OCH_3$ |
| $CH_3$ | $CH_3$ | $CH(CH_3)CN$ | $OCH_3$ |
| $CH_3$ | $CH_2$ | $CH_2OCH_3$ | $OCH_3$ |
| $CH_3$ | $CH_3$ | $CH_2OCH_2CH_3$ | $OCH_3$ |
| $CH_3$ | $CH_3$ | $CH(CH_3)OCH_3$ | $OCH_3$ |
| $CH_3$ | $CH_3$ | $CH_2CH_2OCH_3$ | $OCH_3$ |
| $CH_3$ | $CH_3$ | $CH_2CH_2OCH_2CH_3$ | $OCH_3$ |
| $CH_3$ | $CH_3$ | $CH{=}CH_2OCH(CH_3)_2$ | $OCH_2$ |
| $CH_2$ | $CH_3$ | $CH_2CH_2OC(CH_3)_3$ | $OCH_3$ |
| $CH_3$ | $CH_3$ | $CH_2C(=O)OCH_3$ | $OCH_3$ |
| $CH_2$ | $CH_3$ | $CH_2C(=O)OCH_2CH_2$ | $OCH_3$ |
| $CH_3$ | $CH_3$ | $CH_2C(=O)OCH(CH_3)_2$ | $OCH_3$ |
| $CH_2$ | $CH_3$ | $CH_2C(=O)OC(CH_3)_3$ | $OCH_3$ |
| $CH_3$ | $CH_3$ | $CH(CH_3)C(=O)OCH_3$ | $OCH_3$ |
| $CH_3$ | $CH_3$ | $CH(CH_3)C(=O)OCH_2CH_3$ | $OCH_3$ |

Table 34

| $R^1$ | $R^2$ | $R^3$ | M |
|---|---|---|---|
| $CH_3$ | $CH_2CH_3$ | $CH_2CH{=}CH_3$ | $OCH_3$ |
| $CH_3$ | $CH_2CH_3$ | $CH_2C{\equiv}CH$ | $OCH_3$ |
| $CH_2$ | $CH_2CH_3$ | cyclopropyl | $OCH_3$ |
| $CH_3$ | $CH_2CH_3$ | $CH_2$ (cyclopropyl) | $OCH_2$ |
| $CH_3$ | $CH_2CH_3$ | $CH(CH_2)$ (cyclopropyl) | $OCH_3$ |
| $CH_2$ | $CH_2CH_2$ | $CH_2CN$ | $OCH_3$ |
| $CH_3$ | $CH_2CH_3$ | $CH(CH_2)CN$ | $OCH_8$ |
| $CH_3$ | $CH_2CH_3$ | $CH_2OCH_2$ | $OCH_3$ |
| $CH_2$ | $CH_2CH_8$ | $CH_2OCH_2CH_3$ | $OCH_3$ |
| $CH_2$ | $CH_2CH_3$ | $CH(CH_3)OCH_3$ | $OCH_3$ |
| $CH_3$ | $CH_2CH_3$ | $CH_2CH_2OCH_3$ | $OCH_3$ |
| $CH_3$ | $CH_2CH_3$ | $CH_2CH_2OCH_2CH_3$ | $OCH_3$ |

(continued)

| $R^1$ | $R^2$ | $R^3$ | M |
|---|---|---|---|
| $CH_3$ | $CH_2CH_3$ | $CH_2CH_2OCH(CH_3)_2$ | $OCH_3$ |
| $CH_3$ | $CH_2CH_3$ | $CH_2PH_2OC\ (CH_3)_3$ | $OCH_3$ |
| $CH_3$ | $CH_2CH_3$ | $CH_2C(=O)OCH_3$ | $CH_3$ |
| $CH_3$ | $CH_2CH_3$ | $CH_2C(=O)OCH_2CH_3$ | $OCH_3$ |
| $CH_3$ | $CH_2CH_3$ | $CH_2C(=C)OCH(CH_3)_2$ | $OCH_3$ |
| $CH_2$ | $CH_2CH_3$ | $CH_2C(=O)OC(CH_3)_3$ | $OCH_3$ |
| $CH_3$ | $CH=CH_3$ | $CH(CH_3)C(=O)OCH_3$ | $OCH_3$ |
| $CH_3$ | $CH_2CH_3$ | $CH(CH_3)C(=O)OCH_2CH_3$ | $OCH_8$ |

Table 35

| $R^1$ | $R^2$ | $R^3$ | M |
|---|---|---|---|
| $CH_3$ | $CH_2CH=CH_2$ | $CH_2CH=CH_2$ | $OCH_3$ |
| $CH_3$ | $CH_2C\equiv CH$ | $CH_2C\equiv CH$ | $OCH_3$ |
| $CH_3$ | cyclopropyl | cyclopropyl | $OCH_3$ |
| $CH_3$ | $CH_2$ (cyclopropyl) | $CH_2$(cyclopropyl) | $OCH_3$ |
| $CH_3$ | $CH(CH_3)$ (cyclopropyl) | $CN(CH_3)$ (cyclopropyl) | $OCH_3$ |
| $CH_3$ | $CH_2CN$ | $CH_2CN$ | $OCH_3$ |
| $CH_8$ | $CH(CH_3)CN$ | $CH(CH_2)CN$ | $OCH_3$ |
| $CH_2$ | $CH_2OCH_3$ | $CH_2OCH_2$ | $OCH_3$ |
| $CH_3$ | $CH_2OCH_2CH_2$ | $CH_2OCH_2CH_3$ | $OCH_3$ |
| $CH_2$ | $CH(CH_3)OCH_2$ | $CH(CH_3)OCH_3$ | $OCH_3$ |
| $CH_3$ | $CH_2CH_2OCH_3$ | $CH_2CH_2OCH_3$ | $OCH_3$ |
| $CH_2$ | $CH_2CH_2OCH_2CH_3$ | $CH_2CH_2OCH_2CH_3$ | $OCH_3$ |
| $CH_3$ | $CH_2CH_2OCH(CH_3)_2$ | $CH_2CH_2OCH(CH_2)_2$ | $OCH_3$ |
| $CH_2$ | $CH=CH_2OC\ (CH_3)_2$ | $CH_2CH_2OC(CH_3)_3$ | $OCH_3$ |
| $CH_3$ | $CH_2C(=O)OCH_3$ | $CH_2C(=O)OCH_3$ | $OCH_3$ |
| $CH_2$ | $CH_2C(=O)OCH_2CH_3$ | $CH_2C(=O)OCH_2CH_3$ | $OCH_3$ |
| $CH_3$ | $CH_2C(=O)OCH(CH_3)_2$ | $CH_2C(=O)OCH(CH_3)_3$ | $OCH_3$ |
| $CH_3$ | $CH_2C(=O)OC(CH_2)_3$ | $CH=C(=O)OC(CH_3)_3$ | $OCH_2$ |
| $CH_3$ | $CH(CH_3)C(=O)OCH_3$ | $CH(CH_3)C(=O)OCH_3$ | $OCH_3$ |
| $CH_3$ | $CH(CH_3)C(=O)OCH_2CH_3$ | $CH(CH_3)C(=O)OCH_2CH_3$ | $OCH_3$ |

Table 36

| $R^1$ | $R^2$ | $R^3$ | M |
|---|---|---|---|
| $CH_2$ | $CH_2CH=CH_2$ | H | $N\ (CH_2)_2$ |
| $CH_3$ | $CH_3\equiv CH$ | H | $N(CH_3)_2$ |

(continued)

| $R^1$ | $R^2$ | $R^3$ | M |
|---|---|---|---|
| $CH_3$ | cyclopropyl | H | $N(CH_3)_2$ |
| $CH_3$ | $CH_2$ (cyclopropyl) | H | $N(CH_3)_2$ |
| $CH_3$ | $CH(CH_3)$ (cyclopropyl) | H | $N(CH_3)_2$ |
| $CH_2$ | $CH_2CN$ | H | $N(CH_3)_2$ |
| $CH_2$ | $CH(CH_3)CN$ | H | $N(CH_2)_2$ |
| $CH_3$ | $CH_2OCH_3$ | H | $N(CH_3)_2$ |
| $CH_3$ | $CH_2OCH_2CH_3$ | H | $N(CH_3)_2$ |
| $CH_3$ | $CH(CH_2)OCH_3$ | H | $N(CH_3)_2$ |
| $CH_3$ | $CH_2CH_2OCH_3$ | H | $N(CH_3)_2$ |
| $CH_3$ | $CH_2CH_2OCH_2CH_3$ | H | $N(CH_3)_2$ |
| $CH_3$ | $CH_2CH_2OCH(CH_3)_2$ | H | $N(CH_3)_2$ |
| $CH_3$ | $CH_2CH_2OC(CH_3)_3$ | H | $N(CH_2)_2$ |
| $CH_3$ | $CH_2C(=O)OCH_3$ | H | $N(CH_3)_2$ |
| $CH_3$ | $CH_2C(=O)OCH_2CH_3$ | H | $N(CH_3)_2$ |
| $CH_2$ | $CH_2C(=O)OCH(CH_3)_2$ | H | $N(CH_3)_2$ |
| $CH_3$ | $CH_2C(=O)OC(CH_3)_3$ | H | $N(CH_3)_2$ |
| $CH_3$ | $CH(CH_3)C(=O)OCH_2$ | H | $N(CH_3)_2$ |
| $CH_3$ | $CH(CH_3)C(=O)OCH_2CH_3$ | H | $N(CH_3)_2$ |

Table 37

| $R^1$ | $R^2$ | $R^3$ | M |
|---|---|---|---|
| $CH_2$ | $CH_2CH=CH_2$ | $CH_2$ | $N(CH_3)_2$ |
| $CH_3$ | $CH_2C\equiv CH$ | $CH_3$ | $N(CH_3)_2$ |
| $CH_3$ | cyclopropyl | $CH_3$ | $N(CH_3)_2$ |
| $CH_3$ | $CH_2$(cyclopropyl) | $CH_2$ | $N(CH_2)_2$ |
| $CH_3$ | $CH(CH_3)$ (cyclopropyl) | $CH_2$ | $N(CH_3)_2$ |
| $CH_3$ | $CH_2CN$ | $CH_2$ | $N(CH_3)_3$ |
| $CH_2$ | $CH(CH_3)CN$ | $CH_2$ | $N(CH_3)_2$ |
| $CH_3$ | $CH_2OCH_3$ | $CH_3$ | $N(CH_3)_2$ |
| $CH_3$ | $CH_8OCH_2CH_3$ | $CH_3$ | $N(CH_3)_2$ |
| $CH_2$ | $CH(CH_3)OCH_2$ | $CH_3$ | $N(CH3)_2$ |
| $CH_2$ | $CH_2CH_2OCH_3$ | $CH_2$ | $N(CH_2)_2$ |
| $CH_2$ | $CH_2CH_2OCH_2CH_3$ | $CH_2$ | $N(CH_3)_2$ |
| $CH_3$ | $CH_2CH_2OCH(CH_3)_2$ | $CH_3$ | $N(CH_3)_2$ |
| $CH_3$ | $CH_2CH_2OC(CH_3)_3$ | $CH_3$ | $N(CH_2)_2$ |
| $CH_3$ | $CH_2C(=O)OCH_3$ | $CB_3$ | $N(CH_2)_2$ |
| $CH_3$ | $CH_2C(=O)OCH_2CH_3$ | $CH_3$ | $N(CH_3)_2$ |

(continued)

| $R^1$ | $R^2$ | $R^3$ | M |
|---|---|---|---|
| $CH_3$ | $CH_2C(=O)OCH(CH_3)_2$ | $CH_2$ | $N(CH_3)_2$ |
| $CH_3$ | $CH_2C(=O)OC(CH_3)_3$ | $CH_3$ | $N(CH_3)_2$ |
| $CH_3$ | $CH(CH_3)C(=O)0CH_3$ | $CH_3$ | $N(CH_3)_2$ |
| $CH_2$ | $CH(CH_3)C(=O)OCH_2CH_2$ | $CH_2$ | $N(CH_3)_2$ |

Table 38

| $R^1$ | $R^2$ | $R^3$ | M |
|---|---|---|---|
| $CH_3$ | $CH_2CH=CH_2$ | $CH_2CH_3$ | $N(CH_2)_2$ |
| $CH_3$ | $CH_2C\equiv CH$ | $CH_2CH_3$ | $N(CH_3)_3$ |
| $CH_3$ | cyclopropyl | $CH_2CH_8$ | $N(CH_3)_2$ |
| $CH_3$ | $CH_2$ (cyclopropyl) | $CH_2CH_3$ | $N(CH_3)_2$ |
| $CH_3$ | $CH(CH_3)$ (cyclopropyl) | $CH_2CH_3$ | $N(CH_3)_2$ |
| $CH_2$ | $CH_2CN$ | $CH_2CH_2$ | $N(CH_2)_2$ |
| $CH_3$ | $CH(CH_2)CN$ | $CH_2CH_3$ | $N(CH_3)_2$ |
| $CH_8$ | $CH_2OCH_2$ | $CH_4CH_3$ | $N(CH_2)_2$ |
| $CH_3$ | $CH_2OCH_2CH_3$ | $CH_2CH_8$ | $N\ (CH_2)_2$ |
| $CH_8$ | $CH(CH_3)OCH_2$ | $CH_2CH_8$ | $N(CH_3)_2$ |
| $CH_3$ | $CH_2CH_2OCH_3$ | $CH_2CH_3$ | $N(CH_2)_3$ |
| $CH_8$ | $CH_2CH_2OCH_2CH_3$ | $CH_2CH_3$ | $N(CH_2)_2$ |
| $CH_2$ | $CH_2CH_2OCH(CH_2)_2$ | $CH_2CH_3$ | $N\ (CH_3)_3$ |
| $CH_3$ | $CH_2CH_2OC(CH_3)_3$ | $CH_2CH_3$ | $N(CH_3)_2$ |
| $CH_3$ | $CH_2C(=O)OCH_3$ | $CH_2CH_3$ | $N(CH_2)_2$ |
| $CH_3$ | $CH_2C(=O)OCH_2CH_3$ | $CX_2X_3$ | $N(CH_3)_2$ |
| $CH_3$ | $CH_2C(=O)OCH(CH_3)_2$ | $CH_2CH_3$ | $N(CH_2)_2$ |
| $CH_3$ | $CH_2C(=O)OC(CH_3)_3$ | $CH_2CH_3$ | $N(CH_2)_2$ |
| $CH_2$ | $CH(CH_3)C(=O)OCH_3$ | $CH_2H_3$ | $N(CH)_3)_2$ |
| $CH_3$ | $CH(CH_3)C(=O)OCH_2CH_3$ | $CH_2CH_3$ | $N(CH_3)_2$ |

Table 39

| $R^l$ | $R^2$ | $R^3$ | M |
|---|---|---|---|
| $CH_3$ | H | $CH_2CH=CH_2$ | $N(CH_3)_2$ |
| $CH_3$ | H | $CH_2C\equiv CH$ | $N(CH_3)_2$ |
| $CH_3$ | H | cyclopropyl | $N(CH_3)_2$ |
| $CH_3$ | H | $CH_2$ (cyclopropyl) | $N(CH_2)_2$ |
| $CH_3$ | H | $CH(CH_3)$ (cyclopropyl) | $N(CH_3)_2$ |
| $CH_3$ | H | $CH_2CN$ | $N(CH_3)_2$ |

(continued)

| $R^I$ | $R^2$ | $R^3$ | M |
|---|---|---|---|
| $CH_3$ | H | $CH(CH_3)CN$ | $N (CH_3)_2$ |
| $CH_3$ | H | $CH_4OCH_3$ | $N(CH_3)_3$ |
| $CH_3$ | H | $CH_2OCH_2CH_3$ | $N(CH_3)_2$ |
| $CH_3$ | H | $CH(CH_3)OCH_3$ | $N(CH_3)_2$ |
| $CH_3$ | H | $CH_2CH_2CH_3$ | $N(CH_3)_2$ |
| $CH_3$ | H | $CH_2CH_2OCH_2CH_3$ | $N(CH_3)_2$ |
| $CH_2$ | H | $CH_2CH_2OCH(CH_3)_2$ | $N(CH_3)_2$ |
| $CH_3$ | H | $CH_2CH_2OC(CH_3)_3$ | $N(CH_2)_2$ |
| $CH_3$ | H | $CH_2C(=O)OCH_2$ | $N(CH_3)_2$ |
| $CH_3$ | H | $CH_2C(=O)OCH_2CH_3$ | $N(CH_3)_2$ |
| $CH_3$ | H | $CH_2C(=O)OCH(CH_3)_2$ | $N(CH_2)_2$ |
| $CH_3$ | H | $CH_2C(=O)OC(CH_3)_3$ | $N(CH_3)_2$ |
| $CH_3$ | H | $CH(CH_3)C(=O)OCH_3$ | $N(CH_3)_2$ |
| $CH_3$ | H | $CH(CH_3)C(=O)OCH_2CH_3$ | $N(CH_3)_2$ |

Table 40

| $R^1$ | $R^2$ | $R^3$ | M |
|---|---|---|---|
| $CH_3$ | $CH_3$ | $CH_2CH=CH_2$ | $N(CH_3)_2$ |
| $CH_3$ | $CH_3$ | $CH_2C\equiv CH$ | $N(CH_3)_2$ |
| $CH_3$ | $CH_3$ | cyclopropyl | $N(CH_3)_2$ |
| $CH_3$ | $CH_3$ | $CH_2$(cyclopropyl) | $N(CH_3)_2$ |
| $CH_3$ | $CH_3$ | $CH(CH_3)$ (cyclopropyl) | $N(CH_3)_2$ |
| $CH_3$ | $CH_3$ | $CH_2CN$ | $N(CH_3)_2$ |
| $CH_3$ | $CH_3$ | $CH(CH_3)CN$ | $N(CH_3)_2$ |
| $CH_3$ | $CH_3$ | $CH_2OCH_3$ | $N(CH_3)_2$ |
| $CH_3$ | $CH_3$ | $CH_2OCH_2CH_3$ | $N(CH_3)_2$ |
| $CH_3$ | $CH_3$ | $CH(CH_2)OCH_3$ | $N(CH_3)_2$ |
| $CH_3$ | $CH_3$ | $CH_2CH_2M_3$ | $N(CH_3)_2$ |
| $CH_3$ | $CH_3$ | $CH_2CH_2OCH_2CH_3$ | $N(CH_3)_2$ |
| $CH_3$ | $CH_3$ | $CH_2H_2OCH (CH_3)_2$ | $N(CH_3)_2$ |
| $CH_3$ | $CH_3$ | $CH_2CH_2OC(CH_3)_3$ | $N(CH_3)_2$ |
| $CH_3$ | $CH_3$ | $CH_2C(=O)OCH_3$ | $N(CH_2)_2$ |
| $CH_3$ | $CH_3$ | $CH_2C(=O)OCH_2CH_3$ | $N(CH_3)_2$ |
| $CH_3$ | $CH_3$ | $CH_2C(=O)OCH(CH_3)_2$ | $N(CH_3)_2$ |
| $CH_2$ | $CH_3$ | $CH_2C(=O)OC(CH_3)_3$ | $N(CH_3)_2$ |
| $CH_3$ | $CH_2$ | $CH(CH_3)C(=O)OCH_3$ | $N(CH_3)_2$ |
| $CH_3$ | $CH_3$ | $CH(CH_3)C(=O)OCH_2CH_3$ | $N(CN_3)_2$ |

Table 41

| $R^1$ | $R^2$ | $R^3$ | M |
|---|---|---|---|
| $CH_2$ | $CH_2CH_2$ | $CH_2CH{=}CH_3$ | $N(CH_3)_2$ |
| $CH_3$ | $CH_2CH_3$ | $CH_2C{\equiv}CH$ | $N(CH_3)_2$ |
| $CH_3$ | $CH_2CH_3$ | cyclopropyl | $N(CH_3)_3$ |
| $CH_3$ | $CH_2CH_3$ | $CH_2$ (cyclopropyl) | $N(CH_3)_2$ |
| $CH_3$ | $CH_3CH_3$ | CH ($CH_3$) (cyclopropyl) | $N(CH_3')_{2:}$ |
| $CH_2$ | $CH_2CH_3$ | $CH_2CN$ | $N(CH_3)_2$ |
| $CH_3$ | $CH_2CH_3$ | $CH(CH_3)CN$ | $N(CH_3)_2$ |
| $CH_3$ | $CH_2CH_3$ | $CH_2OCH_3$ | $N(CH_3)_3$ |
| $CH_3$ | $CH_2CH_3$ | $CH_2OCH_2CH_3$ | $N(CH_3)_2$ |
| $CH_3$ | $CH_2CH_3$ | $CH(CH_3)OCH_3$ | $N(CH_3)_2$ |
| $CH_3$ | $CH_2CH_3$ | $CH_2CH_2OCH_3$ | $N(CH_3)_2$ |
| $CH_3$ | $CH_2CH_3$ | $CH_3CH_2OCH_2CH_2$ | $N(CH_3)_2$ |
| $CH_3$ | $CH_2CH_3$ | $CH_2CH_2OCH(CH_3)_2$ | $N(CH_3)_3$ |
| $CH_3$ | $CH_2CH_3$ | $CH_2CH_2OC(CH_3)_3$ | $N(CH_3)_2$ |
| $CH_3$ | $CH_2CH_3$ | $CH_2C(=O)OCH_3$ | $N(CH_3)_2$ |
| $CH_3$ | $CH_2CH_3$ | $CH_2C(=O)OCH_2CH_3$ | $N(CH_3)_2$ |
| $CH_3$ | $CH_2CH_3$ | $CH_2C(=O)OCH(CH_3)_2$ | $N(CH_3)_2$ |
| $CH_3$ | $CH_2CH_3$ | $CH_2C(=O)OC(CH_3)_3$ | $N(CH_3)_2$ |
| $CH_3$ | $CH_2CH_3$ | $CH(CH_3)C(=O)OCH_3$ | $N(CH_3)_2$ |
| $CH_3$ | $CH_2CH_3$ | $CH(CH_3)(=O)OCH_2CH_3$ | $N(CH_3)_2$ |

Table 42

| $R^1$ | $R^2$ | $R^3$ | M |
|---|---|---|---|
| $CH_3$ | $CH_2CH{=}CH_2$ | $CH_2CH{=}CH_2$ | $N(CH_3)_2$ |
| $CH_3$ | $CH_2C{\equiv}CH$ | $CH_2C{\equiv}CH$ | $N(CH_3)_2$ |
| $CH_3$ | cyclopropyl | cyclopropyl | $N(CH_2)_2$ |
| $CH_3$ | $CH_2$ (cyclopropyl) | $CH_2$ (cyclopropyl) | $N(CH_3)_2$ |
| $CH_3$ | $CH(CH_3)$ (cyclopropyl) | $CH(CH_3)$ (cyclopropyl) | $N(CH_3)_2$ |
| $CH_3$ | $CH_2CN$ | $CH_2CN$ | $N(CH_3)_2$ |
| $CH_3$ | $CH(CH_3)CN$ | $CH(CH_3)CN$ | $N(CH_3)_3$ |
| $CH_3$ | $CH_2OCH_3$ | $CH_2OCH_2$ | $N(CH_2)_2$ |
| $CH_3$ | $CH_2OCH_2CH_3$ | $CH_3OCH_2CH_3$ | $N(CH_3)_2$ |
| $CH_3$ | $CH(CH_3)OCH_3$ | $CH(CH_3)OCH_3$ | $N(CH_3)_2$ |
| $CH_3$ | $CH_2CH_2OCH_3$ | $CH_2CH_3OCH_3$ | $N(CH_3)_2$ |
| $CH_3$ | $CH_2CH_2OCH_2CH_3$ | $CH_2CH_2OCH_2CH_2$ | $N(CH_3)_2$ |

(continued)

| $R^1$ | $R^2$ | $R^3$ | M |
|---|---|---|---|
| $CH_3$ | $CH_2CH_2OCH(CH_3)$ | $CH_2CH_2OCH(CH_3)_3$ | $N(CH_3)_3$ |
| $CH_3$ | $CH_2CH_2OC(CH_3)_3$ | $CH_2CH_2OC(CH_3)_3$ | $N(CH_3)_3$ |
| $CH_3$ | $CH_2C(=O)OCH_3$ | $CH_2C(=O)OCH_3$ | $N(CH_3)_2$ |
| $CH_3$ | $CH_2C(=O)OCH_2CH_3$ | $CH_2C(=O)OCH_2CH_3$ | $N(CH_3)_2$ |
| $CH_3$ | $CH_2C(=O)OCH(CH_3)_2$ | $CH_2C(=O)OCH(CH_3)_3$ | $N(CH_3)_2$ |
| $CH_3$ | $CH_2C(=O)OC(CH_3)_3$ | $CH_2C(=O)OC(CH_3)_3$ | $N(CH_3)_2$ |
| $CH_3$ | $CH(CH_3)C(=O)OCH_3$ | $CH(CH_3)C(=O)OCH_3$ | $N(CH_3)_2$ |
| $CH_3$ | $CH(CH_3)C(=O)OCH_2CH_3$ | $CH(CH_3)C(=O)OCH_2CH_3$ | $N(CH_3)_2$ |

Table 43

| $R^1$ | $R^2$ | $R^3$ | M |
|---|---|---|---|
| H | $CH_2C(CH_3)=CH_2$ | H | $OCH_3$ |
| H | $CH_2CH=CHCH_3$ | H | $OCH_3$ |
| H | $CH_2CH=C(CH_3)_2$ | H | $OCH_3$ |
| H | $CH_2CCl=CH_2$ | H | $OCH_3$ |
| H | $CH_2CH=CHCl$ | H | $OCH_3$ |
| H | $CH_2CH=CCl_2$ | H | $OCH_3$ |
| H | $CH_2CBr=CH_2$ | H | $OCR_3$ |
| H | $CH_2CH=CHBr$ | H | $OCH_3$ |
| H | $CH_2CH=CBr_2$ | H | $OCH_3$ |
| H | $CH_2CH_2CH=CH_2$ | H | $OCH_3$ |
| H | $CH(CH_3)CH=CH_2$ | H | $OCH_3$ |
| H | $CH_2C=CCH_3$ | H | $OCH_3$ |
| H | $CH_2C\equiv CCl$ | H | $OCH_3$ |
| H | $CH_2C\equiv CBr$ | H | $OCH_3$ |
| H | $CH_2CH_2C\equiv CH$ | H | $OCH_3$ |
| H | $CH(CH_3)C\equiv CH$ | H | $OCH_3$ |
| H | $CH_2CH_2OH$ | H | $OCH_3$ |
| H | $CH(CH_3)CH_2OH$ | H | $OCH_3$ |
| H | $CH_2CH(CH_3)OH$ | H | $OCH_3$ |

Table 44

| $R^1$ | $R^2$ | $R^3$ | M |
|---|---|---|---|
| H | 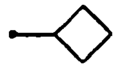 | H | $OCH_3$ |

(continued)

| R1 | R2 | R3 | M |
|---|---|---|---|
| H | 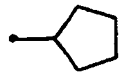 | H | $OCH_3$ |
| H | 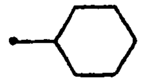 | H | $OCH_3$ |
| H | 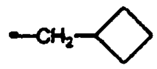 | H | $OCH_3$ |
| H | 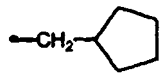 | H | $OCH_3$ |
| H | 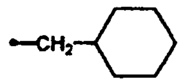 | H | $OCH_3$ |
| H | 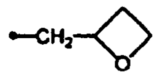 | H | $OCH_3$ |
| H | 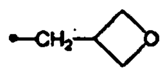 | H | $OCH_3$ |
| H | 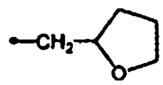 | H | $OCH_3$ |
| H | 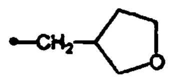 | H | $OCH_3$ |

Table 45

| R1 | R2 | R3 | M |
|---|---|---|---|
| H | 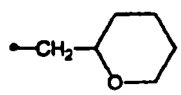 | H | $OCH_3$ |
| H | 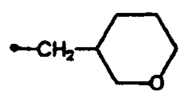 | H | $OCH_3$ |
| H | 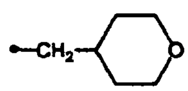 | H | $OCH_3$ |
| H | 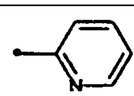 | H | $OCH_3$ |
| H | 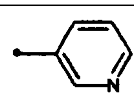 | H | $OCH_3$ |
| H | 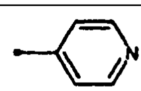 | H | $OCH_3$ |
| H | 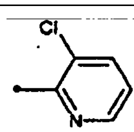 | H | $OCH_3$ |

(continued)

| $R^1$ | $R^2$ | $R^3$ | M |
|---|---|---|---|
| H | 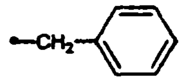 | H | $OCH_3$ |
| H | 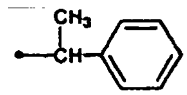 | H | $OCH_3$ |

Table 46

| $R^1$ | $R^2$ | $R^3$ | M |
|---|---|---|---|
| H | 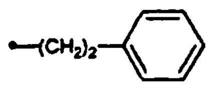 | H | $OCH_3$ |
| H | 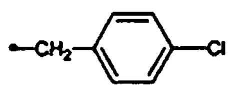 | H | $OCH_3$ |
| H | 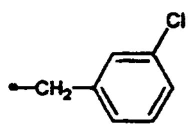 | H | $OCH_3$ |
| H | 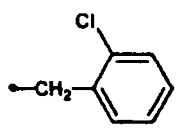 | H | $OCH_3$ |
| H | 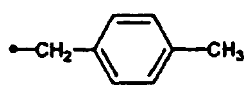 | H | $OCH_3$ |
| H | 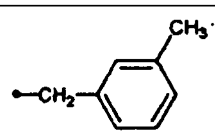 | H | $OCH_3$ |
| H | 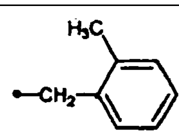 | H | $OCH_3$ |
| H | 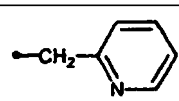 | H | $OCH_3$ |
| H | 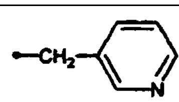 | H | $OCH_3$ |
| H | 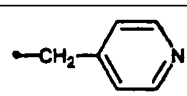 | H | $OCH_3$ |

Table 47

| $R^1$ | $R^2$ | $R^3$ | M |
|---|---|---|---|
| H | $CH_2C(CH_3)=CH_2$ | $CH_3$ | $OCH_3$ |

(continued)

| $R^1$ | $R^2$ | $R^3$ | M |
|---|---|---|---|
| H | $CH_2CH=CHCH_3$ | $CH_3$ | $OCH_3$ |
| H | $CH_2CH=C(CH_3)_2$ | $CH_3$ | $OCH_3$ |
| H | $CH_2CCl=CH_2$ | $CH_3$ | $OCH_3$ |
| H | $CH_2CH=CHCl$ | $CH_3$ | $OCH_3$ |
| H | $CH_2CH=CCl_2$ | $CH_3$ | $OCH_3$ |
| H | $CH_2CBr=CH_2$ | $CH_3$ | $OCH_3$ |
| H | $CH_2CH=CHBr$ | $CH_3$ | $OCH_3$ |
| H | $CH_2CH=CBr_2$ | $CH_3$ | $OCH_3$ |
| H | $CH_2CH_2CH=CH_2$ | $CH_3$ | $OCH_3$ |
| H | $CH(CH_3)CH=CH_2$ | $CH_3$ | $OCH_3$ |
| H | $CH_2C\equiv CCH_3$ | $CH_3$ | $OCH_3$ |
| H | $CH_2C\equiv CCl$ | $CR_3$ | $OCH_3$ |
| H | $CH_2C\equiv CBr$ | $CH_3$ | $OCH_3$ |
| H | $CH_2CH_2C\equiv CH$ | $CH_3$ | $OCH_3$ |
| H | $CH(CH_3)C\equiv CH$ | $CH_3$ | $OCH_3$ |
| H | $CH_2CH_2OH$ | $CH_3$ | $OCH_3$ |
| H | $CH(CH_3)CH_2OH$ | $CH_3$ | $OCH_3$ |
| H | $CH_2CH(CH_3)OH$ | $CH_3$ | $OCH_3$ |

Table 48

| $R^1$ | $R^2$ | $R^3$ | M |
|---|---|---|---|
| H | 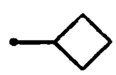 | $CH_3$ | $OCH_3$ |
| H | 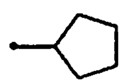 | $CH_3$ | $OCH_3$ |
| H | 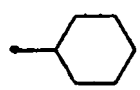 | $CH_3$ | $OCH_3$ |
| H | 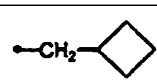 | $CH_3$ | $OCH_3$ |
| H | 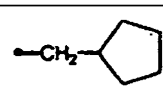 | $CH_3$ | $OCH_3$ |
| H | 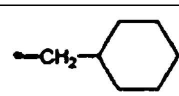 | $CH_3$ | $OCH_3$ |
| H | 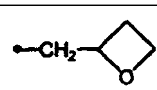 | $CH_3$ | $OCH_3$ |
| H | 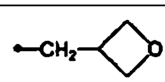 | $CH_3$ | $OCH_3$ |

(continued)

| $R^1$ | $R^2$ | $R^3$ | M |
|---|---|---|---|
| H | 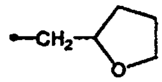 | $CH_3$ | $OCH_3$ |
| H | 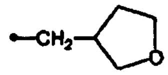 | $CH_3$ | $OCH_3$ |

Table 49

| $R^1$ | $R^2$ | $R^3$ | M |
|---|---|---|---|
| H | 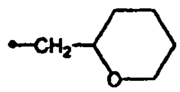 | $CH_3$ | $OCH_3$ |
| H | 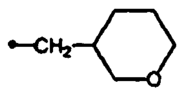 | $CH_3$ | $OCH_3$ |
| H | 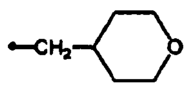 | $CH_3$ | $OCH_3$ |
| H | 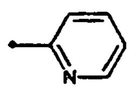 | $CH_3$ | $OCH_3$ |
| H | 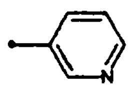 | $CH_3$ | $OCH_3$ |
| H | 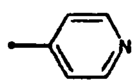 | $CH_3$ | $OCH_3$ |
| H | 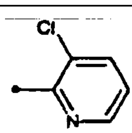 | $CH_3$ | $OCH_3$ |
| H | 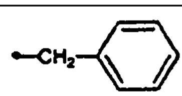 | $CH_3$ | $OCH_3$ |
| H | 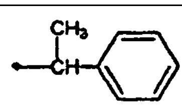 | $CH_3$ | $OCH_3$ |
| H | 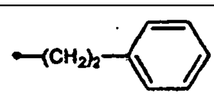 | $CH_3$ | $OCH_3$ |

Table 50

| $R^1$ | $R^2$ | $R^3$ | M |
|---|---|---|---|
| H | 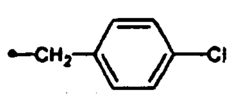 | $CH_3$ | $OCH_3$ |

(continued)

| R1 | R2 | R3 | M |
|---|---|---|---|
| H | 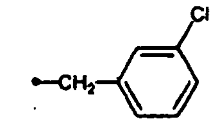 | $CH_3$ | $OCH_3$ |
| H | 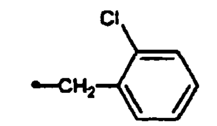 | CH3 | $OCH_3$ |
| H | 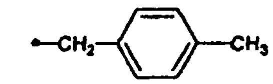 | $CH_3$ | $OCH_3$ |
| H | 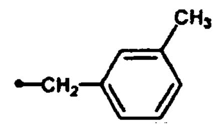 | $CH_3$ | $OCH_3$ |
| H | 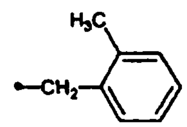 | $CH_3$ | $OCH_3$ |
| H | 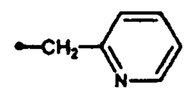 | $CH_3$ | $OCH_3$ |
| H | 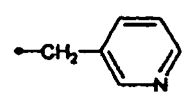 | $CH_3$ | $OCH_3$ |
| H | 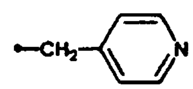 | $CH_3$ | $OCH_3$ |

Table 51

| $R^1$ | $R^2$ | $R^3$ | M |
|---|---|---|---|
| H | $CH_2C(CH_3)=CH_2$ | $CH_2CH_3$ | $OCH_3$ |
| H | $CH_2CH=CHCH_3$ | $CH_2CH_3$ | $OCH_3$ |
| H | $CH_2CH=C(CH_3)_2$ | $CH_2CH_3$ | $OCH_3$ |
| H | $CH_2CCl=CH_2$ | $CH_2CH_3$ | $OCH_3$ |
| H | $CH_2CH=CHCl$ | $CH_2CH_3$ | $OCH_3$ |
| H | $CH_2CH=CCl_2$ | $CH_2CH_3$ | $OCH_3$ |
| H | $CH_2CBr=CH_2$ | $CH_2CH_3$ | $OCH_3$ |
| H | $CH_2CH=CHBr$ | $CH_2CH_3$ | $OCH_3$ |
| H | $CH_2CH=CBr_2$ | $CH_2CH_3$ | $OCH_3$ |
| H | $CH_2CH_2CH=CH_2$ | $CH_2CH_3$ | $OCH_3$ |
| H | $CH(CH_3)CH=CH_2$ | $CH_2CH_3$ | $OCH_3$ |
| H | $CH_2C\equiv CCH_3$ | $CH_2CH_3$ | $OCH_3$ |

(continued)

| $R^1$ | $R^2$ | $R^3$ | M |
|---|---|---|---|
| H | $CH_2C{\equiv}CCl$ | $CH_2CH_3$ | $OCH_3$ |
| H | $CH_2C{\equiv}CBr$ | $CH_2CH_3$ | $OCH_3$ |
| H | $CH_2CH_2C{\equiv}CH$ | $CH_2CH_3$ | $OCH_3$ |
| H | $CH(CH_3)C{\equiv}CH$ | $CH_2CH_3$ | $OCH_3$ |
| H | $CH_2CH_2OH$ | $CH_2CH_3$ | $OCH_3$ |
| H | $CH(CH_3)CH_2OH$ | $CH_2CH_3$ | $OCH_3$ |
| H | $CH_2CH(CH_3)OH$ | $CH_2CH_3$ | $OCH_3$ |

Table 52

| $R^1$ | $R^2$ | $R^3$ | M |
|---|---|---|---|
| H | 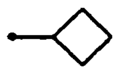 | $CH_2CH_3$ | $OCH_3$ |
| H | 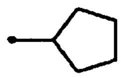 | $CH_2CH_3$ | $OCH_3$ |
| H | 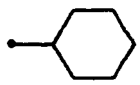 | $CH_2CH_3$ | $OCH_3$ |
| H | 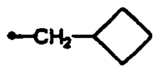 | $CH_2CH_3$ | $OCH_3$ |
| H | 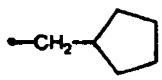 | $CH_2CH_3$ | $OCH_3$ |
| H | 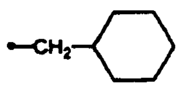 | $CH_2CH_3$ | $OCH_3$ |
| H | 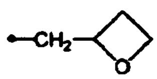 | $CH_2CH_3$ | $OCH_3$ |
| H | 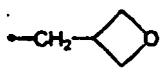 | $CH_2CH_3$ | $OCH_3$ |
| H | 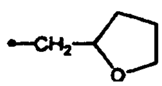 | $CH_2CH_3$ | $OCH_3$ |
| H | 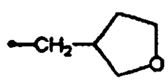 | $CH_2CH_3$ | $OCH_3$ |

able 53

| $R^1$ | $R^2$ | $R^3$ | M |
|---|---|---|---|
| H | 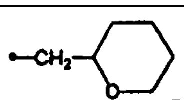 | $CH_2CH_3$ | $OCH_3$ |

(continued)

| R1 | R2 | R3 | M |
|---|---|---|---|
| H | 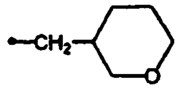 | $CH_2CH_3$ | $OCH_3$ |
| H | 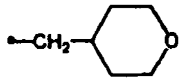 | $CH_2CH_3$ | $OCH_3$ |
| H | 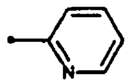 | $CH_2CH_3$ | $OCH_3$ |
| H | 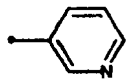 | $CH_2CH_3$ | $OCH_3$ |
| H | 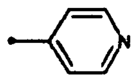 | $CH_2CH_3$ | $OCH_3$ |
| H | 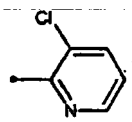 | $CH_2CH_3$ | $OCH_3$ |
| H | 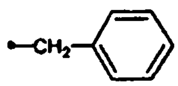 | $CH_2CH_3$ | $OCH_3$ |
| H | 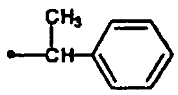 | $CH_2CH_3$ | $OCH_3$ |
| H | 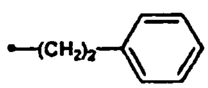 | $CH_2CH_3$ | $OCH_3$ |

Table 54

| R1 | R2 | R3 | M |
|---|---|---|---|
| H | 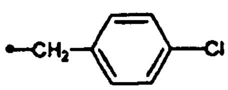 | $CH_2CH_3$ | $OCH_3$ |
| H | 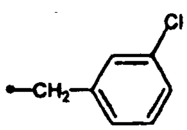 | $CH_2CH_3$ | $OCH_3$ |
| H | 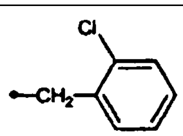 | $CH_2CH_3$ | $OCH_3$ |
| H | 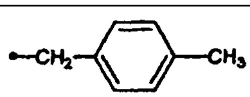 | $CH_2CH_3$ | $OCH_3$ |
| H | 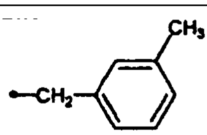 | $CH_2CH_3$ | $OCH_3$ |

(continued)

| $R^1$ | $R^2$ | $R^3$ | M |
|---|---|---|---|
| H | 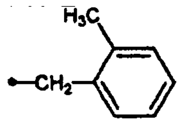 | $CH_2CH_3$ | $OCH_3$ |
| H | 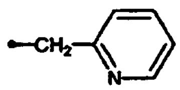 | $CH_2CH_3$ | $OCH_3$ |
| H | 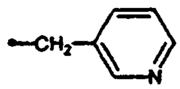 | $CH_2CH_3$ | $OCH_3$ |
| H | 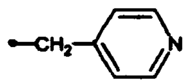 | $CH_2CH_3$ | $OCH_3$ |

Table 55

| $R^1$ | $R^2$ | $R^3$ | M |
|---|---|---|---|
| H | H | $CH_2C(CH_3)=CH_2$ | $OCH_3$ |
| H | H | $CH_2CH=CHCH_3$ | $OCH_3$ |
| H | H | $CH_2CH=C\ (CH_3)_2$ | $OCH_3$ |
| H | H | $CH_2CCl=CH_2$ | $OCH_3$ |
| H | H | $CH_2CH=CHCl$ | $OCH_3$ |
| H | H | $CH_2CH=CCl_2$ | $OCH_3$ |
| H | H | $CH_2CBr=CH_2$ | $OCH_3$ |
| H | H | $CH_2CH=CHBr$ | $OCH_3$ |
| H | H | $CH_2CH=CBr_2$ | $OCH_3$ |
| H | H | $CH_2CH_2CH=CH_2$ | $OCH_3$ |
| H | H | $CH(CH_3)CH=CH_2$ | $OCH_3$ |
| H | H | $CH_2C\equiv CCH_3$ | $OCH_3$ |
| H | H | $CH_2C\equiv CCl$ | $OCH_3$ |
| H | H | $CH_2C\equiv CBr$ | $OCH_3$ |
| H | H | $CH_2CH_2C\equiv CH$ | $OCH_3$ |
| H | H | $CH(CH_3)C\equiv CH$ | $OCH_3$ |
| H | H | $CH_2CH_2OH$ | $OCH_3$ |
| H | H | $CH(CH_3)CH_2OH$ | $OCH_3$ |
| H | H | $CH_2CH(CH_3)OH$ | $OCH_3$ |

Table 56

| $R^1$ | $R^2$ | $R^3$ | M |
|---|---|---|---|
| H | H | 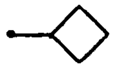 | $OCH_3$ |
| H | H | 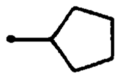 | $OCH_3$ |
| H | H | 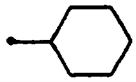 | $OCH_3$ |
| H | H | 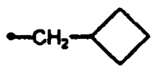 | $OCH_3$ |
| H | H | 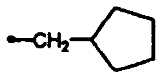 | $OCH_3$ |
| H | H | 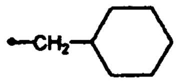 | $OCH_3$ |
| H | H | 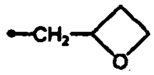 | $OCH_3$ |
| H | H | 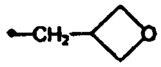 | $OCH_3$ |
| H | H | 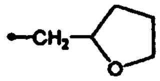 | $OCH_3$ |
| H | H | 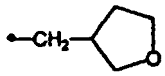 | $OCH_3$ |

Table 57

| $R^1$ | $R^2$ | $R^3$ | M |
|---|---|---|---|
| H | H | 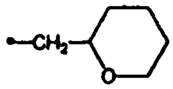 | $OCH_3$ |
| H | H | 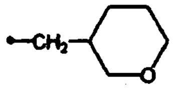 | $OCH_3$ |
| H | H | 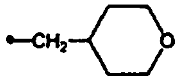 | $OCH_3$ |
| H | H | 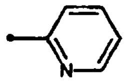 | $OCH_3$ |
| H | H | 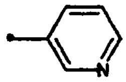 | $OCH_3$ |
| H | H | 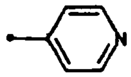 | $OCH_3$ |

(continued)

| $R^1$ | $R^2$ | $R^3$ | M |
|---|---|---|---|
| H | H | 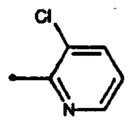 | $OCH_3$ |
| H | H | 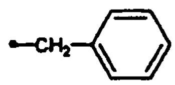 | $OCH_3$ |
| H | H | 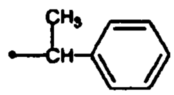 | $OCH_3$ |
| H | H | 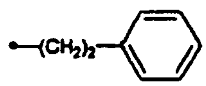 | $OCH_3$ |

Table 58

| $R^1$ | $R^2$ | $R^3$ | M |
|---|---|---|---|
| H | H | 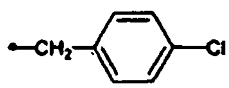 | $OCH_3$ |
| H | H | 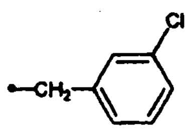 | $OCH_3$ |
| H | H | 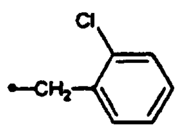 | $OCH_3$ |
| H | H | 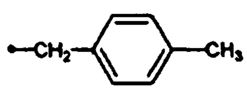 | $OCH_3$ |
| H | H | 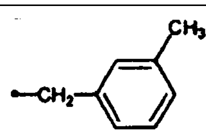 | $OCH_3$ |
| H | H | 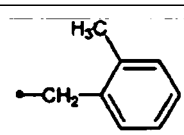 | $OCH_3$ |
| H | H | 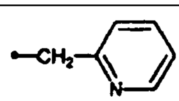 | $OCH_3$ |
| H | H | 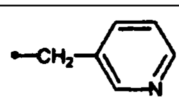 | $OCH_3$ |
| H | H | 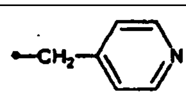 | $OCH_3$ |

Table 59

| $R^1$ | $R^2$ | $R^3$ | M |
|---|---|---|---|
| H | $CH_3$ | $CH_2C(CH_3)=CH_2$ | $OCH_3$ |
| H | $CH_3$ | $CH_2CH=CHCH_3$ | $OCH_3$ |
| H | $CH_3$ | $CH_2CH=C(CH_3)_2$ | $OCH_3$ |
| H | $CH_3$ | $CH_2CCl=CH_2$ | $OCH_3$ |
| H | $CH_3$ | $CH_2CH=CHCl$ | $OCH_3$ |
| H | $CH_3$ | $CH_2CH=CCl_2$ | $OCH_3$ |
| H | $CH_3$ | $CH_2CBr=CH_2$ | $OCH_3$ |
| H | $CH_3$ | $CH_2CH=CHBr$ | $OCH_3$ |
| H | $CH_3$ | $CH_2CH=CBr_2$ | $OCH_3$ |
| H | $CH_3$ | $CH_2CH_2CH=CH_2$ | $OCH_3$ |
| H | CH3 | $CH\ (CH_3)CH=CH_2$ | $OCH_3$ |
| H | $CH_3$ | $CH_2C{\equiv}CCH_3$ | $OCH_3$ |
| H | $CH_3$ | $CH_2C{\equiv}CCl$ | $OCH_3$ |
| H | $CH_3$ | $CH_2C{\equiv}CBr$ | $OCH_3$ |
| H | $CH_3$ | $CH_2CH_2C{\equiv}CH$ | $OCH_3$ |
| H | $CH_3$ | $CH(CH_3)C{\equiv}CH$ | $OCH_3$ |
| H | $CH_3$ | $CH_2CH_2OH$ | $OCH_3$ |
| H | $CH_3$ | $CH(CH_3)CH_2H$ | $OCH_3$ |
| H | $CH_3$ | $CH_2CH\ (CH_3)\ OH$ | $OCH_3$ |

Table 60

| $R^2$ | $R^2$ | $R^3$ | M |
|---|---|---|---|
| H | $CH_3$ | 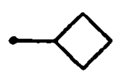 | $OCH_3$ |
| H | $CH_3$ | 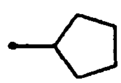 | $OCH_3$ |
| H | $CH_3$ | 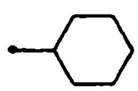 | $OCH_3$ |
| H | $CH_3$ | 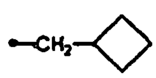 | $OCH_3$ |
| H | $CH_3$ | 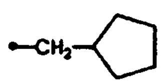 | $OCH_3$ |
| H | $CH_3$ | 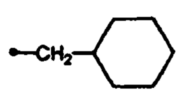 | $OCH_3$ |

(continued)

| R2 | R2 | R3 | M |
|---|---|---|---|
| H | $CH_3$ | 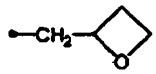 | $OCH_3$ |
| H | $CH_3$ | 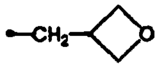 | $OCH_3$ |
| H | $CH_3$ | 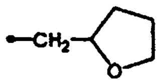 | $OCH_3$ |
| H | $CH_3$ | 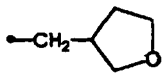 | $OCH_3$ |

Table 61

| $R^1$ | $R^2$ | $R^3$ | M |
|---|---|---|---|
| H | $CH_3$ | 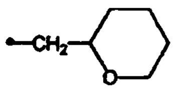 | $OCH_3$ |
| H | $CH_3$ | 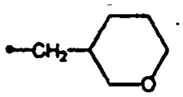 | $OCH_3$ |
| H | $CH_3$ | 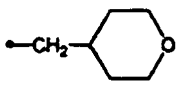 | $OCH_3$ |
| H | $CH_3$ | 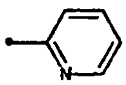 | $OCH_3$ |
| H | $CH_3$ | 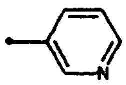 | $OCH_3$ |
| H | $CH_3$ | 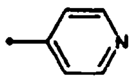 | $OCH_3$ |
| H | $CH_3$ | 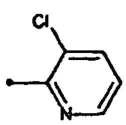 | $OCH_3$ |
| H | $CH_3$ | 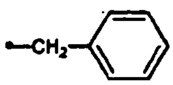 | $OCH_3$ |
| H | $CH_3$ | 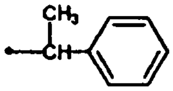 | $OCH_3$ |
| H | $CH_3$ | 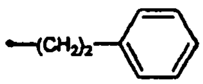 | $OCH_3$ |

Table 62

| $R^2$ | $R^2$ | $R^3$ | M |
|---|---|---|---|
| H | $CH_3$ | 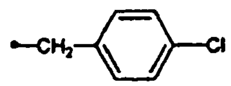 | $OCH_3$ |
| H | $CH_3$ | 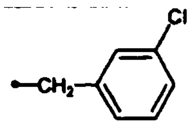 | $OCH_3$ |
| H | $CH_3$ | 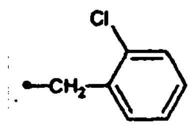 | $OCH_3$ |
| H | $CH_3$ | 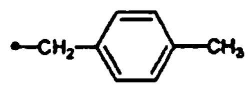 | $OCH_3$ |
| H | $CH_3$ | 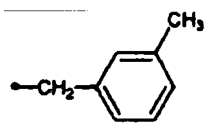 | $OCH_3$ |
| H | $CH_3$ | 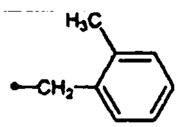 | $OCH_3$ |
| H | $CH_3$ | 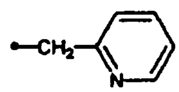 | $OCH_3$ |
| H | $CH_3$ | 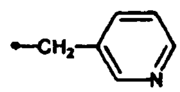 | $OCH_3$ |
| H | $CH_3$ | 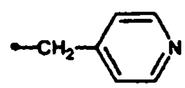 | $OCH_3$ |

Table 63

| $R^1$ | $R^2$ | $R^3$ | M |
|---|---|---|---|
| H | $CH_2CH_3$ | $CH_2C(CH_3)=CH_2$ | $OCH_3$ |
| H | $CH_2CH_3$ | $CH_2CH=CHCH_3$ | $OCH_3$ |
| H | $CH_2CH_3$ | $CH_2CH=C(CH_3)_2$ | $OCH_3$ |
| H | $CH_2CH_3$ | $CH_2CCl=CH_2$ | $OCH_3$ |
| H | $CH_2CH_3$ | $CH_2CH=CHCl$ | $OCH_3$ |
| H | $CH_2CH_3$ | $CH_2CH=CCl_2$ | $OCH_3$ |
| H | $CH_2CH_3$ | $CH_2CBr=CH_2$ | $OCH_3$ |
| H | $CH_2CH_3$ | $CH_2CH=CHBr$ | $OCH_3$ |
| H | $CH_2CH_3$ | $CH_2CH-CBr_2$ | $OCH_3$ |
| H | $CH_2CH_3$ | $CH_2CH_2CH=CH_2$ | $OCH_3$ |

(continued)

| $R^1$ | $R^2$ | $R^3$ | M |
|---|---|---|---|
| H | $CH_2CH_3$ | $CH(CH_3)CH{=}CH_2$ | $CH_3$ |
| H | $CH_2CH_3$ | $CH_2C{\equiv}CCH_3$ | $OCH_3$ |
| H | $CH_2CH_3$ | $CH_2C{\equiv}CCl$ | $OCH_3$ |
| H | $CH_2CH_3$ | $CH_2C{\equiv}CBr$ | $OCH_3$ |
| H | $CH_2CH_3$ | $CH_2CH_2C{\equiv}CH$ | $OCH_3$ |
| H | $CH_2CH_3$ | $CH(CH_3)C{\equiv}CH$ | $OCH_3$ |
| H | $CH_2CH_3$ | $CH_2CH_2OH$ | $OCH_3$ |
| H | $CH_2CH_3$ | $CH\ (CH_3)\ CH_2OH$ | $OCH_3$ |
| H | $CH_2CH_3$ | $CH_2CH(CH_3)OH$ | $OCH_3$ |

Table 64

| $R^1$ | $R^2$ | $R^3$ | M |
|---|---|---|---|
| H | $CH_2CH_3$ | 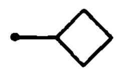 | $OCH_3$ |
| H | $CH_2CH_3$ | 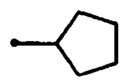 | $OCH_3$ |
| H | $CH_2CH_3$ | 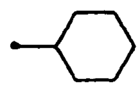 | $OCH_3$ |
| H | $CH_2CH_3$ | 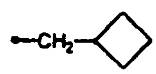 | $OCH_3$ |
| H | $CH_2CH_3$ | 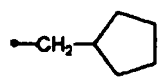 | $OCH_3$ |
| H | $CH_2CH_3$ | 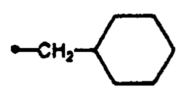 | $OCH_3$ |
| H | $CH_2CH_3$ | 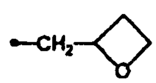 | $OCH_3$ |
| H | $CH_2CH_3$ | 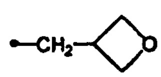 | $OCH_3$ |
| H | $CH_2CH_3$ | 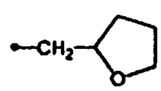 | $OCH_3$ |
| H | $CH_2CH_3$ | 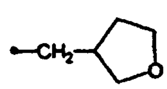 | $OCH_3$ |

Table 65

| $R^1$ | $R^2$ | $R^3$ | M |
|---|---|---|---|
| H | $CH_2CH_3$ | 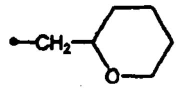 | $OCH_3$ |
| H | $CH_2CH_3$ | 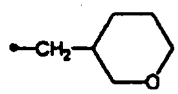 | $OCH_3$ |
| H | $CH_2CH_3$ | 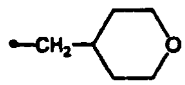 | $OCH_3$ |
| H | $CH_2CH_3$ | 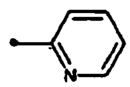 | $OCH_3$ |
| H | $CH_2CH_3$ | 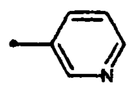 | $OCH_3$ |
| H | $CH_2CH_3$ | 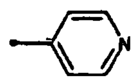 | $OCH_3$ |
| H | $CH_2CH_3$ | 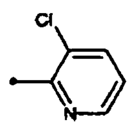 | $OCH_3$ |
| H | $CH_2CH_3$ | 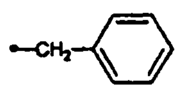 | $OCH_3$ |
| H | $CH_2CH_3$ | 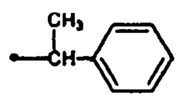 | $OCH_3$ |
| H | $CH_2CH_3$ | 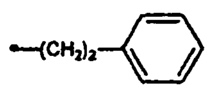 | $OCH_3$ |

Table 66

| $R^1$ | $R^2$ | $R^3$ | M |
|---|---|---|---|
| H | $CH_2cH_3$ | 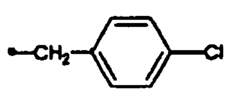 | $OCH_3$ |
| H | $CH_2CH_3$ | 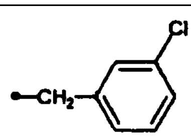 | $OCH_3$ |
| H | $CH_2CH_3$ | 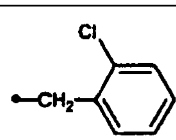 | $OCH_3$ |

(continued)

| $R^1$ | $R^2$ | $R^3$ | M |
|---|---|---|---|
| H | $CH_2CH_3$ | 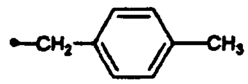 | $OCH_3$ |
| H | $CH_2CH_3$ | 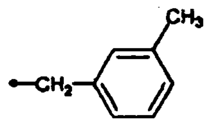 | $OCH_3$ |
| H | $CH_2CH_3$ | 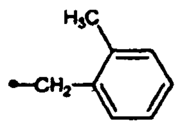 | $OCH_3$ |
| H | $CH_2CH_3$ | 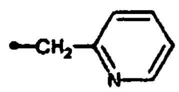 | $OCH_3$ |
| H | $CH_2CH_3$ | 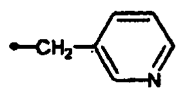 | $OCH_3$ |
| H | $CH_2CH_3$ | 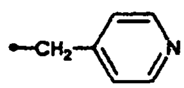 | $OCH_3$ |

Table 67

| $R^1$ | $R^2$ | $R^3$ | M |
|---|---|---|---|
| H | $CH_2CH=CH_2$ | H | $CH_3$ |
| H | $CH_2C\equiv CH$ | H | $CH_3$ |
| H | Cyclopropyl | H | $CH_3$ |
| H | $CH_2$ (cyclopropyl) | H | $CH_3$ |
| H | CH ($CH_3$) (cyclopropyl) | H | $CH_3$ |
| H | $CH_2CN$ | H | $CH_3$ |
| H | $CH(CH_3)CN$ | H | $CH_3$ |
| H | H | $CH_2CH=CH_2$ | $CH_3$ |
| H | H | $CH_2C\equiv CH$ | $CH_3$ |
| H | H | Cyclopropyl | $CH_3$ |
| H | H | $CH_2$(cyclopropyl) | $CH_3$ |
| H | H | $CH(CH_3)$ (cyclopropyl) | $CH_3$ |
| H | H | $CH_2CN$ | $CH_3$ |
| H | H | CH ($CH_3$) CN | $CH_3$ |
| H | $CH_2CH=CH_2$ | $CH_3$ | $CH_3$ |
| H | $CH_2C\equiv CH$ | $CH_3$ | $CH_3$ |
| H | Cyclopropyl | $CH_3$ | $CH_3$ |
| H | $CH_2$(cyclopropyl) | $CH_3$ | $CH_3$ |

(continued)

| $R^1$ | $R^2$ | $R^3$ | M |
|---|---|---|---|
| H | $CH(CH_3)$ (cyclopropyl) | $CH_3$ | $CH_3$ |
| H | $CH_2CN$ | $CH_3$ | $CH_3$ |
| H | $CH(CH_3)CN$ | $CH_3$ | $CH_3$ |
| H | $CH_3$ | $CH_2CH=CH_2$ | $CH_3$ |
| H | $CH_3$ | $CH_2C\equiv CH$ | $CH_3$ |
| H | $CH_3$ | Cyclopropyl | $CH_3$ |
| H | $CH_3$ | $CH_2$ (cyclopropyl) | $CH_3$ |
| H | $CH_3$ | $CH(CH_3)$ (cyclopropyl) | $CH_3$ |
| H | $CH_3$ | $CH_2CN$ | $CH_3$ |
| H | $CH_3$ | $CH(CH_3)CN$ | $CH_3$ |

Table 68

| $R^1$ | $R^2$ | $R^3$ | M |
|---|---|---|---|
| H | $CH_2CH=CH_2$ | H | $OCH_2CH_3$ |
| H | $CH_2C\equiv CH$ | H | $OCH_2CH_3$ |
| H | Cyclopropyl | H | $OCH_2CH_3$ |
| H | $CH_2$(cyclopropyl) | H | $OCH_2CH_3$ |
| H | $CH(CH_3)$ (cyclopropyl) | H | $OCH_2CH_3$ |
| H | $CH_2CN$ | H | $OCH_2CH_3$ |
| H | CH $(CH_3)$ CN | H | $OCH_2CH_3$ |
| H | H | $CH_2CH=CH_2$ | $OCH_2CH_3$ |
| H | H | $CH_2C\equiv CH$ | $OCH_2CH_3$ |
| H | H | Cyclopropyl | $OCH_2CH_3$ |
| H | H | $CH_2$(cyclopropyl) | $OCH_2CH_3$ |
| H | H | $CH(CH_3)$ (cyclopropyl) | $OCH_2CH_3$ |
| H | H | $CH_2CN$ | $OCH_2CH_3$ |
| H | H | CH $(CH_3)$ CN | $OCH_2CH_3$ |
| H | $CH_2CH=CH_2$ | $CH_3$ | $OCH_2CH_3$ |
| H | $CH_2C\equiv CH$ | $CH_3$ | $OCH_2CH_3$ |
| H | Cyclopropyl | $CH_3$ | $OCH_2CH_3$ |
| H | $CH_2$(cyclopropyl) | $CH_3$ | $OCH_2CH_3$ |
| H | $CH(CH_3)$ (cyclopropyl) | $CH_3$ | $OCH_2CH_3$ |
| H | $CH_2CN$ | $CH_3$ | $OCH_2CH_3$ |
| H | CH $(CH_3)CN$ | $CH_3$ | $OCH_2CH_3$ |
| H | $CH_3$ | $CH_2CH=CH_2$ | $OCH_2CH_3$ |
| H | $CH_3$ | $CH_2C\equiv CH$ | $OCH_2CH_3$ |
| H | $CH_3$ | Cyclopropyl | $OCH_2CH_3$ |

(continued)

| $R^1$ | $R^2$ | $R^3$ | M |
|---|---|---|---|
| H | $CH_3$ | $CH_2$ (cyclopropyl) | $OCH_2CH_3$ |
| H | $CH_3$ | $CH(CH_3)$(cyclopropyl) | $OCH_2CH_3$ |
| H | $CH_3$ | $CH_2CN$ | $OCH_2CH_3$ |
| H | $CH_3$ | $CH(CH_3)CN$ | $OCH_2CH_3$ |

A compound represented by the formula (A-2):

[Chemical Formula 44]

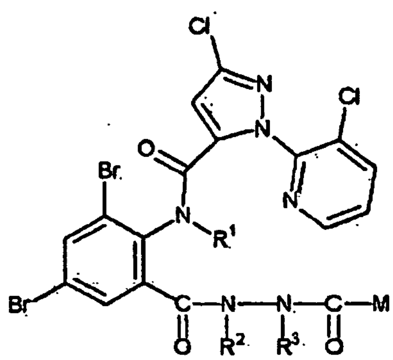

(A-2)

$R^1$, $R^2$, $R^3$ and M in the formula represent the combinations described in Table 1 to Table 68.

A compound represented by the formula (A-3):

[Chemical Formula 45]

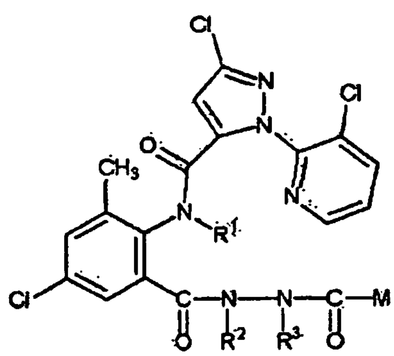

(A-3)

$R^1$, $R^2$, $R^3$ and M in the formula represent the combinations described in Table 1 to Table 68.

A compound represented by the formula (A-4):

[Chemical Formula 46]

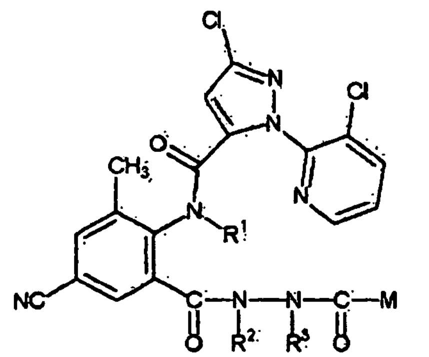

(A-4)

$R^1$, $R^2$, $R^3$ and M in the formula represent the combinations described in Table 1 to Table 68.

A compound represented by the formula (A-5):

[Chemical Formula 47]

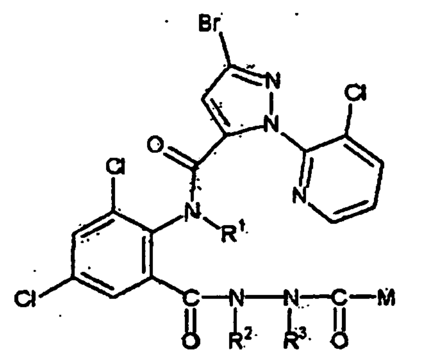

(A-5)

$R^1$, $R^2$, $R^3$ and M in the formula represent the combinations described in Table 1 to Table 68.

A compound represented by the formula (A-6):

[Chemical Formula 48]

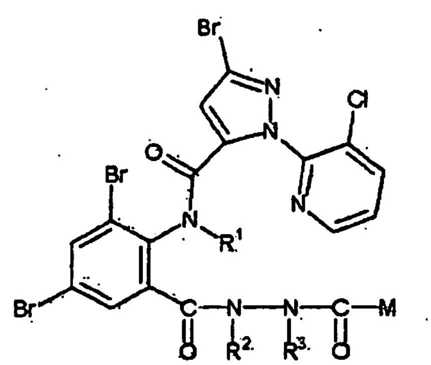

(A-6)

$R^1$, $R^2$, $R^3$ and M in the formula represent the combinations described in Table 1 to Table 68.

A compound represented by the formula (A-7):

[Chemical Formula 49]

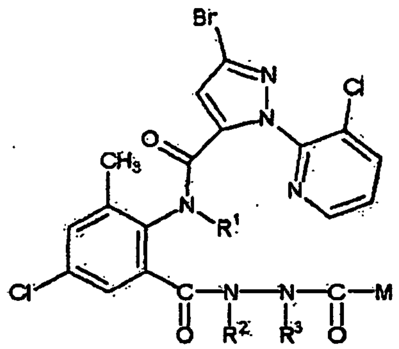

(A-7)

$R^1$, $R^2$, $R^3$ and M in the formula represent the combinations described in Table 1 to Table 68.

A compound represented by the formula (A-8):

[Chemical Formula 50]

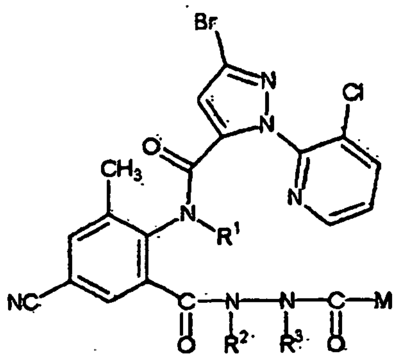

(A-8)

$R^1$, $R^2$, $R^3$ and M in the formula represent the combinations described in Table 1 to Table 68.

A compound represented by the formula (A-9):

[Chemical Formula 51]

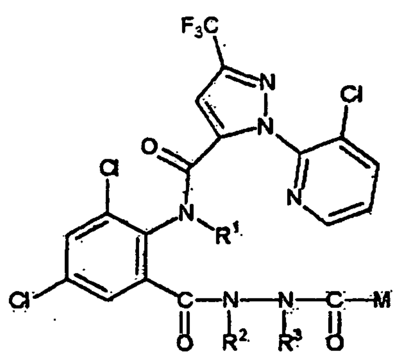

(A-9)

$R^1$, $R^2$, $R^3$ and M in the formula represent the combinations described in Table 1 to Table 68.

A compound represented by the formula (A-10):

[Chemical Formula 52]

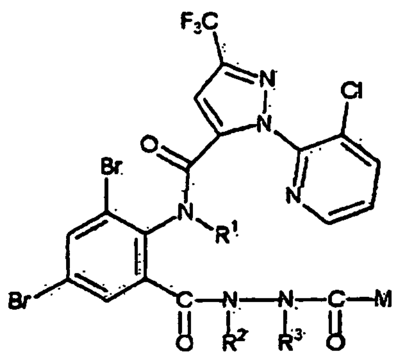

(A-10)

$R^1$, $R^2$, $R^3$ and M in the formula represent the combinations described in Table 1 to Table 68.

A compound represented by the formula (A-11):

[Chemical Formula 53]

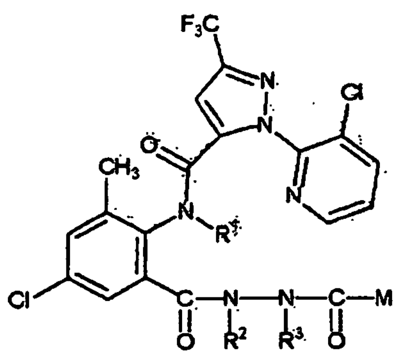

(A-11)

$R^1$, $R^2$, $R^3$ and M in the formula represent the combinations described in Table 1 to Table 68.

A compound represented by the formula (A-12):

[Chemical Formula 54]

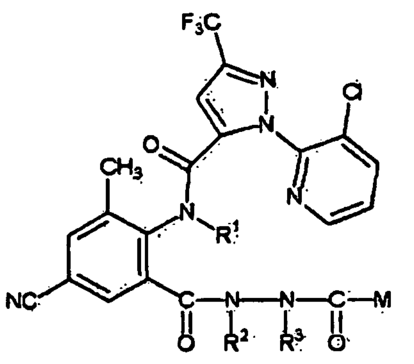

(A-12)

$R^1$, $R^2$, $R^3$ and M in the formula represent the combinations described in Table 1 to Table 68.

A compound represented by the formula (A-13):

[Chemical Formula 55]

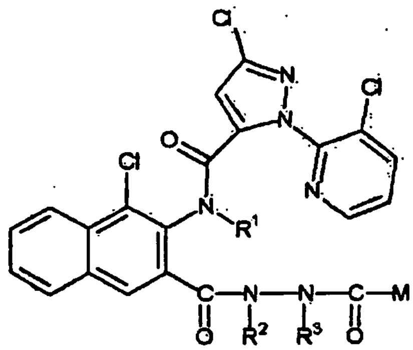

(A-13)

$R^1$, $R^2$, $R^3$ and M in the formula represent the combinations described in Table 1 to Table 68.

A compound represented by the formula (A-14):

[Chemical Formula 56]

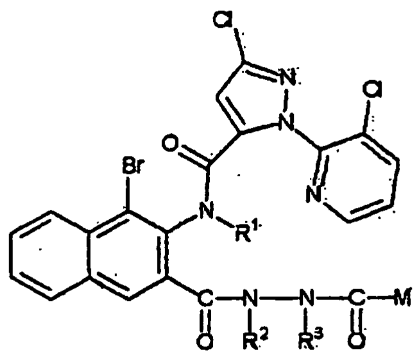

(A-14)

$R^1$, $R^2$, $R^3$ and M in the formula represent the combinations described in Table 1 to Table 68.

A compound represented by the formula (A-15):

[Chemical Formula 57]

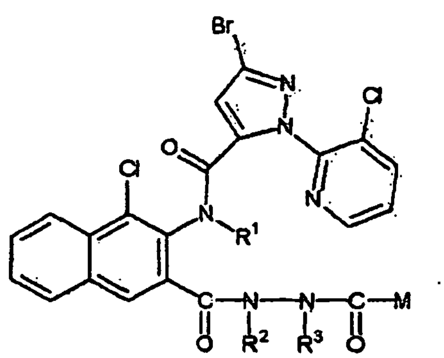

(A-15)

$R^1$, $R^2$, $R^3$ and M in the formula represent the combinations described in Table 1 to Table 68.

A compound represented by the formula (A-16):

[Chemical Formula 58]

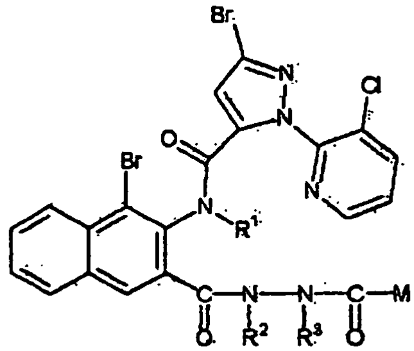

(A-16)

$R^1$, $R^2$ , $R^3$ and M in the formula represent the combinations described in Table 1 to Table 68.

A compound represented by the formula (A-17):

[Chemical Formula 59]

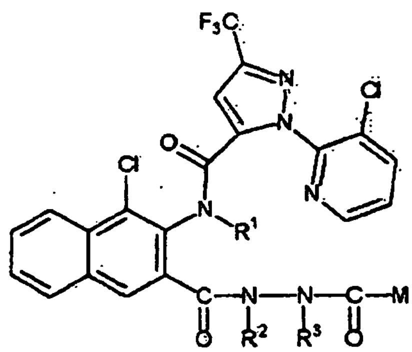

(A-17)

$R^1$, $R^2$, $R^3$ and M in the formula represent the combinations described in Table 1 to Table 68.

A compound represented by the formula (A-18):

[Chemical Formula 60]

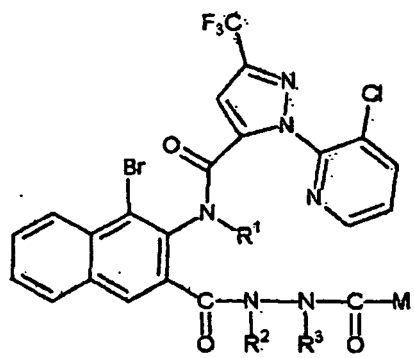

(A-18)

$R^1$, $R^2$ , $R^3$ and M in the formula represent the combinations described in Table 1 to Table 68.

A compound represented by the formula (A-19):

[Chemical Formula 61]

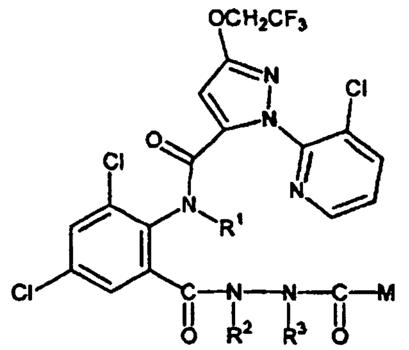

(A-19)

$R^1$, $R^2$, $R^3$ and M in the formula represent the combinations described in Table 1 to Table 68.

A compound represented by the formula (A-20):

[Chemical Formula 62]

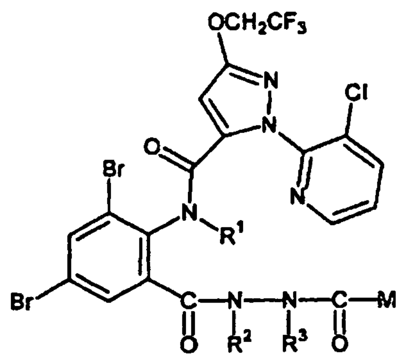

(A-20)

$R^1$, $R^2$, $R^3$ and M in the formula represent the combinations described in Table 1 to Table 68.

A compound represented by the formula (A-21):

[Chemical Formula 63]

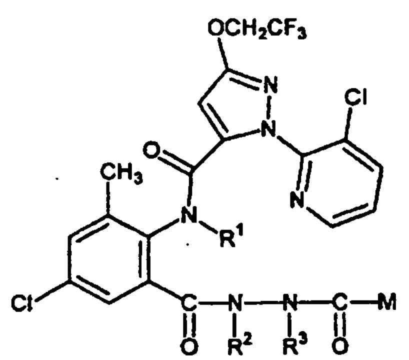

(A-21)

$R^1$, $R^2$, $R^3$ and M in the formula represent the combinations described in Table 1 to Table 68.

A compound represented by the formula (A-22):

[Chemical Formula 64]

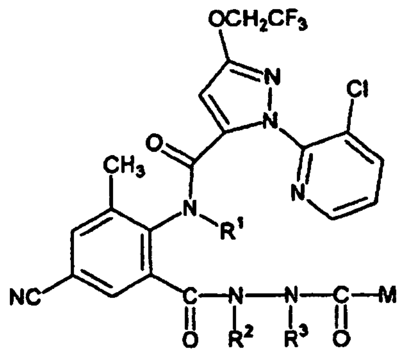

(A-22)

$R^1$, $R^2$, $R^3$ and M in the formula represent the combinations described in Table 1 to Table 68.

A compound represented by the formula (A-23):

[Chemical Formula 65]

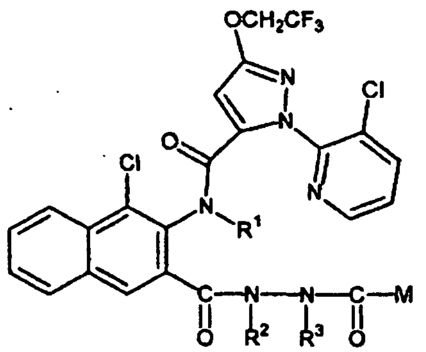

(A-23)

$R^1$, $R^2$, $R^3$ and M in the formula represent the combinations described in Table 1 to Table 68.

A compound represented by the formula (A-24):

[Chemical Formula 66]

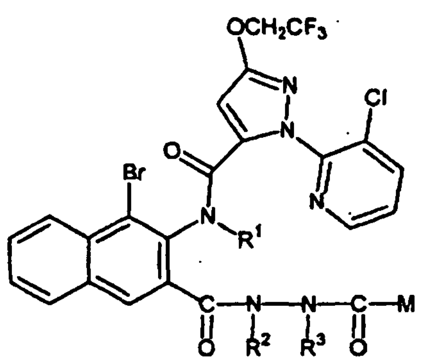

(A-24)

$R^1$, $R^2$, $R^3$ and M in the formula represent the combinations described in Table 1 to Table 68.

A compound represented by the formula (A-25):

[Chemical Formula 67]

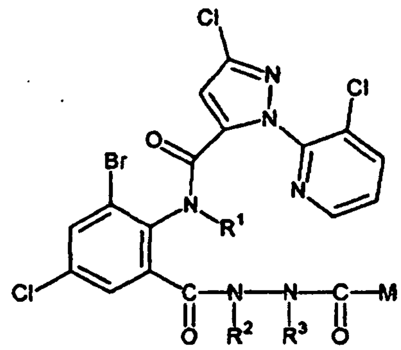

(A-25)

$R^1$, $R^2$, $R^3$ and M in the formula represent the combinations described in Table 1 to Table 68.

A compound represented by the formula (A-26):

[Chemical Formula 68]

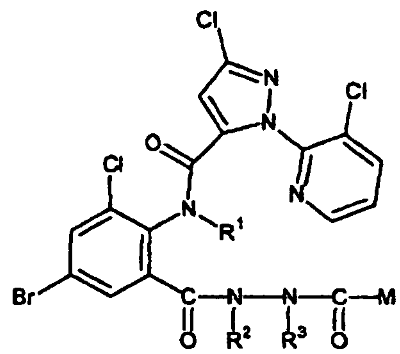

(A-26)

$R^1$, $R^2$, $R^3$ and M in the formula represent the combinations described in Table 1 to Table 68.

A compound represented by the formula (A-27):

[Chemical Formula 69]

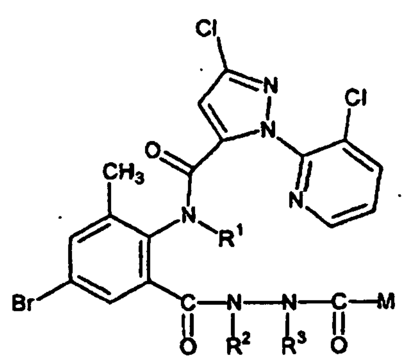

(A-27)

$R^1$, $R^2$, $R^3$ and M in the formula represent the combinations described in Table 1 to Table 68.

A compound represented by the formula (A-28):

[Chemical Formula 70]

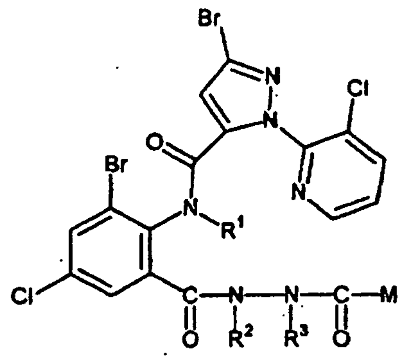

(A-28)

$R^1$, $R^2$, $R^3$ and M in the formula represent the combinations described in Table 1 to Table 68.

A compound represented by the formula (A-29):

[Chemical Formula 71]

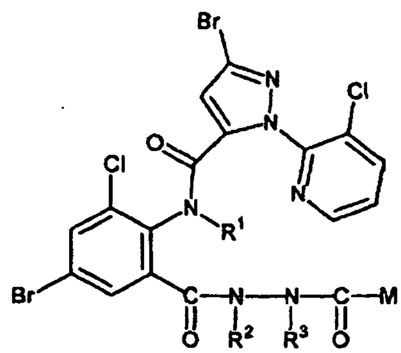

(A-29)

$R^1$, $R^2$, $R^3$ and M in the formula represent the combinations described in Table 1 to Table 68.

A compound represented by the formula (A-30):

[Chemical Formula 72]

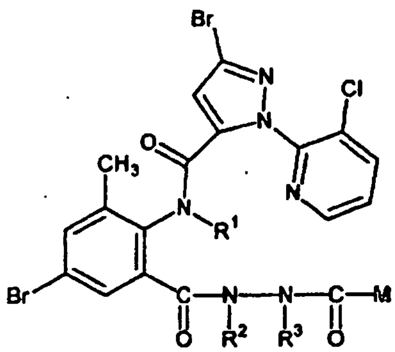

(A-30)

$R^1$, $R^2$, $R^3$ and M in the formula represent the combinations described in Table 1 to Table 68.

A compound represented by the formula (A-31):

[Chemical Formula 73]

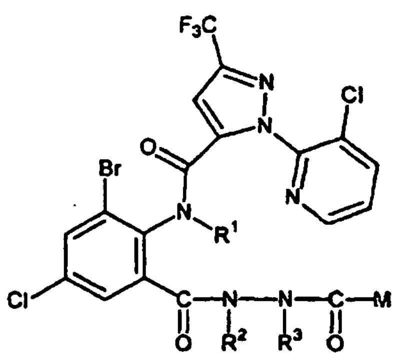

(A-31)

$R^1$, $R^2$ , $R^3$ and M in the formula represent the combinations described in Table 1 to Table 68.

A compound represented by the formula (A-32):

[Chemical Formula 74]

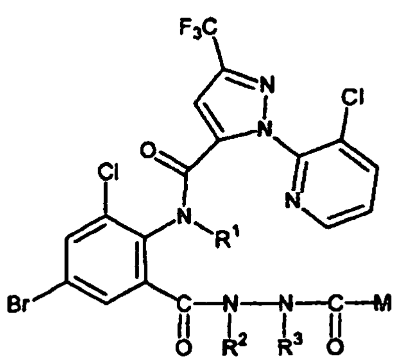

(A-32)

$R^1$, $R^2$, $R^3$ and M in the formula represent the combinations described in Table 1 to Table 68.

A compound represented by the formula (A-33):

[Chemical Formula 75]

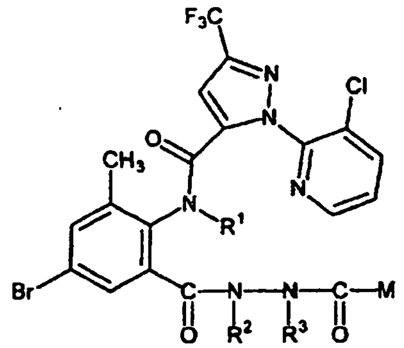

(A-33)

$R^1$, $R^2$, $R^3$ and M in the formula represent the combinations described in Table 1 to Table 68.

A compound represented by the formula (A-34):

[Chemical Formula 76]

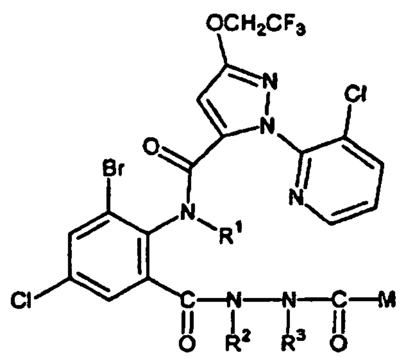

(A-34)

$R^1$, $R^2$, $R^3$ and M in the formula represent the combinations described in Table 1 to Table 68.

A compound represented by the formula (A-35):

[Chemical Formula 77]

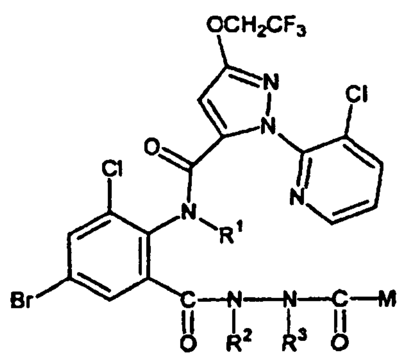

(A-35)

$R^1$, $R^2$, $R^3$ and M in the formula represent the combinations described in Table 1 to Table 68.

A compound represented by the formula (A-36):

[Chemical Formula 78]

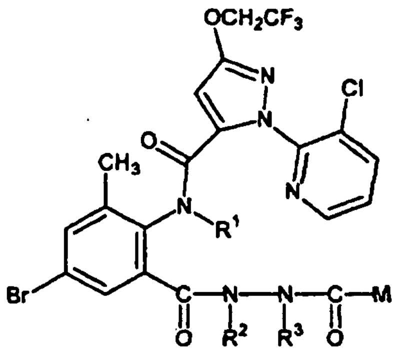

(A-36)

$R^1$, $R^2$, $R^3$ and M in the formula represent the combinations described in Table 1 to Table 68.

A compound represented by the formula (B-1):

[Chemical Formula 79]

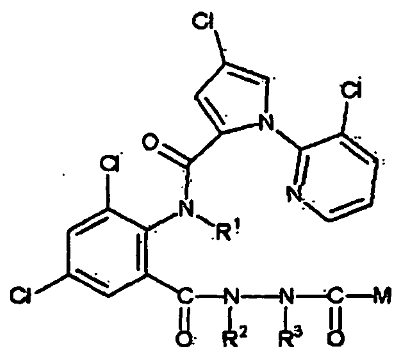

(B-1)

$R^1$, $R^2$, $R^3$ and M in the formula represent the combinations described in Table 1 to Table 68.

A compound represented by the formula (B-2):

[Chemical Formula 80]

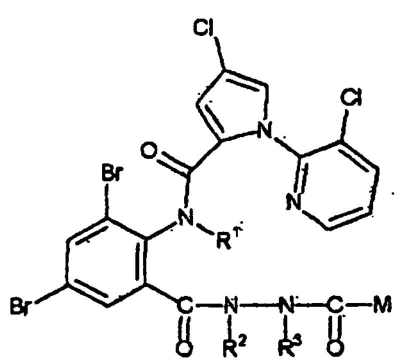

(B-2)

$R^1$, $R^2$, $R^3$ and M in the formula represent the combinations described in Table 1 to Table 68.

A compound represented by the formula (B-3):

[Chemical Formula 81]

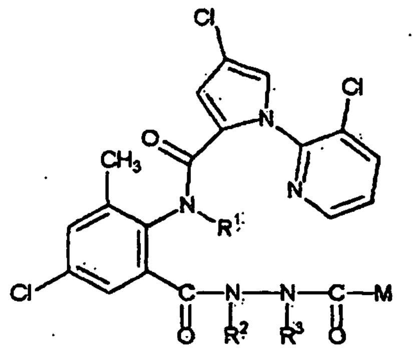

(B-3)

$R^1$, $R^2$, $R^3$ and M in the formula represent the combinations described in Table 1 to Table 68.

A compound represented by the formula (B-4):

[Chemical Formula 82]

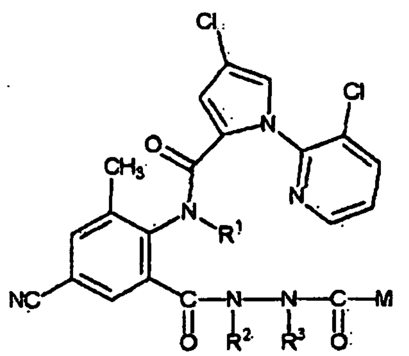

(B-4)

$R^1$, $R^2$, $R^3$ and M in the formula represent the combinations described in Table 1 to Table 68.

A compound represented by the formula (B-5):

[Chemical Formula 83]

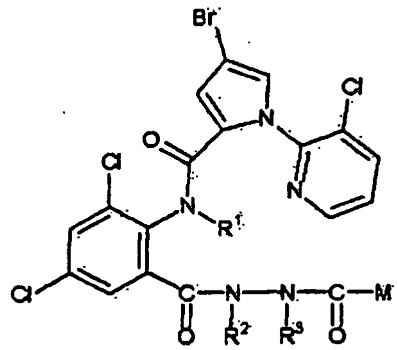

(B-5)

$R^1$, $R^2$, $R^3$ and M in the formula represent the combinations described in Table 1 to Table 68.

A compound represented by the formula (B-6):

[Chemical Formula 84]

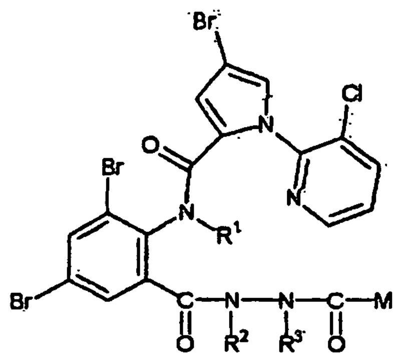

(B-6)

$R^1$, $R^2$, $R^3$ and M in the formula represent the combinations described in Table 1 to Table 68.

A compound represented by the formula (B-7):

[Chemical Formula 85]

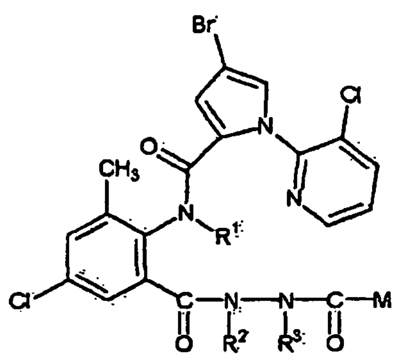

(B-7)

$R^1$, $R^2$ , $R^3$ and M in the formula represent the combinations described in Table 1 to Table 68.

A compound represented by the formula (B-8):

[Chemical Formula 86]

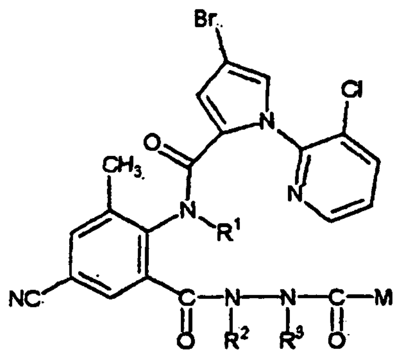

(B-8)

$R^1$, $R^2$, $R^3$ and M in the formula represent the combinations described in Table 1 to Table 68.

A compound represented by the formula (B-9):

[Chemical Formula 87]

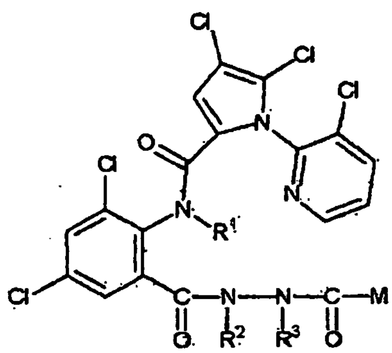

(B-9)

$R^1$, $R^2$, $R^3$ and M in the formula represent the combinations described in Table 1 to Table 68.

A compound represented by the formula (B-10):

[Chemical Formula 88]

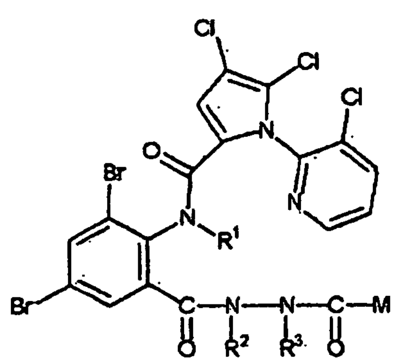

(B-10)

$R^1$, $R^2$, $R^3$ and M in the formula represent the combinations described in Table 1 to Table 68.

A compound represented by the formula (B-11):

[Chemical Formula 89]

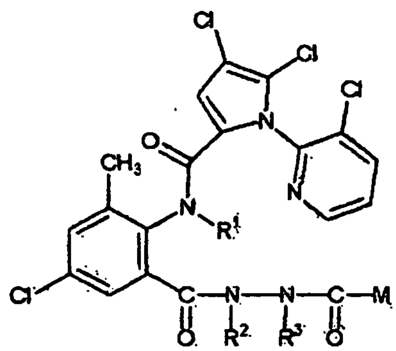

(B-11)

$R^1$, $R^2$, $R^3$ and M in the formula represent the combinations described in Table 1 to Table 68.

A compound represented by the formula (B-12):

[Chemical Formula 90]

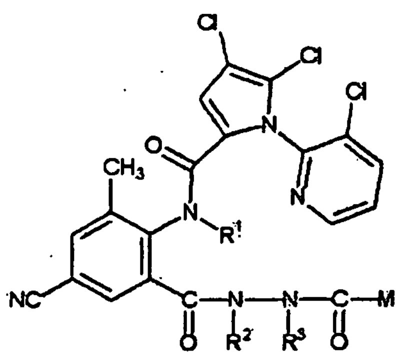

(B-12)

$R^1$, $R^2$, $R^3$ and M in the formula represent the combinations described in Table 1 to Table 68.

A compound represented by the formula (B-13):

[Chemical Formula 91]

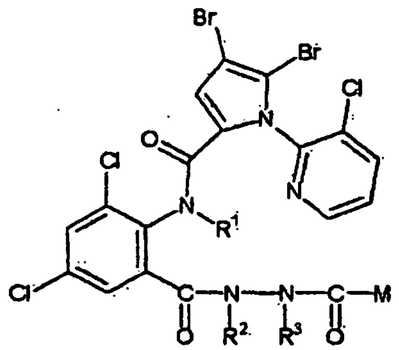

(B-13)

$R^1$, $R^2$, $R^3$ and M in the formula represent the combinations described in Table 1 to Table 68.

A compound represented by the formula (B-14):

[Chemical Formula 92]

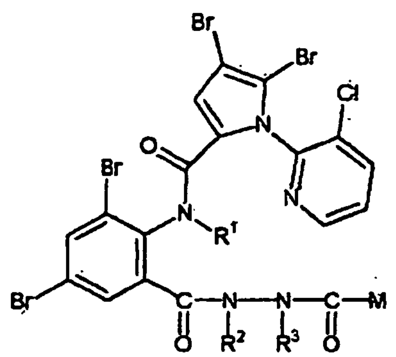

(B-14)

$R^1$, $R^2$, $R^3$ and M in the formula represent the combinations described in Table 1 to Table 68.

A compound represented by the formula (B-15):

[Chemical Formula 93]

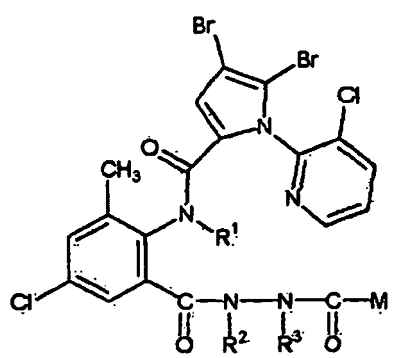

(B-15)

$R^1$, $R^2$, $R^3$ and M in the formula represent the combinations described in Table 1 to Table 68.

A compound represented by the formula (B-16):

[Chemical Formula 94]

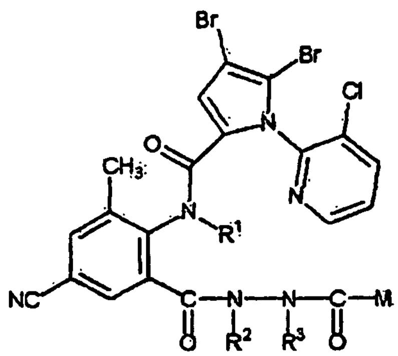

(B-16)

$R^1$, $R^2$, $R^3$ and M in the formula represent the combinations described in Table 1 to Table 68.

A compound represented by the formula (C-1):

[Chemical Formula 95]

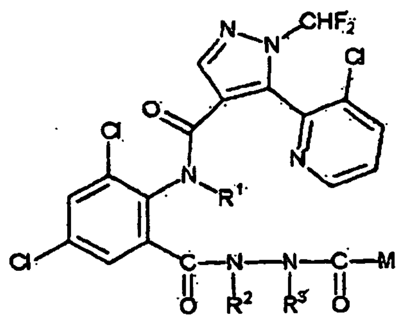

(C-1)

$R^1$, $R^2$, $R^3$ and M in the formula represent the combinations described in Table 1 to Table 68.

A compound represented by the formula (C-2):

[Chemical Formula 96]

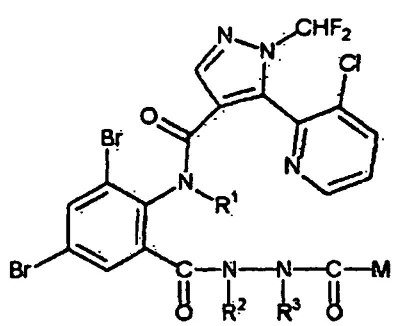

(C-2)

$R^1$, $R^2$, $R^3$ and M in the formula represent the combinations described in Table 1 to Table 68.

A compound represented by the formula (C-3):

[Chemical Formula 97]

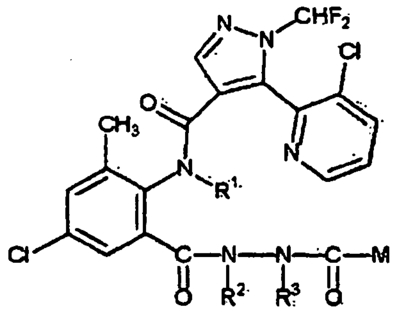

(C-3)

$R^1$, $R^2$, $R^3$ and M in the formula represent the combinations described in Table 1 to Table 68.

A compound represented by the formula (C-4):

[Chemical Formula 98]

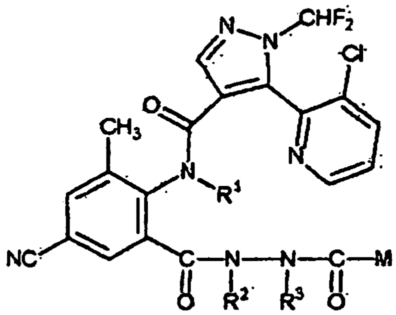

(C-4)

$R^1$, $R^2$ , $R^3$ and M in the formula represent the combinations described in Table 1 to Table 68.

A compound represented by the formula (C-5):

[Chemical Formula 99]

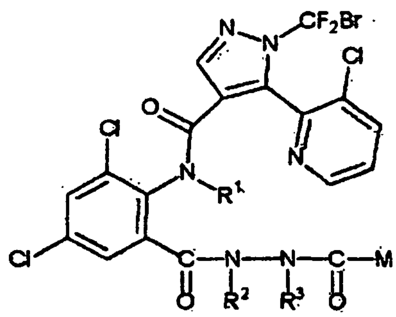

(C-5)

$R^1$, $R^2$, $R^3$ and M in the formula represent the combinations described in Table 1 to Table 68.

A compound represented by the formula (C-6):

[Chemical Formula 100]

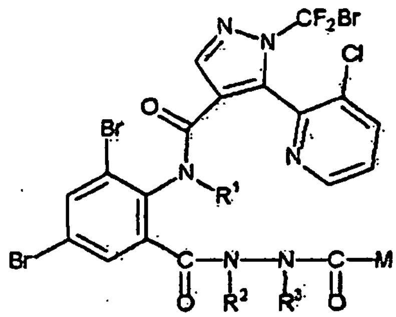

(C-6)

$R^1$, $R^2$, $R^3$ and M in the formula represent the combinations described in Table 1 to Table 68.

A compound represented by the formula (C-7):

[Chemical Formula 101]

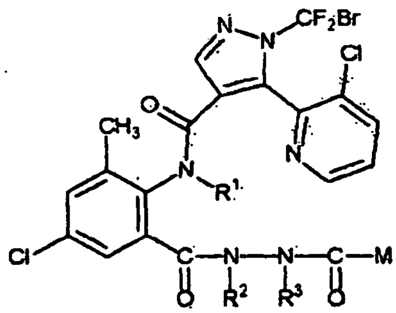

(C-7)

$R^1$, $R^2$, $R^3$ and M in the formula represent the combinations described in Table 1 to Table 68.

A compound represented by the formula (C-8):

[Chemical Formula 102]

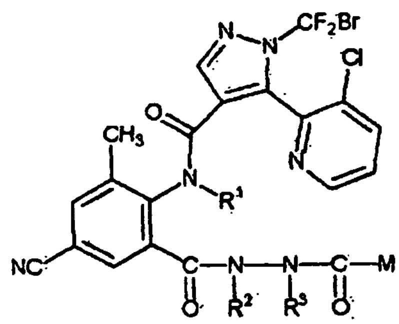

(C-8)

$R^1$, $R^2$, $R^3$ and M in the formula represent the combinations described in Table 1 to Table 68.

A compound represented by the formula (C-9):

[Chemical Formula 103]

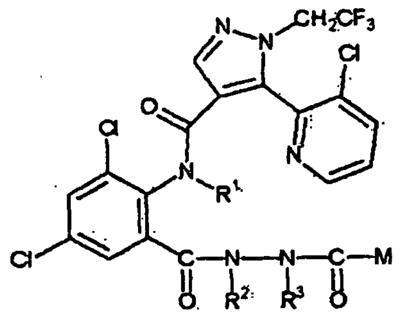

(C-9)

$R^1$, $R^2$, $R^3$ and M in the formula represent the combinations described in Table 1 to Table 68.

A compound represented by the formula (C-10):

[Chemical Formula 104]

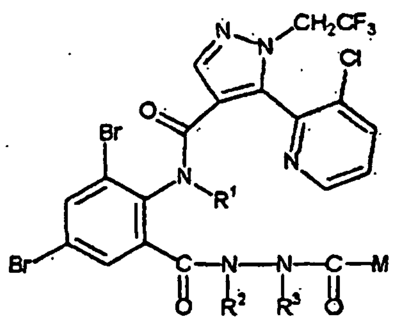

(C-10)

$R^1$, $R^2$, $R^3$ and M in the formula represent the combinations described in Table 1 to Table 68.

A compound represented by the formula (C-11):

[Chemical Formula 105]

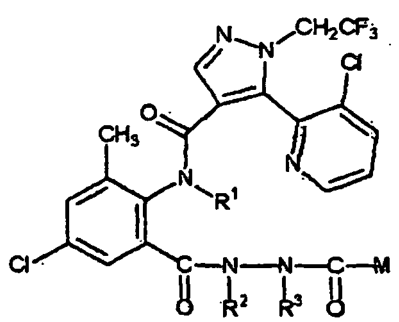

(C-11)

$R^1$ , $R^2$ , $R^3$ and M in the formula represent the combinations described in Table 1 to Table 68.

A compound represented by the formula (C-12):

[Chemical Formula 106]

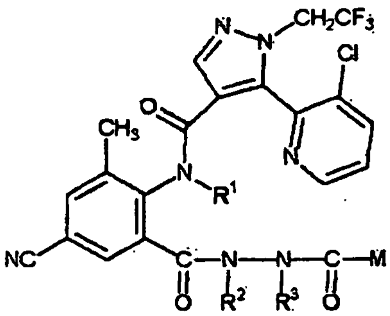

(C-12)

$R^1$, $R^2$, $R^3$ and M in the formula represent the combinations described in Table 1 to Table 68.

A compound represented by the formula (D-1):

[Chemical Formula 107]

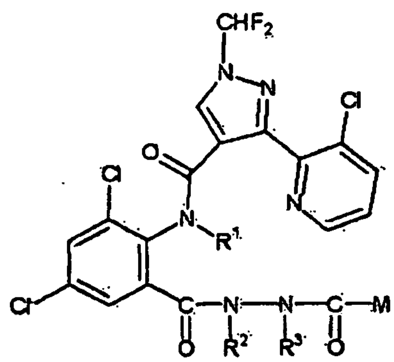

(D-1)

$R^1$, $R^2$, $R^3$ and M in the formula represent the combinations described in Table 1 to Table 68.

A compound represented by the formula (D-2):

[Chemical Formula 108]

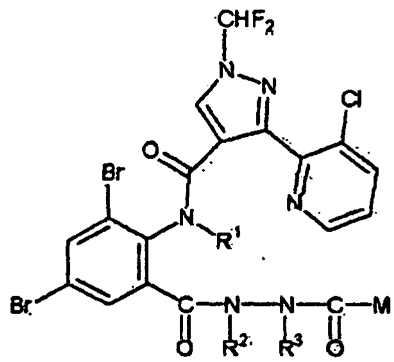

(D-2)

$R^1$, $R^2$, $R^3$ and M in the formula represent the combinations described in Table 1 to Table 68.

A compound represented by the formula (D-3):

[Chemical Formula 109]

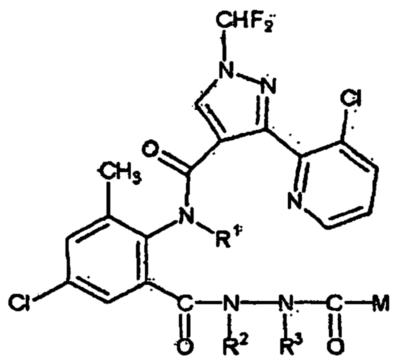

(D-3)

$R^1$, $R^2$, $R^3$ and M in the formula represent the combinations described in Table 1 to Table 68.

A compound represented by the formula (D-4):

[Chemical Formula 110]

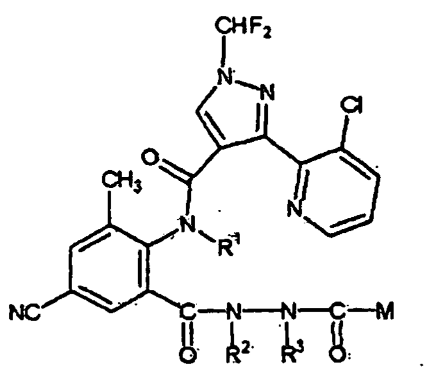

(D-4)

$R^1$, $R^2$, $R^3$ and M in the formula represent the combinations described in Table 1 to Table 68.

A compound represented by the formula (D-5):

[Chemical Formula 111]

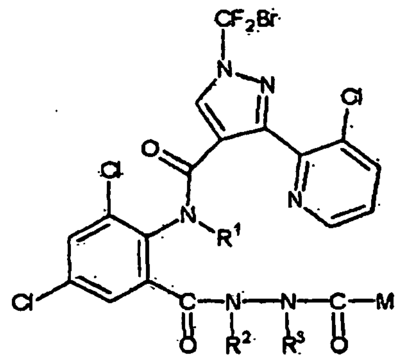

(D-5)

$R^1$, $R^2$, $R^3$ and M in the formula represent the combinations described in Table 1 to Table 68.

A compound represented by the formula (D-6):

[Chemical Formula 112]

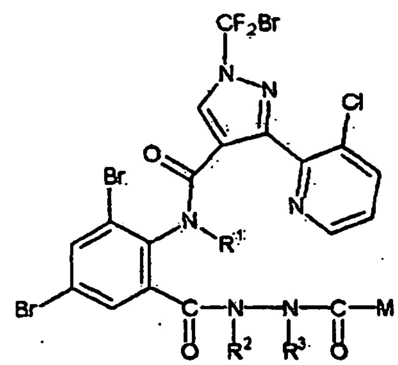

(D-6)

$R^1$, $R^2$, $R^3$ and M in the formula represent the combinations described in Table 1 to Table 68.

A compound represented by the formula (D-7):

[Chemical Formula 113]

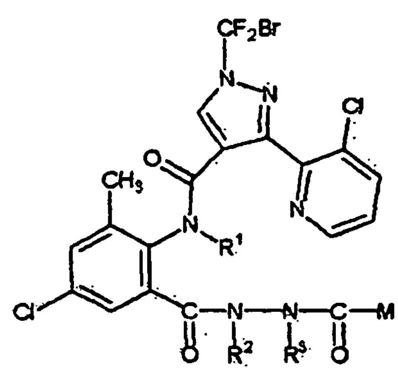

(D-7)

$R^1$, $R^2$, $R^3$ and M in the formula represent the combinations described in Table 1 to Table 68.

A compound represented by the formula (D-8):

[Chemical Formula 114]

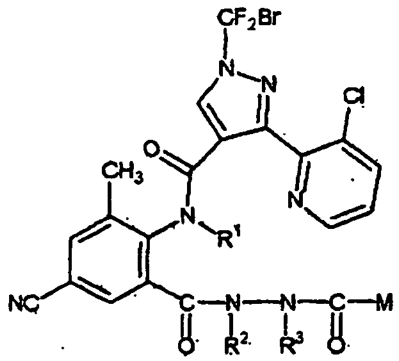

(D-8)

$R^1$, $R^2$, $R^3$ and M in the formula represent the combinations described in Table 1 to Table 68.

A compound represented by the formula (D-9):

[Chemical Formula 115]

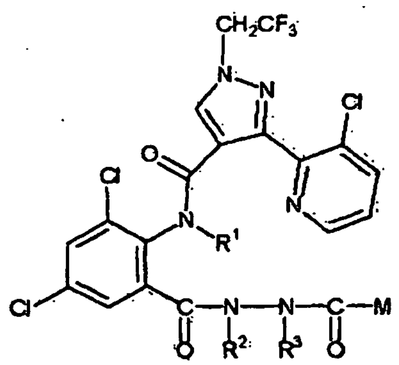

(D-9)

$R^1$, $R^2$, $R^3$ and M in the formula represent the combinations described in Table 1 to Table 68.

A compound represented by the formula (D-10):

[Chemical Formula 116]

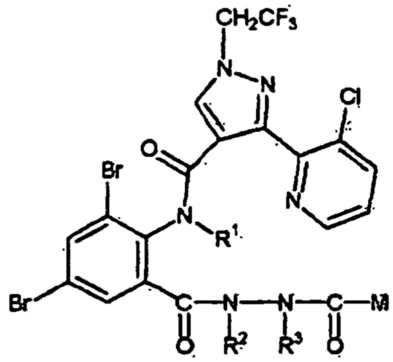

(D-10)

$R^1$, $R^2$, $R^3$ and M in the formula represent the combinations described in Table 1 to Table 68.

A compound represented by the formula (D-11):

[Chemical Formula 117]

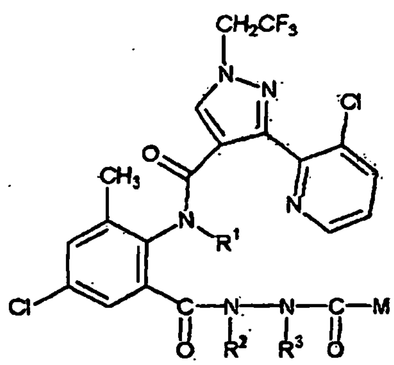

(D-11)

$R^1$, $R^2$, $R^3$ and M in the formula represent the combinations described in Table 1 to Table 68.

A compound represented by the formula (D-12):

[Chemical Formula 118]

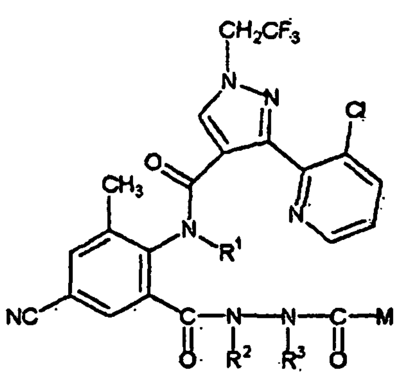

(D-12)

$R^1$, $R^2$, $R^3$ and M in the formula represent the combinations described in Table 1 to Table 68.

Harmful arthropods to which the harmful arthropod controlling agent containing the present compound as an active ingredient exhibits a controlling effect include, for example, harmful insects and harmful acarids, and specific examples thereof include the followings.

Hemiptera:

Planthoppers (Delphacidae) such as small brown planthopper (*Laodelphax striatellus*), brown rice planthopper (*Nilaparvata lugens*), and white-backed rice planthopper (*Sogatella furcifera*); leafhoppers (Deltocephalidae) such as green rice leafhopper (*Nephotettix cincticeps),* green rice leafhopper (*Nephotettix virescens),* and tea green leafhopper (*Empoasca onukii*); aphids (Aphididae) such as cotton aphid (*Aphis gossypii*), green peach aphid (*Myzus persicae),* cabbage aphid *(Brevicoryne brassicae),* spiraea aphid (*Aphis spiraecola*), potato aphid *(Macrosiphum euphorbiae*), foxglove aphid (*Aulacorthum solani*), oat bird-cherry aphid (*Rhopalosiphum padi*), tropical citrus aphid (*Toxoptera citricidus*), and mealy plum aphid *(Hyalopterus pruni);* stink bugs (Pentatomidae) such as green stink bug *(Nezara antennata*), bean bug (*Riptortus clavetus*), rice bug (*Leptocorisa chinensis*), white spotted spined bug *(Eysarcoris parvus),* and stink bug *(Halyomorpha mista);* whiteflies (Aleyrodidae) such as greenhouse whitefly (*Trialeurodes vaporariorum),* sweetpotato whitefly (*Bemisia tabaci*), citrus whitefly *(Dialeurodes citri*), and citrus spiny white fly (*Aleurocanthus spiniferus*); scales (Coccidae) such as Calfornia red scale (*Aonidiella aurantii*), San Jose scale *(Comstockaspis perniciosa),* citrus north scale (*Unaspis citri),* red wax scale (*Ceroplastes rubens*), cottonycushion scale *(Icerya purchasi*), Japanese mealybug *(Planococcus kraunhiae*), Cosmstock mealybug *(Pseudococcus longispinis),* and white peach scale *(Pseudaulacaspis pentagona);* lace bugs (Tingidae); cimices such as *Cimex lectularius;* psyllids (Psyllidae), etc.;

Lepidoptera:

Pyralid moths (Pyralidae) such as rice stem borer (*Chilo suppressalis*), yellow rice borer *(Tryporyza incertulas*), rice leafroller *(Cnaphalocrocis medinalis*), cotton leafroller *(Notarcha derogata),* Indian meal moth *(Plodia interpunctella), Ostrinia furnacalis,* cabbage webworm (*Hellula undalis*), and bluegrass webworm (*Pediasia teterrellus*); owlet moths (Noctuidae) such as common cutworm (*Spodoptera litura*), beet armyworm (*Spodoptera exigua*), armyworm *(Pseudaletia separata),* cabbage armyworm (*Mamestra brassicae),* black cutworm *(Agrotis ipsilon),* beet semi-looper (*Plusia nigrisigna), Thoricoplusia* spp., *Heliothis* spp., and *Helicoverpa* spp.; white butterflies (Pieridae) such as common white (*Pieris rapae);* tortricid moths (Tortricidae) such as *Adoxophyes* spp., oriental fruit moth *(Grapholita molesta*)*,* soybean pod borer *(Leguminivora glycinivorella),* azuki bean podworm (*Matsumuraeses azukivora),* summer fruit tortrix (*Adoxophyes orana fasciata*), smaller tea tortrix *(Adoxophyes* sp.), oriental tea tortrix (*Homona magnanima*), apple tortrix *(Archips fuscocupreanus),* and codling moth *(Cydia pomonella*); leafblotch miners (Gracillariidae) such as tea leafroller (*Caloptilia theivora*), and apple leafminer (*Phyllonorycter ringoneella*); Carposinidae such as peach fruit moth (*Carposina niponensis);* lyonetiid moths (Lyonetiidae) such as *Lyonetia* spp.; tussock moths (Lymantriidae) such as *Lymantria* spp., and *Euproctis* spp.; yponomeutid moths (Yponomeutidae) such as diamondback *(Plutella xylostella);* gelechiid moths (Gelechiidae) such as pink bollworm (*Pectinophora gossypiella),* and potato tubeworm (*Phthorimaea operculella*); tiger moths and allies (Arctiidae) such as fall webworm *(Hyphantria cunea*); tineid moths (Tineidae) such as casemaking clothes moth (*Tinea translucens*), and webbing clothes moth (*Tineola bisselliella*), etc.;

Thysanoptera:

Yellow citrus thrips (*Frankliniella occidentalis*), melon thrips (*Thrips palmi*), yellow tea thrips (*Scirtothrips dorsalis*), onion thrips (*Thrips tabaci*), flower thrips (*Frankliniella intonsa*), etc.;

Diptera:

Housefly (*Musca domestica*), common mosquito (*Culex pipiens pallens*), horsefly (*Tabanus trigonus),* onion maggot (*Hylemya anitqua*), seedcorn maggot *(Hylemya platura), Anopheles sinensis,* rice leafminer (*Agromyza oryzae*), rice leafminer (*Hydrellia griseola),* rice stem maggot *(Chlorops oryzae*), melon fly (*Dacus cucurbitae*), Meditteranean fruit fly (*Ceratitis capitata*), legume leafminer (*Liriomyza trifolii*), tomato leafminer (*Liriomyza sativae*), garden pea leafminer (*Chromatomyia horticola*)*,* etc.;

Coleoptera:

Twenty-eight-spotted ladybird (*Epilachna vigintioctopunctata*)*,* cucurbit leaf beetle (*Aulacophora femoralis*), rice leaf beetle (*Oulema oryzae),* rice curculio (*Echinocnemus squameus*), rice water weevil (*Lissorhoptrus oryzophilus*),

boll weevil (*Anthonomus grandis*), azuki bean weevil (*Callosobruchus chinensis*), hunting billbug (*Sphenophorus venatus*), Japanese beetle (*Popillia japonica*), cupreous chafer (*Anomala cuprea*), corn root worms (*Diabrotica* spp.), Colorado beetle (*Leptinotarsa decemlineata*) click beetles (*Agriotes* spp.), cigarette beetle (*Lasioderma serricorne*), varied carper beetle (*Anthrenus verbasci*), red flour beetle (*Tribolium castaneum*), powder post beetle (*Lyctus brunneus*), whitespotted longicorn beetle (*Anoplophora malasiaca*), pine shoot beetle (*Tomicus piniperda*), etc.;

Orthoptera:

Asiatic locust (*Locusta migratoria*), African mole cricket (*Gryllotalpa africans),* rice grasshopper (*Oxya yezoensis*), rice grasshopper (*Oxya japonica*), etc.;

Hymenoptera:

Cabbage sawfly (*Athalia rosae*), leaf-cutting ant (*Acromyrmex* spp.), fire ant (*Solenopsis* spp.), etc.;

Blattodea:

German cockroach (*Blattella germanica*), smokybrown cockroach (*Periplaneta fuliginosa*)*,* American cockroach (*Periplaneta americana*), *Periplaneta brunnea,* and oriental cockroach (*Blatta orientalis*), etc.;

Spider mites (Tetranychidae) such as two-spotted spider mite (*Tetranychus urticae*)*,* Kanzawa spider mite (*Tetranychus kanzawai*), citrus red mite (*Panonychus citri*)*,* European red mite (*Panonychus ulmi*), and *Oligonychus* spp.; eriophyid mites (Eriophyidae) such as pink citrus rust mite (*Aculops pelekassi*), *Phyllocoptruta citri*, tomato rust mite (*Aculops lycopersici*), purple tea mite (*Calacarus carinatus*), pink tea rust mite (*Acaphylla theavagran*), and *Eriophyes chibaensis;* tarosonemid mites (Tarsonemidae) such as broad mite (*Polyphagotarsonemus latus*); false spider mites (Tenuipalpidae) such as *Brevipalpus phoenicis;* Tuckerellidae; ticks (Ixodidae) such as *Haemaphysalis longicornis, Haemaphysalis flava, Dermacentor taiwanicus, Ixodes ovatus, Ixodes persulcatus, Boophilus microplus,* and *Rhipicephalus sanguineus;* acarid mites (Acaridae) such as mold mite (*Tyrophagus putrescentiae),* and *Tyrophagus similis;* house dust mites (Pyroglyphidae) such as *Dermatophagoides farinae,* and *Dermatophagoides ptrenyssnus;* cheyletide mites (Cheyletidae) such as *Cheyletus eruditus, Cheyletus malaccensis,* and *Cheyletus moorei;* parasitoid mites (Dermanyssidae) such as poultry red mite (*Dermanyssus gallinae*); etc.

The harmful arthropod controlling agent of the present invention can be the present compound as it is. However, it is usually formulated into formulations such as emulsifiable concentrates, oil solutions, dusts, granules, wettable powders, flowable formulations, wettable powders, microcapsule formulations, aerosols, fumigants, poison baits, or resin formulations by mixing the present compound with inert carriers such as solid, liquid or gaseous carriers, and adding surfactants and other auxiliary agents for formulations if necessary. These formulations usually contain 0.1 to 95% by weight of the present compound.

Examples of the solid carrier used for formulation include finely divided powders or granules of clay (e.g., kaolin clay, diatomaceous earth, bentonite, Fubasami clay, and acid clay), synthetic hydrated silicon oxide, talc, ceramics, other inorganic minerals (e.g., sericite, quartz, sulfur, activated carbon, calcium carbonate, and hydrated silica), and chemical fertilizers (e.g., ammonium sulfate, ammonium phosphate, ammonium nitrate, urea, and ammonium chloride).

Examples of the liquid carrier include water, alcohols (e.g., methanol, ethanol, isopropyl alcohol, butanol, hexanol, benzyl alcohol, ethylene glycol, propylene glycol, and phenoxyethanol), ketones (e.g., acetone, methyl ethyl ketone, and cyclohexanone), aromatic hydrocarbons (e.g., toluene, xylene, ethylbenzene, dodecylbenzene, phenylxylylethane, and methylnaphthalene), aliphatic hydrocarbons (e.g., hexane, cyclohexane, kerosene, and gas oil), esters (e.g., ethyl acetate, butyl acetate, isopropyl myristate, ethyl oleate, diisopropyl adipate, diisobutyl adipate, and propylene glycol monomethyl ether acetate), nitriles (e.g., acetonitrile, and isobutyronitrile), ethers (e.g., diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, and 3-methoxy-3-methyl-1-butanol), acid amides (e.g., N,N-dimethylformamide, and N,N-dimethylacetamide), halogenated hydrocarbons (e.g., dichloromethane, trichloroethane, and carbon tetrachloride), sulfoxides (e.g., dimethyl sulfoxide), propylene carbonate and vegetable oils (e.g., soybean oil, and cottonseed oil).

Examples of the gaseous carrier include fluorocarbon, butane gas, LPG (liquefied petroleum gas), dimethyl ether and carbon dioxide gas.

Examples of the surfactant include nonionic surfactants such as polyoxyethylene alkyl ether, polyoxyethylene alkyl aryl ether, and polyethylene glycol fatty acid ester; and anionic surfactants such as alkyl sulfonate, alkylbenzene sulfonate, and alkyl sulfate.

Examples of the other additives for formulations include binders, dispersants, coloring agents and stabilizers, and specific examples thereof include casein, gelatin, saccharides (e.g., starch, gum arabic, cellulose derivatives, and

alginic acid), lignin derivatives, bentonite, synthetic water-soluble polymers (e.g., polyvinyl alcohol, polyvinyl pyrrolidone, and polyacrylic acid), PAP (e.g., isopropyl acid phosphate), BHT (2,6-di-tert-butyl-4-methylphenol), and BHA (a mixture of 2-tert-butyl-4-methoxyphenol and 3-tert-butyl-4-methoxyphenol).

The method for controlling harmful arthropods of the present invention is usually carried out by applying the harmful arthropod controlling agent of the present invention directly to harmful arthropods, or applying to habitats plants, soils and houses of harmful arthropods.

For the method for controlling harmful arthropods of the present invention, the present compound can be used as it is. Usually, the method includes a method comprising formulating the present compound into the harmful arthropod controlling agent of the present invention as described above and applying the harmful arthropod controlling agent to harmful arthropods or a place where harmful arthropods inhabit, for example, by the same method as that of applying a conventional harmful arthropod controlling agent, thereby bringing the harmful arthropod controlling agent to contact with the above harmful arthropods or allowing the harmful arthropods to ingest the harmful arthropod controlling agent. Examples of the place where harmful arthropods inhabit in the present invention include paddy fields, cultivated lands, orchards, non-crop lands, and houses.

Examples of the application method include spraying treatment, soil treatment, seed treatment, and water culture medium treatment.

The spraying treatment in the present invention is a treatment method which comprises treating plant surfaces or harmful arthropods themselves with the active ingredient (the present compound) to produce a controlling effect on harmful arthropods. Specific examples of the spraying treatment include spraying treatment to foliage, and spraying treatment to tree trunks.

The soil treatment is a treatment method which comprises treating soil or an irrigation liquid with the active ingredient for the purpose of allowing the active ingredient to permeate and transfer into the interior of the plant body of a crop to be protected from damage such as ingestion by harmful arthropods, for example, through the root part of the plant, thereby protecting the crop from damage by harmful arthropods. Specific examples of the soil treatment include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, drug solution injection treatment, irrigation of a plant part just above the ground, drug solution drip irrigation, chemigation), seedling raising box treatment (spraying into a seedling raising box, irrigation of a seedling raising box), seedling raising tray treatment (spraying on a seedling raising tray, irrigation of a seedling raising tray), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

The seed treatment is a treating method which comprises applying the active ingredient directly to or around a seed, a seed tuber or a bulb of a crop to be protected from damage such as ingestion by harmful arthropods to produce a controlling effect on harmful arthropods. Specific examples of the seed treatment include spraying treatment, spray coating treatment, immersion treatment, impregnation treatment, coating treatment, film coating treatment, and pellet coating treatment.

The water culture medium treatment is a treating method which comprises treating a water culture medium or the like with an active ingredient for the purpose of allowing the active ingredient to permeate and transfer into the interior of the plant body of a crop to be protected from damage such as ingestion by harmful arthropods, for example, through the root part of the plant, thereby protecting the crop from damage by harmful arthropods. Specific examples of the water culture medium treatment include mixing with a water culture medium, and incorporation into a water culture medium.

When the harmful arthropod controlling agent of the present invention is used for controlling harmful arthropods in the field of agriculture, the application amount thereof is usually from 1 to 10,000 g of the present compound per 10,000 $m^2$ in terms of the amount of the present compound. When a harmful arthropod controlling agent of the present invention is in the form of a formulation such as an emulsifiable concentrate, a wettable powder or a flowable formulation, the harmful arthropod controlling agent is usually applied after it is diluted with water so that the active ingredient concentration becomes 0.01 to 10,000 ppm. When a harmful arthropod controlling agent is in the form of a formulation

such as granules or a powder, the harmful arthropod controlling agent is usually applied as it is.

These harmful arthropod controlling agent and water-dilution thereof can be directly sprayed to harmful arthropods or plants such as crops to be protected from harmful arthropods. Alternatively, soil of a cultivated land can be treated with the harmful arthropod controlling agent or water-dilution thereof in order to control harmful arthropods which inhabit the soil.

The harmful arthropod controlling agent can be in the form of a resin preparation which is processed into a sheet or a string. Such a resin preparation can be applied by winding a crop with a sheet or a string of the resin preparation, putting a string of the resin preparation around a crop so that the crop is surrounded by the string, or laying a sheet of the resin preparation on the soil surface near the root of a crop.

When the harmful arthropod controlling agent of the present invention is used for controlling harmful arthropods living in a house (e.g. fly, mosquito, and cockroach), the application amount thereof is usually from 0.01 to 1,000 mg per 1 $m^2$ in terms of the amount of the present compound in the case of plain surface treatment, and is usually from 0.01 to 500 mg per 1 $m^2$ in terms of the amount of the present compound per in the case of space treatment. When the harmful arthropod controlling agent of the present invention is in the form of a formulation such as an emulsifiable concentrate, a wettable powder or a flowable formulation, the harmful arthropod controlling agent is usually applied after it is diluted with water so that the active ingredient concentration becomes 0.1 to 1,000 ppm. When the harmful arthropod controlling agent of the present invention is in the form of a formulation such as an oil solution, an aerosol formulation, a fumigant or poison bait, the harmful arthropod controlling agent is usually applied as it is.

The present compound can be used a harmful arthropod controlling agent for crop lands such as cultivated lands, paddy fields, lawns and orchards, or non-crop lands.

The harmful arthropod controlling agent of the present invention may further contain, for example, other harmful arthropod controlling agents, acaricides, nematocides, fungicides, herbicides, plant growth regulators, synergists, fertilizers, soil conditioners, and animal feeds.

It is also possible to use the present compound for spraying treatment, soil treatment, seed treatment, and water culture medium treatment as a mixed formulation appropriately prepared by mixing the present compound with harmful organism controlling agents such as insecticides, acaricides, nematocides, fungicides, plant hormone agents, plant growth regulators and herbicides (including isomers and salts thereof), or, for example, synergists, phytotoxicity reducing agents, colorants, and fertilizers.

Examples of the active ingredient of the above other harmful arthropod controlling agents, acaricides and/or nematocides include the followings:

(1) Organophosphorus compounds acephate, Aluminium phosphide, butathiofos, cadusafos, chlorethoxyfos, chlorfenvinphos, chlorpyrifos, chlorpyrifos-methyl, cyanophos : CYAP, diazinon, DCIP (dichlorodiisopropyl ether), dichlofenthion : ECP, dichlorvos : DDVP, dimethoate, dimethylvinphos, disulfoton, EPN, ethion, ethoprophos, etrimfos, fenthion : MPP, fenitrothion : MEP, fosthiazate, formothion, Hydrogen phosphide, isofenphos, isoxathion, malathion, mesulfenfos, methidathion : DMTP, monocrotophos, naled : BRP, oxydeprofos : ESP, parathion, phosalone, phosmet : PMP, pirimiphos-methyl, pyridafenthion, quinalphos, phenthoate:PAP, profenofos, propaphos, prothiofos, pyraclorfos, salithion, sulprofos, tebupirimfos, temephos, tetrachlorvinphos, terbufos, thiometon, trichlorphon : DEP, and vamidothion;

(2) Carbamate compounds alanycarb, bendiocarb, benfuracarb, BPMC carbaryl, carbofuran, carbosulfan, cloethocarb, ethiofencarb, fenobucarb, fenothiocarb, fenoxycarb, furathiocarb, isoprocarb:MIPC, metolcarb, methomyl, methiocarb, NAC, oxamyl, pirimicarb, propoxur : PHC, XMC, thiodicarb, and xylylcarb;

(3) Synthetic pyrethroid compounds acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigmacypermethrin, silafluofen, tefluthrin, tralomethrin, transfluthrin, 2,3,5,6-tetrafluoro-4-(methoxymethyl)benzyl (EZ)-(1RS,3RS;1RS,3SR)-2,2-dimethyl-3-prop-1-enylcyclopropanecarboxylate, 2,3,5,6-tetrafluoro-4-methylbenzyl (EZ)-(1RS,3RS:1RS,3SR)-2,2-dimethyl-3-prop-1-enylcyclopropanecarboxylate, and 2,3,5,6-tetrafluoro-4-(methoxymethyl)benzyl(1RS,3RS:1RS,3SR)-2,2-dimethyl-3-(2-methyl-1-propenyl)cyclopropanecarboxylate;

(4) Nereistoxin compounds cartap, bensultap, thiocyclam, monosultap, and bisultap;

(5) Neonicotinoid compounds imidacloprid, nitenpyram, acetamiprid, thiamethoxam, thiacloprid, dinotefuran, and clothianidin;

(6) Benzoylurea compounds chlorfluazuron, bistrifluron, diafenthiuron, diflubenzuron, fluazuron, flucycloxuron, flufenoxuron, hexaflumuron, lufenuron, novaluron, noviflumuron, teflubenzuron, and triflumuron;

(7) Phenyl pyrazole compounds acetoprole, ethiprole, fipronil, vaniliprole, pyriprole, and pyrafluprole;

(B) Bt toxin insecticides viable spores of *Bacillus thuringinesis* and crystal toxins produced therefrom, and a mixture thereof;

(9) Hydrazine compounds chromafenozide, halofenozide, methoxyfenozide, and tebufenozide;

(10) Organic chlorine compounds aldrin, dieldrin, dienochlor, endosulfan, and methoxychlor;

(11) Natural insecticides machine oil, and nicotine-sulfate;

(12) Other insecticides avermectin-B, bromopropylate, buprofezin, chlorphenapyr, cyromazine, D-D(1,3-Dichloropropene), emamectin-benzoate, fenazaquin, flupyrazofos, hydroprene, indoxacarb, metoxadiazone, A(milbemycin-A), pymetrozine, pyridalyl, pyriproxyfen, spinosad, sulfluramid, tolfenpyrad, triazamate, flubendiamide, SI-0009, cyflumetofen, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, nidinotefuran, Potassium oleate, protrifenbute, spiromesifen, Sulfur, metaflumizone, and spirotetramat;

Acaricides acequinocyl, amitraz, benzoximate, bromopropylate, chinomethionat, chlorobenzilate, CPCBS (chlorfenson), clofentezine, dicofol, etoxazole, fenbutatin oxide, fenothiocarb, fenpyroximate, fluacrypyrim, fluproxyfen, hexythiazox, propargite:BPPS, polynactins, pyridaben, Pyrimidifen, tebufenpyrad, tetradifon, spirodiclofen, amidoflumet, Bifenazate, and Cyflumetofen;

Nematocides (nematocidal active ingredients) DCIP, fosthiazate, levamisol, methyisothiocyanate, and morantel tartarate.

Examples

Hereinafter, the present invention will be explained in more detail by way of Production Examples, Formulation Examples, and Test Examples, but the present invention is not limited to these Examples.

First, Production Examples of the present compound will be explained.

Production Example 1

(1) A mixture of 10.7 g of 3-bromo-1H-pyrazole, 11.8 g of 2,3-dichloropyridine, 57.3 g of cesium carbonate and 80 mL of N,N-dimethylformamide was stirred at 100°C for 8 hours. After cooling to room temperature and adding water, the reaction mixture was extracted twice with methyl tert-butyl ether. The organic layers were combined, washed sequentially with water and a saturated sodium chloride solution, dried over magnesium sulfate, and concentrated under reduced pressure. The resulting residue was subjected to silica gel column chromatography to obtain 12.9 g of 2-(3-bromo-1H-pyrazol-1-yl)-3-chloropyridine. 2-(3-bromo-1H-pyrazol-1-yl)-3-chloropyridine

$^1$H-NMR ($CDCl_3$, TMS) δ (ppm): 6.51 (1H, d, J=2Hz), 7.31 (1H, dd, J=8Hz, 4Hz), 7.91 (1H, dd, J=8Hz, 1Hz), 8.04 (1H, d, J=2Hz), 8.45 (1H, dd, J=4Hz, 1Hz)

(2) To a mixture of 5.0 g of 2-(3-bromo-1H-pyrazol-1-yl)-3-chloropyridine and 30 mL of tetrahydrofuran, 11.7 mL of a heptane/tetrahydrofuran/ethylbenzene solution of 2.0 M lithium diisopropylamide was added dropwise at -78°C. To the reaction mixture, a mixture of 3 g of ethyl formate and 10 mL of tetrahydrofuran were added dropwise at -78°C, followed by stirring at room temperature for 2 hours. After adding water, the reaction mixture was extracted with ethyl acetate. The organic layer was washed with water, dried over anhydrous magnesium sulfate, and concentrated under reduced pressure. The resulting residue was subjected to silica gel column chromatography to obtain 3.0 g of the compound (4-1).

The compound (4-1)

[Chemical Formula 119]

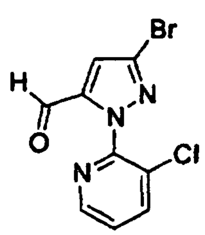

(4-1)

$^1$H-NMR ($CDCl_3$, TMS) δ (ppm): 7.11 (1H, s), 7.47 (1H, dd, J=8Hz, 5Hz), 7.96 (1H, dd, J=8Hz, 1Hz), 8.52 (1H, dd, J=5Hz, 1Hz), 9.79 (1H, s)

(3) To a mixture of 0.61 g of ethylhydrazine oxalate, 1.0 g of 6,8-dibromo-2H-3,1-benzoxazine-2,4-1H-dion

[Chemical Formula 120]

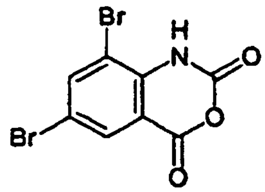

(a compound descried in Journal of Organic Chemistry (1947), 12, 743-51) and 10 mL of tetrahydrofuran, 1.12 g of potassium carbonate was added under ice cooling, followed by stirring at room temperature for 1.5 hours. After adding water, the reaction mixture was extracted with ethyl acetate. The organic layer was washed sequentially with water and a saturated sodium chloride solution, dried over anhydrous magnesium sulfate, and concentrated under reduced pressure. The resulting residue was subjected to silica gel column chromatography to obtain 0.44 g of N-(2-amino-3,5-dibromobenzoyl)-N-ethylhydrazine and 0.13 g of N-(2-amino-3,5-dibromobenzoyl)-N'-ethylhydrazine.

N-(2-amino-3,5-dibromobenzoyl)-N-ethylhydrazine

$^1$H-NMR ($CDCl_3$, TMS) δ (ppm): 1.25 (3H, t, J=7Hz), 3.52 (2H, q, J=7Hz), 4.38 (2H, brs), 4.81 (2H, brs), 7.21 (1H, d, J=2Hz), 7.59 (1H, d, J=2Hz)

N-(2-amino-3,5-dibromobenzoyl)-N'-ethylhydrazine $^1$H-NMR ($CDCl_3$, TMS) δ (ppm): 1.15 (3H, t, J=7Hz), 2.95 (2H, q, J=7Hz), 4.78 (1H, brs), 6.02 (2H, brs), 7.38 (1H, d, J=2Hz), 7.52 (1H, brs), 7.64 (1H, d, J=2Hz)

(4) To a mixture of 0.42 g of N-(2-amino-3,5-dibromobenzoyl)-N-ethylhydrazine and 3 mL of pyridine, 0.15 g of methyl chloroformate was added under ice cooling, followed by stirring under ice cooling for 1 hour. After adding water, the reaction mixture was concentrated under reduced pressure. Water was added to the resulting residue, followed by extraction with ethyl acetate. The organic layer was washed sequentially with water and a saturated sodium chloride solution, dried over anhydrous magnesium sulfate, and concentrated under reduced pressure. The resulting residue was subjected to silica gel column chromatography to obtain 0.42 g of N-(2-amino-3,5-dibromobenzoyl)-N-ethyl-N'-methoxycarbonylhydrazine. N-(2-amino-3,5-dibromobenzoyl)-N-ethyl-N'-methoxycarbonylhydrazine

$^1$H-NMR ($CDCl_3$, TMS) δ (ppm): 1.21 (3H, t, J=7Hz), 3.62 (2H, q, J=7Hz), 3.78 (3H, s), 4.95 (2H, brs), 6.96 (1H, brs), 7.26 (1H, d, J=2Hz), 7.59 (1H, d, J=2Hz)

(5) A mixture of 0.15 g of N-(2-amino-3,5-dibromobenzoyl)-N-ethyl-N'-methoxycarbonylhydrazine, 2 mL of N, N-dimethylformamide, 0.050 g of bromoacetonitrile and 0.079 g of potassium carbonate was stirred at room temperature for 4 hours. After adding water, the reaction mixture was extracted with methyl tert-butyl ether. The organic layer was washed with water, dried over anhydrous magnesium sulfate, and concentrated under reduced pressure. The resulting residue was subjected to silica gel column chromatography to obtain 0.15 g of the compound (6-1).

The compound (6-1)

[Chemical Formula 121]

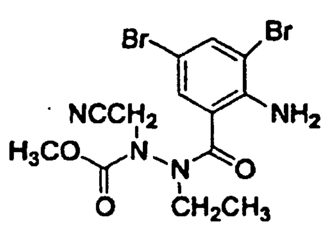

(6-1)

$^1$H-NMR (DMSO-$d_6$, 80°C, TMS) δ (ppm): 1.16 (3H, t, J=7Hz), 3.45-3.55 (2H, m), 3.77 (3H, s), 4.62-4.73 (2H, m), 5.26 (2H, brs), 7.26 (1H, d, J=2Hz), 7.66 (1H, d, J=2Hz)

(6) A mixture of 0.14 g of the above compound (6-1), 0.092 g of the above compound (4-1), 0.095 g of o-chloranil, p-toluenesulfonic acid monohydrate (catalytic amount), copper iodide (catalytic amount) and 1.5 mL of 1,4-dioxane was stirred in a nitrogen atmosphere under heat-refluxing conditions for 6 hours. After cooling to room temperature and adding water, the reaction mixture was extracted with ethyl acetate. The organic layer was washed sequentially with an aqueous 2 N sodium hydroxide solution, water and a saturated sodium chloride solution, dried over anhydrous magnesium sulfate, and concentrated under reduced pressure. The resulting residue was subjected to silica gel column chromatography to obtain 0.19 g of the following present compound (1-1).

The present compound (1-1)

[Chemical Formula 122]

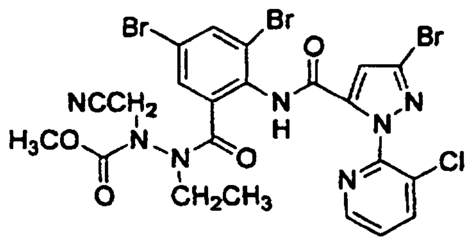

(1-1)

$^1$H-NMR ($CDCl_3$, TMS) δ (ppm): 1.08-1.14 (3.0H, m), 3.22 (1.1H, s), 3.33-3.52 (1.5H, m), 3.60-4.13 (3.8H, m), 4.68-4.84 (0.6H, m), 7.15-7.25 (1.0H, m), 7.30-7.31 (0.6H, m), 7.42-7.50 (1.4H, m), 7.64-7.70 (1.0H, m), 7.91-7.94 (1.0H, m), 8.48-8.57 (1.0H, m), 9.26-9.35 (1.0H, m)

Production Example 2

(1) In place of N-(2-amino-3,5-dibromobenzoyl)-N-ethylhydrazine of Production Example 1 (4), N-(2-amino-3,5-dibromobenzoyl)-N'-ethylhydrazine (a compound obtained in Production Example 1 (3)) is used to obtain N-(2-amino-3,5-dibromobenzoyl)-N'-ethyl-N'-methoxycarbonylhydrazine.
(2) In place of N-(2-amino-3,5-dibromobenzoyl)-N-ethyl-N'-methoxycarbonylhydrazine of Production Example 1 (5), N-(2-amino-3,5-dibromobenzoyl)-N'-ethyl-N'-methoxycarbonylhydrazine is.used to obtain the compound (6-2). The compound (6-2)

[Chemical Formula 123]

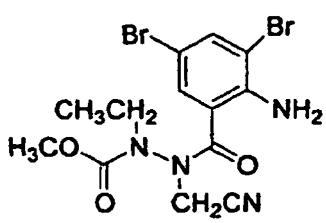

(6-2)

(3) In place of the compound (6-1) of Production Example 1 (6), the compound (6-2) is used to obtain the following present compound (1-2). The present compound (1-2)

[Chemical Formula 124]

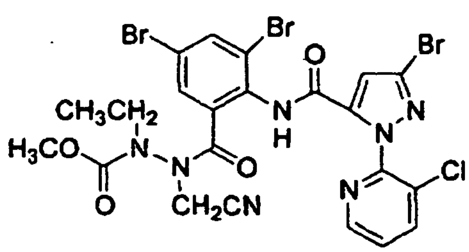

(1-2)

Production Example 3

(1) To a mixture of 10.0 g of 6,8-dibromo-2H-3,1-benzoxazine-2,4-1H-dion and 90 mL of tetrahydrofuran, 1.58 g of methylhydrazine was added under ice cooling, followed by stirring at room temperature for 4 hours. After adding water, the reaction mixture was extracted with ethyl acetate. The organic layer was washed with a saturated sodium chloride solution, dried over anhydrous magnesium sulfate, and concentrated under reduced pressure. The resulting residue was subjected to silica gel column chromatography to obtain 4.64 g of N-(2-amino-3,5-dibromobenzoyl)-N-methylhydrazine and 0.75 g of N-(2-amino-3,5-dibromobenzoyl)-N'-methylhydrazine.
N-(2-amino-3,5-dibromobenzoyl)-N-methylhydrazine

$^1$H-NMR ($CDCl_3$, TMS) δ (ppm): 3.25 (3H, s), 4.55 (2H, brs), 4.89 (2H, brs), 7.23 (1H, s), 7.59 (1H, s)

N-(2-amino-3,5-dibromobenzoyl)-N'-methylhydrazine

$^1$H-NMR (DMSO-$d_6$, TMS) δ (ppm): 2.51 (3H, s), 5.11 (1H, brs), 6.54 (2H, s), 7.63 (1H, d, J=2Hz), 7.73 (1H, d, J=2Hz), 10.06 (1H, brs)

(2) To a mixture of 3.40 g of N-(2-amino-3,5-dibromobenzoyl)-N-methylhydrazine and 30 mL of tetrahydrofuran, 2.2 g of triethylamine and 2.0 g methyl chloroformate were added sequentially under ice cooling,, followed by stirring at room temperature. After adding water, the reaction mixture was extracted with ethyl acetate. The organic layer was washed with a saturated sodium chloride solution, dried over anhydrous magnesium sulfate, and concentrated under reduced pressure. The resulting residue was subjected to silica gel column chromatography to obtain 1.10 g of N-(2-amino-3,5-dibromobenzoyl)-N-methyl-N'-methoxycarbonylhydrazine. N-(2-amino-3,5-dibromobenzoyl)-N-methyl-N'-methoxycarbonylhydrazine

$^1$H-NMR ($CDCl_3$, TMS) δ (ppm): 3.28 (3H, s), 3.76 (3H, s), 4.96 (2H, brs), 7.00 (1H, brs), 7.27 (1H, d, J=2Hz), 7.59 (1H, d, J=2Hz)

(3) In place of N-(2-amino-3,5-dibromobenzoyl)-N-ethyl-N'-methoxycarbonylhydrazine of Production Example 1 (5), N-(2-amino-3,5-dibromobenzoyl)-N-methyl-N'-methoxycarbonylhydrazine is used to obtain the compound (6-3). The compound (6-3)

[Chemical Formula 125]

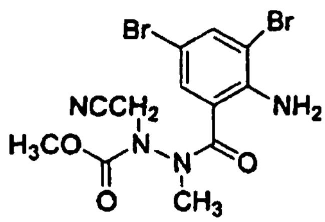

(6-3)

(4) In place of the compound (6-1) of Production Example 1 (6), the compound (6-3).is used to obtain the following present compound (1-3).

The present compound (1-3)

[Chemical Formula 126]

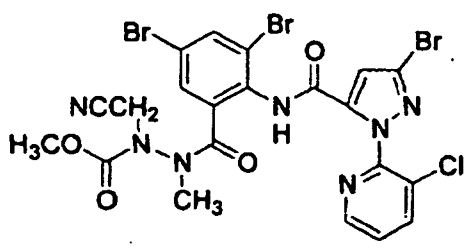

(1-3)

Production Example 4

(1) To a mixture of 0.33 g of N-(2-amino-3,5-dibromobenzoyl)-N'-methylhydrazine (a compound obtained in Production Example 3 (1)) and 3 mL of pyridine, 0.097 g of methyl chloroformate was added under ice cooling, followed by stirring under ice cooling for 1 hour. To the reaction mixture, 0.032 g of methyl chloroformate was further added under ice cooling, followed by stirring under ice cooling for 1 hour. After adding water, the reaction mixture was concentrated under reduced pressure. Water was added to the resulting residue, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.31 g of N-(2-amino-3,5-dibromobenzoyl)-N'-methyl-N'-methoxycarbonylhydrazine. N-(2-amino-3,5-dibromobenzoyl)-N'-methyl-N'-methoxycarbonylhydrazine.

$^1$H-NMR ($CDCl_3$, TMS) δ (ppm): 3.26 (3.0H, s), 3.78 (3.0H, brs), 6.06 (2.0H, brs), 7.51 (1.0H, brs), 7.65 (1.0H, brs), 7.83 (0.5H, brs), 8.25 (0.5H, brs)

(2) In place of N-(2-amino-3,5-dibromobenzoyl)-N-ethyl-N'-methoxycarbonylhydrazine of Production Example 1 (5), N-(2-amino-3,5-dibromobenzoyl)-N'-methyl-N'-methoxycarbonylhydrazine is used to obtain the compound (6-4).

The compound (6-4)

[Chemical Formula 127]

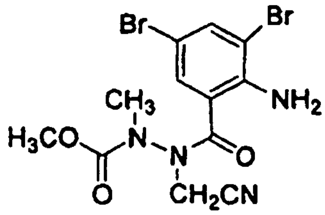

(6-4)

(3) In place of the compound (6-1) of Production Example 1 (6), the compound (6-4) is used to obtain the following present compound (1-4).
The present compound (1-4)

[Chemical Formula 128]

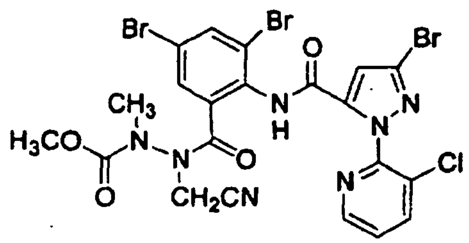

(1-4)

Production Example 5

(1) A mixture of 2.0 g of 2-[3-bromo-1-(3-chloro-2-pyridinyl)-1H-pyrazol-5-yl]-6,8-dibromo-4H-3,1-benzoxazin-4-one, 0.58 g of 2-hydrazinopyridine, 0.74 g of potassium carbonate and 50 mL of tetrahydrofuran was stirred at room temperature for 1 hour. After adding water, the reaction mixture was extracted with ethyl acetate. The organic layer was washed with a saturated sodium chloride solution, dried over anhydrous magnesium sulfate, and concentrated under reduced pressure. The resulting solid was washed with chloroform to obtain 1.86 g of the compound (2-1). The compound (2-1)

[Chemical Formula 129]

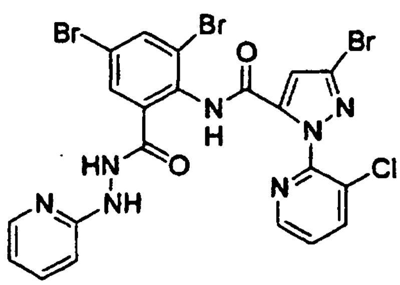

(2-1)

$^1$H-NMR (DMSO-D$_6$) δ: 6.59 (1H, d, J=8Hz), 6.70 (1H, dd, J=8Hz, 5Hz), 7.38 (1H, s), 7.41-7.46 (1H, m), 7.61 (1H, dd, J=8Hz, 5Hz), 7.78 (1H, d, J=2Hz), 8.01-8.04 (1H, m), 8.15-8.18 (2H, m), 8.30 (1H, s), 8.52 (1H, dd, J=5Hz, 2Hz), 10.27 (1H, brs), 10.54 (1H, brs)

(2) To a mixture of 0.50 g of the compound (2-1) and 5 mL of pyridine, 115 μL of methyl chloroformate was added dropwise under ice cooling, followed by stirring at room temperature for 1 hour. After adding water, the reaction was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.40 g of the present compound (1-5).
The present compound (1-5)

[Chemical Formula 130]

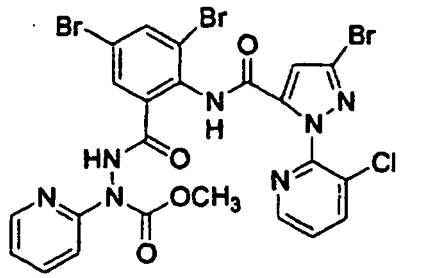

(1-5)

$^1$H-NMR (DMSO-$D_6$) δ: 3.62 (3H, s), 7.18 (1H, dd, J=7Hz, 5Hz), 7.31 (1H, s), 7.61 (1H, dd, J=8Hz, 5Hz), 7.67 (1H, d, J=8Hz), 7.76-7.82 (2H, m), 8.16 (1H, dd, J=8Hz, 1Hz), 8.20 (1H, d, J=2Hz), 8.28 (1H, d, J=7Hz), 8.50 (1H, dd, J=5Hz, 1Hz), 10.48 (1H, s), 11.13 (1H, s)

Production Example 6

(1) In place of 2-[3-bromo-1-(3-chloro-2-pyridinyl)-1H-pyrazol-5-yl]-6,8-dibromo-4H-3,1-benzoxazin-4-one of Production Example 5(1), 2-[3-bromo-1-(3-chloro-2-pyridinyl)-1H-pyrazol-5-yl]-6-chloro-8-methyl-4H-3,1-benzoxazin-4-one was used to obtain the compound (2-2). The compound (2-2)

[Chemical Formula 131]

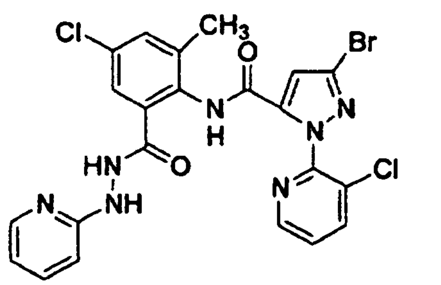

(2-2)

$^1$H-NMR (DMS O-$D_6$) δ: 2.18 (3H, s), 6.60 (1H, d, J=9Hz), 6.69 (1H, dd, J=7Hz, 5Hz), 7.29 (1H, s), 7.41-7.45 (1H, m), 7.49 (1H, d, J=2Hz), 7.55 (1H, d, J=2Hz), 7.61 (1H, dd, J=8Hz, 5Hz), 8.02-8.04 (1H, m), 8.18 (1H, dd, J=8Hz, 2Hz), 8.28 (1H, s), 8.51 (1H, dd, J=5Hz, 2Hz), 10.20 (1H, s), 10.28 (1H, s)

(2) In place of the compound (2-1) of Production Example 5 (2), the compound (2-2) was used to obtain the present compound (1-6).
The present compound (1-6)

[Chemical Formula 132]

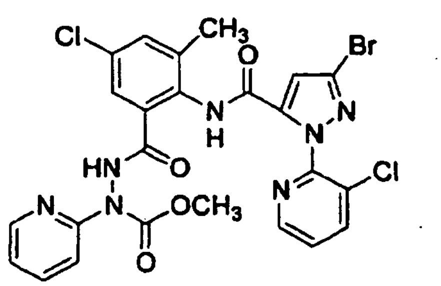

(1-6)

$^1$H-NMR (DMSO-$D_6$) δ: 2.18 (3H, s), 3.63 (3H, s), 7.17 (1H, ddd, J=7Hz, 5Hz, 1Hz), 7.21 (1H, s), 7.48 (1H, d, J=2Hz), 7.58-7.63 (2H, m), 7.69 (1H, d, J=8Hz), 7.78-7.82 (1H, m), 8.17 (1H, dd, J=8Hz, 2Hz), 8.27-8.29 (1H, m), 8.50 (1H, dd, J=5Hz, 2Hz), 10.20 (1H, brs), 11.06 (1H, brs).

Production Example 7

(1) A mixture of 1.0 g of 2-[3-bromo-1-(3-chloro-2-pyridinyl)-1H-pyrazol-5-yl]-6,8-dibromo-4H-3,1-benzoxazin-4-one, 0.37 g of an aqueous 70% allylhydrazine solution and 25 mL of tetrahydrofuran was stirred at room temperature for 1 hour. After adding water, the reaction mixture was extracted with ethyl acetate. The organic layer was washed with a saturated sodium chloride solution, dried over anhydrous magnesium sulfate, and concentrated under reduced pressure. The resulting residue was subjected to column chromatography to obtain 0.82 g of a mixture of the compound (2-3) and the compound (2-4).
The compound (2-3)

[Chemical Formula 133]

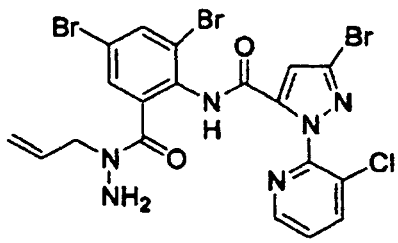

(2-3)

Compound (2-4)

[Chemical Formula 134]

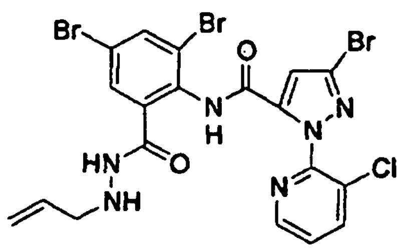

(2-4)

(2) To a mixture of 0.82 g of the mixture of the compound (2-3) and the compound (2-4), and 8 mL of pyridine, 400 μL of methyl chloroformate was added dropwise under ice cooling, followed by stirring at room temperature for 3 hours. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.53 g of the present compound (1-7) and 0.23 g of the present compound (1-8).
The present compound (1-7)

[Chemical Formula 135]

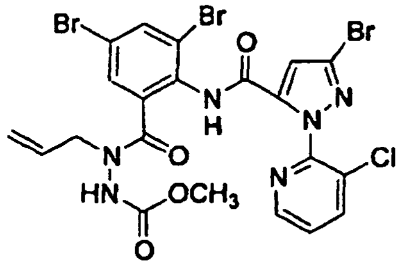

(1-7)

$^1$H-NMR (DMSO-$D_6$) δ: 3.49 (3.0H, s), 3.61-3.82 (1.5H, m), 4.34-4.48 (0.5H, m), 5.03-5.26 (2.0H, m), 5.53-5.73 (1.0H, m), 7.35-7.44 (2.0H, m), 7.61-7.65 (1.0H, m), 8.08-8.13 (1.0H, m), 8.17-8.21 (1.0H, m), 8.51 (1.0H, dd, J=5Hz, 1Hz), 9.05 (0.7H, s), 9.70 (0.3H, s), 10.25 (0.7H, s), 10.68 (0.3H, s)
The present compound (1-8)

[Chemical Formula 136]

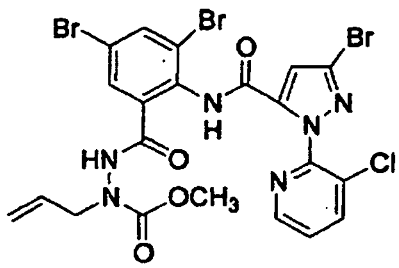

(1-8)

$^1$H-NMR (DMSO-$D_6$) δ: 3.52 (3H, brs), 3.87 (2H, brs), 5.00-5.08 (2H, m), 5.64-5.74 (1H, m), 7.45 (1H, s), 7.58-7.62 (2H, m), 8.13-8.19 (2H, m), 8.48 (1H, d, J=5Hz), 10.50 (1H, s), 10.62 (1H, s)

Production Example 8

(1) In place of 2-[3-bromo-1-(3-chloro-2-pyridinyl)-1H-pyrazol-5-yl]-6,8-dibromo-4H-3,1-benzoxazin-4-one of Production Example 7 (1), 2-[3-bromo-1-(3-chloro-2-pyridinyl)-1H-pyrazol-5-yl]-6,8-dichloro-4H-3,1-benzoxazin-4-one was used to obtain a mixture of the compound (2-5) and the compound (2-6).
The compound (2-5)

[Chemical Formula 137]

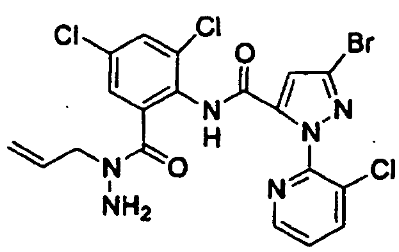

(2-5)

The compound (2-6)

[Chemical Formula 138]

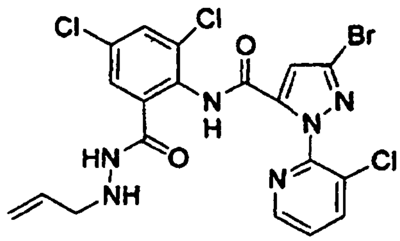

(2-6)

(2) In place of the mixture of the compound (2-3) and the compound (2-4) of Production Example 7 (2), the mixture of the compound (2-5) and the compound (2-6) was used to obtain the present compound (1-9) and the present compound (1-10).
The present compound (1-9)

[Chemical Formula 139]

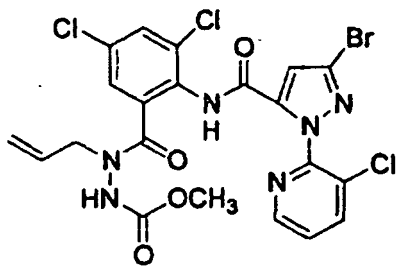

(1-9)

$^1$H-NMR (DMSO-$D_6$) δ: 3.48 (3.0H, s), 3.63-4.43 (2.0H, brm), 5.04-5.27 (2.0H, m), 5.58-5.73 (1.0H, m), 7.26-7.44 (2.0H, m), 7.62-7.65 (1.0H, m), 7.85-7.91 (1.0H, m), 8.20 (1.0H, d, J=8Hz), 8.50 (1.0H, d, J=5Hz), 9.17 (0.7H, s), 9.72 (0.3H, s), 10.24 (0.7H, s), 10.69 (0.3H, s)
The present compound (1-10)

[Chemical Formula 140]

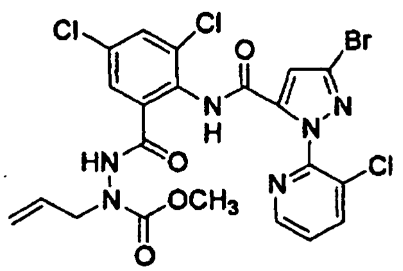

(1-10)

$^1$H-NMR (DMSO-$D_6$) δ: 3.53 (3H, brs), 3.89 (2H, brs); 5.00-5.08 (2H, m), 5.65-5.75 (1H, m), 7.44 (2H, brs), 7.60 (1H, dd, J=8Hz, 5Hz), 7.96 (1H, d, J=2Hz), 8.16 (1H, dd, J=8Hz, 1Hz), 8.48 (1H, dd, J=5Hz, 1Hz), 10.53 (1H, s), 10.65 (1H, s)

Production Example 9

(1) A mixture of 1.5 g of 2-[3-bromo-1-(3-chloro-2-pyridinyl)-1H-pyrazol-5-yl]-6-chloro-8-methyl-4H-3,1-benzoxazin-4-one, 0.68 g of an aqueous 70% allylhydrazine solution and 40 mL of tetrahydrofuran was stirred at room temperature for 1 hour. After adding water, the reaction mixture was extracted with ethyl acetate. The organic layer was washed with a saturated sodium chloride solution, dried over anhydrous magnesium sulfate, and concentrated under reduced pressure. The resulting residue was subjected to column chromatography to obtain 0.75 g of the compound (2-7) and 0.37 g of the compound (2-8).
The compound (2-7)

[Chemical Formula 141]

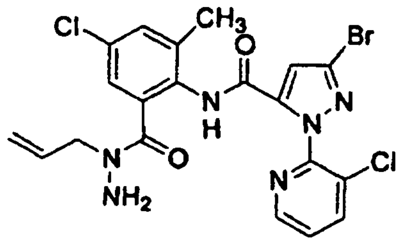

(2-7)

$^1$H-NMR (DMSO-$D_6$) δ: 2.15 (3.0H, s), 3.59-3.62 (0.6H, m), 4.06-4.09 (1.4H, m), 4.41 (1.4H, s), 4.81 (0.6H, s), 5.02-5.23 (2.0H, m), 5.62-5.75 (1.0H, m), 7.15 (0.3H, d, J=2Hz), 7.21 (0.7H, d, J=2Hz), 7.33 (0.7H, s), 7.36-7.37 (1.0H, m), 7.47 (0.3H, d, J=2Hz), 7.61-7.65 (1.0H, m), 8.17-8.21 (1.0H, m), 8.49-8.52 (1.0H, m), 10.12 (0.7H, s), 10.39 (0.3H, s)
The compound (2-8)

[Chemical Formula 142]

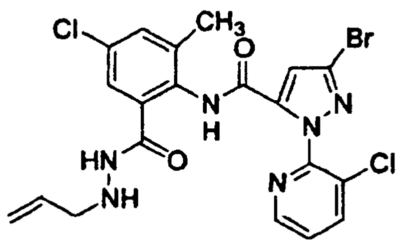

(2-8)

$^1$H-NMR (DMSO-$D_6$) δ: 2.16 (3H, s), 3.37-3.40 (2H, m), 5.02-5.15 (3H, m), 5.76-5.86 (1H, m), 7.28 (1H, d, J=2Hz), 7.38 (1H, s), 7.49 (1H, d, J=2Hz), 7.60 (1H, dd, J=8Hz, 5Hz), 8.16 (1H, dd, J=8Hz, 2Hz), 8.48 (1H, dd, J=5Hz, 2Hz), 9.76-9.78 (1H, m), 10.22 (1H, s)

(2) To a mixture of 0.65 g of the compound (2-7) and 23 mL of pyridine, 0..14 g of methyl chloroformate was added dropwise under ice cooling, followed by stirring at room temperature for 3 hours. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.04 g of the present compound (1-11) .
The present compound (1-11)

[Chemical Formula 143]

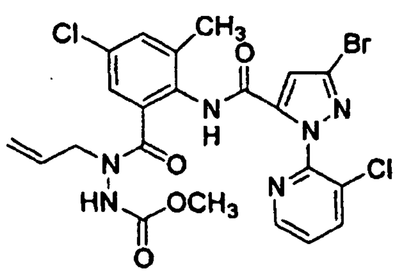

(1-11)

$^1$H-NMR ($CDCl_3$) δ: 2.04 (3H, s), 3.57 (3H, s), 3.74-4.20 (2H, brm), 5.19-5.29 (2H, m), 5.62-5.70 (1H, m), 7.03 (1H, s), 7.07 (1H, s), 7.21 (1H, s), 7.38 (1H, dd, J=8Hz, 5Hz), 7.52 (1H, s), 7.86 (1H, dd, J=8Hz, 1Hz), 8.45 (1H, dd, J=5Hz, 1Hz), 9.73 (1H, brs)

Production Example 10

To a mixture of 0.26 g of the compound (2-8) and 9 mL of pyridine, 0.35 g of methyl chloroformate was added dropwise under ice cooling, followed by stirring at room temperature for 4 hours. After adding water, the reaction mixture

was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.15 g of the present compound (1-12).
The present compound (1-12)

[Chemical Formula 144]

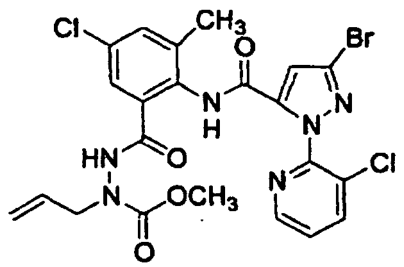

(1-12)

$^1$H-NMR ($CDCl_3$) δ: 2.20 (3H, s), 3.74 (3H, brs), 4.16-4.19. (2H, m), 5.15-5.21 (2H, m), 5.77-5.87 (1H, m), 7.04 (1H, s), 7.29-7.39 (3H, m), 7.85-7.91 (2H, m), 8.43 (1H, dd, J=5Hz, 2Hz), 9.46 (1H, s)

Production Example 11

(1) A mixture of 0.41 g of 2-[3-bromo-1-(3-chloro-2-pyridinyl)-1H-pyrazol-5-yl]-6,8-dibromo-4H-3,1-benzoxazin-4-one, 0.21 g of propargyl hydrazine dihydrochloride (a compound described in Synlett (2004), 2355-2356), 1.02 g of potassium carbonate and 7 mL of N,N-dimethylformamide was stirred at room temperature for 1 hour. After adding water, the reaction mixture was extracted with methyl tert-butyl ether. The organic layer was washed sequentially with water and a saturated sodium chloride solution, dried over anhydrous magnesium sulfate, and concentrated under reduced pressure. The resulting residue was subjected to column chromatography to obtain 0.12 g of a mixture of the compound (2-9) and the compound (2-10).
The compound (2-9)

[Chemical Formula 145]

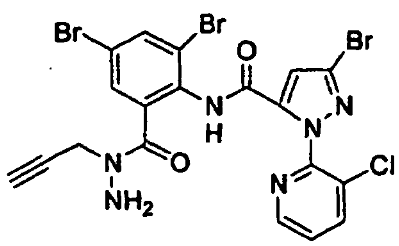

(2-9)

The compound (2-10)

[Chemical Formula 146]

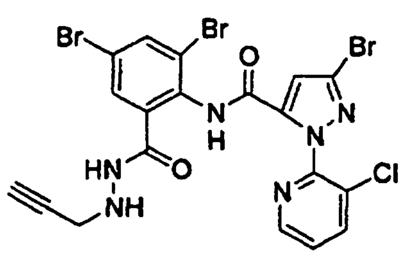

(2-10)

(2) To a mixture of 0.12 g of the above mixture of the compound (2-9) and the compound (2-10) and 2 mL of

pyridine, 60 μL of methyl chloroformate was added dropwise under ice cooling, followed by stirring at room temperature for 40 minutes. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.022 g of the present compound (1-31) and 0.088 g of the present compound (1-32).
The present compound (1-31)

[Chemical Formula 147]

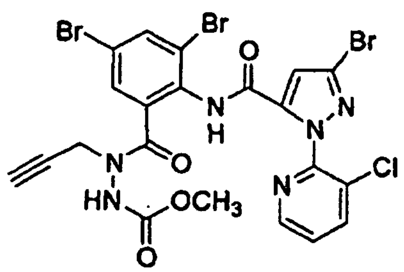

(1-31)

$^1$H-NMR ($CDCl_3$) δ: 2.25 (1H, s), 3.62-3.86 (3H, m), 4.02-4.47 (2H, brm), 7.20 (1H, s), 7.38-7.47 (2H, m), 7.62-7.66 (2H, m), 7.87 (1H, d, J=8Hz), 8.48 (1H, d, J=5Hz), 9.33 (1H, s)
The present compound (1-32)

[Chemical Formula 148]

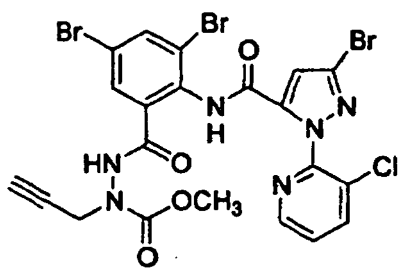

(1-32)

$^1$H-NMR ($CDCl_3$) δ: 2.22 (1H, s), 3.69 (3H, brs), 4.31 (2H, s), 7.10-7.16 (1H, m), 7.35-7.40 (1H, m), 7.64 (1H, brs), 7.80-7.88 (2H, m), 8.15 (1H, brs), 8.45 (1H, d, J=5Hz), 9.00 (1H, brs)

Production Example 12

(1) To a mixture of 1.85 g of methyl carbazate and 60 mL of tetrahydrofuran, 6.0 g of 6,8-dibromo-2H-3,1-benzoxazine-2,4-1H-dion was added under ice cooling, followed by stirring under ice cooling for 3 hours. After warming to room temperature, 0.46 g of methyl carbazate was further added to the reaction mixture, followed by stirring at room temperature for 15 hours. The reaction mixture was concentrated under reduced pressure and water was added to the resulting residue, and then the remaining solid was collected by filtration. The solid was washed sequentially with water and ethyl acetate to obtain 4.96 g of N-(2-amino-3,5-dibromobenzoyl)-N'-methoxycarbonylhydrazine. N-(2-amino-3,5-dibromobenzoyl)-N'-methoxycarbonylhydrazine $^1$H-NMR (DMSO-$d_6$) δ: 3.63 (3H, s), 6.55 (2H, s), 7.71 (1H, s), 7.79 (1H, s), 9.25 (1H, s), 10.32 (1H, s)

(2) To a mixture of 2.0 g of N-(2-amino-3,5-dibromobenzoyl)-N'-methoxycarbonylhydrazine, 0.90 g of potassium carbonate and 25 mL of N,N-dimethylformamide, 0.58 g of propargyl bromide was added dropwise under ice cooling, followed by stirring at room temperature for 2 hours and further stirring at 50°C for 4 hours. After cooling to room temperature and adding water, the reaction mixture was extracted with ethyl acetate. The organic layer was washed with water, dried over anhydrous magnesium sulfate, and concentrated under reduced pressure. The resulting residue was subjected to silica gel column chromatography to obtain 0.72 g of the compound (6-5).
The compound (6-5)

[Chemical Formula 149]

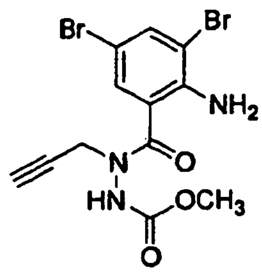

(6-5)

$^1$H-NMR ($CDCl_3$) δ: 2.35 (1H, t, J=3Hz), 3.76 (3H, s), 4.44 (2H, brs), 4.98 (2H, brs), 7:06 (1H, brs), 7.34 (1H, d, J=2Hz), 7.61 (1H, d, J=2Hz)

(3) A mixture of 0.31 g of 3-bromo-1-(3-chloro-2-pyridinyl)-1H-pyrazole-5-carboxylic acid, 0.32 g of oxalyl dichloride, a drop of N,N-dimethylformamide and 5 mL of toluene was stirred at room temperature for 1 hour. The reaction mixture was concentrated under reduced pressure to prepare an acid chloride.
Then, a mixture of the acid chloride, 0.35 g of the compound (6-5), 5 drops of pyridine and 5 drops of xylene was stirred at 100˚C for 1 hour. After cooling to room temperature and adding water, the reaction mixture was extracted with ethyl acetate. The organic layer was washed with water, dried over anhydrous magnesium sulfate, and concentrated under reduced pressure. The resulting residue was subjected to silica gel column chromatography to obtain 0.43 g of the present compound (1-31).

Production Example 13

(1) In place of 6,8-dibromo-2H-3,1-benzoxazine-2,4-1H-dion of Production Example 12 (1), 6-chloro-8-methyl-2H-3,1-benzoxazine-2,4-1H-dion was used to obtain N-(2-amino-5-chloro-3-methylbenzoyl)-N'-methoxycarbonylhydrazine. N-(2-amino-5-chloro-3-methylbenzoyl)-N'-methoxycarbonylhydrazine.
$^1$H-NMR ($CDCl_3$) δ: 2.13 (3H, s), 3.79 (3H, s), 5.50 (2H, s), 6.81 (1H, brs), 7.13 (1H, d, J=2Hz), 7.30 (1H, d, J=2Hz), 7.88 (1H, brs)

(2) In place of N-(2-amino-3,5-dibromobenzoyl)-N'-methoxycarbonylhydrazine of Production Example 12 (2), N-(2-amino-5-chloro-3-methylbenzoyl)-N'-methoxycarbonylhydrazine was used to obtain the compound (6-6).
The compound (6-6)

[Chemical Formula 150]

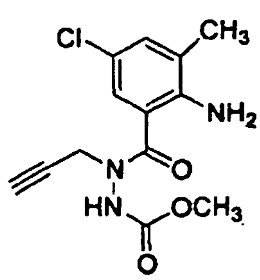

(6-6)

$^1$H-NMR ($CDCl_3$) δ: 2.13 (3H, s), 2.33 (1H, t, J=3Hz), 3.74 (3H, s), 4.44 (4H, brs), 7.08-7.13 (3H, m)

(3) In place of the compound (6-5) of Production Example 12 (3), the compound (6-6) was used to obtain the present compound (1-35).
The present compound (1-35)

[Chemical Formula 151]

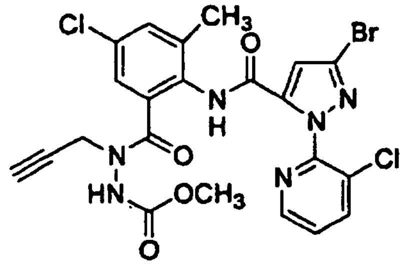

(1-35)

$^1$H-NMR ($CDCl_3$) δ: 2.08 (3H, s), 2.23 (1H, s), 3.60-3.77 (3H, m), 3.98-4.62 (2H, m), 7.08 (2H, brs), 7.18 (1H, s), 7.37 (1H, dd, J=8Hz, 5Hz), 7.70 (1H, s), 7.85 (1H, dd, J=8Hz, 1Hz), 8.46 (1H, dd, J=5Hz, 1Hz), 9.55 (1H, brs)

Production Example 14

(1) A mixture of 0.50 g of 2-[3-bromo-1-(3-chloro-2-pyridinyl)-1H-pyrazol-5-yl]-6-chloro-8-methyl-4H-3,1-benzoxazin-4-one, 0.45 g of benzylhydrazine dihydrochloride, 1.21 g of potassium carbonate and 10 mL of N,N-dimethylformamide was stirred at room temperature for 1 hour. After adding water, the reaction mixture was extracted with ethyl acetate. The organic layer was washed sequentially with water and a saturated sodium chloride solution, dried over anhydrous magnesium sulfate, and concentrated under reduced pressure. The resulting residue was subjected to column chromatography to obtain 0.31 g of a mixture of the compound (2-11) and the compound (2-12).
The compound (2-11)

[Chemical Formula 152]

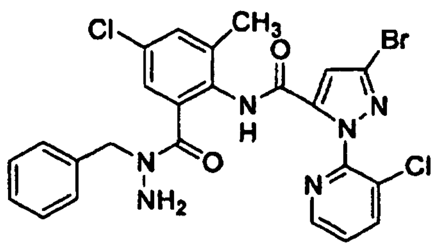

(2-11)

The compound (2-12)

[Chemical Formula 153]

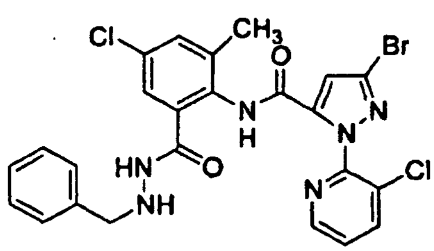

(2-12)

(2) To a mixture of 0.31 g of the above mixture of the compound (2-11) and the compound (2-12) and 4 mL of pyridine, 260 μL of methyl chloroformate was added dropwise under ice cooling, followed by stirring at room temperature for 18 hours. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.090 g of the present compound (1-57) and 0.057 g of the present compound (1-58).
The present compound (1-57)

[Chemical Formula 154]

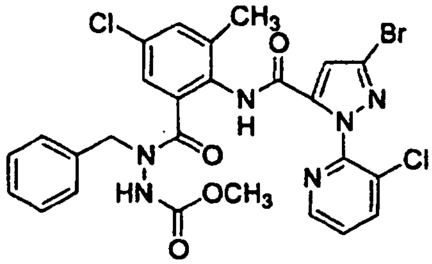

(1-57)

$^1$H-NMR (DMSO-$D_6$, 100°C) δ: 2.13 (3H, s), 3.48 (3H, s), 4.65 (2H, brs), 7.03-7.10 (2H, m), 7.23-7.26 (5H, m), 7.37 (1H, brs), 7.57 (1H, dd, J=8Hz, 5Hz), 8.10 (1H, d, J=8Hz), 8.45 (1H, d, J=5Hz), 8.85 (1H, brs), 9.62 (1H, brs) The present compound (1-58)

[Chemical Formula 155]

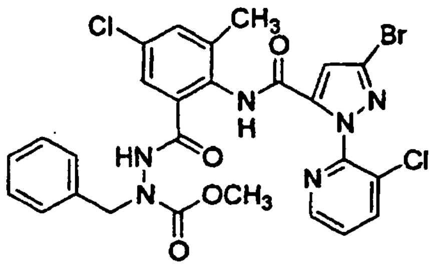

(1-58)

$^1$H-NMR (DMSO-$D_6$) δ: 2.22 (3H, s), 3.55-3.68 (3H, m), 4.43 (2H, brs), 7.17-7.27 (6H, m), 7.37 (1H, s), 7.48-7.53 (1H, m), 7.56 (1H, d, J=2Hz), 8.06 (1H, d, J=7Hz), 8.40 (1H, d, J=5Hz), 10.24 (1H, s), 10.58 (1H, s)

Production Example 15

(1) A mixture of 0.61 g of 2-[3-bromo-1-(3-chloro-2-pyridinyl)-1H-pyrazol-5-yl]-6,8-dibromo-4H-3,1-benzoxazin-4-one, 0.33 g of cyclohexylhydrazine hydrochloride, 0.60 g of potassium carbonate and 10 mL of N,N-dimethylformamide was stirred at room temperature for 2 hours. After adding water, the reaction mixture was extracted with methyl tert-butyl ether. The organic layer was washed sequentially with water and a saturated sodium chloride solution, dried over anhydrous magnesium sulfate, and concentrated under reduced pressure. The resulting residue was subjected to column chromatography to obtain 0.39 g of the compound (2-13).
The compound (2-13)

[Chemical Formula 156]

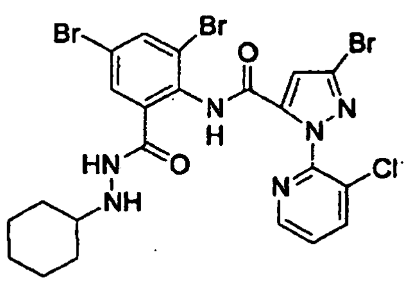

(2-13)

$^1$H-NMR ($CDCl_3$) δ: 1.17-1.19 (4H, m), 1.55-1.85 (6H, m), 2.73-2.79 (1H, m), 4.60 (1H, brs), 7.29 (1H, s), 7.34 (1H, dd, J=8Hz, 5Hz), 7.38 (1H, d, J=2Hz), 7.65 (1H, d, J=2Hz), 7.77 (1H, brs), 7.84 (1H, dd, J=8Hz, 2Hz), 8.45 (1H, dd, J=5Hz, 2Hz), 9.57 (1H, s)

(2) To a mixture of 0.25 g of the compound (2-13) and 3 mL of pyridine, 290 μL of methyl chloroformate was

added dropwise under ice cooling, followed by stirring at room temperature for 20 hours. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.23 g of the present compound (1-59).
The present compound (1-59)

[Chemical Formula 157]

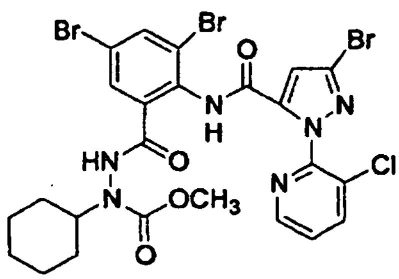
(1-59)

$^1$H-NMR ($CDCl_3$) δ: 0.87-1.80 (10H, m), 3.65 (3H, brs), 4.01 (1H, brs), 7.22 (1H, s), 7.35 (1H, dd, J=8Hz, 5Hz), 7.52 (1H, brs), 7.75 (1H, d, J=2Hz), 7.81-7.87 (2H, m), 8.41 (1H, dd, J=5Hz, 2Hz), 9.42 (1H, s)

Production Example 16

(1) In place of 2-[3-bromo-1-(3-chloro-2-pyridinyl)-1H-pyrazol-5-yl]-6,8-dibromo-4H-3,1-benzoxazin-4-one of Production Example 15 (1), 2-[3-bromo-1-(3-chloro-2-pyridinyl)-1H-pyrazol-5-yl]-6-chloro-8-methyl-4H-3,1-benzoxazin-4-one was used to obtain the compound (2-14). The compound (2-14)

[Chemical Formula 158]

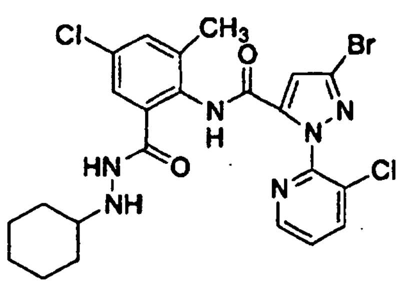
(2-14)

$^1$H-NMR ($CDCl_3$) δ: 1.13-1.28 (4H, m), 1.60-1.86 (6H, m), 2.15 (3H, s), 2.75-2.80 (1H, m), 4.66 (1H, brs), 7.11 (1H, s), 7.16 (1H, d, J=2Hz), 7.23 (1H, d, J=2Hz), 7.37 (1H, dd, J=8Hz, 5Hz), 7.66 (1H, s), 7.84 (1H, dd, J=8Hz, 1Hz), 8.46 (1H, dd, J=5Hz, 1Hz), 9.92 (1H, s)

(2) In place of the compound (2-13) of Production Example 15 (2), the compound (2-14) was used to obtain the present compound (1-60).
The present compound (1-60)

[Chemical Formula 159]

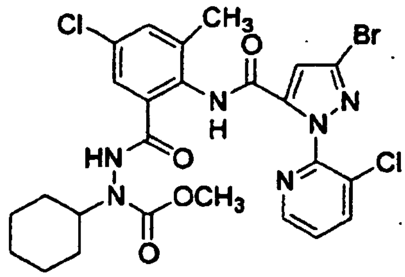

(1-60)

$^1$H-NMR ($CDCl_3$) δ: 0.87-1.88 (10H, m), 2.20 (3H, s), 3.69 (3H, brs), 4.07 (1H, brs), 7.05 (1H, s), 7.30-7.33 (2H, m), 7.37 (1H, dd, J=8, 5Hz), 7.53 (1H, s), 7.85 (1H, dd, J=8Hz, 2Hz), 8.43 (1H, dd, J=5Hz, 2Hz), 9.48 (1H, s)

Production Example 17

(1) A mixture of 0.60 g of 2-[3-bromo-1-(3-chloro-2-pyridinyl)-1H-pyrazol-5-yl]-6,8-dibromo-4H-3,1-benzoxazin-4-one, 0.41 g of 2-hydroxyethylhydrazine and 20 mL of N,N-dimethylformamide was stirred at room temperature for 0.5 hours. After adding water, the reaction mixture was extracted with methyl tert-butyl ether. The organic layer was washed sequentially with water and a saturated sodium chloride solution, dried over anhydrous magnesium sulfate, and concentrated under reduced pressure. The resulting residue was subjected to column chromatography to obtain 0.21 g of the compound (2-15).
The compound (2-15)

[Chemical Formula 160]

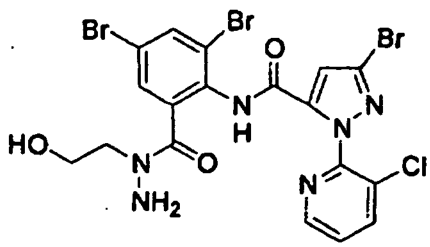

(2-15)

$^1$H-NMR (DMSO-$D_6$) δ: 3.40-3.57 (4.0H, m), 4.51 (1.3H, s), 4.73 (0.7H, t, J=5Hz), 4.81 (0.7H, s), 5.00 (0.3H, brs), 7.35 (0.3H, s), 7.41 (0.7H, s), 7.54 (0.6H, d, J=2Hz), 7.61-7.64 (1.4H, m), 7.95 (0.7H, d, J=2Hz), 8.03 (0.3H, d, J=2Hz), 8.16-8.20 (1.0H, m), 8.49-8.52 (1.0H, m), 10.37 (0.7H, s), 10.55 (0.3H, s)

(2) To a mixture of 0.21 g of the compound (2-15) and 3 mL of pyridine, 40 μL of methyl chloroformate was added dropwise under ice cooling, followed by stirring under ice cooling for 1 hour. To the reaction mixture, 25 μL of methyl chloroformate was added dropwise, followed by stirring at room temperature for 1.5 hours. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.12 g of the present compound (1-61).
The present compound (1-61)

[Chemical Formula 161]

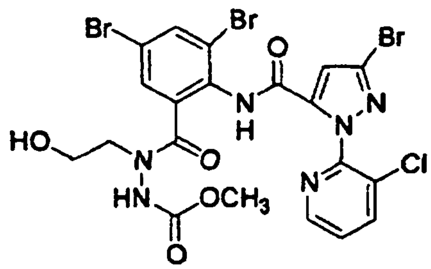

(1-61)

$^1$H-NMR (DMSO-$D_6$) δ: 3.26-3.56 (7.0H, m), 4.72 (1.0H, s), 7.39-7.49 (2.0H, m), 7.60-7.65 (1.0H, m), 8.04-8.09 (1.0H, m), 8.16-8.21 (1.0H, m), 8.49-8.51 (1.0H, m), 8.87 (0.2H, s), 9.16 (0.6H, s), 9.74 (0.2H, s), 10.19 (0.6H, s), 10.53 (0.4H, s)

Production Example 18

(1) To a mixture of 1.3 g of N'-isopropylidenehydrazinecarboxylic acid tert-butyl ester (a compound described in Synlett (2004), 2355-2356), 0.44 g of potassium hydroxide, 0.20 g of tetrabutyl ammonium sulfate and 20 mL of toluene, 1.05 g of 1,1,3-trichloro-2-propene was added dropwise at 50˚C. The reaction mixture was stirred at 80˚C for 3.5 hours and 0.44 g of 1,1,3-trichloro-2-propene was further added, followed by stirring at 80˚C for 1 hour. After cooling and adding water, the reaction mixture was extracted with ethyl acetate. The organic layer was washed sequentially with water and a saturated sodium chloride solution, dried over anhydrous magnesium sulfate, and concentrated under reduced pressure. The resulting residue was subjected to column chromatography to obtain 0.74 g of a mixture of N-(3,3-dichloro-2-propenyl)-N'-isopropylidenehydrazinecarboxylic acid tert-butyl ester and N-(3,3-dichloro-2-propenyl)hydrazinecarboxylic acid tert-butyl ester. N-(3,3-dichloro-2-propenyl)-N'-isopropylidenehydrazinecarboxylic acid tert-butyl ester $^1$H-NMR ($CDCl_3$) δ: 1.47 (9H, s), 1.88 (3H, s), 2.07 (3H, s), 4.18 (2H, d, J=7Hz), 6.02 (1H, t, J=7Hz) N-(3,3-dichloro-2-propenyl)hydrazinecarboxylic acid tert-butyl ester
$^1$H-NMR ($CDCl_3$) δ: 1.48 (9H, s), 4.01 (2H, brs), 4.11 (2H, d, J=7Hz), 5.99 (1H, t, J=7Hz)

(2) A mixture of 0.74 g of the mixture of N-(3,3-dichloro-2-propenyl)-N'-isopropylidenehydrazinecarboxylic acid tert-butyl ester and N-(3,3-dichloro-2-propenyl)hydrazinecarboxylic acid tert-butyl ester and 20 mL of a hydrogen chloride-ethanol solution was stirred at room temperature for 16 hours. The reaction mixture was concentrated under reduced pressure to obtain 0.48 g of 3,3-dichloro-2-propenylhydrazine hydrochloride. 3,3-dichloro-2-propenylhydrazine hydrochloride $^1$H-NMR (DMSO-$D_6$) δ: 3.36 (2H, brs), 3.63 (2H, d, J=7Hz), 6.28 (1H, t, J=7Hz), 9.36 (2H, s)

(3) A mixture of 0.63 g of 2-[3-bromo-1-(3-chloro-2-pyridinyl)-1H-pyrazol-5-yl]-6,8-dibromo-4H-3,1-benzoxazin-4-one, 0.48 g of 3,3-dichloro-2-propenylhydrazine hydrochloride, 0.93 g of potassium carbonate and 10 mL of N,N-dimethylformamide was stirred at room temperature for 1.5 hours. After adding water, the reaction mixture was extracted with methyl tert-butyl ether. The organic layer was washed sequentially with water and a saturated sodium chloride solution, dried over anhydrous magnesium sulfate, and concentrated under reduced pressure. The resulting residue was subjected to column chromatography to obtain 0.23 g of the compound (2-16) and 0.18 g of the compound (2-17).
The compound (2-16)

[Chemical Formula 162]

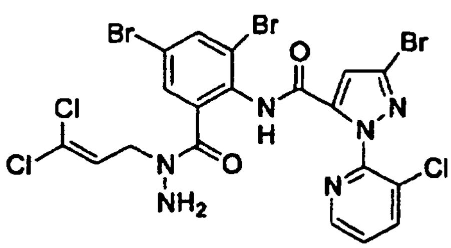

(2-16)

$^1$H-NMR ($CDCl_3$) δ: 3.97 (2.0H, brs), 4.27-4.33 (2.0H, m), 5.88-5.90 (1.0H, m), 7.15 (0.5H, s), 7.23 (0.5H, s), 7.34-7.43 (2.0H, m), 7.59-7.64 (1.0H, m), 7.85-7.92 (1.0H, m), 8.46-8.50 (1.0H, m), 9.30 (0.5H, brs), 9.43 (0.5H, brs)
The compound (2-17)

[Chemical Formula 163]

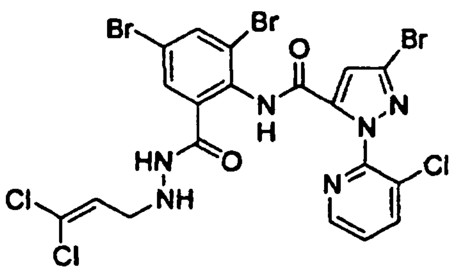

(2-17)

H-NMR ($CDCl_3$) δ: 3.62 (2H, t, J=7Hz), 4.76-4.80 (1H, m), 5.99 (1H, t, J=7Hz), 7.16 (1H, s), 7.36 (1H, dd, J=8Hz, 5Hz),

7.43 (1H, d, J=2Hz), 7.67-7.71 (2H, m), 7.86 (1H, dd, J=8Hz, 1Hz), 8.45 (1H, dd, J=5Hz, 1Hz), 9.15 (1H, s)

(4) To the mixture of 0.17 g of the compound (2-16) and 3 mL of pyridine, 75 μL of methyl chloroformate was added dropwise under ice cooling. While the reaction mixture is stirred at room temperature for 8 hour, 75 μL of methyl chloroformate was added three times. After adding water, the reaction mixture was concentrated under reduced pressure. To the resulting residue, water was added, followed by extraction with ethyl acetate. The organic layer was washed with a saturated sodium chloride solution, dried over anhydrous magnesium sulfate, and concentrated under reduced pressure. The resulting residue was subjected to silica gel column chromatography to obtain 0.11 g of the present compound (1-63).

The present compound (1-63)

[Chemical Formula 164]

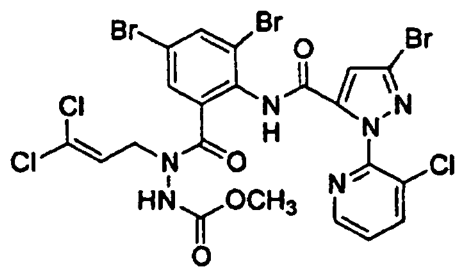

(1-63)

$^1$H-NMR ($CDCl_3$) δ: 3.58-3.82 (3H, brm), 4.22-4.24 (2H, m), 5.96 (1H, t, J=7Hz), 7.13 (1H, s), 7.39 (1H, dd, J=8Hz, 5Hz), 7.59 (1H, brs), 7.82 (1H, d, J=2Hz), 7.87 (1H, dd, J=8Hz, 2Hz), 8.11 (1H, brs), 8.42 (1H, dd, J=5Hz, 1Hz), 8.97 (1H, brs)

Production Example 19

In place of the compound (2-16) of Production Example 18 (4), the compound (2-17) was used to obtain the present compound (1-64).

The present compound (1-64)

[Chemical Formula 165]

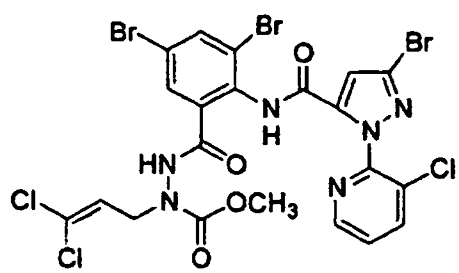

(1-64)

$^1$H-NMR ($CDCl_3$) δ: 3.64 (3H, s), 4.31 (2H, d, J=7Hz), 5.87 (1H, t, J=7Hz), 7.17 (1H, s), 7.40-7.49 (3H, m), 7.68 (1H, s), 7.91 (1H, dd, J=8Hz, 2Hz), 8.49 (1H, dd, J=5Hz, 2Hz), 9.10 (1H, s)

Production Example 20

(1) To a mixture of 1.5 g of N-(2-amino-3,5-dibromobenzoyl)-N'-methoxycarbonylhydrazine (a compound obtained in Production Example 12(1)), 0.68 g of potassium carbonate and 18 mL of N,N-dimethylformamide, 0.50 g of (bromomethyl)cyclopropane was added dropwise under ice cooling, followed by stirring at room temperature for 20 hours and further stirring at 60˚C for 4 hours. After cooling to room temperature and adding water, the reaction mixture was extracted with ethyl acetate. The organic layer was washed with water, dried over anhydrous magnesium sulfate, and concentrated under reduced pressure. The resulting residue was subjected to silica gel column chromatography to obtain 0.39 g of the compound (6-7). The compound (6-7)

[Chemical Formula 166]

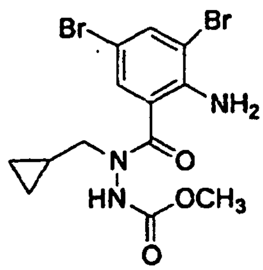

(6-7)

$^1$H-NMR ($CDCl_3$) δ: 0.18-0.24 (2H, m), 0.54-0.57 (2H, m), 0.97-1.06 (1H, m), 3.44 (2H, brs), 3.76 (3H, s), 4.96 (2H, brs), 7.10 (1H, brs), 7.28 (1H, d, J=2Hz), 7.58 (1H, d, J=2Hz)

(2) A mixture of 0.31 g of 3-bromo-1-(3-chloro-2-pyridinyl)-1H-pyrazole-5-carboxylic acid, 0.35 g of oxalyl dichloride, 3 drops of N,N-dimethylformamide and 3 mL of toluene was stirred at room temperature for 1 hour. The reaction mixture was concentrated under reduced pressure to prepare an acid chloride.

Then, the mixture of the acid chloride, 0.39 g of the compound (6-7), 500 μL of pyridine and 500 μL of xylene was stirred at 100°C for 1 hour. After cooling to room temperature and adding water, the reaction mixture was extracted with ethyl acetate. The organic layer was washed sequentially with 1 N hydrochloric acid, water and a saturated sodium chloride solution, dried over anhydrous magnesium sulfate, and concentrated under reduced pressure. The resulting residue was subjected to silica gel column chromatography to obtain the present compound (1-67).

The present compound (1-67)

[Chemical Formula 167]

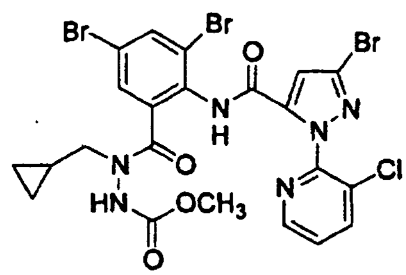

(1-67)

$^1$H-NMR ($CDCl_3$) δ: 0.16 (2H, brs), 0.40 (2H, brs), 0.78-0.90 (1H, m), 3.12-3.83 (5H, m), 7.28 (1H, s), 7.38 (1H, dd, J=8Hz, 5Hz), 7.45 (1H, d, J=2Hz), 7.59 (1H, s), 7.61 (1H, d, J=2Hz), 7.85 (1H, dd, J=8Hz, 2Hz), 8.44 (1H, dd, J=5Hz, 2Hz), 9.62 (1H, brs)

The present compounds produced by processes according to those in Production Examples described above are shown in Table 69 to Table 73 together with the compound numbers.

[Chemical Formula 168]

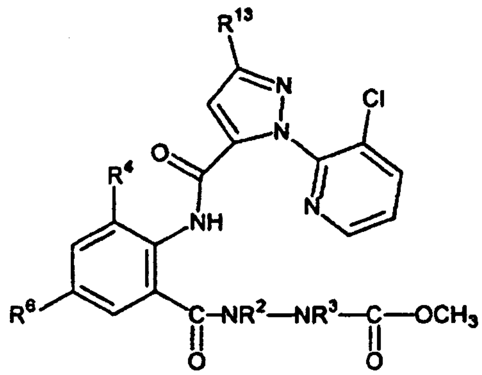

(1)

Table 69

| Compound Number | $R^2$ | $R^3$ | $R^4$ | $R^6$ | $R^{13}$ | Properties |
|---|---|---|---|---|---|---|
| 1-1 | $CH_2CH_3$ | $CH_2CN$ | Br | Br | Br | Described in Production Example 1 (6) |
| 1-2 | $CH_2CN$ | $CH_2CH_3$ | Br | Br | Br | |
| 1-3 | $CH_3$ | $CH_2CN$ | Br | Br | Br | |
| 1-4 | $CH_2CN$ | $CH_3$ | Br | Br | Br | |
| 1-5 | H | 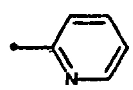 | Br | Br | Br | Described in Production Example 5 (2) |
| 1-6 | H | 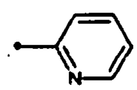 | $CH_3$ | Cl | Br | Described in Production Example 6 (2) |
| 1-7 | $CH_2CH=CH_2$ | H | Br | Br | Br | Described in Production Example 7 (2) |
| 1-8 | H | $CH_2CH=CH_2$ | Br | Br | Br | Described in Production Example 7 (2) |
| 1-9 | $CH_2CH=CH_2$ | H | Cl | Cl | Br | Described in Production Example 8 (2) |
| 1-10 | H | $CH_2CH=CH_2$ | Cl | Cl | Br | Described in Production Example 8 (2) |
| 1-11 | $CH_2CH=CH_2$ | H | $CH_3$ | Cl | Br | Described in Production Example 9 (2) |
| 1-12 | H | $CH_2CH=CH_2$ | $CH_3$ | Cl | Br | Described in Production Example 10 |

Table 70

| Compound Number | $R^2$ | $R^3$ | $R^4$ | $R^6$ | $R^{13}$ | Properties |
|---|---|---|---|---|---|---|
| 1-13 | $CH_2CH=CH_2$ | H | $CH_3$ | CN | Br | |
| 1-14 | H | $CH_2CH=CH_2$ | $CH_3$ | CN | Br | |

(continued)

| Compound Number | $R^2$ | $R^3$ | $R^4$ | $R^6$ | $R^{13}$ | Properties |
|---|---|---|---|---|---|---|
| 1-15 | $CH_2CH=CH_2$ | H | Br | Br | Cl | |
| 1-16 | H | $CH_2CH-CH_2$ | Br | Br | Cl | |
| 1-17 | $CH_2CH=CH_2$ | H | Cl | Cl | Cl | |
| 1-18 | H | $CH_2CH=CH_2$ | Cl | Cl | Cl | |
| 1-19 | $CH_2CH=CH_2$ | H | $CH_3$ | Cl | Cl | |
| 1-20 | H | $CH_2CH=CH_2$ | $CH_3$ | Cl | Cl | |
| 1-21 | $CH_2CH=CH_2$ | H | $CH_3$ | CN | Cl | |
| 1-22 | H | $CH_2CH=CH_2$ | $CH_3$ | CN | Cl | |
| 1-23 | $CH_2CH=CH_2$ | H | Br | Br | $CF_3$ | |
| 1-24 | H | $CH_2CH=CH_2$ | Br | Br | $CF_3$ | |
| 1-25 | $CH_2cH=CH_2$ | H | Cl | Cl | $CF_3$ | |
| 1-26 | H | $CH_2CH=CH_2$ | Cl | Cl | $CF_3$ | |
| 1-27 | $CH_2CH=CH_2$ | H | $CH_3$ | Cl | $CF_3$ | |
| 1-28 | H | $CH_2CH=CH_2$ | $CH_3$ | Cl | $CF_3$ | |
| 1-29 | $CH_2CH=CH_2$ | H | $CH_3$ | CN | $CF_3$ | |
| 1-30 | H | $CH_2CH=CH_2$ | $CH_3$ | CN | $CF_3$ | |

Table 71

| Compound Number | $R^2$ | $R^3$ | $R^4$ | $R^6$ | $R^{13}$ | Properties |
|---|---|---|---|---|---|---|
| 1-31 | $CH_2C\equiv CH$ | H | Br | Br | Br | Described in Production Example 11(2) |
| 1-32 | H | $CH_2C\equiv CH$ | Br | Br | Br | Described in Production Example 11(2) |
| 1-33 | $CH_2C\equiv CH$ | H | Cl | Cl | Br | |
| 1-34 | H | $CH_2C\equiv CH$ | Cl | Cl | Br | |
| 1-35 | $CH_2C\equiv CH$ | H | $CH_3$ | Cl | Br | Described in Production Example 13(3) |
| 1-36 | H | $CH_2C\equiv CH$ | $CH_3$ | Cl | Br | |
| 1-37 | $CH_2C\equiv CH$ | H | $CH_3$ | CN | Br | |
| 1-38 | H | $CH_2C\equiv CH$ | $CH_3$ | CN | Br | |
| 1-39 | $CH_2C\equiv CH$ | H | Br | Br | Cl | |
| 1-40 | H | $CH_2C\equiv CH$ | Br | Br | Cl | |
| 1-41 | $CH_2C\equiv CH$ | H | Cl | Cl | Cl | |
| 1-42 | H | $CH_2C\equiv CH$ | Cl | Cl | Cl | |
| 1-43 | $CH_2C\equiv CH$ | H | $CH_3$ | Cl | Cl | |
| 1-44 | H | $CH_2C\equiv CH$ | $CH_3$ | Cl | Cl | |
| 1-45 | $CH_2C\equiv CH$ | H | $CH_3$ | CN | Cl | |
| 1-46 | H | $CH_2C\equiv CH$ | $CH_3$ | CN | Cl | |
| 1-47 | $CH_2C\equiv CH$ | H | Br | Br | $CF_3$ | |
| 1-48 | H | $CH_2C\equiv CH$ | Br | Br | $CF_3$ | |

Table 72

| Compound Number | $R^2$ | $R^3$ | $R^4$ | $R^6$ | $R^{13}$ | Properties |
|---|---|---|---|---|---|---|
| 1-49 | $CH_2C{\equiv}CH$ | H | Cl | Cl | $CF_3$ | |
| 1-50 | H | $CH_2C{\equiv}CH$ | Cl | Cl | $CF_3$ | |
| 1-51 | $CH_2C{\equiv}CH$ | H | $CH_3$ | Cl | $CF_3$ | |
| 1-52 | H | $CH_2C{\equiv}CH$ | $CH_3$ | Cl | $CF_3$ | |
| 1-53 | $CH_2C{\equiv}CH$ | H | $CH_3$ | CN | $CF_3$ | |
| 1-54 | H | $CH_2C{\equiv}CH$ | $CH_3$ | CN | $CF_3$ | |
| 1-55 | 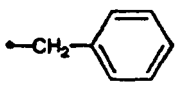 | H | Br | Br | Br | |
| 1-56 | H | 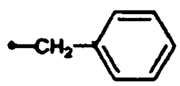 | Br | Br | Br | |
| 1-57 | 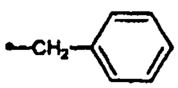 | H | $CH_3$ | Cl | Br | Described in Production Example 14(2) |
| 1-58 | H | 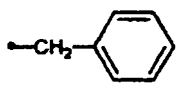 | $CH_3$ | Cl | Br | Described in Production Example 14 (2) |
| 1-59 | H | 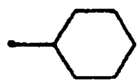 | Br | Br | Br | Described in Production Example 15(2) |
| 1-60 | H | 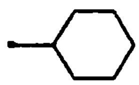 | $CH_3$ | Cl | Br | Described in Production Example 15 (2) |

Table 73

| Compound Number | $R^2$ | $R^3$ | $R^4$ | $R^6$ | $R^{13}$ | Properties |
|---|---|---|---|---|---|---|
| 1-61 | $CH_2CH_2OH$ | H | Br | Br | Br | Described in Production Example 17 (2) |
| 1-62 | $CH_2CH_2OH$ | H | $CH_3$ | Cl | Br | |
| 1-63 | $CH_2CH{=}CCl_2$ | H | Br | Br | Br | Described in Production Example 18(4) |
| 1-64 | H | $CH_2CH{=}CCl_2$ | Br | Br | Br | Described in Production Example 19 |
| 1-65 | $CH_2CH{=}CCl_2$ | H | $CH_3$ | Cl | Br | |
| 1-66 | H | $CH_2CH{=}CCl_2$ | $CH_3$ | Cl | Br | |
| 1-67 | 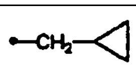 | H | Br | Br | Br | Described in Production Example 20(2) |
| 1-68 | 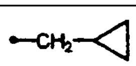 | H | $CH_3$ | Cl | Br | |

Hereinafter, Formulation Examples are shown. All parts are by weight.

Formulation Example 1

Into a mixture of 35 parts of xylene and 35 parts of N,N-dimethylformamide, 10 parts of each of the present compounds (1-1) to (1-68) is dissolved, and then 14 parts of polyoxyethylene styrylphenyl ether and 6 parts of calcium dodecylbenzenesulfonate are added, followed by stirring to obtain a 10% emulsifiable concentrate.

Formulation Example 2

To a mixture of 4 parts of sodium lauryl sulfate, 2 parts of calcium ligninsulfonate, 20 parts of synthetic hydrous silicon oxide fine powder and 54 parts of diatomaceous earth, 20 parts of each of the present compounds (1-1) to (1-68) is added, followed by stirring to obtain a 20% wettable formulation.

Formulation Example 3

To 2 parts of each of the present compounds (1-1) to (1-68), 1 part of synthetic hydrous silicon oxide fine powder, 2 parts of calcium ligninsulfonate, 30 parts of bentonite and 65 parts of kaolin clay are added, and then stirred thoroughly. Then, an appropriate amount of water is added to the mixture, followed by stirring, granulation with a granulator and forced-air drying to obtain a 2% granular formulation.

Formulation Example 4

Into an appropriate amount of acetone, 1 part of each the present compounds (1-1) to (1-68) is dissolved, and then 5 parts of synthetic hydrous silicon oxide fine powder, 0.3 parts of PAP and 93.7 parts of fubasami clay are added, followed by stirring and removal of acetone from the mixture by evaporation to obtain a 1% powder formulation.

Formulation Example 5

A mixture of 10 parts of each of the present compounds (1-1) to (1-68), 35 parts of white carbon containing 50 parts of polyoxyethylene alkyl ether sulfate ammonium salt, and 55 parts of water is finely ground by a wet grinding method to obtain a 10% flowable formulation.

Formulation Example 6

In 5 parts of xylene and 5 parts of trichloroethane, 0.1 parts of each of the present compounds (1-1) to (1-68) is dissolved, and then solution is mixed with 89.9 parts of deodorized kerosene to obtain a 0.1% oil solution.

Formulation Example 7

In 0.5 mL of acetone, 10 mg of each of the present compounds (1-1) to (1-68) is dissolved and the solution is mixed uniformly with 5 g of a solid feed powder for an animal (solid feed powder for rearing and breeding CE-2, manufactured by CLEA Japan, Inc.), and then dried by evaporation of acetone to obtain a poison bait.
Hereinafter, harmful arthropod controlling activity of the present compound is shown by Test Examples.

Test Example 1

The flowable formulation of each of the present compounds (1-1), (1-5), (1-6), (1-7), (1-8), (1-9), (1-10), (1-12), (1-57), (1-58), (1-59), (1-60), (1-61), (1-63) and (1-64) obtained in Formulation Example 5 was diluted with water so that the active ingredient concentration became 500 ppm. Further, the flowable formulation of each of the present compounds (1-11), (1-31) and (1-32) was diluted with water so that the active ingredient concentration became 200 ppm. Thus, test spray solutions were prepared.
On the other hand, cabbage was planted in a polyethylene cup, and grown until the third true leaf or the fourth true leaf was developed. The test spray solution as described above was sprayed in an amount of 20 mL/cup on the cabbage. After the spray solution on the cabbage was dried, 10 third-instar larvae of diamondback moths (Plutella *xylostella)* were placed on the cabbage. After 5 days, the number of diamondback moths was counted, and the controlling value was calculated by the following equation. Controlling value (%) = $\{1 - (Cb \times Tai) / (Cai \times Tb)) \times 100$ wherein

Cb: the number of worms in an untreated group before treatment;

Cai: the number of worms in an untreated group on observation;

Tb: the number of worms in a treated group before treatment;

Tai: the number of worms in a treated group on observation.

As a result, the group treated with each of the present compounds (1-1), (1-5), (1-6), (1-7), (1-8), (1-9), (1-10), (1-11), (1-12), (1-31), (1-32), (1-57), (1-58), (1-59), (1-60), (1-61), (1-63) and (1-64) exhibited a controlling value of 100%.

Test Example 2

The flowable formulation of each of the present compounds (1-1), (1-5), (1-6), (1-7), (1-8), (1-9), (1-10), (1-12), (1-57), (1-58), (1-59), (1-61), (1-63) and (1-64) obtained in Formulation Example 5 was diluted with water so that the active ingredient concentration became 500 ppm. Further, the flowable formulation of each of the present compounds (1-11), (1-31) and (1-32) was diluted with water so that the active ingredient concentration became 200 ppm. Thus, test spray solutions were prepared.
On the other hand, cucumber was planted in a polyethylene cup, and was grown until the first true leaf was developed. About 30 cotton aphids (*Aphis gossypii*) were placed on the cucumber. One day after, the test spray solution as described above was sprayed in an amount of 20 mL/cup on the cucumber. Six days after spraying, the number of cotton aphids was counted, and a controlling value was calculated by the following equation.

$$\text{Controlling value (\%)} = \{1 - (Cb \times Tai)/(Cai \times Tb)\} \times 100$$

As a result, the group treated with each of the present compounds (1-1), (1-5), (1-6), (1-7), (1-8), (1-9), (1-10), (1-11), (1-12), (1-31), (1-32), (1-57), (1-58), (1-59), (1-61), (1-63) and (1-64) exhibited a controlling value of 90% or more.

Test Example 3

The flowable formulation of each of the present compounds (1-1), (1-5), (1-6), (1-7), (1-8), (1-9), (1-10), (1-12), (1-57), (1-58), (1-59), (1-60), (1-61), (1-63) and (1-64) obtained in Formulation Example 5 was diluted with water so that the active ingredient concentration became 500 ppm. Further, the flowable formulation of each of the present compounds (1-11), (1-31) and (1-32) was diluted with water so that the active ingredient concentration became 200 ppm. Thus, test spray solutions were prepared.
On the other hand, cabbage was planted in a polyethylene cup, and grown until the third true leaf or the fourth true leaf was developed. The test spray solution as described above was sprayed in an amount of 20 mL/cup on the cabbage. After the spray solution sprayed on the cabbage was dried, 10 fourth-instar larvae of common cutworm (*Spodoptera litura*) were placed on the cabbage. After 4 days, the number of common cutworm surviving on the cabbage leaves was counted, and a controlling value was calculated by the following equation.

$$\text{Controlling value (\%)} = \{1 - (Cb \times Tai)/(Cai \times Tb)\} \times 100$$

As a result, the group treated with each of the present compounds (1-1), (1-5), (1-6), (1-7), (1-8), (1-9), (1-10), (1-11), (1-12), (1-31), (1-32), (1-57), (1-58), (1-59), (1-60), (1-61), (1-63) and (1-64) exhibited a controlling value of 80% or more.

Test Example 4

The flowable formulation of each of the present compounds (1-1), (1-5), (1-6), (1-7), (1-8), (1-9), (1-10), (1-12), (1-60), (1-63) and (1-64) obtained in Formulation Example 5 was diluted with water so that the active ingredient concentration became 500 ppm. Further, the flowable formulation of each of the present compounds (1-11), (1-31) and (1-32) was diluted with water so that the active ingredient concentration became 200 ppm. Thus, test spray solutions were prepared.
On the other hand, 20 mL of the test spray solution as described above was sprayed to an apple seedling (28 day-old seeding, tree height: about 15 cm) planted in a plastic cup. The apple seedling was air-dried to such an extent that the spray solution sprayed on the apple seedling was dried, and about 30 first-instar larvae of summer fruit tortrix (*Adoxophyes*

orana *fasciata*) were released. Seven days after spraying, the number of worms surviving on the apple seedling was counted, and a controlling value was calculated by the following equation.

$$\text{Controlling value (\%)} = (1 - (Cb \times Tai)/(Cai \times Tb)) \times 100$$

As a result, the group treated with each of the present compounds (1-1), (1-5), (1-6), (1-7), (1-8), (1-9), (1-10), (1-11), (1-12), (1-31), (1-32), (1-60), (1-63) and (1-64) exhibited a controlling value of 100%.

Test Example 5

The flowable formulation of each of the present compounds (1-1), (1-5), (1-6), (1-7), (1-8), (1-9), (1-10), (1-12), (1-57), (1-58), (1-61), (1-63) and (1-64) obtained in Formulation Example 5 was diluted with water so that the active ingredient concentration became 500 ppm. Further, the flowable formulation of each of the present compounds (1-31) and (1-32) was diluted with water so that the active ingredient concentration became 200 ppm. Thus, test spray solutions were prepared.
On the other hand, cucumber was planted in a polyethylene cup, and was grown until the first true leaf was developed. The test spray solution as described above was sprayed in an amount of 20 mL/cup on the cucumber. After the spray solution on the cucumber was dried, the first true leaf was cut and then placed on a filter paper (diameter: 70 mm) containing water in a polyethylene cup (diameter: 110 mm). On the cucumber leaf, 20 larvae of yellow citrus thrips (*Franklinella occidentalis*) were released, and the polyethylene cup was capped. Seven days after spraying, the percentage of leaf area damaged by the insect was examined and a controlling value was calculated by the following equation.

$$\text{Controlling value (\%)} = (1 - (Cb \times Tai)/(Cai \times Tb)) \times 100$$

As a result, the group treated with each of the present compounds (1-1), (1-5), (1-6), (1-7), (1-8), (1-9), (1-10), (1-12), (1-31), (1-32), (1-57), (1-58), (1-61), (1-63) and (1-64) exhibited a controlling value of 100%.

Industrial Applicability

The present compound has an excellent controlling activity against harmful arthropods and is therefore useful as an active ingredient of a harmful arthropod controlling agent.

**Claims**

**1.** A hydrazide compound represented by the formula (1)

[Chemical Formula 1]:

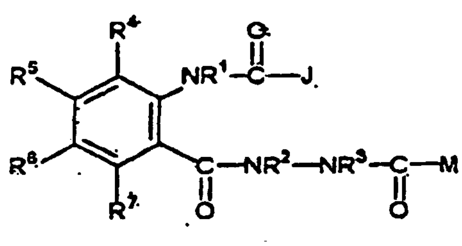

(1)

wherein

$R^1$ represents a hydrogen atom, an optionally halogenated C1-C6 alkyl group, a C2-C6 cyanoalkyl group, a C2-C6 alkoxyalkyl group, an optionally halogenated C3-C6 alkenyl group, an optionally halogenated C3-C6 alkynyl group, or a C7-C9 phenylalkyl group whose benzene ring moiety is optionally substituted with a substituent A shown below;
each of $R^2$ and $R^3$ independently represents a hydrogen atom, an optionally halogenated C1-C6 alkyl group,

or $R^6$ {in which at least one of $R^2$ and $R^3$ is $R^2$,
$R^6$ represents a C1-C6 alkyl group substituted with at least one substituent selected from the group D shown below, an optionally halogenated C3-C6 alkenyl group, an optionally halogenated C3-C6 alkynyl group, a C3-C6 cycloalkyl group optionally substituted with a substituent B shown below, a 5- to 6-membered heteroaryl group optionally substituted with a substituent A shown below, a 3- to 8-membered non-aromatic heterocyclic group optionally substituted with a substituent B shown below, an optionally halogenated C1-C6 alkylthio group, an "optionally halogenated C1-C6 alkylsulfinyl group, or an optionally halogenated C1-C6 alkylsulfonyl group,
group D:

(a) a cyano group,
(b) a nitro group,
(c) -C(=O)$OR^{101}$ ($R^{101}$ represents a hydrogen atom or a C1-C4 alkyl group),
(d) -C(=O)$NR^{102}R^{103}$ (each of $R^{102}$ and $R^{103}$ independently represents a hydrogen atom or a C1-C4 alkyl group),
(e) -C(=O)$R^{104}$ ($R^{204}$ represents a hydrogen atom, an optionally halogenated C1-C6 alkyl group, or a phenyl group optionally substituted with a substituent A shown below),
(f) $-CR^{105}$=N-$OR^{106}$ ($R^{105}$ represents a hydrogen atom, a C1-C4 alkyl group, or a phenyl group optionally substituted with a substituent A shown below, and $R^{106}$ represents a hydrogen atom or a C1-C4 alkyl group),
(g) $-OR^{107}$ ($R^{107}$ represents a hydrogen atom, a C1-C4 alkyl group, or a phenyl group optionally substituted with a substituent A shown below),
(h) $-5(O)_jR^{108}$ ($R^{100}$ represents a hydrogen atom, a C1-C4 alkyl group, or a phenyl group optionally substituted with a substituent A shown below, and j represents an integer of 0 to 2),
(i) $-NR^{109}R^{110}$ (each of $R^{109}$ and $R^{110}$ independently represents a hydrogen atom, a C1-C4 alkyl group, or a phenyl group optionally substituted with a substituent A shown below),
(j) a C3-C6 cycloalkyl group optionally substituted with a substituent B shown below,
(k) a phenyl group optionally substituted with a substituent A shown below,
(l) a 5- to 6-membered heteroaryl group optionally substituted with a substituent A shown below,
(m) a 3- to 8-membered non-aromatic heterocyclic group optionally substituted with a substituent B shown below, and
(n) a styryl group whose benzene ring moiety is optionally be substituted with a substituent A shown below);

$R^4$ represents a halogen atom, or an optionally halogenated C1-C6 alkyl group;
each of $R^5$, $R^6$ and $R^7$ independently represents a hydrogen atom, a halogen atom, a cyano group, or an optionally halogenated C1-C6 alkyl group, or
$R^5$ and $R^6$ may be combined to forme a 1,3-butadiene-1,4-diyl group optionally substituted with a substituent C shown below;
M represents $-R^8$, $-OR^9$, $-SR^{10}$, or $-NR^{11}R^{12}$ {in which $R^8$ represents a hydrogen atom, an optionally halogenated C1-C6 alkyl group, a C2-C6 alkoxyalkyl group, an optionally halogenated C2-C6 alkenyl group, or an optionally halogenated C2-C6 alkynyl group, each of $R^9$, $R^{10}$, $R^{11}$ and $R^{12}$ independently represents an optionally halogenated C1-C6 alkyl group, a C3-C6 alkoxyalkyl group, an optionally halogenated C3-C6 alkenyl group, or an optionally halogenated C3-C6 alkynyl group};
J represents any one of J1 to J3 shown below:

[Chemical Formula 2]

J1: 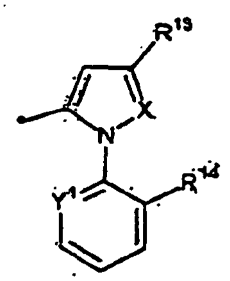

J2: 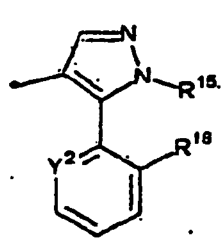

J3: 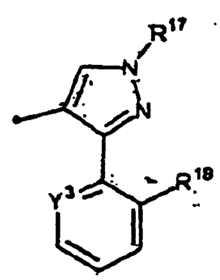

{in which X represents a nitrogen atom or $CR^{19}$;
$Y^1$ represents a nitrogen atom or $CR^{20}$;

$Y^2$ represents a nitrogen atom or $CR^{21}$;
$Y^3$ represents a nitrogen atom or $CR^{22}$;
each of $R^{13}$ and $R^{19}$ independently represents a hydrogen atom, a halogen atom, a cyano group, an optionally halogenated C1-C6 alkyl group, an optionally halogenated C1-C6 alkoxy group, an optionally halogenated C1-C6 alkylthio group, an optionally halogenated C1-C6 alkylsulfinyl group, or an optionally halogenated C1-C6 alkylsulfonyl group;
$R^{15}$ and $R^{11}$ each independently represents an optionally halogenated C1-C6 alkyl group;
each of $R^{14}$, $R^{16}$, $R^{28}$, $R^{20}$, $R^{21}$ and $R^{22}$ independently represents a hydrogen atom, a halogen atom, or an optionally halogenated C1-C6 alkyl group};

substituent A: a substituent selected from the group consisting of a halogen atom, a cyano group, a nitro ground, an optionally halogenated C1-C6 alkyl group, and an optionally halogenated C1-C6 alkoxy group;
substituent B: a substituent selected from the group consisting of a halogen atom and a C1-C6 alkyl group; and
substituent C: a substituent selected from the group consisting of a halogen atom, a cyano group and an optionally halogenated C1-C6 alkyl group.

**2.** The hydrazide compound according to claim 1, wherein, in the formula (1), $R^G$ is a C1-C6 alkyl group substituted with at least one substituent selected from the group D1 shown below, an optionally halogenated C3-C6 alkenyl group, an optionally halogenated C3-C6 alkynyl group, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B;
group D1 : (a) a cyano group, (g) $-OR^{107}$ ($R^{107}$ represents a hydrogen atom, a C1-C4 alkyl group or a phenyl group optionally substituted with a substituent A), (j) a C3-C6 cycloalkyl group optionally substituted with a substituent B, (k) a phenyl group optionally substituted with a substituent A, (1) a 5- to 6-membered heteroaryl group optionally substituted with a substituent A, and (m) a 3-to 8-membered non-aromatic heterocyclic group optionally substituted with a substituent B.

**3.** The hydrazide compound according to claim 2, wherein, in the formula (1), $R^G$ is a C1-C6 alkyl group substituted with at least one substituent selected from the group D2 shown below, an optionally halogenated C3-C6 alkenyl group, or an optionally halogenated C3-C6 alkynyl group;
group D2: (a) a cyano group and (g) $-OR^{107}$ ($R^{107}$ represents a hydrogen atom, a C1-C4 alkyl group or a phenyl group optionally substituted with a substituent A).

**4.** The hydrazide compound according to claim 2, wherein, in the formula (1), $R^G$ is a C1-C6 alkyl group substituted with -at least one substituent selected from the group D3 shown below, a C3-C6 cycloalkyl group optionally substituted with a substituent B, a 5- to 6-membered heteroaryl group optionally substituted with a substituent A, or a 3- to 8-membered non-aromatic heterocyclic group optionally substituted with a substituent B;
group D3: (j) a C3-C6 cycloalkyl group optionally substituted with a substituent B, (k) a phenyl group optionally substituted with a substituent A, (1) a 5- to 6-membered heteroaryl group optionally substituted with a substituent A, and (m) a 3- to 8-membered non-aromatic heterocyclic group optionally substituted with a substituent B.

**5.** The hydrazide compound according to claim 3, wherein, in the formula (1), one of $R^2$ and $R^3$ is $R^2$, and the other one is a hydrogen atom or an optionally halogenated C1-C6 alkyl group, and $R^6$ is a C1-C6 alkyl group substituted with a cyano group, an optionally halogenated C3-C6 alkenyl group, or an optionally halogenated C3-C6 alkynyl group.

**6.** The hydrazide compound according to claim 1, wherein, in the formula (1), $R^G$ is a C1-C6 alkyl group substituted with at least one substituent selected from the group D.

**7.** The hydrazide compound according to claim 6, wherein in the formula (1), $R^G$ is a C1-C6 alkyl group substituted with a cyano group.

**8.** The hydrazide compound according to claim 7, wherein, in the formula (1), $R^2$ is a C1-C6 alkyl group substituted with a cyano group; and $R^3$ is a hydrogen atom or an optionally halogenated C1-C6 alkyl ground.

**9.** The hydrazide compound according to claim 7, wherein, in the formula (1), $R^2$ is a hydrogen atom or an optionally halogenated C1-C6 alkyl group; and $R^3$ is a C1-C6 alkyl group substituted with a cyano group.

**10.** A harmful arthropod controlling agent comprising the hydrazide compound according to any one of claims 1 to 9 as an active ingredient.

**11.** Use of the hydrazide compound according to any one of claims 1 to 9 as an active ingredient of a harmful arthropod controlling agent.

**12.** A method for controlling harmful arthropods, which comprises applying the hydrazide compound according to any one of claims 1 to 9 directly to harmful arthropods, or applying to habitats of harmful arthropods, wherein the term "habitats" means plants, soits and houses.

**13.** Use of the hydrazide compound according to any one of claims 1 to 9 for the production of a harmful arthropods controlling agent.

**Patentansprüche**

**1.** Eine Hydrazidverbindung, dargestellt durch die Formel (1)

[Chemische Formel 1]:

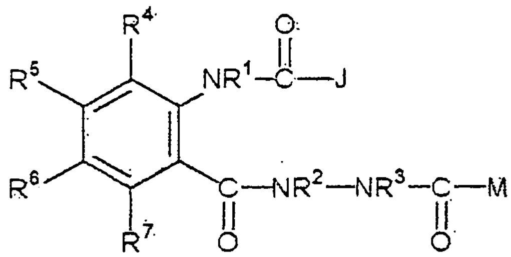

(1)

wobei

$R^1$ ein Wasserstoffatom, einen gegebenenfalls halogenierten C1-C6-Alkylrest, einen C2-C6-Cyanoalkylrest, einen C2-C6-Alkoxyalkylrest, einen gegebenenfalls halogenierten C3-C6-Alkenylrest, einen gegebenenfalls halogenierten C3-C6-Alkinylrest oder einen C7-C9-Phenylalkylrest, dessen Benzolringeinheit gegebenenfalls mit einem nachstehend gezeigten Substituenten A substituiert ist, bedeutet;
jedes von $R^2$ und $R^3$ unabhängig ein Wasserstoffatom, einen gegebenenfalls halogenierten C1-C6-Alkylrest oder $R^G$ bedeutet {wobei mindestens eines von $R^2$ und $R^3$ $R^G$ ist, $R^G$ einen C1-C6-Alkylrest, substituiert mit mindestens einem Substituenten, ausgewählt aus der nachstehend gezeigten Gruppe D, einen gegebenenfalls halogenierten C3-C6-Alkenylrest, einen gegebenenfalls halogenierten C3-C6-Alkinylrest, einen C3-C6-Cycloalkylrest, gegebenenfalls substituiert mit einem nachstehend gezeigten Substituenten B, einen 5- bis 6-gliedrigen Heteroarylrest, gegebenenfalls substituiert mit einem nachstehend gezeigten Substituenten A, einen 3- bis 8-gliedrigen nichtaromatischen heterocyclischen Rest, gegebenenfalls substituiert mit einem nachstehend gezeigten Substituenten B, einen gegebenenfalls halogenierten C1-C6-Alkylthiorest, einen gegebenenfalls halogenierten C1-C6-Alkylsulfinylrest oder einen gegebenenfalls halogenierten C1-C6-Alkylsulfonylrest bedeutet,
Gruppe D:

(a) eine Cyanogruppe,
(b) eine Nitrogruppe,
(c) -C(=O)$OR^{101}$ ($R^{101}$ bedeutet ein Wasserstoffatom oder einen C1-C4-Alkylrest),
(d) -C(=O)$NR^{102}R^{103}$ (jedes von $R^{102}$ und $R^{103}$ bedeutet unabhängig ein Wasserstoffatom oder einen C1-C4-Alkylrest),
(e) -C(=O)$R^{104}$ ($R^{104}$ bedeutet ein Wasserstoffatom, einen gegebenenfalls halogenierten C1-C6-Alkylrest oder einen Phenylrest, gegebenenfalls substituiert mit einem nachstehend gezeigten Substituenten A),
(f) -$CR^{105}$=N-$OR^{106}$ ($R^{105}$ bedeutet ein Wasserstoffatom, einen C1-C4-Alkylrest oder einen Phenylrest, gegebenenfalls substituiert mit einem nachstehend gezeigten Substituenten A, und $R^{106}$ bedeutet ein Wasserstoffatom oder einen C1-C4-Alkylrest),
(g) -$OR^{107}$ ($R^{107}$ bedeutet ein Wasserstoffatom, einen C1-C4-Alkylrest oder einen Phenylrest, gegebenenfalls substituiert mit einem nachstehend gezeigten Substituenten A),

(h) $-S(O)_jR^{108}$ ($R^{108}$ bedeutet ein Wasserstoffatom, einen C1-C4-Alkylrest oder einen Phenylrest, gegebenenfalls substituiert mit einem nachstehend gezeigten Substituenten A, und j bedeutet eine ganze Zahl von 0 bis 2),

(i) $-NR^{109}R^{110}$ (jedes von $R^{109}$ und $R^{110}$ bedeutet unabhängig ein Wasserstoffatom, einen C1-C4-Alkylrest oder einen Phenylrest, gegebenenfalls substituiert mit einem nachstehend gezeigten Substituenten A),

(j) ein C3-C6-Cycloalkylrest, gegebenenfalls substituiert mit einem nachstehend gezeigten Substituenten B,

(k) ein Phenylrest, gegebenenfalls substituiert mit einem nachstehend gezeigten Substituenten A,

(l) ein 5- bis 6-gliedriger Heteroarylrest, gegebenenfalls substituiert mit einem nachstehend gezeigten Substituenten A,

(m)ein 3- bis 8-gliedriger nicht-aromatischer heterocyclischer Rest, gegebenenfalls substituiert mit einem nachstehend gezeigten Substituenten B, und

(n) ein Styrylrest, dessen Benzolringeinheit gegebenenfalls mit einem nachstehend gezeigten Substituenten A substituiert ist};

$R^4$ ein Halogenatom oder einen gegebenenfalls halogenierten C1-C6-Alkylrest bedeutet; jedes von $R^5$, $R^6$ und $R^7$ unabhängig ein Wasserstoffatom, ein Halogenatom, eine Cyanogruppe oder einen gegebenenfalls halogenierten C1-C6-Alkylrest bedeutet oder

$R^5$ und $R^6$ vereinigt sein können, um einen 1,3-Butadien-1,4-diylrest, gegebenenfalls substituiert mit einem nachstehend gezeigten Substituenten C, zu bilden;

M die Bedeutung $-R^8$, $-OR^9$, $-SR^{10}$ oder $-NR^{11}R^{12}$ hat {wobei $R^8$ ein Wasserstoffatom, einen gegebenenfalls halogenierten C1-C6-Alkylrest, einen C2-C6-Alkoxyalkylrest, einen gegebenenfalls halogenierten C2-C6-Alkenylrest oder einen gegebenenfalls halogenierten C2-C6-Alkinylrest bedeutet, jedes von $R^9$, $R^{10}$, $A^{11}$ und $R^{12}$ unabhängig einen gegebenenfalls halogenierten C1-C6-Alkylrest, einen C3-C6-Alkoxyalkylrest, einen gegebenenfalls halogenierten C3-C6-Alkenylrest oder einen gegebenenfalls halogenierten C3-C6-Alkinylrest bedeutet};

J eines von nachstehend gezeigten J1 bis J3 bedeutet:

[Chemische Formel 2]

J1: 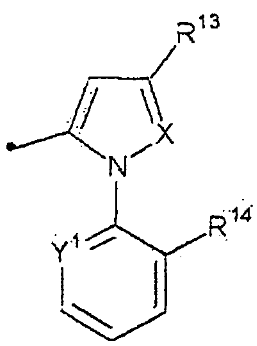

J2: 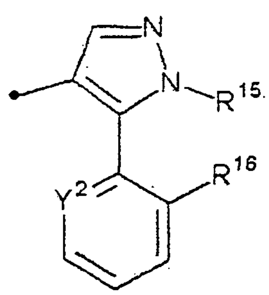

J3: 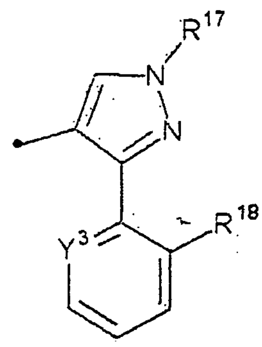

{wobei X ein Stickstoffatom oder $CR^{19}$ bedeutet;

$Y^1$ ein Stickstoffatom oder $CR^{20}$ bedeutet;

$Y^2$ ein Stickstoffatom oder $CR^{21}$ bedeutet;

$Y^3$ ein Stickstoffatom oder $CR^{22}$ bedeutet;

jedes von $R^{13}$ und $R^{19}$ unabhängig ein Wasserstoffatom, ein Halogenatom, eine Cyanogruppe, einen gegebenenfalls halogenierten C1-C6-Alkylrest, einen gegebenenfalls halogenierten C1-C6-Alkoxyrest, einen gegebenenfalls halogenierten C1-C6-Alkylthiorest, einen gegebenenfalls halogenierten C1-C6-Alkylsulfinylrest oder einen gegebenenfalls halogenierten C1-C6- Alkylsulfonylrest bedeutet;

$R^{15}$ und $R^{17}$ jeweils unabhängig einen gegebenenfalls halogenierten C1-C6-Alkylrest bedeuten;

jedes von $R^{14}$, $R^{16}$, $R^{18}$, $R^{20}$, $R^{21}$ und $R^{22}$ unabhängig ein Wasserstoffatom, ein Halogenatom oder einen gegebenenfalls halogenierten C1-C6-Alkylrest bedeutet}; Substituent A: ein Substituent, ausgewählt aus der Gruppe bestehend aus einem Halogenatom, einer Cyanogruppe, einer Nitrogruppe, einem gegebenenfalls halogenierten C1-C6-Alkylrest und einem gegebenenfalls halogenierten C1-C6-Alkoxyrest; Substituent B: ein Substituent, ausgewählt aus der Gruppe bestehend aus einem Halogenatom und einem C1-C6-Alkylrest; und

Substituent C: ein Substituent, ausgewählt aus der Gruppe bestehend aus einem Halogenatom, einer Cyanogruppe und einem gegebenenfalls halogenierten C1-C6-Alkylrest.

**2.** Die Hydrazidverbindung nach Anspruch 1, wobei in der Formel (1) $R^G$ ein C1-C6-Alkylrest, substituiert mit mindestens einem Substituenten, ausgewählt aus der nachstehend gezeigten Gruppe D1, ein gegebenenfalls halogenierter C3-C6-Alkenylrest, ein gegebenenfalls halogenierter C3-C6-Alkinylrest, ein C3-C6-Cycloalkylrest, gegebenenfalls substituiert mit einem Substituenten B, ein 5- bis 6-gliedriger Heteroarylrest, gegebenenfalls substituiert mit einem Substituenten A, oder ein 3- bis 8-gliedriger nicht-aromatischer heterocyclischer Rest, gegebenenfalls substituiert mit einem Substituenten B, ist;
Gruppe D1: (a) eine Cyanogruppe, (g) $-OR^{107}$ ($R^{107}$ bedeutet ein Wasserstoffatom, einen C1-C4-Alkylrest oder einen Phenylrest, gegebenenfalls substituiert mit einem Substituenten A), (j) ein C3-C6-Cycloalkylrest, gegebenenfalls substituiert mit einem Substituenten B, (k) ein Phenylrest, gegebenenfalls substituiert mit einem Substituenten A, (1) ein 5- bis 6-gliedriger Heteroarylrest, gegebenenfalls substituiert mit einem Substituenten A, und (m) ein 3- bis 8-gliedriger nicht-aromatischer heterocyclischer Rest, gegebenenfalls substituiert mit einem Substiuenten B.

**3.** Die Hydrazidverbindung nach Anspruch 2, wobei in der Formel (1) $R^G$ ein C1-C6-Alkylrest, substituiert mit mindestens einem Substituenten, ausgewählt aus der nachstehend gezeigten Gruppe D2, ein gegebenenfalls halogenierter C3-C6-Alkenylrest oder ein gegebenenfalls halogenierter C3-C6-Alkinylrest ist;
Gruppe D2: (a) eine Cyanogruppe und (g) $-OR^{107}$ ($R^{107}$ bedeutet ein Wasserstoffatom, einen C1-C4-Alkylrest oder einen Phenylrest, gegebenenfalls substituiert mit einem Substituenten A).

**4.** Die Hydrazidverbindung nach Anspruch 2, wobei in der Formel (1) $R^G$ ein C1-C6-Alkylrest, substituiert mit mindestens einem Substituenten, ausgewählt aus der nachstehend gezeigten Gruppe D3, ein C3-C6-Cycloalkylrest, gegebenenfalls substituiert mit einem Substituenten B, ein 5- bis 6-gliedriger Heteroarylrest, gegebenenfalls substituiert mit einem Substituenten A, oder ein 3- bis 8-gliedriger nicht-aromatischer heterocyclischer Rest, gegebenenfalls substituiert mit einem Substituenten B, ist; Gruppe D3: (j) ein C3-C6-Cycloalkylrest, gegebenenfalls substituiert mit einem Substituenten B, (k) ein Phenylrest, gegebenenfalls substituiert mit einem Substituenten A, (1) ein 5- bis 6-gliedriger Heteroarylrest, gegebenenfalls substituiert mit einem Substituenten A, und (m) ein 3- bis 8-gliedriger nicht-aromatischer heterocyclischer Rest, gegebenenfalls substituiert mit einem Substituenten B.

**5.** Die Hydrazidverbindung nach Anspruch 3, wobei in der Formel (1) eines von $R^2$ und $R^3$ $R^G$ ist und das andere ein Wasserstoffatom oder ein gegebenenfalls halogenierter Cl-C6-Alkylrest ist und $R^G$ ein C1-C6-Alkylrest, substituiert mit einer Cyanogruppe, ein gegebenenfalls halogenierter C3-C6-Alkenylrest oder ein gegebenenfalls halogenierter C3-C6-Alkinylrest ist.

**6.** Die Hydrazidverbindung nach Anspruch 1, wobei in der Formel (1) $R^G$ ein C1-C6-Alkylrest, substituiert mit mindestens einem Substituenten, ausgewählt aus der Gruppe D, ist.

**7.** Die Hydrazidverbindung nach Anspruch 6, wobei in der Formel (1) $R^G$ ein C1-C6-Alkylrest, substituiert mit einer Cyanogruppe, ist.

**8.** Die Hydrazidverbindung nach Anspruch 7, wobei in der Formel (1) $R^2$ ein C1-C6-Alkylrest, substituiert mit einer Cyanogruppe, ist; und $R^3$ ein Wasserstoffatom oder ein gegebenenfalls halogenierter C1-C6-Alkylrest ist.

**9.** Die Hydrazidverbindung nach Anspruch 7, wobei in der Formel (1) $R^2$ ein Wasserstoffatom oder ein gegebenenfalls halogenierter C1-C6-Alkylrest ist; und $R^3$ ein C1-C6-Alkylrest, substituiert mit einer Cyanogruppe, ist.

**10.** Ein Mittel zur Bekämpfung schädlicher Arthropoden, umfassend die Hydrazidverbindung nach einem der Ansprüche 1 bis 9 als einen Wirkstoff.

**11.** Verwendung der Hydrazidverbindung nach einem der Ansprüche 1 bis 9 als ein Wirkstoff eines Mittels zur Bekämpfung schädlicher Arthropoden.

**12.** Ein Verfahren zur Bekämpfung schädlicher Arthropoden, welches Aufbringen der Hydrazidverbindung nach einem der Ansprüche 1 bis 9 direkt auf schädliche Arthropoden oder Aufbringen auf Lebensräume schädlicher Arthropoden, wobei der Begriff "Lebensräume" Pflanzen, Böden und Häuser bedeutet, umfasst.

**13.** Verwendung der Hydrazidverbindung nach einem der Ansprüche 1 bis 9 zur Herstellung eines Mittels zur Bekämpfung schädlicher Arthropoden.

## Revendications

**1.** Composé hydrazide représenté par la formule (1) :

[Formule chimique 1] :

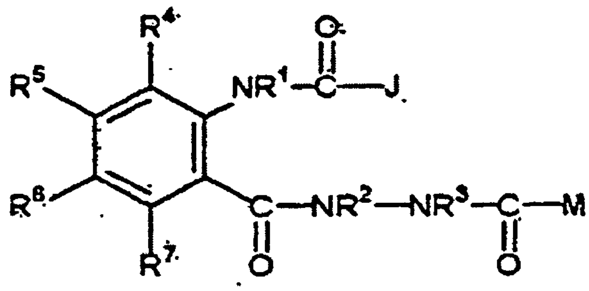

(1)

dans laquelle

$R^1$ représente un atome d'hydrogène, un groupe alkyle en $C_1$ à $C_6$ éventuellement halogéné, un groupe cyanoalkyle en $C_2$ à $C_6$, un groupe alcoxyalkyle en $C_2$ à $C_6$, un groupe alcényle en $C_3$ à $C_6$ éventuellement halogéné, un groupe alcynyle en $C_3$ à $C_6$ éventuellement halogéné, ou un groupe phénylalkyle en $C_7$ à $C_9$ dont le fragment de cycle benzène est éventuellement substitué par un substituant A indiqué ci-dessous ;
chacun de $R^2$ et $R^3$ représente indépendamment un atome d'hydrogène, un groupe alkyle en $C_1$ à $C_6$ éventuellement halogéné, ou $R^G$ {où au moins l'un de $R^2$ et $R^3$ est $R^G$, $R^G$ représente un groupe alkyle en $C_1$ à $C_6$ substitué par au moins un substituant choisi dans le groupe D indiqué ci-dessous, un groupe alcényle en $C_3$ à $C_6$ éventuellement halogéné, un groupe alcynyle en $C_3$ à $C_6$ éventuellement halogéné, un groupe cycloalkyle en $C_3$ à $C_6$ éventuellement substitué par un substituant B indiqué ci-dessous, un groupe hétéroaryle à 5 ou 6 chaînons éventuellement substitué par un substituant A indiqué ci-dessous, un groupe hétérocyclique non aromatique à 3 à 8 chaînons éventuellement substitué par un substituant B indiqué ci-dessous, un groupe alkylthio en $C_1$ à $C_6$ éventuellement halogéné, un groupe alkylsulfinyle en $C_1$ à $C_6$ éventuellement halogéné, ou un groupe alkylsulfonyle en $C_1$ à $C_6$ éventuellement halogéné ;
groupe D :

(a) un groupe cyano,
(b) un groupe nitro,
(c) -C(=O)OR$^{101}$ (où $R^{101}$ représente un atome d'hydrogène ou un groupe alkyle en $C_1$ à $C_4$),
(d) -C(=O)NR$^{102}$R$^{103}$ (où chacun de $R^{102}$ et $R^{103}$ représente indépendamment un atome d'hydrogène ou un groupe alkyle en $C_1$ à $C_4$),
(e) -C(=O)R$^{104}$ (où $R^{104}$ représente un atome d'hydrogène, un groupe alkyle en $C_1$ à $C_6$ éventuellement halogéné, ou un groupe phényle éventuellement substitué par un substituant A indiqué ci-dessous),
(f) -CR$^{105}$=N-OR$^{106}$ (où $R^{105}$ représente un atome d'hydrogène, un groupe alkyle) en $C_1$ à $C_4$, ou un groupe phényle éventuellement substitué par un substituant A indiqué ci-dessous, et $R^{106}$ représente un atome d'hydrogène ou un groupe alkyle en $C_1$ à $C_4$),
(g) -OR$^{107}$ (où $R^{107}$ représente un atome d'hydrogène, un groupe alkyle en $C_1$ à $C_4$, ou un groupe phényle éventuellement substitué par un substituant A indiqué ci-dessous),
(h) -S(O)$_j$R$^{108}$ (où $R^{108}$ représente un atome d'hydrogène, un groupe alkyle en $C_1$ à $C_4$, ou un groupe phényle éventuellement substitué par un substituant A indiqué ci-dessous, et j représente un entier de 0 à 2),
(i) -NR$^{109}$R$^{110}$ (où chacun de $R^{109}$ et $R^{110}$ représente indépendamment un atome d'hydrogène, un groupe alkyle en $C_1$ à $C_4$, ou un groupe phényle éventuellement substitué par un substituant A indiqué ci-dessous),
(j) un groupe cycloalkyle en $C_3$ à $C_6$ éventuellement substitué par un substituant B indiqué ci-dessous,
(k) un groupe phényle éventuellement substitué par un substituant A indiqué ci-dessous,
(l) un groupe hétéroaryle à 5 ou 6 chaînons éventuellement substitué par un substituant A indiqué ci-dessous,
(m) un groupe hétérocyclique non aromatique à 3 à 8 chaînons éventuellement substitué par un substituant B indiqué ci-dessous, et
(n) un groupe styryle dont le fragment de cycle benzène est éventuellement substitué par un substituant A indiqué ci-dessous} ;

$R^4$ représente un atome d'halogène, ou un groupe alkyle en $C_1$ à $C_6$ éventuellement halogéné ;
chacun de $R^5$ $R^6$ et $R^7$ représente indépendamment un atome d'hydrogène, un atome d'halogène, un groupe cyano, ou un groupe alkyle en $C_1$ à $C_6$ éventuellement halogéné, ou bien
$R^5$ et $R^6$ peuvent être combinés pour former un groupe 1,3-butadiène-1,4-diyle éventuellement substitué par un substituant C indiqué ci-dessous ;
M représente $-R^8$, $-OR^9$, $-SR^{10}$, ou $-N^{11}R^{12}$ {où $R^8$ représente un atome d'hydrogène, un groupe alkyle en $C_1$ à $C_6$ éventuellement halogéné, un groupe alcoxyalkyle en $C_2$ à $C_6$, un groupe alcényle en $C_2$ à $C_6$ éventuellement halogéné, ou un groupe alcynyle en $C_2$ à $C_6$ éventuellement halogéné, chacun de $R^9$, $R^{10}$, $R^{11}$ et $R^{12}$ représente indépendamment un groupe alkyle en $C_1$ à $C_6$ éventuellement halogéné, un groupe alcoxyalkyle en $C_3$ à $C_6$, un groupe alcényle en $C_3$ à $C_6$ éventuellement halogéné, ou un groupe alcynyle en $C_3$ à $C_6$ éventuellement halogéné} ;
J représente l'un quelconque de J1 à J3 indiqués ci-dessous :

[Formule chimique 2]

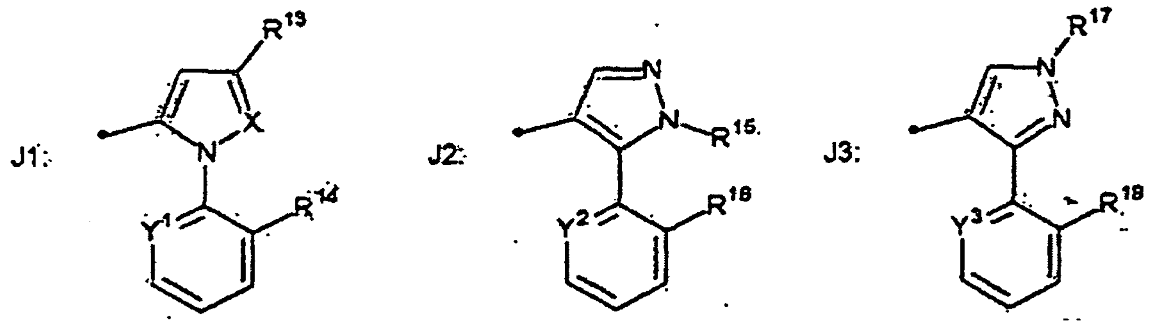

{où X représente un atome d'azote ou $CR^{19}$ ;
$Y^1$ représente un atome d'azote ou $CR^{20}$ ;
$Y^2$ représente un atome d'azote ou $CR^{21}$ ;
$Y^3$ représente un atome d'azote ou $CR^{22}$ ;
chacun de $R^{13}$ et $R^{19}$ représente indépendamment un atome d'hydrogène, un atome d'halogène, un groupe cyano, un groupe alkyle en $C_1$ à $C_6$ éventuellement halogéné,
un groupe alcoxy en $C_1$ à $C_6$ éventuellement halogéné, un groupe alkylthio en $C_1$ à $C_6$ éventuellement halogéné, un groupe alkylsulfinyle en $C_1$ à $C_6$ éventuellement halogéné, ou un groupe alkylsulfonyle en $C_1$ à $C_6$ éventuellement halogéné ;
chacun de $R^{15}$ et $R^{17}$ représente indépendamment un groupe alkyle en $C_1$ à $C_6$ éventuellement halogéné ;
chacun de $R^{14}$, $R^{16}$, $R^{18}$, $R^{20}$, $R^{21}$ et $R^{22}$ représente indépendamment un atome d'hydrogène, un atome d'halogène, ou un groupe alkyle en $C_1$ à $C_6$ éventuellement halogéné} ;
substituant A : un substituant choisi dans l'ensemble constitué par un atome d'halogène, un groupe cyano, un groupe nitro, un groupe alkyle en $C_1$ à $C_6$ éventuellement halogéné, et un groupe alcoxy en $C_1$ à $C_6$ éventuellement halogéné ;
substituant B : un substituant choisi dans l'ensemble constitué par un atome d'halogène et un groupe alkyle en $C_1$ à $C_6$ ; et
substituant C : un substituant choisi dans l'ensemble constitué par un atome d'halogène, un groupe cyano et un groupe alkyle en $C_1$ à $C_6$ éventuellement halogéné.

**2.** Composé hydrazide selon la revendication 1, dans lequel, dans la formule (1), $R^G$ est un groupe alkyle en $C_1$ à $C_6$ substitué par au moins un substituant choisi dans le groupe D1 indiqué ci-dessous, un groupe alcényle en $C_3$ à $C_6$ éventuellement halogéné, un groupe alcynyle en $C_3$ à $C_6$ éventuellement halogéné, un groupe cycloalkyle en $C_3$ à $C_6$ éventuellement substitué par un substituant B, un groupe hétéroaryle à 5 ou 6 chaînons éventuellement substitué par un substituant A, ou un groupe hétérocyclique non aromatique à 3 à 8 chaînons éventuellement

substitué par un substituant B ;
groupe D1 : (a) un groupe cyano, (g) -$OR^{107}$ (où $R^{107}$ représente un atome d'hydrogène, un groupe alkyle en $C_1$ à $C_4$ ou un groupe phényle éventuellement substitué par un substituant A), (j) un groupe cycloalkyle en $C_3$ à $C_6$ éventuellement substitué par un substituant B, (k) un groupe phényle éventuellement substitué par un substituant A, (l) un groupe hétéroaryle à 5 ou 6 chaînons éventuellement substitué par un substituant A, et (m) un groupe hétérocyclique non aromatique à 3 à 8 chaînons éventuellement substitué par un substituant B.

**3.** Composé hydrazide selon la revendication 2, dans lequel, dans la formule (1), $R^G$ est un groupe alkyle en $C_1$ à $C_6$ substitué par au moins un substituant
choisi dans le groupe D2 indiqué ci-dessous, un groupe alcényle en $C_3$ à $C_6$ éventuellement halogéné, ou un groupe alcynyle en $C_3$ à $C_6$ éventuellement halogéné ;
groupe D2 : (a) un groupe cyano et (g) -$OR^{107}$ (où $R^{107}$ représente un atome d'hydrogène, un groupe alkyle en $C_1$ à $C_4$ ou un groupe phényle éventuellement substitué par un substituant A).

**4.** Composé hydrazide selon la revendication 2, dans lequel, dans la formule (1), $R^G$ est un groupe alkyle en $C_1$ à $C_6$ éventuellement substitué par au moins un substituant choisi dans le groupe D3 indiqué ci-dessous, un groupe cycloalkyle en $C_3$ à $C_6$ éventuellement substitué par un substituant B, un groupe hétéroaryle à 5 ou 6 chaînons éventuellement substitué par un substituant A, ou un groupe hétérocyclique non aromatique à 3 à 8 chaînons éventuellement substitué par un substituant B ;
groupe D3 : (j) un groupe cycloalkyle en $C_3$ à $C_6$ éventuellement substitué par un substituant B, (k) un groupe phényle éventuellement substitué par un substituant A, (l) un groupe hétéroaryle à 5 ou 6 chaînons éventuellement substitué par un substituant A, et (m) un groupe hétérocyclique non aromatique à 3 à 8 chaînons éventuellement substitué par un substituant B.

**5.** Composé hydrazide selon la revendication 3, dans lequel, dans la formule (1), l'un de $R^2$ et $R^3$ est $R^G$, et l'autre est un atome d'hydrogène ou un groupe alkyle en $C_1$ à $C_6$ éventuellement halogéné, et $R^G$ est un groupe alkyle en $C_1$ à $C_6$ éventuellement substitué par un groupe cyano, un groupe alcényle en $C_3$ à $C_6$ éventuellement halogéné, ou un groupe alcynyle en $C_3$ à $C_6$ éventuellement halogéné.

**6.** Composé hydrazide selon la revendication 1, dans lequel, dans la formule (1), $R^G$ est un groupe alkyle en $C_1$ à $C_6$ éventuellement substitué par au moins un substituant choisi dans le groupe D.

**7.** Composé hydrazide selon la revendication 6, dans lequel, dans la formule (1), $R^G$ est un groupe alkyle en $C_1$ à $C_6$ substitué par un groupe cyano.

**8.** Composé hydrazide selon la revendication 7, dans lequel, dans la formule (1), $R^2$ est un groupe alkyle en $C_1$ à $C_6$ substitué par un groupe cyano ; et $R^3$ est un atome d'hydrogène ou un groupe alkyle en $C_1$ à $C_6$ éventuellement halogéné.

**9.** Composé hydrazide selon la revendication 7, dans lequel, dans la formule (1), $R^2$ est un atome d'hydrogène ou un groupe alkyle en $C_1$ à $C_6$ éventuellement halogéné ; et $R^3$ est un groupe alkyle en $C_1$ à $C_6$ substitué par un groupe cyano.

**10.** Agent de lutte contre les arthropodes nuisibles comprenant le composé hydrazide selon l'une quelconque des revendications 1 à 9 à titre d'ingrédient actif.

**11.** Utilisation du composé hydrazide selon l'une quelconque des revendications 1 à 9 à titre d'ingrédient actif d'un agent de lutte contre les arthropodes nuisibles.

**12.** Procédé de lutte contre les arthropodes nuisibles, qui comprend l'application du composé hydrazide selon l'une quelconque des revendications 1 à 9 directement sur les arthropodes nuisibles, ou l'application à l'habitat des arthropodes nuisibles, dans lequel le terme "habitat" signifie les plantes, les sols et les maisons.

**13.** Utilisation du composé hydrazide selon l'une quelconque des revendications 1 à 9 pour la production d'un agent de lutte contre les arthropodes nuisibles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0170671 A
- WO 03015518 A
- WO 03016284 A
- WO 03016300 A
- WO 03024222 A


### Non-patent literature cited in the description


- Greene's Protective Groups in Organic Synthesis. WILEY
- Organic Functional Group Preparations. vol. 1, 359-376
- **Stanley R. Sandler ; Wolf Karo.** Organic Functional Group Preparations. vol. 1, 434-465
- *Journal of Organic Chemistry,* 1947, vol. 12, 743-51
- *Synlett,* 2004, 2355-2356